# EUROPEAN PATENT SPECIFICATION

(11) **EP 1 192 165 B1**
(45) Date of publication and mention of the grant of the patent: **22.09.2004**
(21) Application number: 00942807.9
(22) Date of filing: 15.06.2000
(51) Int. Cl.: C07D 513/16, C07D 519/00, C07D 495/06, C07D 491/06, A61K 31/55, A61K 31/4353, A61P 25/00

(54) **SUBSTITUTED HETEROCYCLE FUSED GAMMA-CARBOLINES**
SUBSTITUIERTE HETEROCYCLYLKONDENSIERTE GAMMA CARBOLINE
GAMMA-CARBOLINES FUSIONNEES A HETEROCYCLES SUBSTITUES

(30) Priority: 15.06.1999 US 139321 P
(43) Date of publication of application: 03.04.2002
(73) Proprietor: Bristol-Myers Squibb Pharma Company, Princeton, New Jersey 08543-4000 (US)
(72) Inventor: ROBICHAUD, Albert, J., Landenberg, PA 19350 (US); LEE, Taekyu, Wilmington, DE 19808 (US); DENG, Wei, Wilmington, DE 19803 (US); MITCHELL, Ian S., Wilmington, DE 19806 (US); HAYDAR, Simon, Niskayuna, NY 12309 (US); CHEN, Wenting, Exton, PA 19341 (US); MC CLUNG, Christopher, D., Wilmington, DE 19805 (US); CALVELLO, Emilie, J., B., Bryn Mawr, PA 19010 (US); ZAWROTNY, David, M., Moorestown, NJ 08057 (US)
(74) Representative: Beacham, Annabel Rose
(86) International application number: PCT/US2000/016373
(87) International publication number: WO 2000/077010

(56) References cited:
- US-A- 4 219 550
- US-A- 4 238 607
- DATABASE CAPLUS [Online] Chemical Abstracts Service; 84 : 59411, XP002153997 & KHIM. GETEROTSIKL. SOEDIN., no. 9, 1975, pages 1262-6, & CHEMICAL ABSTRACTS, vol. 84, no. 9, 1 March 1976 (1976-03-01) Columbus, Ohio, US; abstract no. 59411, ORLOVA ETAL.: "Synthesis of 2,3,4,5-tetrahydro-1,5-benzox and thi)azepines and their use for synthesis of condensed indoles"

## Description

### FIELD OF THE INVENTION

The present invention is directed to certain novel compounds represented by structural Formula (I-a) 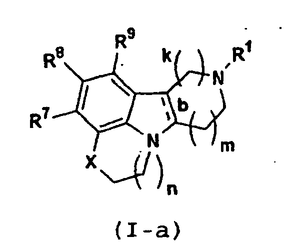 or pharmaceutically acceptable salt forms thereof, wherein R¹, R⁵, R^{6a}, R^{6b}, R⁷, R⁸, R⁹, X, b, k, m, and n, and the dashed lines are described herein. The invention is also concerned with pharmaceutical formulations comprising these novel compounds as active ingredients and the use of the novel compounds and their formulations in the treatment of certain disorders. The compounds of this invention are serotonin agonists and antagonists and are useful in the control or prevention of central nervous system disorders including obesity, anxiety, depression, psychosis, schizophrenia, sleep disorders, sexual disorders, migraine, conditions associated with cephalic pain, social phobias, and gastrointestinal disorders such as dysfunction of the gastrointestinal tract motility.

### BACKGROUND OF THE INVENTION

There exists a substantial correlation for the relationship between 5-HT2 receptor modulation and a variety of diseases and therapies. To date, three subtypes of the 5-HT2 receptor class have been identified, 5-HT2A, 5-HT2B, and 5-HT2C. Prior to the early 1990's the 5-HT2C and 5-HT2A receptors were referred to as 5-HT1C and 5-HT2, respectively.

The agonism or antagonism of 5-HT2 receptors, either selectively or nonselectively, has been associated with the treatment of various central nervous system (CNS) disorders. Ligands possessing affinity for the 5-HT2 receptors have been shown to have numerous physiological and behavioral effects (Trends in Pharmacological Sciences, 11, 181, 1990). In the recent past the contribution of serotonergic activity to the mode of action of antidepressant drugs has been well documented. Compounds that increase the overall basal tone of serotonin in the CNS have been successfully developed as antidepressants. The serotonin selective reuptake inhibitors (SSRI) function by increasing the amount of serotonin present in the nerve synapse. These breakthrough treatments, however, are not without side effects and suffer from delayed onset of action (Leonard, J. Clin. Psychiatry, 54(suppl), 3, 1993). Due to the mechanism of action of the SSRIs, they effect the activity of a number of serotonin receptor subtypes. This non-specific modulation of the serotonin family of receptors most likely plays a significant role in the side effect profile. In addition, these compounds often have a high affinity for a number of the serotonin receptors as well as a multitude of other monoamine neurotransmitters and nuisance receptors. Removing some of the receptor cross reactivity would allow for the examination and possible development of potent therapeutic ligands with an improved side effect profile.

There is ample evidence to support the role of selective 5-HT2 receptor ligands in a number of disease therapies. Modulation of 5-HT2 receptors has been associated with the treatment of schizophrenia and psychoses (Ugedo, L., et.al., Psychopharmacology, 98, 45, 1989). Mood, behavior and hallucinogenesis can be affected by 5-HT2 receptors in the limbic system and cerebral cortex. 5-HT2 receptor modulation in the hypothalamus can influence appetite, thermoregulation, sleep, sexual behavior, motor activity, and neuroendocrine function (Hartig, P., et.al., Annals New York Academy of Science, 149, 159). There is also evidence indicating that 5-HT2 receptors mediate hypoactivity, effect feeding in rats, and mediate penile erections (Pyschopharmacology, 101, 57, 1990).

Compounds exhibiting selectivity for the 5-HT2B receptor are useful in treating conditions such as tachygastria, hypermotility associated with irritable bowel disorder, constipation, dyspepsia, and other peripherally mediated conditions.

5-HT2A antagonists have been shown to be effective in the treatment of schizophrenia, anxiety, depression, and migraines (Koek, W., Neuroscience and Behavioral reviews, 16, 95, 1996). Aside from the beneficial antipsychotic effects, classical neuroleptic are frequently responsible for eliciting acute extrapyramidal side effects and neuroendocrine disturbances. These compounds generally possess signifcant dopamine D2 receptor affinity (as well as other nuisance receptor affinity) which frequently is associated with extra pyramidal symptoms and tardive dyskinesia, thus detracting from their efficacy as front line treatments in schizophrenia and related disorders. Compounds possessing a more favorable selectivity profile would represent a possible improvement for the treatment of CNS disorders.

U.S. Patent Numbers 3,914,421; 4,013,652; 4,115,577; 4,183,936; and 4,238,607 disclose pyridopyrrolobenzheterocycles of formula: 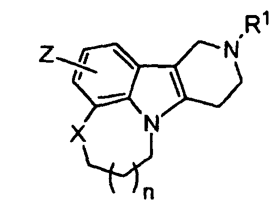 where X is O, S, S(=O), or SO₂; n is 0 or 1; R¹ is various carbon substituents, and Z is a monosubstituent of H, methyl, or chloro.

U.S. Patent Number 4,219,550 discloses pyridopyrrolobenzheterocycles of formula: 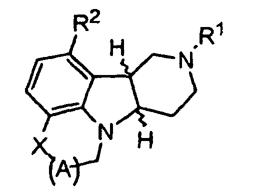 where X is O or S; R¹ is C₁₋₄ alkyl or cyclopropyl; R² is H, CH₃, OCH₃, Cl, Br, F, or CF₃; and (A) is -CH₂-, -CH(CH₃)-, or -CH₂CH₂-.

US 3,299,078 describes pyridopyrroloindoles and quinolines which have analgesic, anti-pyratic, anti-inflammatory, anti-serotonin and central nervous system stimulant activity.

### SUMMARY OF THE INVENTION

One object of the present invention is to provide novel compounds which are useful as agonists or antagonists of 5-HT2 receptors, more specifically 5-HT2A and 5-HT2C receptors, or pharmaceutically acceptable salts or prodrugs thereof.

It is another object of the present invention to provide pharmaceutical compositions comprising a pharmaceutically acceptable carrier and a therapeutically effective amount of at least one of the compounds of the present invention or a pharmaceutically acceptable salt thereof.

It is another object of the present invention to provide compounds for use in therapy. More specifically, the present invention provides compounds for use in treating central nervous system disorders including obesity, schizophrenia, and depression.

These and other objects, which will become apparent during the following detailed description, have been achieved by the inventors' discovery that compounds of Formula (I-a): 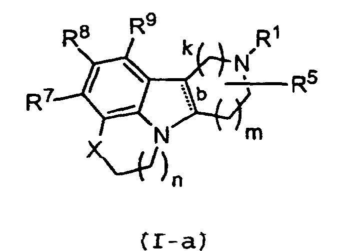 or pharmaceutically acceptable salt or prodrug forms thereof, wherein R¹, R⁵, R⁷, R⁸, R⁹, X, b, k, m, and n are defined below, are effective agonists or antagonists of 5-HT2 receptors.

### DETAILED DESCRIPTION OF THE EMBODIMENTS

Thus, in a first embodiment, the present invention provides a novel compound of Formula (I-a): 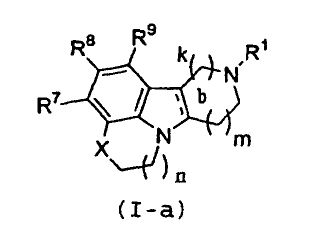 or pharmaceutically acceptable salt forms thereof, wherein:
b is a single bond;
X is -S- or -O-;
R¹ is selected from
   - (CH₂)₃C(=O)(4-fluoro-phenyl),
   - (CH₂)₃C(=O)(4-bromo-phenyl),
   - (CH₂)₃C(=O)(4-methyl-phenyl),
   - (CH₂)₃C(=O)(4-methoxy-phenyl),
   - (CH₂)₃C(=O)(4-(3,4-dichloro-phenyl)phenyl),
   - (CH₂)₃C(=O)(3-methyl-4-fluoro-phenyl),
   - (CH₂)₃C(=O)(2,3-dimethoxy-phenyl),
   - (CH₂)₃C(=O)(phenyl),
   - (CH₂)₃C(=O)(4-chloro-phenyl),
   - (CH₂)₃C(=O)(3-methyl-phenyl),
   - (CH₂)₃C(=O)(4-t-butyl-phenyl) ,
   - (CH₂)₃C(=O)(3,4-difluoro-phenyl),
   - (CH₂)₃C(=O)(2-methoxy-5-fluoro-phenyl),
   - (CH₂)₃C(=O)(4-fluoro-1-naphthyl),
   - (CH₂)₃C(=O)(benzyl),
   - (CH₂)₃C(=O)(4-pyridyl),
   - (CH₂)₃C(=O)(3-pyridyl),
   - (CH₂)₃CH(OH)(4-fluoro-phenyl),
   - (CH₂)₃CH(OH)(4-pyridyl),
   - (CH₂)₃CH(OH)(2,3-dimethoxy-phenyl),
   - (CH₂)₃S(3-fluoro-phenyl),
   - (CH₂)₃S(4-fluoro-phenyl),
   - (CH₂)₃S(=O)(4-fluoro-phenyl),
   - (CH₂)₃SO₂(3-fluoro-phenyl),
   - (CH₂)₃SO₂(4-fluoro-phenyl),
   - (CH₂)₃O(4-fluoro-phenyl),
   -(CH₂)₃O(phenyl),
   - (CH₂)₃O(3-pyridyl),
   - (CH₂)₃O(4-pyridyl),
   - (CH₂)₃O(2-NH₂-phenyl),
   - (CH₂)₃O(2-NH₂-5-F-phenyl),
   - (CH₂)₃O(2-NH₂-4-F-phenyl),
   - (CH₂)₃O(2-NH₂-3-F-phenyl),
   - (CH₂)₃O(2-NH₂-4-Cl-phenyl),
   - (CH₂)₃O(2-NH₂-4-OH-phenyl),
   - (CH₂)₃O(2-NH₂-4-Br-phenyl),
   - (CH₂)₃O(2-NHC(=O)Me-4-F-phenyl),
   - (CH₂)₃O(2-NHC(=O)Me-phenyl),
   - (CH₂)₃NH(4-fluoro-phenyl),
   - (CH₂)₃N(methyl)(4-fluoro-phenyl),
   -(CH₂)₃CO₂(ethyl),
   -(CH₂)₃C(=O)N(methyl)(methoxy),
   -(CH₂)₃C(=O)NH(4-fluoro-phenyl),
   - (CH₂)₂NHC(=O)(phenyl),
   -(CH₂)₂NMeC(=O)(phenyl),
   -(CH₂)₂NHC(=O)(2-fluoro-phenyl),
   -(CH₂)₂NMeC(=O)(2-fluoro-phenyl),
   -(CH₂)₂NHC(=O)(4-fluoro-phenyl),
   - (CH₂)₂NMeC(=O)(4-fluoro-phenyl),
   - (CH₂)₂NHC(=O)(2,4-difluoro-phenyl),
   - (CH₂)₂NMeC(=O)(2,4-difluoro-phenyl),
   - (CH₂)₃(3-indolyl),
   - (CH₂)₃(1-methyl-3-indolyl),
   - (CH₂)₃(1-indolyl),
   - (CH₂)₃(1-indolinyl),
   - (CH₂)₃(1-benzimidazolyl),
   - (CH₂)₃(1H-1,2,3-benzotriazol-1-yl),
   - (CH₂)₃(1H-1,2,3-benzotriazol-2-yl),
   - (CH₂)₂(1H-1,2,3-benzotriazol-1-yl),
   - (CH₂)₂(1H-1,2,3-benzotriazol-2-yl),
   - (CH₂)₃(3,4 dihydro-1(2H)-quinolinyl),
   - (CH₂)₂C(=O)(4-fluoro-phenyl),
   - (CH₂)₂C(=O)NH(4-fluoro-phenyl),
   - CH₂CH₂(3-indolyl),
   -CH₂CH₂(1-phthalimidyl),
   - (CH₂)₄C(=O)N(methyl)(methoxy),
   - (CH₂)₄CO₂(ethyl),
   - (CH₂)₄C(=O)(phenyl),
   - (CH₂)₃CH(phenyl)₂,
   - CH₂CH₂CH=C(phenyl)₂,
   - CH₂CH₂CH=CMe(4-F-phenyl),
   - (CH₂)₃CH(4-fluoro-phenyl)₂,
   - CH₂CH₂CH=C(4-fluoro-phenyl)₂,
   - (CH₂)₂(2,3-dihydro-1H-inden-2-yl),
   - (CH₂)₃C(=O)(2-NH₂-phenyl),
   - (CH₂)₃C(=O)(2-NH₂-5-F-phenyl),
   - (CH₂)₃C(=O)(2-NH₂-4-F-phenyl),
   - (CH₂)₃C(=O)(2-NH₂-3-F-phenyl),
   - (CH₂)₃C(=O)(2-NH₂-4-Cl-phenyl),
   -(CH₂)₃C(=O)(2-NH₂-4-OH-phenyl),
   - (CH₂)₃C(=O)(2-NH₂-4-Br-phenyl),
   - (CH₂)₃(1H-indazol-3-yl),
   - (CH₂)₃(5-F-1H-indazol-3-yl),
   - (CH₂)₃(7-F-1H-indazol-3-yl),
   - (CH₂)₃(6-Cl-1H-indazol-3-yl),
   - (CH₂)₃(6-Br-1H-indazol-3-yl),
   - (CH₂)₃C(=O)(2-NHMe-phenyl),
   - (CH₂)₃(1-benzothien-3-yl),
   - (CH₂)₃(6-F-1H-indol-1-yl),
   - (CH₂)₃(5-F-1H-indol-1-yl),
   - (CH₂)₃(6-F-2,3-dihydro-1H-indol-1-yl),
   - (CH₂)₃(5-F-2,3-dihydro-1H-indol-1-yl),
   - (CH₂)₃(6-F-1H-indol-3-yl),
   - (CH₂)₃(5-F-1H-indol-3-yl),
   - (CH₂)₃(5-F-1H-indol-3-yl),
   - (CH₂)₃(9H-purin-9-yl),
   - (CH₂)₃(7H-purin-7-yl),
   - (CH₂)₃(6-F-1H-indazol-3-yl),
   - (CH₂)₃C(=O)(2-NHSO₂Me-4-F-phenyl),
   - (CH₂)₃C(=O)(2-NHC(=O)Me-4-F-phenyl),
   - (CH₂)₃C(=O)(2-NHC(=O)Me-phenyl),
   - (CH₂)₃C(=O)(2-NHCO₂Et-4-F-phenyl),
   - (CH₂)₃C(=O)(2-NHC(=O)NHEt-4-F-phenyl),
   - (CH₂)₃C(=O)(2-NHCHO-4-F-phenyl),
   - (CH₂)₃C(=O)(2-OH-4-F-phenyl),
   -(CH₂)₃C(=O)(2-MeS-4-F-phenyl),
   - (CH₂)₃C(=O)(2-NHSO₂Me-4-F-phenyl),
   - (CH₂)₂C(Me)CO₂Me,
   - (CH₂)₂C(Me)CH(OH)(4-F-phenyl)₂,
   - (CH₂)₂C(Me)CH(OH)(4-Cl-phenyl)₂,
   - (CH₂)₂C(Me)C(=O)(4-F-phenyl),
   - (CH₂)₂C(Me)C(=O)(2-MeO-4-F-phenyl),
   - (CH₂)₂C(Me)C(=O)(3-Me-4-F-phenyl),
   - (CH₂)₂C(Me)C(=O)(2-Me-phenyl),
   - (CH₂)₂C(Me)C(=O)phenyl, 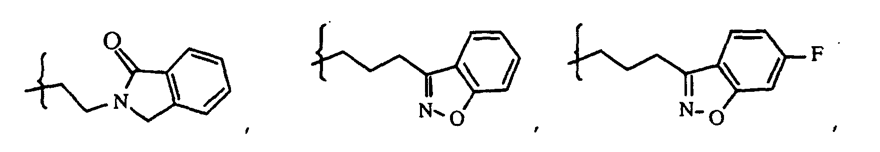 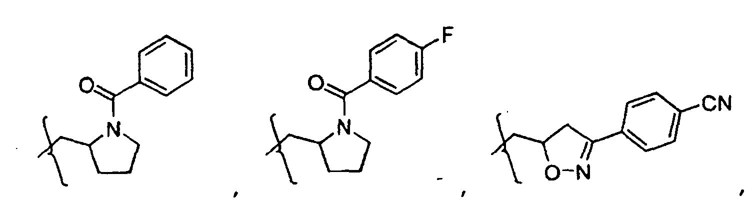
   and 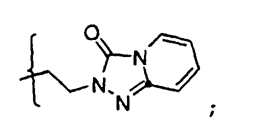
R⁷, R⁸, and R⁹, at each occurrence, are independently selected from
   hydrogen, fluoro, chloro, bromo, cyano, methyl, ethyl, propyl, isopropyl, butyl, t-butyl, nitro, trifluoromethyl, methoxy, ethoxy, isopropoxy, trifluoromethoxy, phenyl, benzyl,
   HC(=O)-, methylC(=O)-, ethylC(=O)-, propylC(=O)-, isopropylC(=O)-, n-butylC(=O)-, isobutylC(=O)-, secbutylC(=O)-, tertbutylC(=O)-, phenylC(=O)-,
   methylC(=O)NH-, ethylC(=O)NH -, propylC(=O)NH-, isopropylC(=O)NH-, n-butylC(=O)NH-, isobutylC(=O)NH-, secbutylC(=O)NH-, tertbutylC(=O)NH-, phenylC(=O)NH-, methylamino-, ethylamino-, propylamino-, isopropylamino-, n-butylamino-, isobutylamino-, secbutylamino-, tertbutylamino-, phenylamino-,
   provided that two of substituents R⁷, R⁸, and R⁹, are independently selected from hydrogen, fluoro, chloro, bromo, cyano, methyl, ethyl, propyl, isopropyl, butyl, t-butyl, nitro, trifluoromethyl, methoxy, ethoxy, isopropoxy, and trifluoromethoxy;
k is 1 or 2;
m is 1 or 2; and
n is 1 or 2.

[21] In a preferred embodiment of the present invention, the compound of Formula (I-a) is selected from Formula (II-a) or Formula (III-a): 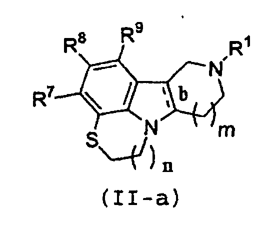 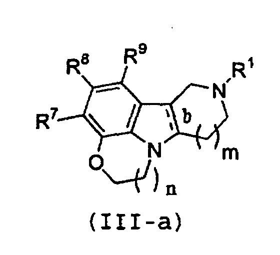 wherein:
b is a single bond, wherein the bridge hydrogens are in a cis position;
R¹ is selected from
   - (CH₂)₃C(=O)(4-fluoro-phenyl),
   - (CH₂)₃C(=O)(4-bromo-phenyl),
   - (CH₂)₃C(=O)(4-methyl-phenyl),
   - (CH₂)₃C(=O)(4-methoxy-phenyl),
   - (CH₂)₃C(=O)(4-(3,4-dichloro-phenyl)phenyl),
   - (CH₂)₃C(=O)(3-methyl-4-fluoro-phenyl),
   -(CH₂)₃C(=O)(2,3-dimethoxy-phenyl),
   - (CH₂)₃C(=O)(phenyl),
   - (CH₂)₃C(=O)(4-chloro-phenyl),
   - (CH₂)₃C(=O)(3-methyl-phenyl),
   - (CH₂)₃C(=O)(4-t-butyl-phenyl),
   - (CH₂)₃C(=O)(3,4-difluoro-phenyl),
   - (CH₂)₃C(=O)(2-methoxy-5-fluoro-phenyl),
   - (CH₂)₃C(=O)(4-fluoro-1-naphthyl),
   - (CH₂)₃C(=O)(benzyl),
   - (CH₂)₃C(=O)(4-pyridyl),
   - (CH₂)₃C(=O)(3-pyridyl),
   - (CH₂)₃CH(OH)(4-fluoro-phenyl),
   - (CH₂)₃CH(OH)(4-pyridyl),
   - (CH₂)₃CH(OH)(2,3-dimethoxy-phenyl),
   - (CH₂)₃S(3-fluoro-phenyl),
   - (CH₂)₃S(4-fluoro-phenyl),
   - (CH₂)₃S(=O)(4-fluoro-phenyl),
   - (CH₂)₃SO₂(3-fluoro-phenyl),
   - (CH₂)₃SO₂(4-fluoro-phenyl),
   - (CH₂)₃O(4-fluoro-phenyl),
   -(CH₂)₃O(phenyl),
   - (CH₂)₃NH(4-fluoro-phenyl),
   - (CH₂)₃N(methyl)(4-fluoro-phenyl),
   - (CH₂)₃CO₂(ethyl),
   - (CH₂)₃C(=O)N(methyl)(methoxy),
   - (CH₂)₃C(=O)NH(4-fluoro-phenyl),
   - (CH₂)₂NHC(=O)(phenyl),
   - (CH₂)₂NMeC(=O)(phenyl),
   - (CH₂)₂NHC(=O)(2-fluoro-phenyl),
   - (CH₂)₂NMeC(=O)(2-fluoro-phenyl),
   - (CH₂)₂NHC(=O)(4-fluoro-phenyl),
   - (CH₂)₂NMeC(=O)(4-fluoro-phenyl),
   - (CH₂)₂NHC(=O)(2,4-difluoro-phenyl),
   - (CH₂)₂NMeC(=O)(2,4-difluoro-phenyl),
   - (CH₂)₃(3-indolyl),
   - (CH₂)₃(1-methyl-3-indolyl),
   - (CH₂)₃(1-indolyl),
   - (CH₂)₃(1-indolinyl),
   - (CH₂)₃(1-benzimidazolyl),
   - (CH₂)₃(2H-1,2,3-benzotriazol-1-yl),
   - (CH₂)₃(1H-1,2,3-benzotriazol-2-yl),
   - (CH₂)₂(1H-1,2,3-benzotriazol-1-yl),
   - (CH₂)₂(1H-1,2,3-benzotriazol-2-yl),
   -(CH₂)₃(3,4 dihydro-1(2H)-quinolinyl),
   - (CH₂)₂C(=O)(4-fluoro-phenyl),
   - (CH₂)₂C(=O)NH(4-fluoro-phenyl),
   -CH₂CH₂(3-indolyl),
   - CH₂CH₂(1-phthalimidyl),
   - (CH₂)₄C(=O)N(methyl)(methoxy),
   - (CH₂)₄CO₂(ethyl),
   - (CH₂)₄C(=O)(phenyl),
   - (CH₂)₄(cyclohexyl),
   -(CH₂)₃CH(phenyl)₂,
   - CH₂CH₂CH=C(phenyl)₂,
   -CH₂CH₂CH=CMe(4-F-phenyl),
   - (CH₂)₃CH(4-fluoro-phenyl)₂,
   -CH₂CH₂CH=C(4-fluoro-phenyl)₂,
   - (CH₂)₂(2,3-dihydro-1H-inden-2-yl),
   - (CH₂)₃C(=O)(2-NH₂-phenyl),
   -(CH₂)₃C(=O)(2-NH₂-5-F-phenyl),
   - (CH₂)₃C(=O)(2-NH₂-4-F-phenyl),
   - (CH₂)₃C(=O)(2-NH₂-3-F-phenyl),
   - (CH₂)₃C(=O)(2-NH₂-4-Cl-phenyl),
   - (CH₂)₃C(=O)(2-NH₂-4-OH-phenyl),
   -(CH₂)₃C(=O)(2-NH₂-4-Br-phenyl),
   - (CH₂)₃(1H-indazol-3-yl),
   - (CH₂)₃(5-F-1H-indazol-3-yl),
   - (CH₂)₃(7-F-1H-indazol-3-yl),
   - (CH₂)₃(6-Cl-1H-indazol-3-yl),
   -(CH₂)₃(6-Br-1H-indazol-3-yl),
   -(CH₂)₃C(=O)(2-NHMe-phenyl),
   - (CH₂)₃(1-benzothien-3-yl),
   - (CH₂)₃(6-F-1H-indol-1-yl),
   - (CH₂)₃(5-F-1H-indol-1-yl),
   - (CH₂)₃(6-F-2,3-dihydro-1H-indol-1-yl),
   - (CH₂)₃(5-F-2,3-dihydro-1H-indol-1-yl),
   - (CH₂)₃(6-F-1H-indol-3-yl),
   - (CH₂)₃(5-F-1H-indol-3-yl),
   - (CH₂)₃(5-F-1H-indol-3-yl),
   - (CH₂)₃(9H-purin-9-yl),
   - (CH₂)₃(7H-purin-7-yl),
   - (CH₂)₃(6-F-1H-indazol-3-yl),
   - (CH₂)₃C(=O)(2-NHSO₂Me-4-F-phenyl),
   - (CH₂)₃C(=O)(2-NHC(=O)Me-4-F-phenyl),
   - (CH₂)₃C(=O)(2-NHC(=O)Me-4-F-phenyl),
   - (CH₂)₃C(=O)(2-NHCO₂Et-4-F-phenyl),
   -(CH₂)₃C(=O)(2-NHC(=O)NHEt-4-F-phenyl),
   - (CH₂)₃C(=O)(2-NHCHO-4-F-phenyl),
   - (CH₂)₃C(=O)(2-OH-4-F-phenyl),
   - (CH₂)₃C(=O)(2-MeS-4-F-phenyl),
   - (CH₂)₃C(=O)(2-NHSO₂Me-4-F-phenyl),
   -(CH₂)₂C(Me)CO₂Me,
   - (CH₂)₂C(Me)CH(OH)(4-F-phenyl)₂,
   - (CH₂)₂C(Me)CH(OH)(4-Cl-phenyl)₂,
   - (CH₂)₂C(Me)C(=O)(4-F-phenyl),
   - (CH₂)₂C(Me)C(=O)(2-MeO-4-F-phenyl),
   - (CH₂)₂C(Me)C(=O)(3-Me-4-F-phenyl),
   - (CH₂)₂C(Me)C(=O)(2-Me-phenyl) ,
   - (CH₂)₂C(Me)C(=O)phenyl, 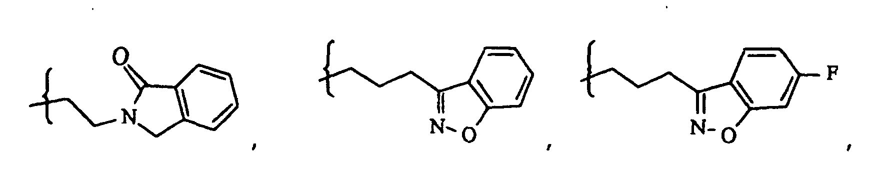 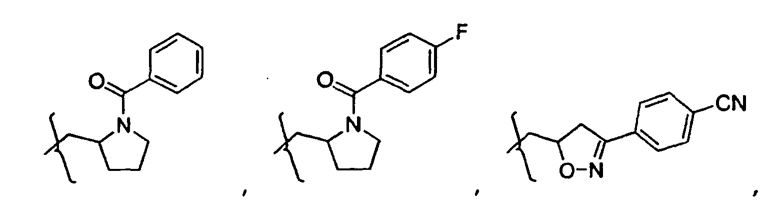
   and 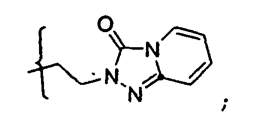
R⁷, R⁸, and R⁹, at each occurrence, are independently selected from hydrogen, fluoro, chloro, bromo, cyano, methyl, ethyl, propyl, isopropyl, butyl, t-butyl, nitro, trifluoromethyl, methoxy, ethoxy, isopropoxy, trifluoromethoxy, methylC(=O)-, ethylC(=O)-, propylC(=O)-, isopropylC(=O)-, methylC(=O)NH-, ethylC(=O)NH -, propylC(=O)NH-, isopropylC(=O)NH, methylamino-, ethylamino-, propylamino-, and isopropylamino-,
provided that two of substituents R⁷, R⁸, and R⁹, are independently selected from hydrogen, fluoro, chloro, methyl, trifluoromethyl, methoxy, and trifluoromethoxy.

In a more preferred embodiment of the present invention, are compounds of Formula (I-a) selected from Table 1.

In a more preferred embodiment of the present invention, are compounds of Formula (I-a) selected from Table 2.

In an even further more preferred embodiment of the present invention, are compounds of Formula (I-a) selected from Table 3.

In an even further more preferred embodiment of the present invention, are compounds of Formula (I-a) selected from Table 4.

In a second embodiment, the present invention provides a pharmaceutical composition comprising a compound of Formula (I-a) or a pharmaceutically acceptable salt form thereof and a pharmaceutically acceptable carrier.

In a third embodiment, the present invention provides a compound of Formula (I-a) or a pharmaceutically acceptable salt form thereof for use in therapy.

In a preferred embodiment the compound is a 5HT2a antagonist.

In another preferred embodiment the compound is a 5HT2c agonist.

In a more preferred embodiment the present invention provides a compound of Formula (I-a) or a pharmaceutically acceptable salt form thereof for use in treating central nervous system disorders including obesity, anxiety, depression, psychosis, schizophrenia, sleep disorders, sexual disorders, migraine, conditions associated with cephalic pain, social phobias, and gastrointestinal disorders such as dysfunction of the gastrointestinal tract motility.

In a further preferred embodiment the central nervous system disorder comprises obesity.

In another further preferred embodiment the central nervous system disorder comprises schizophrenia.

In another further preferred embodiment the central nervous system disorder comprises depression.

In another further preferred embodiment the central nervous system disorder comprises anxiety.

In a fifth embodiment the present invention provides the use of novel compounds of Formula (I-a) or pharmaceutically acceptable salt forms thereof in the manufacture of a medicament for the treatment of central nervous system disorders including obesity, anxiety, depression, psychosis, schizophrenia, sleep disorders, sexual disorders, migraine, conditions associated with cephalic pain, social phobias, and gastrointestinal disorders, in particular for the treatment of obesity, schizophrenia or depression.

### DEFINITIONS

The compounds herein described may have asymmetric centers. Compounds of the present invention containing an asymmetrically substituted atom may be isolated in optically active or racemic forms. It is well known in the art how to prepare optically active forms, such as by resolution of racemic forms or by synthesis from optically active starting materials. Many geometric isomers of olefins, C=N double bonds, and the like can also be present in the compounds described herein, and all such stable isomers are contemplated in the present invention. Cis and trans geometric isomers of the compounds of the present invention are described and may be isolated as a mixture of isomers or as separated isomeric forms. All chiral, diastereomeric, racemic forms and all geometric isomeric forms of a structure are intended, unless the specific stereochemistry or isomeric form is specifically indicated.

The numbering of the tetracyclic ring-system present in the compounds of Formula (I-a), as defined by nomenclature known to one skilled in the art, is shown for two examples in Formula (I'), when k is 1, m is 1, and n is 1; and in Formula (I''), when k is 1, m is 1, and n is 2: 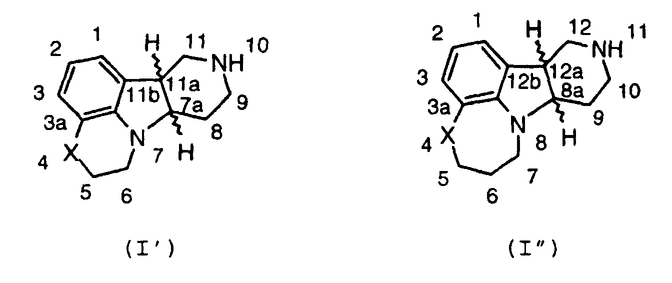 The tetracyclic ring-system present in compounds of Formula (I) occur as "cis" or "trans" isomers when the carbon-carbon bond b in Formula (I) is a single bond. As such, the terms "cis" and "trans", in conjunction with the tetracyclic ring structure, refer to the configuration of hydrogen atoms on carbon atoms 7a and 11a in Formula (I') or, for example, on carbon atoms 8a and 12a in Formula (I"), above. When both hydrogens are on the same side of the mean plane determined by the octahydro tetracyclic moiety then the configuration is designated "cis", if not, the configuration is designated "trans". It is understood that the above example is for demonstrative puproses only and not intended to limit the scope of the tetracyclic ring-system present in compounds of Formula (I). As such, it is understood that one skilled in the art of organic chemistry can apply the above numbering system to other values of k, m, and n in the scope of compounds of Formula (I) to deterine the appropriate numbering. Additional Examples of the numbering of the tetracyclic ring-system are further provided below in the synthetic Examples. Lastly, it is understood that the use of "cis" or "trans" in the identification of the tetracyclic ring-system is not meant to construe the configuration of any other cis or trans geometric isomer in the molecule, for example, cis or trans butene.

The term "substituted," as used herein, means that any one or more hydrogens on the designated atom is replaced with a selection from the indicated group, provided that the designated atom's normal valency is not exceeded, and that the substitution results in a stable compound. When a substituent is keto (i.e., =O), then 2 hydrogens on the atom are replaced.

When any variable (e.g., R²) occurs more than one time in any constituent or formula for a compound, its definition at each occurrence is independent of its definition at every other occurrence. Thus, for example, if a group is shown to be substituted with 0-2 R², then said group may optionally be substituted with up to two R² groups and R² at each occurrence is selected independently from the definition of R². Also, combinations of substituents and/or variables are permissible only if such combinations result in stable compounds.

When a bond to a substituent is shown to cross a bond connecting two atoms in a ring, then such substituent may be bonded to any atom on the ring. When a substituent is listed without indicating the atom via which such substituent is bonded to the rest of the compound of a given formula, then such substituent may be bonded via any atom in such substituent. Combinations of substituents and/or variables are permissible only if such combinations result in stable compounds.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like.

The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in *Remington's Pharmaceutical Sciences,* 17th ed., Mack Publishing Company, Easton, PA, 1985, p. 1418, the disclosure of which is hereby incorporated by reference.

"Stable compound" and "stable structure" are meant to indicate a compound that is sufficiently robust to survive isolation to a useful degree of purity from a reaction mixture, and formulation into an efficacious therapeutic agent.

### SYNTHESIS

Throughout the details of the invention, the following abbreviations are used with the following meanings:

| Reagents: | |
|---|---|
| MCPBA | m-chloroperoxybenzoic acid |
| DIBAL | diisobutyl aluminum hydride |
| Et₃N | triethylamine |
| TFA | trifluoroacetic acid |
| LAH | lithium aluminum hydride |
| NBS | N-bromo succinimide |
| Red-Al | Sodium bis(2-methoxyethoxy)aluminum hydride |
| Pd₂dba₃ | Tris(dibenzylideneacetone)dipalladium(0) |
| ACE-Cl | 2-chloroethylchloroformate |

| Solvents: | |
|---|---|
| THF | tetrahydrofuran |
| MeOH | methanol |
| EtOH | ethanol |
| EtOAc | ethyl acetate |
| HOAc | acetic acid |
| DMF | dimethyl formamide |
| DMSO | dimethyl sulfoxide |
| DME | dimethoxyethane |
| Et₂O | diethylether |
| iPrOH | isopropanol |
| MEK | methyl ethyl ketone |

| Others: | |
|---|---|
| Ar | aryl |
| Ph | phenyl |
| Me | methyl |
| Et | ethyl |
| NMR | nuclear magnetic resonance |
| MHz | megahertz |
| BOC | tert-butoxycarbonyl |
| CBZ | benzyloxycarbonyl |
| Bn | benzyl |
| Bu | butyl |
| Pr | propyl |
| cat. | catalytic |
| mL | milliliter |
| nM | nanometer |
| ppm | part per million |
| mmol | millimole |
| mg | milligram |
| g | gram |
| kg | kilogram |
| TLC | thin layer chromatography |
| HPLC | high pressure liquid chromatography |
| RPM | revolutions per minute |
| rt | room temperature |
| aq. | aqueous |
| sat. | saturated |

The compounds of the present invention can be prepared in a number of ways well known to one skilled in the art of organic synthesis. The compounds of the present invention can be synthesized using the methods described below, together with synthetic methods known in the art of synthetic organic chemistry, or variations thereon as appreciated by those skilled in the art. Preferred methods include, but are not limited to, those described below. All references cited herein are hereby incorporated in their entirety herein by reference.

The novel compounds of this invention may be prepared using the reactions and techniques described in this section. The reactions are performed in solvents appropriate to the reagents and materials employed and are suitable for the transformations being effected. Also, in the description of the synthetic methods described below, it is to be understood that all proposed reaction conditions, including choice of solvent, reaction atmosphere, reaction temperature, duration of the experiment and workup procedures, are chosen to be the conditions standard for that reaction, which should be readily recognized by one skilled in the art. It is understood by one skilled in the art of organic synthesis that the functionality present on various portions of the molecule must be compatible with the reagents and reactions proposed. Such restrictions to the substituents which are compatible with the reaction conditions will be readily apparent to one skilled in the art and alternate methods must then be used.

The preparation of compounds of Formula (I) of the present invention may be carried out in a convergent or sequential synthetic manner. Detailed synthetic preparations of the compounds of Formula (I) are shown in the following reaction schemes. The skills required in preparation and purification of the compounds of Formula (I) and the intermediates leading to these compounds are known to those in the art. Purification procedures include, but are not limited to, normal or reverse phase chromatography, crystallization, and distillation.

Several methods for the preparation of the compounds of the present invention are illustrated in the schemes and examples shown below. The substitutions are as described and defined above.

Compounds of Formula (I) of this invention may be prepared as shown in Scheme 1. Thus, preparation of an aryl hydrazine (III) is accomplished, for example, by treatment of a corresponding substituted aniline (II) with NaNO₂ followed by reduction of the N-nitroso intermediate with a reducing agent such as LAH or zinc and an organic acid, such as acetic acid or trifluoroacetic acid at low temperature. Assembly of the core tetracyclic intermediate indole (V) is accomplished by Fischer indole cyclization of the aryl hydrazine and a suitably substituted ketone (i.e. (IV)) by methods described by, but not limited to, R.J. Sundberg, "Indoles, Best Synthetic Methods" **1996**, Academic Press, San Diego, CA. For example, treatment of the aryl hydrazine (III) as the free base or the corresponding mineral acid salt with the ketone (IV) (R¹ = H, Bn, CBZ, CO₂Et, etc) in an alcoholic solvent in the presence of mineral acid affords the indoles (V) as the free bases (after treatment with aq. NaOH). Reduction of the indoles to the corresponding cis-or trans substituted dihydroindoles is accomplished by, for example, treatment with hydrogen in the presence of a catalyst such as platinum oxide or palladium on carbon, or with a metal such as zinc and a mineral acid such as hydrochloric acid, or with sodium and liquid ammonia, or with borane-amine complex such as borane-triethylamine in tetrahydofuran, or preferably by treatment with NaCNBH₃ in an acid such as acetic or trifluoroacetic acid.

The corresponding enantiomers can be isolated by separation of the racemic mixture of (I) on a chiral stationary phase column utilizing normal or reverse phase HPLC techniques, the details of which are described in the examples. Alternatively, a diastereomeric mixture of (I) can be prepared by treatment of (I, R¹ = H) with an appropriate chiral acid (or suitably activated derivative), for example dibenzoyl tartrate or the like (see, for example, Kinbara, K., et. al., *J. Chem*. *Soc.,* Perkin *Trans. 2*, **1996**, 2615; and Tomori, H., et. al., *Bull*. *Chem*. *Soc. Jpn*., **1996**, 3581). The diastereomers would then be separated by traditional techniques (i.e. silica chromatography, crystallization, HPLC, etc) followed by removal of the chiral auxiliary to afford enantiomerically pure (I).

In the cases where the carboline nitrogen has been protected (VI) (i.e. R¹ = Boc, Bn, CBZ, CO₂R), it may be removed under a variety of conditions as described in Greene, T.W., Wuts, P.G.W., "Protective Groups in Organic Synthesis, 2nd Edition", John Wiley and Sons, Inc., New York, pages 309-405, **1991**. The free secondary amine could then be alkylated, for example, by treatment with a suitably substituted alkyl halide (R¹Cl, or R¹I) and a base to afford additional compounds of type (I), as described, for example, by Glennon, R.A., et. al., *Med. Chem. Res.,* **1996**, 197.

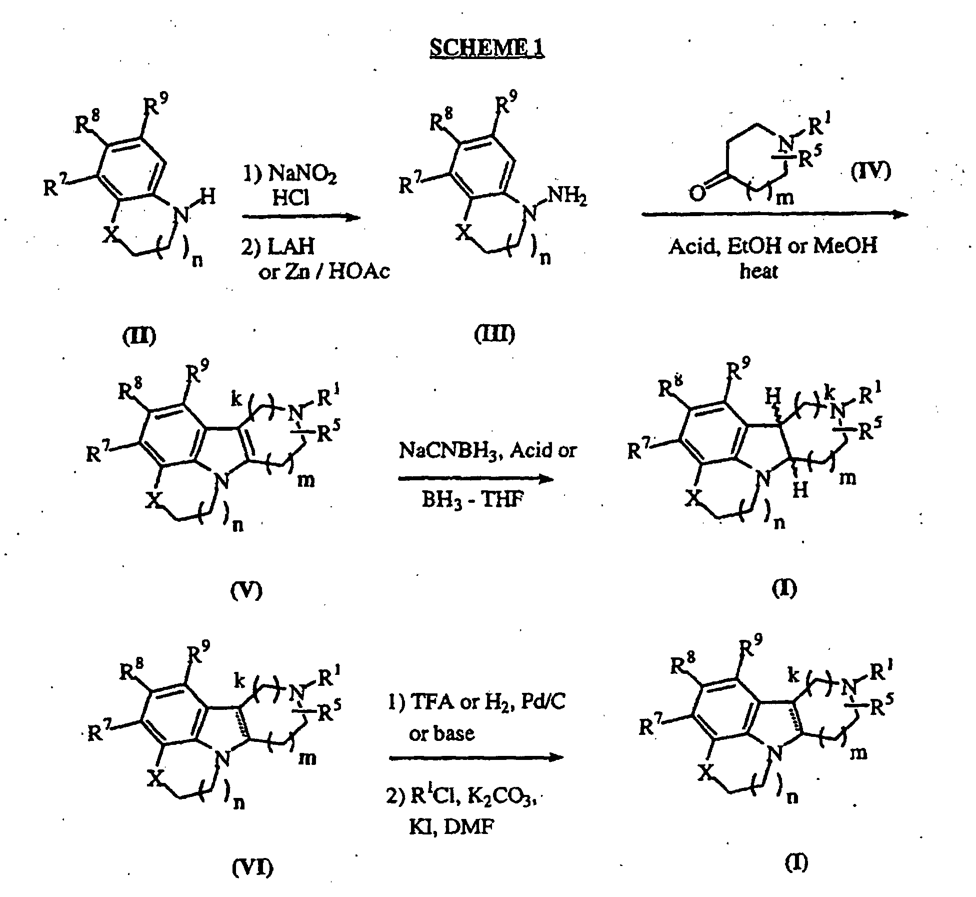

Alternatively, compounds of Formula (I) can be prepared as described in Scheme 2. Treatment of an ortho halonitrobenzene compound (VII) with a nucleophilic alkyl halide (X = OH, SH, NHR, (VIII)) (as described by Kharasch, N., Langford, R.B., *J. Org. Chem.,* **1963,** 1903) and a suitable base followed by subsequent reduction of the corresponding nitroaryl derivative to the aniline (IX). The reduction may be accomplished with a variety of reducing agents, for example, LAH, SnCl₂, NaBH₄, N₂H₄, etc. or with hydrogen in the presence of a suitable catalyst, such as palladium on carbon, or platinum oxide, etc., (see Hudlicky, M., "Reductions in Organic Chemistry", Ellis Horwood, Ltd., Chichester, UK, **1984**). Formation of the aryl hydrazine (X) may be accomplished as described previously in Scheme 1 or more directly by treatment of the aniline (IX) with aq. hydrochloric acid, stannous chloride and NaNO₂ at room temperature (see, Buck, J.S., Ide, W.S., *Org*. *Syn., Coll*. *Vol*., 2, **1943**, 130). This primary aryl hydrazine (X) can then be cyclized under Fischer indole cyclization conditions as detailed above for compound (V), to afford the indole (XI) as the corresponding salt. Upon treatment of the indole (XI) with a base such potassium hydroxide or potassium t-butoxide in a solvent such as DME or THF affords the tetracyclic indole intermediates (V). These indoles can also be reduced to the corresponding cis-or trans indolines (I) as described previously in Scheme 1.

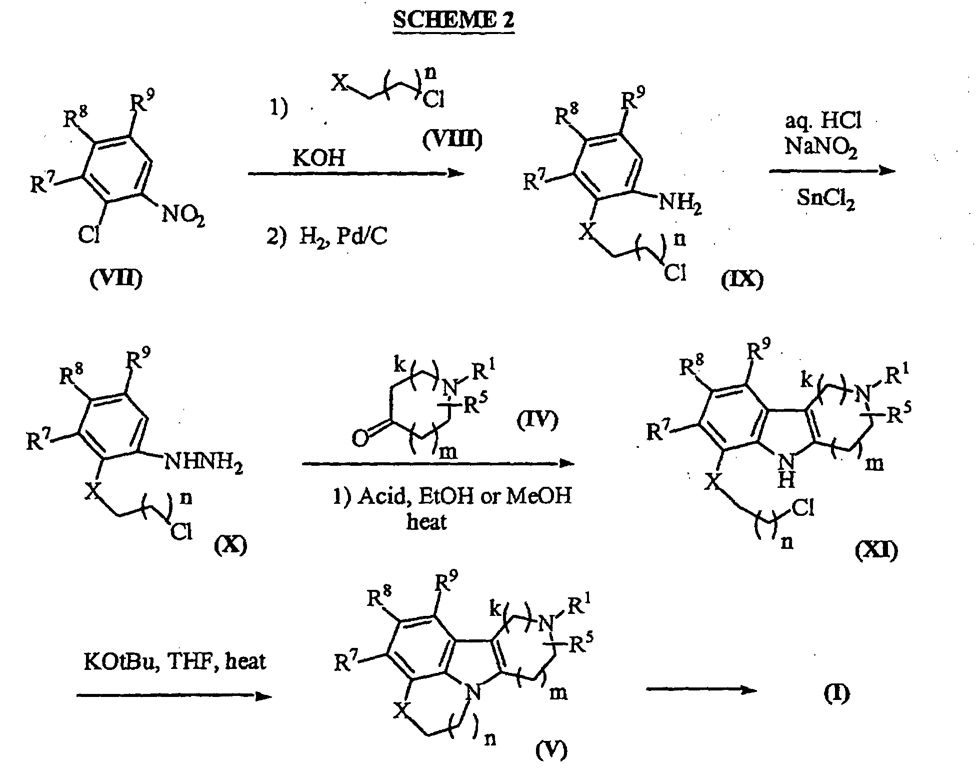

Still another related route to compounds of Formula (I) is shown in Scheme 3. Initiating the synthesis with a nitrobenzene derivative such as (XII), this approach allows for a variety of derivatization. More highly substituted nitrobenzenes can be obtained by traditional synthetic manipulation (*i*.*e*. aromatic substitution) and are known by those in the art (see Larock, R.C., *Comprehensive Organic Transformations,* VCH Publishers, New York, **1989**). Treatment of nitrobenzene derivative with a reducing agent such as LAH, etc., as described previously (see Hudlicky, et. al.), affords the corresponding aniline intermediate. Subsequent formation of the hydrazine followed by Fischer indole cyclization with a suitably functionalized ketone as described above (*i.e.* Scheme 1, (III) to (V)) affords the g-carboline indole (XIII). At this point the fused ring may be appended by condensation of a haloalkyl carboxylic acid or a related activated carboxylic acid (*i.e.* acid chloride, mixed anhydride, etc.) such as (XIV). Reduction of the resultant heterocyclic carbonyl may be effected with various reducing agents, for example, sodium borohydride, diisobutyl aluminum hydride and the like (see Larock; R.C., *Comprehensive Organic Transformations,* VCH Publishers, New York, **1989** and/or Hudlicky, M., "Reductions in Organic Chemistry", Ellis Horwood, Ltd., Chichester, UK, **1984**) to afford the tetracyclic indoles (V). Further reduction of the indole (V) to the indolines (I) is as described previously in Scheme 1.

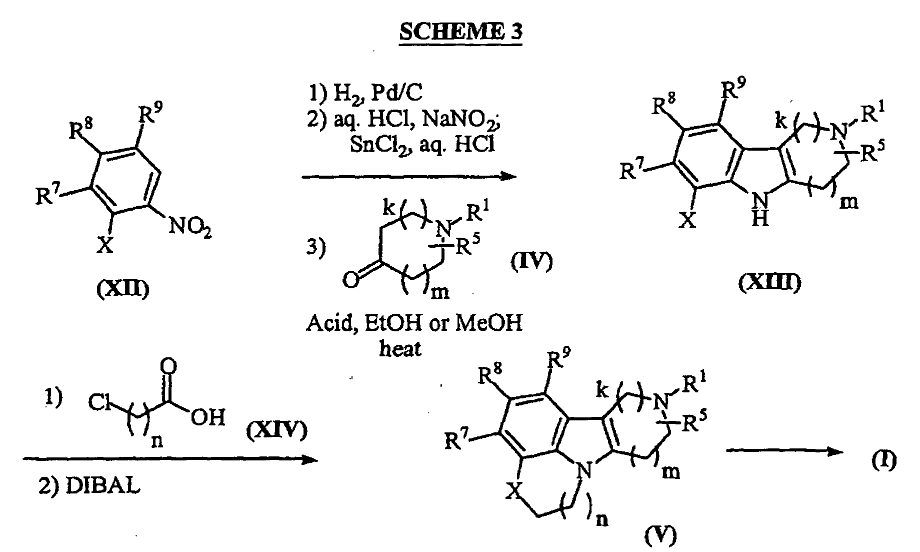

Preparation of the aniline precursors (II) to the Fischer indole cyclizations is shown in Scheme 4. Treatment of a suitably ortho-functionalized aniline XVI) with a chloroalkyl carboxylic acid or ester (or equivalent substrate, *i.e.* acrylic acid, acryloyl chloride, etc.) and concomitant condensation, followed by reduction of the resultant heterocyclic carbonyl with a reducing agent such as LAH, DIBAL, or Red-Al affords the fused heterocyclic benzene derivatives (II). More diverse intermediates of (II) may be obtained by formation of the ortho substitiuted aniline from the corresponding ortho substituted nitobenzenes and concomitant reduction of the nitro moiety as described above. Furthermore, aromatic substitution of the fluoro (or other halo derived nitrobenzene) functionality of (XV) for an oxygen, or sulphur moiety is accomplished, for example, by treatment of (XV) with a nucleophile, such as sodium sulfide or an alcohol, followed by formation of the requisite thiophenol or phenol, respectively, using standard techniques known by those in the art (see Larock, R.C., *Comprehensive Organic Transformations,* VCH Publishers, New York, **1989**, page 481). Reduction of the nitro as before affords the substituted anilines (XVI).

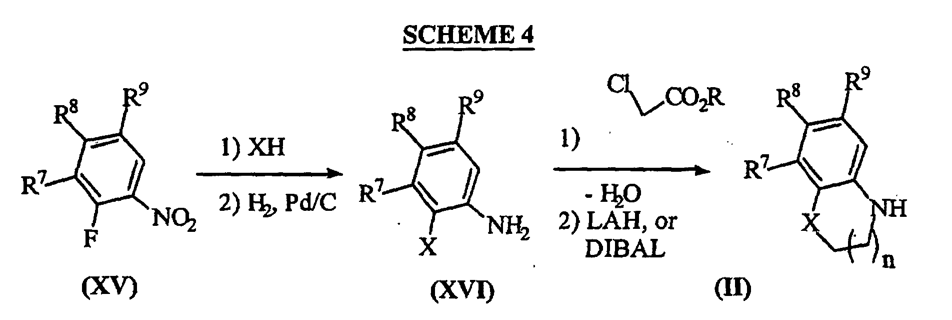

An alternate approach to the substituted fused anilines (II) is shown in Scheme 5. Treatment of the phenol (X = OH), thiophenol (X = SH), or other nucleophilically aromatic substituted derivative (XVII) with, for example, a haloalkyl carboxylic acid (or equivalent activated haloalkylcarboxylic acid, (*i.e.* acid halide, mixed anhydride, acrylic acid, acryloyl chloride, etc.), affords the derivative (XVIII) which when treated under Friedel-Crafts acylation conditions (see Ed. G.A. Olah, "Friedel-Crafts and Related Reactions", J. Wiley and Sons, New York, **1964**, Vol 3, Pts 1 and 2 or Chem. Rev., 1955, 229, or Olah, G.A., "Friedel-Crafts Chemistry", Wiley Interscience, New York, **1973,** for varying conditions and protocols), i.e. strong Lewis acids (AlCl₃, FeCl₃, etc.), affords the cyclic alkylphenones (XIX). Incorporation of the nitrogen functionality can be accomplished in several ways. For example, Schmidt rearrangement (as described by Smith, P.A.S., *J. Am. Chem. Soc*., **1948**, 320) is effected by treatment of the carbonyl derivative (XIX) with NaN₃ and methanesulfonic acid to afford the bicyclic lactam (XX). Alternatively, this transformation may be carried out under Hoffmann rearrangement protocol (see, for example, Dike, S.Y., et. al., *Bioorg. Med. Chem. Lett.,* **1991**, 383), by initial formation of the oxime derivative of (XXI) by treatment with hydroxylamine hydrochloride. Subsequent rearrangement to the lactam is efficiently accomplished by heating in polyphosphoric acid to afford the lactam (XX). Reduction of the lactam (XX) can be accomplished with a variety of reducing agents, for example, DIBAL, Red-Al and the like to afford the aniline (II).

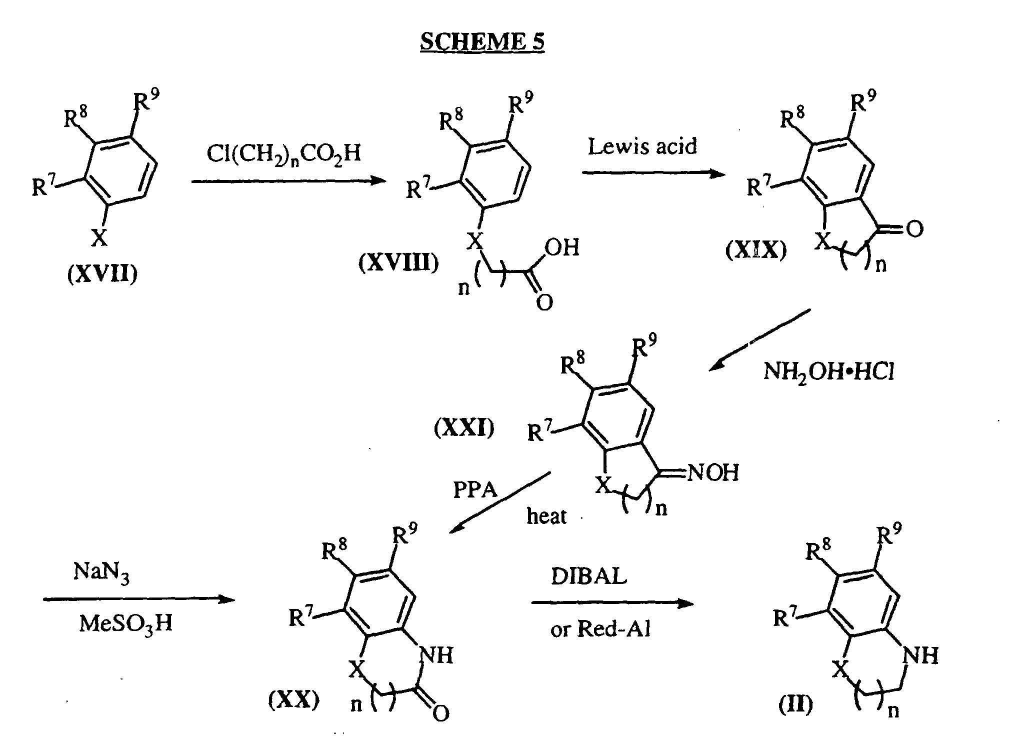

The preparation of compounds of Formula (I) with additional diversity of functionalization of the aromatic A ring of the tetracycle is shown in Scheme 6 and Scheme 7 and described here. Due to the nature of the synthetic route of Scheme 1 to derivatives of Formula (I), compounds with halogen substituents on the A-ring are difficult to prepare. However, bromination of the indolines (I, R⁸ = H) when the amine is protected, for example, with the Boc or CBZ protecting groups, with, for example, NBS in DMF affords the R⁸ brominated derivatives (XXII). These activated aryl derivatives (XXII) act as excellent counterparts for a number of important synthetic transformations.

For example, biaryl coupling is accomplished under Suzuki coupling protocol. For a review and leading references of palladium catalyzed cross coupling reactions, see Miyaura, N., Suzuki, A., *Chem. Rev.*, **1995**, 2457. One such procedure entails treatment of the aryl bromide (XXII) with a functionalized aryl boronic acid (XXIII) in the presence of a catalytic Pd(0) species, such as Pd(PPh₃)₄, Pd(PPh₃)₂Cl₂, Pd(OAC)₂, Pd₂(dba)₃ and a suitable ligand such as PPh₃, AsPh₃, etc., or other such Pd(0) catalyst, and a base such as Na₂CO₃ or Et₃N in a suitable solvent such as DMF, toluene, THF, DME or the like, to afford the indolines (XXIV). Alternatively formation of the indole boronic acid from the bromine derivative (XXII) (i.e. (I, R⁸ = B(OH)₂)) would allow for greater diversity in the subsequent coupling of this indole boronic acid with commercially available haloaromatic derivatives in a similar Suzuki coupling strategy as described above to afford the indolines (XXIV).

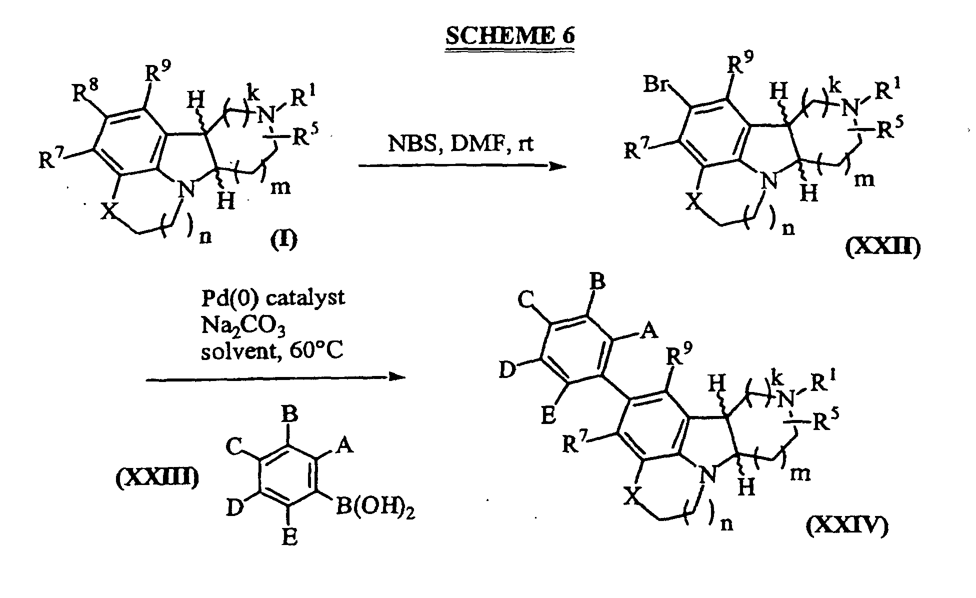

Similarly biaryl coupling of the bromine derivatives (XXV), readily obtained by the synthetic sequence exemplified in Scheme 2, (starting with the suitably functionalized bromo nitrobenzenes (II)), is shown in Scheme 7. This approach allows for the preparation of biaryl indoles as well as the corresponding indoline derivatives. Protection of the amine functionality must be carried out if R¹ = H (see Greene et.al for protections of amines). This is readily accomplished, for example, by treatment of bromo derivatives (XXV) with (Boc)₂O in aqueous sodium hydroxide and dioxane. Subsequent Suzuki coupling with a variety of aryl boronic acids is carried out as described above in Scheme 6, to afford the biaryl adducts (XXVI). This protocol is amenable to R⁷, R⁸, and R⁹ bromide, iodide, triflates, and/or diazo derivatives (see Miyaura, N., Suzuki, A., *Chem. Rev.*, **1995**, 2457, for a review of aryl couplings).

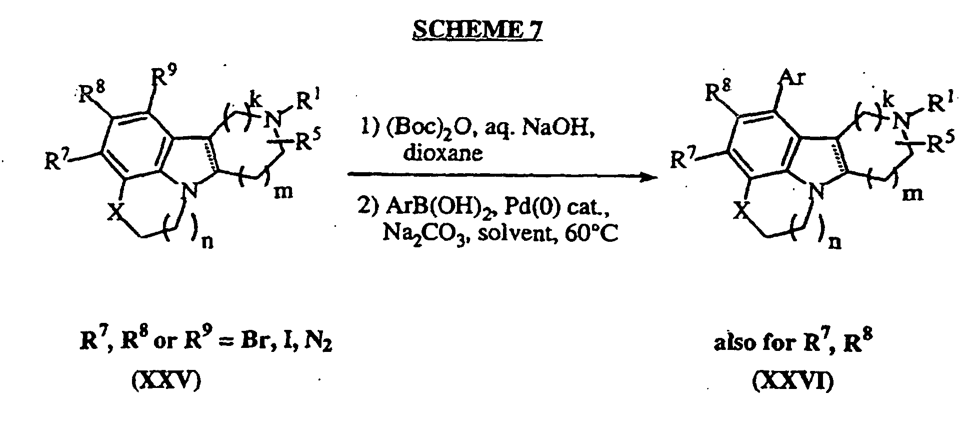

Furthermore and as an extension of this approach to a rapid preparation of a large array of biaryl indole and indoline derivatives, these bromide derivatives (XXV) can be bound to a solid support and the Suzuki couplings can be carried out on solid support (see XXVIII) as illustrated in Scheme 8. Towards that end treatment of indoline (XXV) with TFA in CH₂Cl₂, to remove the Boc protecting group, followed extraction from aqueous base provides the free amine (XXXVII). The free amine can be loaded onto a suitable solid support such as (XXVIII) using conditions well known to those skilled in the art. Thus, p-nitrophenylchloroformate Wang resin (XXVIII) which can be obtained commercially from sources such as Novabiochem, Inc. is swollen in a suitable solvent such as N-methyl pyrrolidinone and treated with 1.5 equiv. of amine to afford the functionalized resin (XXIX). Suzuki couplings are then carried out in array format by treatment of resins (XXIX) with a suitable palladium source such as Pd(PPh₃)₄ or Pd(dppf)Cl₂ and a suitable base such as 2M aqueous K₂CO₃ or Na₂CO₃ or triethylamine with an excess (typically 5 equivalents) of an aryl boronic acid (procedures for solid-phase Suzuki and other palladium couplings are well-known by those in the art, see for instance L.A. Thompson and J.A. Ellman, *Chem. Rev.* **1996**, *96*, (1), 555-600). The coupling may be repeated to ensure complete conversion to the desired coupled product. Cleavage from the solid support by treatment with TFA affords the corresponding indoles and indolines (XXX) as their TFA salts.

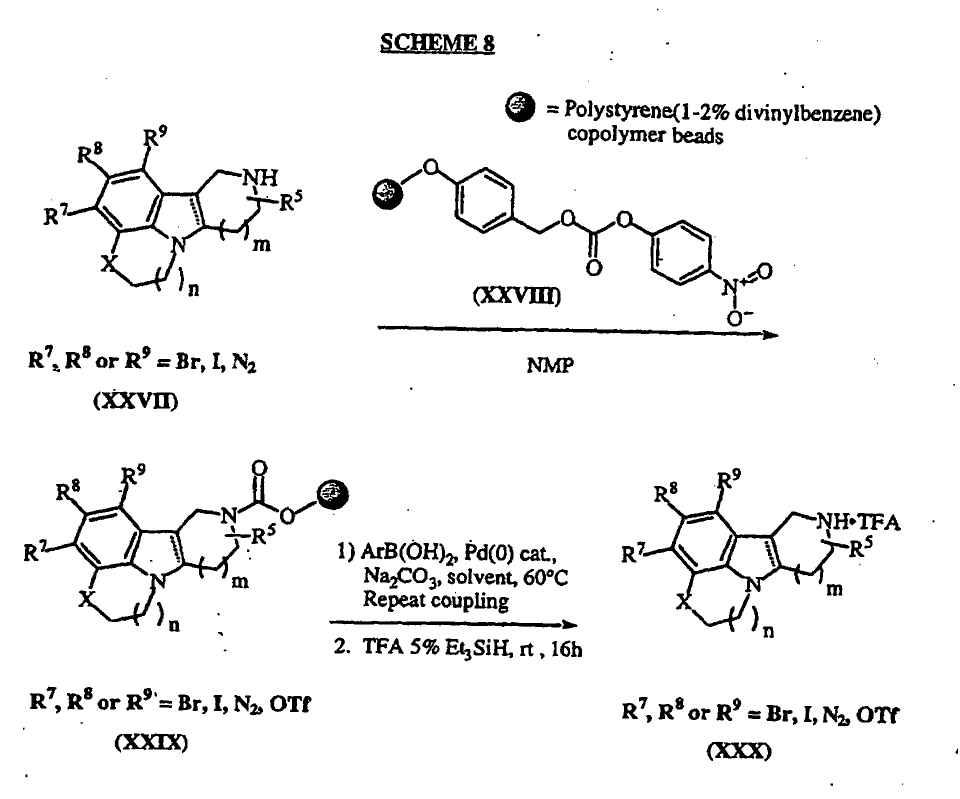

In addition, there exists a wide range of procedures and protocols for functionalizing haloaromatics, aryldiazonium and aryltriflate compounds. These procedures are well known by those in the art and described, for example, by Stanforth, S.P., *Tetrahedron,* **1998,** 263; Buchwald, S.L., et. al., *J. Am. Chem. Soc.,* **1998,** 9722; Stille, J.K., et. al., *J. Am. Chem. Soc.,* **1984,** 7500. Among these procedures are biaryl couplings, alkylations, acylations, aminations, and amidations. The power of palladium catalyzed functionalization of aromatic cores has been explored in depth in the last decade. An excellent review of this field can be found in J. Tsuji, "Palladium Reagents and Catalysts, Innovations in Organic Synthesis", J. Wiley and Sons, New York, **1995**.

One such method to prepare compounds of Formula (I) with substituted R¹ sidechains in a more direct manner is shown in Scheme 9. Alkylation of the indole or indoline derivatives (I, R¹ = H) with a haloalkyl ester, such as ClCH₂(CH₂)ₚCO₂Me, in the presence of NaI or KI and a base such as K₂CO₃, Na₂CO₃ or the like, in dioxane or THF or other such solvent while heating (see Glennon, R.A., et. al., *Med. Chem. Res.,* **1996**, 197) affords the R¹ alkylated esters. Subsequent formation of the activated amides (XXXI) is accomplished by treatment of the ester with N,O-dimethylhydroxylamine hydrochloride and a Lewis acid such as trimethylaluminum or triethylaluminum in toluene (see, for example, Golec, J.M.C., et. al., *Tetrahedron*, **1994**, 809) at 0°C. Treatment of the amide (XXXI) with a variety of organometallic agents, such as Grignard reagents R^{1a}MgBr, alkyl and aryl lithium reagents etc. (see Sibi, M.P., et. al., *Tetrahedron Lett.,* **1992**, 1941; and more generally House, H.O., *Modern Synthetic Reactions,* W.A. Benjamin, Inc., Menlo Park, CA., **1972**), in a suitable solvent such as THF, ether, etc. at low temperatures affords the substituted ketones (XXXII).

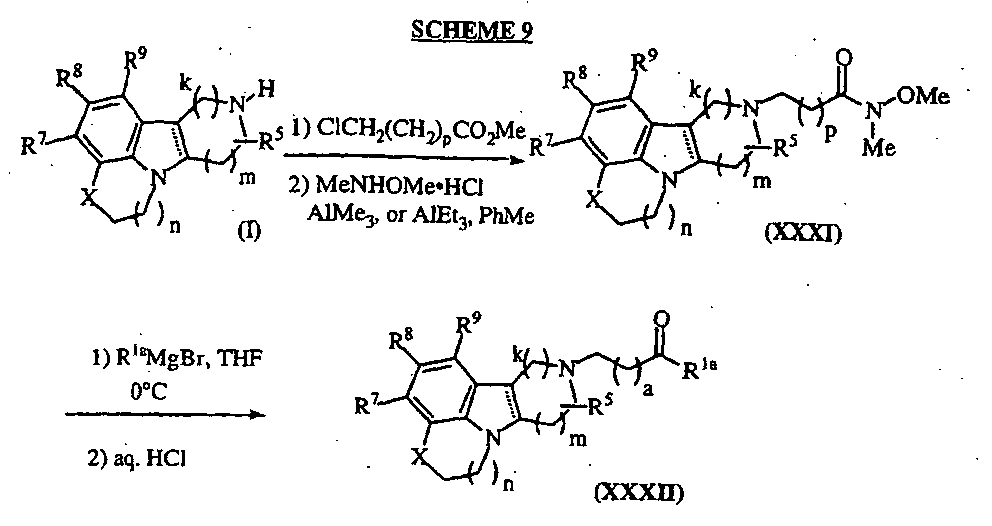

Preparation of compounds of Formula (I) where m=0, k = 1 is outlined in Scheme 10 and described here. Fischer indole cyclization of the previously described hydrazine (III) with a known protected 2,3-dioxopyrolidine (Carlson, E.H., et. al., *J*. *Org*. *Chem*., **1956**, 1087) under a variety of typical cyclization conditions affords the tetracyclic indole (XXXIII). The reduction may be accomplished with a variety of reducing agents, for example, LAH, DIBAL, etc., to yield the pyrole fused indole (XXXIV). This derivative can then be deprotected and subsequently alkylated as described previously (see Greene, T.W., Wuts, P.G.W., "Protective Groups in Organic Synthesis, 2nd Edition", John Wiley and Sons, Inc., New York, **1991,** and Scheme 1), to give the R¹ alkylated indole analogs (XXXV). Alternatively, reduction of the indole to the indoline, as described previously (see Scheme 1), followed by deprotection of the benzyl group to give (XXXVI) and alkylation gives access to the corresponding R¹ alkylated indoline derivatives (XXXVII). All the previously described methods to functionalize the aromatic ring, and to afford derivatives of varying R¹ sidecahins are applicable to these cores.

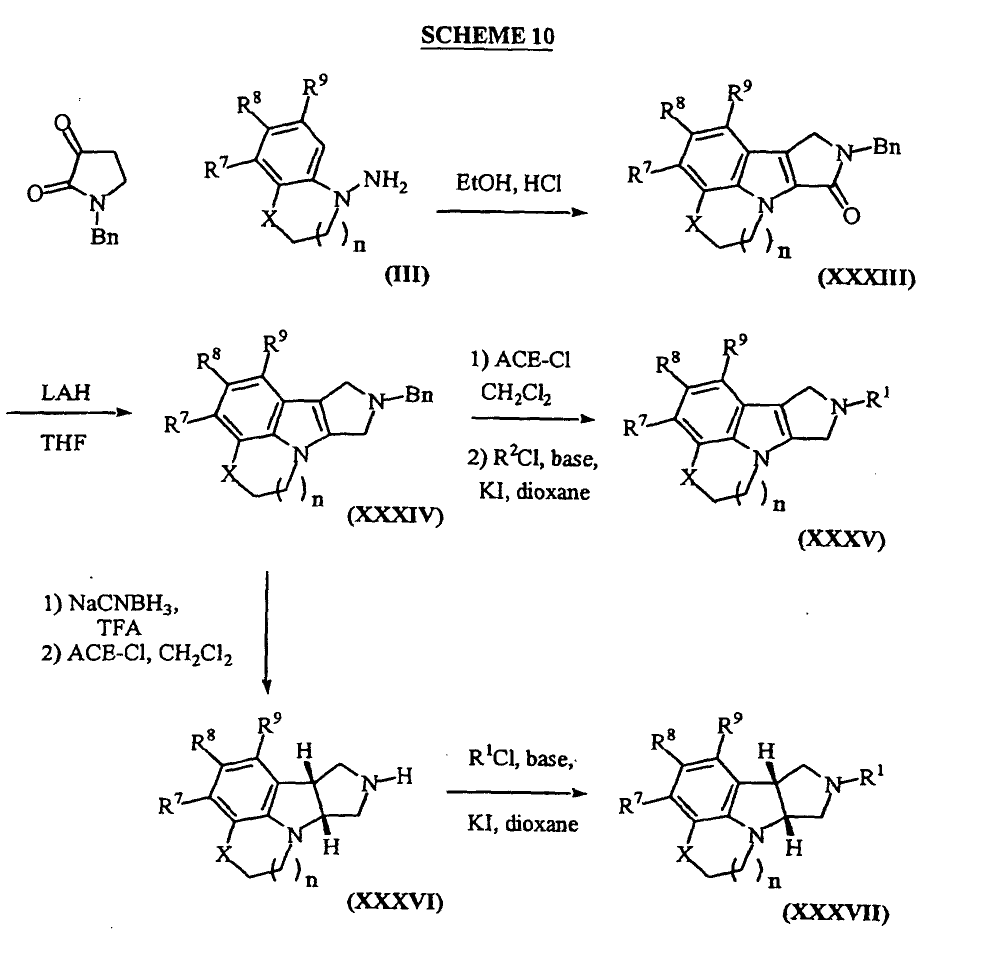

### EXAMPLES

Chemical abbreviations used in the Examples are defined above. The detailed processes for preparing the compounds of Formula (I) are illustrated by the following Examples. It is, however, understood that this invention is not limited to the specific details of these examples. The Examples as set forth below are intended to demonstrate the scope of the invention but are not intended to limit the scope of the invention. Proton nuclear magnetic resonance spectra (¹H NMR) were measured in chloroform-d (CDCl₃) unless otherwise specified and the peaks are reported in parts per million (ppm) downfield from tetramethylsilane (TMS). The coupling patterns are reported as follows: s, singlet; d, doublet; dd, doublet of doublets; t, triplet; q, quartet; m, multiplet; bs, broad singlet; bm, broad multiplet.

### EXAMPLE 178

### 4-(cis-(8a,12a)-3-chloro-6,7,9,10,12,12a-hexahydro-5H-pyrido[4,3-b][1,4]thiazepino[2,3,4-hi]indol-11(8aH)-yl)-1-(4-fluorophenyl)-1-butanone

Cis-(8a,12a)-3-chloro-6,7,8a,9,10,11,12,12a-octahydro-5*H*-pyrido[4,3-*b*][1,4]thiazepino[2,3,4-*hi*]indole (90 mg, 0.32 mmol), 4-chloro-4'-fluorobutyrobenzophenone (161 mg, 0.8 mmol), KI (10 mg) and K₂CO₃ (132, 0. 96 mmol) were suspended in dioxane (0.6 mL). The resulting mixture was heated at reflux for 24 h. After it was cooled to 23°C the reaction mixture was partitioned between H₂O-CHCl₃ (1:1, 40 mL). The layers were separated and the aqueous layer was back-extracted with CHCl₃ (2 x 30 mL). The extracts were combined, dried (MgSO₄) and concentrated *in vacuo*. Purification of this residue by column silica gel chromatography eluting with CHCl₃ (100%), then 50:1 CHCl₃-MeOH provided the title compound as a semi-solid (90 mg, 25%). ¹H NMR (CD₃OD, 300 MHz) δ 8.99 (dd, 2H, J = 8.8, 5.5 Hz), 7.12 (t, 2H, J = 8.4 Hz), 6.72 (s, 2H), 4.03-3.91 (m, 1H), 3.77-3.62 (m, 1H), 3.27-3.00 (m, 1H), 3.09-2.81 (m, 7H), 2.78-2.69 (m, 3H), 2.42-2.32 (m, 2H), 2.30-2.19 (m, 1H), 2.16-1.75 (m, 4H) ppm.

### EXAMPLE 179

### 4-(cis-(8a,12a)-3-methyl-6,7,9,10,12,12a-hexahydro-5H-pyrido[4,3-b][1,4]thiazepino[2,3,4-hi]indol-11(8aH)-yl)-1-(4-fluorophenyl)-1-butanone

The cis-(8a,12a)-3-methyl-6,7,8a,9,10,11,12,12a-octahydro-5*H*-pyrido[4,3-*b*][1,4]thiazepino[2,3,4-*hi*]indole (0.033 g, 0.09 mmol) was combined with 4-chloro-4'-fluorobutyrophenone (0.0176 g, 0.09 mmol) of KI (0.0179 g, 0.108 mmol), K₂CO₃ (0.062 g, 0.45 mmol) and 1,2-dioxane (0.7 mL). This mixture was refluxed for 4 days. Water was added and the layers were separated. The aqueous layer was extracted with CHCl₃ (3 x 15 mL) and the combined organics were washed with brine, water and dried (Na₂SO₄) and evaporated. The yellow oil was purified by preparatory silica gel TLC (70% EtOAc/Hexanes) affording the title compound (0.017 g, 45%) as a clear colorless oil. ¹H NMR (CD₃OD, 300 MHz) δ 8.02 (q, 2H, J = 5.5, 3.7 Hz), 7.16 (t, 2H, J = 2.9 Hz), 6.73 (d, 1H, J = 7.7 Hz), 6.53 (d, 1H, J = 8 Hz), 3.94 - 4.05 (m, 1H), 4.6 - 4.78 (m, 1H), 3.18 - 3.24 (m, 1H), 3.05 - 3.16 (m, 3H), 2.97 (t, 2H, J = 7.3 Hz), 2.65 - 2.81 (m, 2H), 2.28 - 2.48 (m, 2H), 2.15 (s, 3H), 2.0 - 2.18 (m, 1H), 1.82 - 2.0 (m, 5H) ppm.

### EXAMPLE 180

### cis-(8a,12a)-11-{3-[(4-fluorophenyl)sulfanyl]propyl}-6,7,8a,9,10,12,12a-octahydro-5H-pyrido[4,3-b][1,4]thiazepino[2,3,4-hi]indole

To a solution of cis-(8a,12a)-6,7,8a,9,10,11,12,12a-octahydro-5*H*-pyrido[4,3-*b*][1,4]thiazepino[2,3,4-*hi*]indole (100 mg, 0.32 mmol) in 1,4-dioxane (2 mL) was added 3-chloro-1-(3-flourophenylthio)propane (65.4 mg, 0.32 mmol), potassium iodide (64 mg, 0.38 mmol), and potassium carbonate ( 133 mg, 0.96 mmol). This mixture was heated at reflux with stirring for 60 hours. At which point 1 equivilant (32.4 mg, 0.32 mmol) of TEA was added and then heated at reflux for another 3 days, followed by thin layer chromatography (9:1 CH₂Cl₂:MeOH). After 132 hours water was added and organic layer was extracted with EtOAc (3 x 50 mL), and the extracts combined and concentrated to yield 170 mg of crude oil. Column chromatagraphy (gradient: 1% and 10% MeOH in CH₂Cl₂) was used to purify cis-(8a,12a)-11-{3-[(4-fluorophenyl)sulfanyl]propyl}-6,7,8a,9,10,12,12a-octahydro-5H-pyrido[4,3-b][1,4]thiazepino[2,3,4-hi]indole (20 mg, 16%). ¹H NMR (CDCl₃, 300 MHz): δ 7.26-7.21 (m, 2H), 7.08-7.00 (m, 1H), 6.94 (dd, 1H, J = 7.7 Hz, J = 7.7 Hz), 6.87-6.81 (m, 2H), 6.5 (t, 1H, J = 7.3 Hz), 3.86-3.76 (m, 1H), 3.59-3.49 (m, 1H), 3.27-3.25 (m, 1H), 3.17-2.91 (m, 4H), 2.75-2.72 (m, 1H), 2.61-2.58 (m, 1H), 2.45-2.39 (m, 2H), 2.31-2.2.2 (m, 1H), 2.18-2.02 (m, 3H), 1.99-1.82 (m, 3H), 1.50 (s-broad, 1H), 1.25 (s, 2H) ppm. Mass Spec (ESI): 415 (base M+H).

### EXAMPLE 181

### 4-(cis-(8a,12a)-6,7,9,10,12,12a-hexahydro-5H-pyrido[4,3-b][1,4]thiazepino[2,3,4-hi]indol-11(8aH)-yl)-1-(4-fluorophenyl)-1-butanol

To 4-(cis-(8a,12a)-6,7,9,10,12,12a-hexahydro-5H-pyrido[4,3-b][1,4]thiazepino[2,3,4-hi]indol-11(8aH)-yl)-1-(4-fluorophenyl)-1-butanone (25 mg, 0.06 mmol) was added methanol (1 mL). The flask was cooled to 0°C in an ice bath. Sodium cyanoborohydride was added (38 mg, 0.35 mmol) slowly portionwise. The reaction mixture was allowed to warm to room temperature over a 1 hour. Acetic acid was added ( 5 drops), then concetrated under reduced pressure to yield a residue. The residue was extracted with dichloromethane (1 x 50 mL), washed with sodium bicarbonate (1 x 25 mL) and brine (1x 25 mL), then dried (sodium sulfate), and concentrated to an oil under reduced pressure. The hydrochloride salt was formed by taking oil up in minimal amount of chloroform, then adding hydogen chloride in ether (1M) until percipitation. The solid was filtered off to give the title compound (21.1 mg, 81%). ¹H NMR (CD₃OD, 300 MHz): δ 7.40-7.37 ( m, 2H), 7.06 (t, 2H, J = 8.7 Hz), 6.95 (d, 2H, J = 8.1 Hz), 6.66 (t, 1H, J = 7.4 Hz), 4.8-4.7 (m, 1H), 3.83 (m, 1H), 3.1-3.53 (m, 3H), 3.45-3.30 (m, 2H), 3.22-3.18 (m, 3H), 3.18 (m, 1 H), 2.91 (m, 1 H), 2.59 (m, 1H), 2.37 (m, 1 H), 2.0- 2.2 (m, 5H) ppm. Mass Spec (ESI): 399 (base M+H).

### EXAMPLE 182

### cis-4-((6b,10a)-1,2,6b,9,10,10a-hexahydro[1,4]oxazino[2,3,4-hi]pyrido[4,3-b]indol-8(7H)-yl)-1-(4-fluorophenyl)-1-butanone

The title compound was prepared from addition of 3-chloro-4'-fluorobutyrophenone to cis-(8a,12a)-6,7,8a,9,10,11,22,12a-octahydro-5*H*-[1,4]oxazepino[2,3,4-*hi*]pyrido[4,3-*b*]indole following General procedure A of Example 197. ¹H NMR (300 MHz, CDCl₃) δ 7.94 - 8.00 (m, 2H), 7.08 - 7.11 (m, 2H), 6.58 - 6.70 (m, 3H), 4.39 - 4.43 (m, 2H), 3.17 - 3.23 (m, 4H), 2.97 - 3.09 (m, 4H), 2.66 - 2.80 (m, 2H), 2.37 - 2.52 (m, 2H), 1.90 - 2.10 (m, 4H). MS - ESI: 381 [MH]⁺

### EXAMPLE 185

### cis-(6b,10a)-8-[4-(4-fluorophenyl)butyl]-6-(trifluoromethyl)-1,2,6b,7,8,9,10,10a-octahydropyrido[4,3-b][1,4]thiazino[2,3,4-hi]indole

1-(4-Fluorophenyl)-4-(6-(trifluoromethyl)-1,2,9,10-tetrahydro[1,4]oxazino[2,3,4-*hi*]pyrido[4,3-*b*]indol-8(7*H*)-yl)-1-butanone (34.5 mg, 0.07 mmol) was dissolved in trifluoroacetic acid (0.5 mL) and cooled to 0°C in an ice bath. Sodium cyanoborohydride (14 mg, 0.22 mmol) was added slowly then stirred at 0°C for 1 hour. 1N aqueous HCl (0.5 mL) was added and reaction heated at reflux for 0.5 hours. 50% Sodium hydroxide was added until pH >11 and extracted with dichloromethane (2x 20 mL), dried (sodium sulfate) and concentrated to give the title compound. ¹H NMR (CD₃OD, 300 MHz): δ 7.18-7.15 (m, 2H), 7.12-6.91 (m, 3H), 6.81 (d, 1H, J = 6.8 Hz), 3.69-3.65 (m, 1H), 3.56-3.28 (m,4H),3.12-3.10 (m, 1H), 2.93-2.87 (m, 1H), 2.71-2.56 (m, 2H), 2.35-2.22 (m, 1H), 2.21-2.01 (m, 1H), 1.95-1.78 (m, 1H), 1.75-1.47 (m, 4H), 1.37-1.13 (m,2H), 0.87-0.71 (m, 1H) ppm.

### EXAMPLE 186

### 4-(trans(8a,12a)-6,7,9,10,12,12a-hexahydro-5H-pyrido[4,3-b][1,4]thiazepino[2,3,4-hi]indol-11(8aH)-yl)-1-(4-fluorophenyl)-1-butanone

4-(Cis-(8a,12a)-6,7,9,10,12,12a-hexahydro-5H-pyrido[4,3-b][1,4]thiazepino[2,3,4-hi]indol-11(8aH)-yl)-1-(4-fluorophenyl)-1-butanone (70 mg, 0.282 mmol), potassium iodide (28.2 mg, 0.17 mmol), potassium carbonate (84.5 mg, 0.61 mmol), and 4-chloro-4'fluorbutyrophenone (57mg, 0.28 mmol) were combined in 1,4-dioxane (4mL) and heated at reflux for 48 hours. The reaction was diluted with water (15 mL) and extracted with diethyl ether (3 x 25 mL), and concentrated to a residue. The residue was purified on a chiralcel OD column (8% 2-propanol in hexanes) to give (1 mg, 0.5%) of each enantiomer of the title compound. ¹H NMR (CD₃OD, 300 MHz): δ 8.07 (t, 2H, J = 7.4 Hz), 7.19 (t, 2H, J = 7.5 Hz), 6.84 (dd, 2H, J = 8 Hz, J = 7.7 Hz), 6.64 (t, 1H, J = 7.7 Hz), 3.70-3.61 (m, 1H), 3.52-3.45 (m, 2H), 3.18-3.01 (m, 3H), 2.90-2.82 (m, 1H), 2.63-2.58 (m, 3H), 2.21-1.96 (m, 7H), 1.73-1.63 (m, 1H), 0.91-0.80 (m, 1H) ppm.

### EXAMPLE 187

### 4-(cis-(8a,12a)-2-methoxy-6,7,9,10,12,12a-hexahydro-5H-pyrido[4,3-b][1,4]thiazepino[2,3,4-hi]indol-11(8aH)-yl)-1-(4-fluorophenyl)-1-butanone

Cis-(8a,12a)-2-methoxy-6,7,8a,9,10,11,12,12a-octahydro-5*H*-pyrido[4,3-*b*][1,4]thiazepino[2,3,4-*hi*]indole (43 mg, 0.16 mmol) was dissolved in 1.2 mL of MEK. KI (27 mg, 0.16 mmol) and K₂CO₃ (66 mg, 0.48 mmol), and 2a (112 mg, 0.56 mmol) were added. The suspension was refluxed for 48 hrs and then cooled to rt. The suspension was filtered and the residue was washed with CH₂Cl₂ (5ml). The solution was concentrated in vacuo. The residue was pruified by column chromatography (10% MeOH-CH₂Cl₂) to afford the title compound (67mg, 95%) as a white amorphous solid. ¹H NMR (CDCl₃, 300 MHz) δ 7.95-8.02 (m, 2 H), 7.08-7.12 (m, 2H), 6.42 (dd, 2H, 2.2Hz, 8.8 Hz), 3.60-3.80 (m, 5H), 3.40-3.58 (m, 2H), 3.15-3.25 (m, 1H), 2.90-3.10 (m, 4H), 2.70-2.88 (m, 2H), 2.50-2.68 (m, 1H), 2.39 (dt, 2H, 3.7 Hz, 7.4 Hz), 2.24 (dt, 1H, 4.1 Hz, 11.0 Hz), 1.70-2.10 (m, 5H) ppm. MS (ESI): 441.1 (M+H).

### EXAMPLE 188

### cis-4-((6b,10a)-1,2,6b,9,10,10a-hexahydropyrido[4,3-b][1,4]thiazino[2,3,4-hi]indol-8(7H)-yl)-1-(4-fluorophenyl)-1-butanone

The title compound (55.9mg, 50%) was prepared by the method of Example 187 from cis-(6b,10a)-1,2,6b,7,8,9,10,10a-octahydropyrido[4,3-*b*][1,4]thiazino[2,3,4-*hi*]indole (99mg, 0.43 mmol), 4-chloro-4'-fluorobutyrophenone (112 mg, 0.56 mmol), KI (71 mg, 0.43 mmol), and K₂CO₃ (177 mg, 1.28 mmol) after chromatographic purification as a white amorphous solid. The enantiomers of the title compound were separated on a Chiracel OD column using isocratic 6% IPA/hexane as the eluent. ¹H NMR (CDCl₃, 300 MHz) δ 7.26-8.01 (m, 2 H), 7.12 (t, 2H, 8.4 Hz), 6.81 (t, 2H, 7.7 Hz), 6.19 (t, 1H, 7.6 Hz), 3.38-3.62 (m, 2H), 3.25-3.37 (m, 1H), 2.85-3.20 (m, 5H), 2.70-2.85 (m, 1H), 2.50-2.70 (m, 1H), 2.45-2.68 (m, 2H), 2.20 (dt, 1H, 3.0 Hz, 11.4 Hz), 1.70-2.10 (m, 5H) ppm. MS (ESI): 397.2 (base, M+H).

### EXAMPLE 192

### 4-(cis-(8a,12a)-6,7,9,10,12,12a-hexahydro-5H-pyrido[4,3-b][1,4]thiazepino[2,3,4-hi]indol-11(8aH)-yl)-1-(4-bromophenyl)-1-butanone

The title compound (932 mg, 81%) was prepared by the method of Example 187 from cis-(8a,12a)-6,7,8a,9,10,11,12,12a-octahydro-5*H*-pyrido[4,3-*b*][1,4]thiazepino[2,3,4-*hi*]indole (594.00 mg, 2.44 mmol), 4-chloro-4'-fluorobutyrophenone (831.00 mg, 3.18 mmol), KI (406.00 mg, 2.44 mmol), and K₂CO₃ (638.00 mg, 7.33 mmol) after chromatographic purification as a white amorphous solid. ¹H NMR (CDCl₃, 300 MHz) δ 7.87-7.92 (m, 2 H), 7.64-7.68 (m, 2H), 6.94-6.99 (m, 2H), 6.67 (t, 1H, 7.4 Hz), 3.70-3.90 (m, 2H), 3.41-3.68 (m, 4H), 2.30-3.40 (m, 1H), 3.00-3.29 (m, 5H), 2.80-2.98 (m, 1H), 2.61-2.68 (t, 1H, 11.7 Hz), 1.90-2.50 (m, 6H) ppm. MS (CI, NH3): 473 (base, M+H).

### EXAMPLE 193

### (8aS,12aR)-11-{3-[(4-fluorophenyl)sulfonyl]propyl}-6,7,8a,9,10,12,12a-octahydro-5H-pyrido[4,3-b][1,4]thiazepino[2,3,4-hi]indole

The title compound (188.00 mg, 86%) was prepared by the method of Example 187 from (8a*S*,12a*R*)-6,7,8a,9,10,11,12,12a-octahydro-5*H*-pyrido[4,3-*b*][1,4]thiazepino[2,3,4-*hi*]indole (139.00 mg, 0.49 mmol), 3-(3-fluorophenylsulfonyl)propyl chloride (116.00 mg, 0.49 mmol), KI (48.00 mg, 0.29 mmol), and K₂CO₃ (135.00 mg, 0.98 mmol) after chromatographic purification as a white amorphous solid. ¹H NMR (CDCl₃, 300 MHz) δ 7.71 (bd, 1 H, 6.6 Hz), 7.54-7.65 (m, 2H), 7.36-7.40 (m, 1H), 6.93 (dd, 1H, 1.1 Hz, 7.7 Hz), 6.62 (m, 1H), 3.73-3.76 (m, 1H), 3.45-3.52 (m, 1H), 3.18-3.30 (m, 3H), 2.90-3.18 (m, 3H), 2.57-2.62 (m, 1H), 2.41-2.55 (m, 1H), 2.17-2.41 (m, 3H), 1.95-2.17 (m, 2H), 1.65-1.94 (m, 5H) ppm. MS (ESI): 447.2 (base, M+H).

### EXAMPLE 194

### 4-(cis-(8a,12a)-6,7,9,10,12,12a-hexahydro-5H-pyrido[4,3-b][1,4]thiazepino[2,3,4-hi]indol-11(8aH)-yl)-1-(3',4'-dichloro[1,1'biphenyl]-4-yl)-1-butanone

4-(Cis-(8a,12a)-6,7,9,10,12,12a-hexahydro-5H-pyrido[4,3-b][1,4]thiazepino[2,3,4-hi]indol-11(8aH)-yl)-1-(4-bromophenyl)-1-butanone (123.9 mg, 0.26 mmol) was dissloved in DME (4 mL). 2M aqueous sodium carbonate (0.75 mL) was added. The 3,4-dichlorophenylboronic acid (100.4 mg, 0.53 mmol) was added, followed by Pd₂(dba)₃ (13.5 mg, .013 mmol). PPh₃ (13.8 mg, 0.053 mmol) was added. The reaction flask was degassed and kept under a nitrogen atmosphere. The suspension was refluxed for 18 hrs cooled to rt. The reaction was concentrated in vacuo, after which water (10 mL) and EtOAc (10 mL) were added. The layers were separated and the aqueous phase was extraced with EtOAc (2 x 10 mL). The combined organic layers were washed with brine (2 x 10 mL), dried, and concentrated to afford a crude brown amorphous solid (187 mg). The residue was pruified by column chromatography (20-40% EtOAc/Hexane) to afford the title compound (140.0 mg, 100%) as a white amorphous solid. ¹H NMR (CDCl₃, 300 MHz) δ 8.03-8.06 (m, 1 H), 7.43-7.71 (m, 6H), 6.93 (dd, 1H, 1.1 Hz), 6.84 (bd, 1H, 6.6 Hz), 6.58-6.63 (m, 1H), 3.70-3.90 (m, 1H), 3.50-3.60 (m, 1H), 3.15-3.30 (m, 1H), 2.90-3.18 (m, 4H), 2.50-2.80 (m, 2H), 2.20-2.50 (m, 3H), 1.50-2.20 (m, 8H) ppm. MS (ESI): 537.2 (base, M+H).

### EXAMPLE 197

### 4-((8aS,12aR)-6,7,9,10,12,12a-hexahydro-5H-pyrido[4,3-b][1,4]thiazepino[2,3,4-hi]indol-22(8aH)-yl)-1-(4-methylphenyl)-1-butanone

### General Procedure A:

To a suspension of (8a*S*,12a*R*)-6,7,8a,9,10,11,12,12a-octahydro-5*H*-pyrido[4,3-*b*][1,4]thiazepino[2,3,4-*hi*]indole ( 0.5 mmol) in 1,4-dioxane (3 mL) was added the corresponding chlorobutyrophenone (0.5 - 1.0 mmol), potassium iodine (100 mg) and potassium carbonate (300 mg). The reaction mixture was heated at reflux for 2 days. The solvent was removed under reduced pressure. The residue was treated with water (50 mL) and extracted with diethyl ether ( 3 x 50 mL). The ether extract was washed with brine (150 mL), dried over MgSO₄, filtered and concentrated to a residue. The residue was purified by flash column chromatography (Silica gel, CH₂Cl₂:CH₃OH 9:1). The product was dissolved in ether (2 mL) and stirred at 0°C for 10 minutes, added 1N HCl in ether (0.5 mL) at 0°C. The white crystalline solid was collected by filtration to give the title compound in 50 - 90% yield.

### General procedure B:

To a suspension of (8a*S*,12a*R*)-6,7,8a,9,10,11,12,12a-octahydro-5*H*-pyrido[4,3-*b*][1,4]thiazepino[2,3,4-*hi*]indole ( 0.5 mmol) in 1,4-dioxane (3 mL) was added the corresponding alkyl halide (0.5 - 1.0 mmol), potassium iodine (100 mg) and triethylamine (1.5 mmol). The reaction mixture was heated at reflux for 2 days. The solvent was removed under reduced pressure. The residue was treated with water (50 mL) and extracted with diethyl ether ( 3 x 50 mL). The ether extract was washed with brine (150 mL), dried over MgSO₄, filtered and concentrated to a residue. The residue was purified by flash column chromatography (Silica gel, CH₂Cl₂:CH₃OH 9:1). The product was dissolved in ether (2 mL) and stirred at 0°C for 10 minutes, added 1N HCl in ether (0.5 mL) at 0°C. The white crystalline solid was collected by filtration to give the title compound in 50 - 90% yield.

The title compound was prepared from addition of 4-chloro-4'-methylbutyrophenone to (8a*S*,12a*R*)-6,7,8a,9,10,11,12,12a-octahydro-5*H*-pyrido[4,3-*b*][1,4]thiazepino[2,3,4-*hi*]indole following General procedure A above. ¹H NMR (300 MHz, CDCl₃) δ 7.86 (d, J = 8.0 Hz, 2H), 7.25 (d, J = 8.0 Hz, 2H), 6.94 (d, J = 7.7 Hz, 1H), 6.84 (d, J = 7.3 Hz, 1H), 6.61 (dd, J = 7.7 Hz, 7.3 Hz, 1H), 3.72 - 3.86 (m, 2H), 3.44 - 3.59 (m, 2H), 3.22 - 3.27 (m, 1H), 2.98 - 3.14 (m, 7H), 2.41 (s, 3H), 2.68 - 2.84 (m, 2H), 1.89 - 2.16 (m, 6H) ppm. MS - ESI: 407 [MH]⁺

### EXAMPLE 198

### 4-((8aS,12aR)1-6,7,9,10,12,12a-hexahydro-5H-pyrido[4,3-b][1,4]thiazepino[2,3,4-hi]indol-11(8aH)-yl)-1-(4-fluorophenyl)-1-butanone

The title compound was prepared from addition of 4-chloro-4'-fluorobutyrophenone to (8a*S*,12a*R*)-6,7,8a,9,10,11,12,12a-octahydro-5*H*-pyrido[4,3-*b*][1,4]thiazepino[2,3,4-*hi*]indole following General procedure A of Example 197. ¹H NMR (300 MHz, CDCl₃) δ 7.97 - 8.02 (m, 2H), 7.10 - 7.16 (m, 2H), 6.95 (d, J = 8.0 Hz, 1H), 6.85 (d, J = 7.3 Hz, 1H), 6.62 (dd, J = 7.2 Hz, 7.3 Hz, 1H), 3.76 - 3.86 (m, 1H), 3.44 - 3.59 (m, 2H), 3.24 - 3.30 (m, 1H), 2.90 - 3.14 (m, 4H), 2.68 - 2.84 (m, 4H), 2.24 - 2.58 (m, 4H), 1.99 - 2.11(m, 4H) ppm. MS - ESI: 411 [MH]⁺

### EXAMPLE 199

### 4-((8aS,12aR)1-6,7,9,10,12,12a-hexahydro-5H-pyrido[4,3-b][1,4]thiazepino[2,3,4-hi]indol-11(8aH)-yl)-1-(4-methoxyphenyl)-1-butanone

The title compound was prepared from addition of 4-chloro-4'-methoxybutyrophenone to (8a*S*,12a*R*)-6,7,8a,9,10,11,12,12a-octahydro-5*H*-pyrido[4,3-*b*][1,4]thiazepino[2,3,4-*hi*]indole following General procedure A of Example 197. ¹H NMR (300 MHz, CDCl₃) δ 7.93 - 7.99 (m, 2H), 6.88 - 6.98 (m, 3H), 6.84 (d, J = 7.0 Hz, 1H), 6.61 (dd, J = 8.0 Hz, 7.3 Hz, 1H), 3.87 (s, 3H), 3.70 - 3.90 (m, 2H), 3.48 - 3.58 (m, 1H), 3.22-3.27 (m, 1H), 2.90 - 2.99 (m, 4H), 2.62 - 2.80 (m, 4H), 2.27 - 2.42(m, 4H), 1.90 - 2.13 (m, 4H) ppm. MS - ESI: 423 [MH]⁺

### EXAMPLE 200

### 3-((8aS,12aR)-6,7,9,10,12,12a-hexahydro-5H-pyrido[4,3-b][1,4]thiazepino[2,3,4-hi]indol-11(8aH)-yl)-1-(4-fluorophenyl)-1-propanone

The title compound was prepared from addition of 3-chloro-4'-fluoropropiophenone to (8a*S*,12a*R*)-6,7,8a,9,10,11,12,12a-octahydro-5*H*-pyrido[4,3-*b*][1,4]thiazepino[2,3,4-*hi*]indole following General procedure A of Example 197. ¹H NMR (300 MHz, CDCl₃) δ 7.98 - 8.03 (m, 2H), 7.12 - 7.18 (m, 2H), 6.88 (d, J = 7.6 Hz, 1H), 6.87 (d, J = 6.2 Hz, 1H), 6.65 (dd, J = 7.7 Hz, 7.3 Hz, 1H), 3.79 - 3.88 (m, 1H), 3.7 (s, 2H), 3.50 - 3.60 (m, 1H), 3.25 - 3.38 (m, 3H), 2.89 - 3.01 (m, 7H), 1.90 - 2.15 (m, 4H). MS - ESI: 397 [MH]⁺

### EXAMPLE 201

### (8aS,12aR)-11-{3-[(4-fluorophenyl)sulfonyl]propyl}-6,7,8a,9,10,11,12,12a-octahydro-5H-pyrido[4,3-b][1,4]thiazepino[2,3,4-hi]indole

The title compound was prepared from addition of 3-chloro-1-[(4-fluorophenyl)sulfonyl)propane to (8a*S*,12a*R*)-6,7,8a,9,10,11,12,12a-octahydro-5*H*-pyrido[4,3-*b*][1,4]thiazepino[2,3,4-*hi*]indole following General procedure A of Example 197. ¹H NMR (300 MHz, CDCl₃) δ 7.90 - 7.96 (m, 2H), 7.22 - 7.28 (m, 2H), 9.38 (d, J = 7.7 Hz, 1H), 6.82 (d, J = 6.6 Hz, 1H), 6.61 (dd, J = 7.7 Hz, 7.3 Hz, 1H), 3.72 - 3.81 (m, 1H), 3.45 - 3.55 (m, 1H), 3.15 - 3.29 (m, 4H), 3.02 - 3.12 (m, 2H), 2.92 - 2.99 (m, 1H), 2.57 - 2.62 (m, 1H), 2.46 - 2.55 (m, 1H), 2.30 - 2.37 (m, 2H), 2.18 - 2.27 (m, 1H), 1.94 - 2.09 (m, 2H), 1.78 - 1.92 (m, 4H) ppm. MS (CI, NH₃) *m*/*e* 446 (base, M+H⁺).

### EXAMPLE 202

### (8aS,12aR)-11-{3-[(4-fluorophenyl)sulfinyl]propyl}-6,7,8a,9,10,11,12,12a-octahydro-5H-pyrido[4,3-b][1,4]thiazepino[2,3,4-hi]indole

The title compound was prepared from addition of 3-chloro-1-[(4-fluorophenyl)sulfinyl]propane to (8a*S*,12a*R*)-6,7,8a,9,10,11,12,12a-octahydro-5*H*-pyrido[4,3-*b*][1,4]thiazepino[2,3,4-*hi*]indole following General procedure A of Example 197. ¹H NMR (300 MHz, CD₃OD) δ 7.65 - 7.80 (m, 2H), 7.27 - 7.31 (m, 2H), 6.95 (d, J = 8.1 Hz, 2H), 6.63 (dd, J = 8.1 Hz, 7.7 Hz, 1H), 3.63 - 3.93 (m, 1H), 3.38 - 3.62 (m, 4H), 3.10 - 3.25 (m, 4H), 3.26 -3.36 (m, 2H), 2.92 - 3.09 (m, 3H), 2..50 - 2.62 (m, 1H), 2.30 - 2.42 (m, 1H), 1.94 - 2.28 (m, 4H) ppm. MS (CI, NH₃) *m*/*e* 430 (base, 287).

### EXAMPLE 203

### (8aS,12aR)-11-[3-(4-fluorophenoxy)propyl]-6,7,8a,9,10,12,12a-octahydro-5H-pyrido[4,3-b][1,4]thiazepino[2,3,4-hi]indole

The title compound was prepared from addition of 3-chloro-1-(4-fluorophenoxy)propane (8a*S*,12a*R*)-6,7,8a,9,10,11,12,12a-octahydro-5*H*-pyrido[4,3-*b*][1,4]thiazepino[2,3,4-*hi*]indole following General procedure A of Example 197. ¹H NMR (300 MHz, CDCl₃) δ 6.91-7.00 (m, 3H), 6.79-6.87 (m, 3H), 6.62 (dd, J = 7.7 Hz, 7.3 Hz, 1H), 3.97 (t, J = 6.2, 2H), 3.70 - 3.87 (m, 1H), 3.50-3.60 (m, 1H), 3.18 - 3.31 (m, 2H), 2.90 - 3.12 (m, 2H), 2.70 - 2.80 (m, 2H), 2.40-2.62 (m, 2H), 2.22 - 2.38 (m, 1H), 1.90 - 2.11 (m, 7H) ppm. MS - ESI: 399 [MH]⁺

### EXAMPLE 204

### (8aS,12aR)-11-(3-phenoxypropyl)-6,7,8a,9,10,12,12a-octahydro-5H-pyrido[4,3-b][1,4]thiazepino[2,3,4-hi]indole

The title compound was prepared from addition of 3-chloro-1-phenoxypropane (8a*S*,12a*R*)-6,7,8a,9,10,11,12,12a-octahydro-5*H*-pyrido[4,3-*b*][1,4]thiazepino[2,3,4-*hi*]indole following General procedure B of Example 197. ¹H NMR (300 MHz, CDCl₃) δ 7.25 - 7.30 (m, 2H), 6.85 - 6.97 (m, 5H), 6.62 (dd, J = 7.7 Hz, 7.3 Hz, 1H), 4.02 (t, J = 6.2 Hz, 2H), 3.78 - 3.88 (m, 1H), 3.50 - 3.60 (m, 1H), 3.17 - 3.31 (m, 2H), 2.90 - 3.10 (m, 2H), 2.72 - 2.86 (m, 2H), 2.51 - 2.58 (m, 2H), 2.30 - 2.37 (m, 1H), 1.92-2.15 (m, 7H) ppm. MS (CI, NH₃) *m*/*e* 380 (base, M+H⁺).

### EXAMPLE 205

### (8aS,12aR)-11-[3-[(4-fluorophenyl)sulfanyl]propyl]-6,7,8a,9,10,12,12a-octahydro-5H-pyrido[4,3-b][1,4]thiazepino[2,3,4-hi]indole

The title compound was prepared from addition of 3-chloro-1-(4-fluorophenylthio)propane to (8a*S*,12a*R*)-6,7,8a,9,10,11,12,12a-octahydro-5*H*-pyrido[4,3-*b*][1,4]thiazepino[2,3,4-*hi*]indole following General procedure B of Example 197. ¹H NMR (300 MHz, CDCl₃) δ 7.31 - 7.36 ( m, 2H), 6.93 - 7.02 (m, 4H), 6.84 (d, J = 7.3 Hz, 1H), 6.2 (dd, J = 7.3 Hz, 7.3 Hz, 1H), 3.76 - 3.84 (m, 1H), 3.48 - 3.59 (m, 1H), 3.24 - 3.28 (m, 2H), 2.88 - 3.17 (m, 6H), 2.60 - 2.74 (m, 2H), 2.25 - 2.45 (m, 2H), 2.00 - 2.11 (m, 2H), 1.77 - 1.93 (m, 4H) ppm. MS (CI, NH₃) *m*/*e* 414 (base, M+H⁺).

### EXAMPLE 206

### N-[3-((8aS,12aR)-6,7,9,10,12,12a-hexahydro-5H-pyrido[4,3-b][1,4]thiazepino[2,3,4-hi]indol-11(8aH)-yl)propyl]-4-fluoroaniline

The title compound was prepared from addition of 3-chloropropyl-4-fluorophenylamine to (8a*S*,12a*R*)-6,7,8a,9,10,11,12,12a-octahydro-5*H*-pyrido[4,3-*b*][1,4]thiazepino[2,3,4-*hi*]indole following General procedure B of Example 197. ¹H NMR (300 MHz, CDCl₃) δ 6.97 ( d, J = 8.1 Hz, 1H), 6.93 - 7.02 (m, 3H), 6.64 (dd, J = 7.7 Hz, 7.3 Hz, 1H), 6.47 - 6.52 (m, 2H), 3.75 - 3.85 (m, 1H), 3.46 - 3.56 (m, 1H), 3.25 - 3.35 (m, 2H), 2.91- 3.20 (m, 6H), 2.60 - 2.74 (m, 2H), 1.91 - 2.17 (m, 8H) ppm. MS (CI, NH₃) *m*/*e* 397 (base, M+H⁺).

### EXAMPLE 207

### N-[3-((8aS,12aR)-6,7,9,10,12,12a-hexahydro-5H-pyrido[4,3-b] [1,4]thiazepino[2,3,4-hi]indol-11(8aH)-yl)propyl]-4-fluoro-N-methylaniline

The title compound was prepared from addition of 3-chloropropyl-4-fluorophenylmethylamine to (8a*S*,12a*R*)-6,7,8a,9,10,11,12,12a-octahydro-5*H*-pyrido[4,3-*b*][1,4]thiazepino[2,3,4-*hi*]indole following General procedure B of Example 197. ¹H NMR (300 MHz, CDCl₃) δ 6.84 - 6.99 (m, 4H), 6.59 - 6.67 (m, 3H), 3.77 - 3.90 (m, 1H), 3.47 - 3.59 (m, 1H), 3.19 - 3.33 (m, 4H), 2.67 - 3.09 (m, 4H), 2.87 (s, 3H), 2.33 - 2.37 (m, 3H), 1.76 - 2.17 (m, 7H) ppm. MS (CI, NH₃) *m*/*e* 411 (base, M+H⁺).

### EXAMPLE 208

### 4-((8aS,12aR)-6,7,9,10,12,12a-hexahydro-5H-pyrido[4,3-b][1,4]thiazepino[2,3,4-hi]indol-11(8aH)-yl)-1-(4-pyridinyl)-1-butanone

The title compound was prepared from addition of 4-chloro-1-(4-pyridyl)butan-1-one to (8a*S*,12a*R*)-6,7,8a,9,10,11,12,12a-octahydro-5*H*-pyrido[4,3-b] [1,4]thiazepino[2,3,4-*hi*]indole following General procedure A of Example 197. ¹H NMR (300 MHz, CDCl₃) δ 8.79 (dd, J = 5.9 Hz, 1.5 Hz, 2H), 7.73 (dd, J = 6.2 Hz, 1.8 Hz, 2H), 6.93 (d, J = 7.7 Hz, 1H), 6.82 (d, J = 7.4 Hz, 1H), 6.59 (dd, J = 7.7 Hz, 7.4 Hz, 1H), 3.64 - 3.82 (m, 4H), 3.46 - 3.56 (m, 2H), 3.19 - 3.24 (m, 2H), 2.88 - 3.06 (m, 4H), 2.60 - 2.75 (m, 2H), 2.28 - 2.42 (m, 2H), 1.87 - 2.09 (m, 4H) ppm. MS (CI, NH₃) *m*/*e* 393 (base, M+H⁺).

### EXAMPLE 209

### 4-((8aS,12aR)-6,7,9,10,12,12a-hexahydro-5H-pyrido[4,3-b][1,4]thiazepino[2,3,4-hi]indol-11(8aH)-yl)-1-(3-pyridinyl)-1-butanone

The title compound was prepared from addition of 4-chloro-1-(3-pyridyl)butan-1-one to (8a*S*,12a*R*)-6,7,8a,9,10,11,12,12a-octahydro-5*H*-pyrido[4,3-*b*][1,4]thiazepino[2,3,4-*hi*]indole following General procedure A of Example 197. ¹H NMR (300 MHz, CDCl₃) δ 9.18 (d, J = 2.2 Hz, 1H), 8.76 (dd, J = 4.7 Hz, 1.8 Hz, 1H), 8.23 (dt, J = 8.1 Hz, 1.8 Hz, 1H), 7.40 (dd, J = 8.1 Hz, 4.8 Hz, 1H), 6.91 - 6.95 (m, 1H), 6.82 - 6.87 (m, 1H), 6.57 - 6.63 ( m, 1H), 3.49 - 3.83 (m, 4H), 3.06 - 3.25 (m, 2H), 3.01 (t, J = 7.0 Hz, 2H), 2.52 - 2.94 (m, 4H), 2.26 - 2.39 (m, 2H), 1.83 - 2.10 (m, 6H) ppm. MS (CI, NH₃) *m*/*e* 393 (base, M+H⁺).

### EXAMPLE 213

### cis-4-((6b,10a)-5-methyl-1,2,6b,9,10,10a-hexahydropyrido[4,3-b][1,4]thiazino[2,3,4-hi]indol-8(7H)-yl)-1-(4-fluorophenyl)-1-butanone

The title compound was prepared from addition of the 4-chloro-4'-fluorobutyrophenone to cis-(6b,10a)-5-methyl-1,2,6b,7,8,9,10,10a-octahydropyrido[4,3-*b*][1,4]thiazino[2,3,4-*hi*]indole following General procedure A of Example 197. ¹H NMR (300 MHz, CDCl₃) δ 7.90 - 8.03 (m, 2H), 6.81 - 7.16 (m, 4H), 3.75 - 3.80 (m, 1H), 3.39 - 3.52 (m, 2H), 3.18 - 3.24 (m, 2H), 3.06 - 3.13 (m, 2H), 2.84 - 2.94 (m, 1H), 1.92 - 2.52 (m, 10H), 2.24 (s, 3H) ppm. MS - ESI: 411 [MH]⁺

### EXAMPLE 214

### (8aS,12aR)-11-[3-(6-fluoro-1,2-benzisoxazol-3-yl)propyl]-6,7,8a,9,10,12,12a-octahydro-5H-pyrido[4,3-b][1,4]thiazepino[2,3,4-hi]indole

The title compound was prepared from addition of 3-(3-chloropropyl)-6-fluorobenzo[d]isoxazole to (8a*S*,12a*R*)-6,7,8a,9,10,11,12,12a-octahydro-5*H*-pyrido[4,3-*b*][1,4]thiazepino[2,3,4-*hi*]indole benzothiazepine following General procedure A of Example 197. ¹H NMR (300 MHz, CDCl₃) δ 7.66 (dd, J = 8.4 Hz, 5.1 Hz, 1H), 7.23 (dd, J = 8.5 Hz, 1.8 Hz, 1H), 7.06 (ddd, J = 8.7 Hz, 8.8 Hz, 2.2 Hz, 1H), 6.93 (dd, J = 7.7 Hz, 0.9 Hz, 1H), 6.84 (d, J = 6.6 Hz, 1H), 6.61 (dd, J = 7.7 Hz, 7.3 Hz, 1H), 3.70 - 3.83 (m, 1H), 3.48 - 3.56 (m, 1H), 3.23 - 3.27 (m, 1H), 2.91 - 3.12 (m, 5H), 2.71 - 2.77 (m, 1H), 2.61 - 2.65 (m, 1H), 2.39 - 2.46.(m, 2H) , 2.24 - 2.28 (m, 1H), 1.90 - 2.11 (m, 4H), 1.84 - 1.88 (m, 3H) ppm. MS - ESI: 424 [MH]⁺

### EXAMPLE 215

### (8aS,12aR)-11-[3-(1,2-benzisoxazol-3-yl)propyl]-6,7,8a,9,10,12,12a-octahydro-5H-pyrido[4,3-b][1,4]thiazepino[2,3,4-hi]indole

The title compound was prepared from addition 3-(3-chloropropyl)benzo[d]isoxazole to (8a*S*,12a*R*)-6,7,8a,9,10,11,12,12a-octahydro-5*H*-pyrido[4,3-*b*][1,4]thiazepino[2,3,4-*hi*]indole following General procedure A of Example 197. ¹H NMR (300 MHz, CDCl₃) δ 7.67 (dd, J = 7.7 Hz, 1.1 Hz, 1H), 7.53 - 7.55 (m, 2H), 7.27 - 7.33 (m, 1H), 6.94 (dd, J = 7.7 Hz, 1.1 Hz, 1H), 6.84 (d, J = 6.6 Hz, 1H), 6.61 (dd, J = 7.6 Hz, 7.4 Hz, 1H), 3.76 - 3.84 (m, 1H), 3.48 - 3.58 (m, 1H), 3.23 - 3.27 (m, 1H), 2.91 - 3.17 (m, 5H), 2.67 - 2.82 (m, 2H), 2.45 - 2.51 (m, 2H), 2.24 - 2.38 (m, 1H), 1.89 - 2.14 (m, 7H) ppm. MS (CI, NH₃) *m*/*e* 405 (base, M+H⁺).

### EXAMPLE 219

### ethyl 4-((8aS,12aR)-6,7,9,10,12,12a-hexahydro-5H-pyrido[4,3-b][1,4]thiazepino[2,3,4-hi]indol-11(8aH)-yl)butanoate

### General procedure:

To a suspension of to (8a*S*,12a*R*)-6,7,8a,9,10,11,12,12a-octahydro-5*H*-pyrido[4,3-*b*][1,4]thiazepino[2,3,4-*hi*]indole (3 mmol) in 1,4-dioxane (18 mL) was added the corresponding alkyl halide (3.3 mmol), potassium iodine (100 mg) and potassium carbonate (900 mg), the reaction mixture was heated at reflux for 2 days. The solvent was removed under reduced pressure. The residue was treated with water (50 mL) and extracted with diethyl ether ( 3 x 50 mL). The ether extract was washed with brine (150 mL), dried over MgSO₄, filtered and concentrated to a residue. The residue was purified by flash column chromatography (Silica gel, CH₂Cl₂:CH₃OH 9:1) to give the title compound in 47% - 64% yields.

The title compound was prepared from addition of ethyl 4-chlorobutanoate to (8a*S*,12a*R*)-6,7,8a,9,10,11,12,12a-octahydro-5*H*-pyrido[4,3-*b*][1,4]thiazepino[2,3,4-*hi*]indole following the General procedure above. ¹H NMR (300 MHz, CDCl₃) δ 6.94 (bd, J = 7.7 Hz, 1H), 6.86 (bd, J = 6.9 Hz, 1H), 6.62 (dd, J = 7.4 Hz, 7.3 Hz, 1H), 4.08 - 4.15 (m, 2H), 3.77 - 3.86 (m, 1H), 3.47 -3.59 (m, 2H), 3.10 - 3.29 (m, 2H), 2.89 - 3.08 (m, 2H), 2.64 - 2.82 (m, 2H), 2.31 - 2.44 (m, 4H), 1.83 - 2.12 (m, 7H), 1.23 - 1.27 (m, 3H) ppm. MS - ESI: 361 [MH]⁺

### EXAMPLE 220

### ethyl 5-((8aS,12aR)-6,7,9,10,12,12a-hexahydro-5H-pyrido[4,3-b][1,4]thiazepino[2,3,4-hi]indol-11(8aH)-yl)pentanoate

The title compound was prepared from addition of ethyl 5-chloropentanoate to to (8a*S*,12a*R*)-6,7,8a,9,10,11,12,12a-octahydro-5*H*-pyrido[4,3-*b*][1,4]thiazepino[2,3,4-*hi*]indole following the General procedure of Example 219. ¹H NMR (300 MHz, CDCl₃) δ 6.95 (bd, J = 7.7 Hz, 1H), 6.86 (bd, J = 7.4 Hz, 1H), 6.62 (dd, J = 7.7 Hz, 7.3 Hz, 1H), 4.08 - 4.15 (m, 2H), 3.77 - 3.87 (m, 1H), 3.47 - 3.59 (m, 1H), 3.21 - 3.28 (m, 2H), 2.89 - 3.08 (m, 2H), 2.64 - 2.84 (m, 2H), 2.29 -2.34 (m, 5H), 1.90 - 2.16 (m, 5H), 1.55 - 1.64 (m, 4H), 1.22 - 1.27 (m, 3H) ppm. MS - ESI: 375 [MH]⁺

### EXAMPLE 221

### 4-((8aS,12aR)-6,7,9,10,12,12a-hexahydro-5H-pyrido[4,3-b][1,4]thiazepino[2,3,4-hi]indol-11(8aH)-yl)-N-methoxy-N-methylbutanamide

### General procedure:

A solution of 2M trimethylaluminium in toluene (6.0 mmol) was added to a stirred mixture of N,O - dimethyl hydroxyamine hydrochloride (2.0 mmol) in dry toluene (20 mL) at 0°C. The resultant mixture was stirred at room temperature for 1 hour and added to a solution of ethyl 4-((8aS,12aR)-6,7,9,10,12,12a-hexahydro-5H-pyrido[4,3-b][1,4]thiazepino[2,3,4-hi]indol-11(8aH)-yl)butanoate or ethyl 5-((8aS,12aR)-6,7,9,10,12,12a-hexahydro-5H-pyrido[4,3-b][1,4]thiazepino[2,3,4-hi]indol-11(8aH)-yl)pentanoate from Example 219 and Example 220 in toluene (1 mL) at 0°C. The mixture was stirred at 0°C for 2 hours, then at room temperature for 3 hours. 1M tartaric acid (27 mL) was added slowly to the reaction at 0°C and stirred at 0°C for 30 minutes. The reaction mixture was extracted with CHCl₃ (3 x 50 mL). The organic layer was washed with brine, dried over Na₂SO₄, filtered and concentrated to a residue. The residue was purified by flash column chromatography (Silica gel, CH₂Cl₂:CH₃OH 9:1) to obtain the title compound in 70% - 90% yields.

The title compound was prepared from the corresponding ester ethyl 4-((8aS,12aR)-6,7,9,10,12,12a-hexahydro-5H-pyrido[4,3-b][1,4]thiazepino[2,3,4-hi]indol-11(8aH)-yl)butanoate following the General procedure above. ¹H NMR (300 MHz, CDCl₃) δ 6.94 (bd, J = 7.7 Hz, 1H), 6.85 (bd, J = 6.6 Hz, 1H), 6.61 (dd, J = 7.6 Hz, 7.4 Hz, 1H), 3.77 - 3.86 (m, 1H), 3.67 (s, 3H), 3.48 - 3.59 ( m, 1H), 3.21 - 3.28 ( m, 2H), 3.17 (s, 3H), 2.89 - 3.08 (m, 2H), 2.71 - 2.84 (m, 2H), 2.29 - 2.52 (m, 5H), 1.84 - 2.16 (m, 7H) ppm. MS - ESI: 376 [MH]⁺

### EXAMPLE 222

### 5-((8aS,12aR)-6,7,9,10,12,12a-hexahydro-5H-pyrido[4,3-b][1,4]thiazepino[2,3,4-hi]indol-11(8aH)-yl)-N-methoxy-N-methylpentanamide

The title compound was prepared from the corresponding ester ethyl 5-((8aS,12aR)-6,7,9,10,12,12a-hexahydro-5H-pyrido[4,3-b][1,4]thiazepino[2,3,4-hi]indol-11(8aH)-yl)pentanoate following the General procedure of Example 221. ¹H NMR (300 MHz, CDCl₃) δ 6.95 (bd, J = 7.7 Hz, 1H), 6.86 (bd, J = 7.4 Hz, 1H), 6.62 (dd, J = 7.7 Hz, 7.4 Hz, 1H), 3.77 - 3.87 (m, 1H), 3.67 (s, 3H), 3.47 - 3.59 ( m, 1H), 3.23 - 3.29 ( m, 2H), 3.16 (s, 3H), 2.77 - 3.06 (m, 4H), 2.33 - 2.44 (m, 5H), 1.91 - 2.13 (m, 5H), 1.61 - 1.65 (m, 4H) ppm. MS (CI, NH₃) *m*/*e* 389 (base, M+H⁺).

### EXAMPLE 223

### 4-((8aS,12aR)-6,7,9,10,12,12a-hexahydro-5H-pyrido[4,3-b][1,4]thiazepino[2,3,4-hi]indol-11(8aH)-yl)-1-(4-fluoro-3-methylphenyl)-1-butanone

### General procedure:

To a solution of 4-((8aS,12aR)-6,7,9,10,12,12a-hexahydro-5H-pyrido[4,3-b][1,4]thiazepino[2,3,4-hi]indol-11(8aH)-yl)-N-methoxy-N-methylbutanamide or 5-((8aS,12aR)-6,7,9,10,12,12a-hexahydro-5H-pyrido[4,3-b][1,4]thiazepino[2,3,4-hi]indol-11(8aH)-yl)-N-methoxy-N-methylpentanamide from Example 221 and Example 222 (0.1 mmol) in THF (2 mL) or diethyl ether (2 mL) at ambient temperature, was added the corresponding aryl magnesium bromide ( 0.5 mmol) in THF (or diethyl ether) dropwise. The resultant mixture was stirred at room temperature for 2 to 5 hours. Added several drops of conc. HCl, extracted with CH₂Cl₂ (15 mL). The organic layer was washed with sat. NaHCO₃ (15 mL), brine (15 mL), dried over Na₂SO₄, filtered and concentrated to a residue. The result residue was purified by preparative TLC (Silica gel; CH₂Cl₂:CH₃OH 9:1) to afford the title compounds in 70% -90% yields.

The title compound was prepared from addition of 4-((8aS,12aR)-6,7,9,10,12,12a-hexahydro-5H-pyrido[4,3-b][1,4]thiazepino[2,3,4-hi]indol-11(8aH)-yl)-N-methoxy-N-methylbutanamide following the General procedure above. ¹H NMR (300 MHz, CD₃OD) δ 7.84 - 7.94 (m, 2H), 7.08 - 7.16 (m, 1H), 6.92 -6.99 (m, 2H), 6.64 - 6.72 (m, 1H), 3.79 - 3.93 (m, 1H), 3.48 - 3.70 (m, 3H), 3.36 - 3.47 ( m, 1H), 2.98 - 3.14 ( m, 7H), 2.84 - 2.94 (m, 1H), 2.58 - 2.68 (m, 1H), 2.31 (bs, 3H), 1.89 - 2.21 (m, 6H) ppm. MS (CI, NH₃) *m*/*e* 424 (base, M+H⁺).

### EXAMPLE 224

### 4-((8aS,12aR)-6,7,9,10,12,12a-hexahydro-5H-pyrido[4,3-b][1,4]thiazepino[2,3,4-hi]indol-11(8aH)-yl)-1-phenyl-1-butanone

The title compound was prepared from addition of phenyl magnesium bromide to 4-((8aS,12aR)-6,7,9,10,12,12a-hexahydro-5H-pyrido[4,3-b][1,4]thiazepino[2,3,4-hi]indol-11(8aH)-yl)-N-methoxy-N-methylbutanamide following the General procedure of Example 223. ¹H NMR (300 MHz, CDCl₃) δ 7.96 (d, J = 7.3 Hz, 2H), 7.55 - 7.97 (m, 1H), 7.44 - 7.49 (m, 2H), 7.18 - 7.33 (m, 3H), 4.72 - 4.82 (m, 1H), 4.25 - 4.50 (m, 2H), 3.90 - 4.06 ( m, 3H), 3.60 - 3.76 ( m, 3H), 2.99 - 3.24 (m, 7H), 2.54 - 2.60 (m, 1H), 2.28 - 2.40 (m, 2H), 2.11 - 2.26 (m, 1H) ppm. MS (CI, NH₃) *m*/*e* 392 (base, M+H⁺).

### EXAMPLE 225

### 4-((8aS,12aR)-6,7,9,10,12,12a-hexahydro-5H-pyrido[4,3-b][1,4]thiazepino[2,3,4-hi]indol-11(8aH)-yl)-1-(4-chlorophenyl)-1-butanone

The title compound was prepared from addition of 4-chlorophenyl magnium bromide to 4-((8aS,12aR)-6,7,9,10,12,12a-hexahydro-5H-pyrido[4,3-b][1,4]thiazepino[2,3,4-hi]indol-11(8aH)-yl)-N-methoxy-N-methylbutanamide following the General procedure of Example 223. ¹H NMR (300 MHz, CD₃OD) δ 7.97 - 8.02 (m, 2H), 7.50 - 7.56 (m, 2H), 6.95 - 7.02 (m, 2H), 6.62 -6.72 (m, 1H), 3.80 - 3.90 (m, 1H), 3.40 - 3.59 (m, 8H), 2.99 - 3.24 (m, 6H), 2.85 - 2.95 (m, 1H), 1.96 - 2.21(m, 4H) ppm. MS (CI, NH₃) *m*/*e* 426 (base, M+H⁺).

### EXAMPLE 226

### 4-((8aS,12aR)-6,7,9,10,12,12a-hexahydro-5H-pyrido[4,3-b][1,4]thiazepino[2,3,4-hi]indol-11(8aH)-yl)-1-(3-methylphenyl)-1-butanone

The title compound was prepared from addition of m-tolyl magnesium chloride to 4-((8aS,12aR)-6,7,9,10,12,12a-hexahydro-5H-pyrido[4,3-b][1,4]thiazepino[2,3,4-hi]indol-11(8aH)-yl)-N-methoxy-N-methylbutanamide following the General procedure of Example 223. ¹H NMR (300 MHz, CD₃OD) δ 7.79 - 7.84 (m, 2H), 7.38 - 7.44 (m, 2H), 6.95 - 7.05 (m, 2H), 6.62 -6.72 (m, 1H), 3.80 - 3.90 (m, 1H), 3.40 - 3.59 (m, 8H), 2.99 - 3.24 (m, 6H), 2.85 - 2.95 (m, 1H), 2.38 (s, 3H), 1.96 - 2.21(m, 4H) ppm. MS (CI, NH₃) *m*/*e* 406 (base, M+H⁺).

### EXAMPLE 227

### 4-((8aS,12aR)-6,7,9,10,12,12a-hexahydro-5H-pyrido[4,3-b][1,4]thiazepino[2,3,4-hi]indol-11(8aH)-yl)-1-(4-tert-butylphenyl)-1-butanone

The title compound was prepared from addition of 4-*tert*-butylphenyl magnesium bromide to 4-((8aS,12aR)-6,7,9,10,12,12a-hexahydro-5H-pyrido[4,3-b][1,4]thiazepino[2,3,4-hi]indol-11(8aH)-yl)-N-methoxy-N-methylbutanamide following the General procedure of Example 223. ¹H NMR (300 MHz, CD₃OD) δ 7.97 - 8.02 (m, 2H), 7.50 - 7.56 (m, 2H), 6.92 - 7.00 (m, 2H), 6.65 -6.75 (m, 1H), 3.80 - 3.90 (m, 1H), 3.40 - 3.59 (m, 4H), 3.26 - 3.38 (m, 4H), 2.99 - 3.24 (m, 6H), 2.85 - 2.95 (m, 1H), 1.96 - 2.21(m, 4H), 1.32 (s, 9H) ppm. MS (CI, NH₃) m/e 448 (base, M+H⁺).

### EXAMPLE 228

### 4-((8aS,12aR)-6,7,9,10,12,12a-hexahydro-5H-pyrido[4,3-b][1,4]thiazepino[2,3,4-hi]indol-11(8aH)-yl)-1-(3,4-difluorophenyl)-1-butanone

The title compound was prepared from addition of 3,4-difluorophenyl magnesium bromide to 4-((8aS,12aR)-6,7,9,10,12,12a-hexahydro-5H-pyrido[4,3-b][1,4]thiazepino[2,3,4-hi]indol-11(8aH)-yl)-N-methoxy-N-methylbutanamide following the General procedure of Example 223. ¹H NMR (300 MHz, CD₃OD) δ 7.88 - 7.94 (m, 2H), 7.38 - 7.44 (m, 1H), 6.92 - 7.00 (m, 2H), 6.65 -6.75 (m, 1H), 3.80 - 3.90 (m, 1H), 3.40 - 3.59 (m, 4H), 3.26 - 3.38 (m, 4H), 2.99 - 3.24 (m, 6H), 2.85 - 2.95 (m, 1H), 1.96 - 2.21(m, 4H) ppm. MS (CI, NH₃) *m*/*e* 428 (base, M+H⁺).

### EXAMPLE 229

### 4-((8aS,12aR)-6,7,9,10,12,12a-hexahydro-5H-pyrido[4,3-b][1,4]thiazepino[2,3,4-hi]indol-11(8aH)-yl)-1-(5-fluoro-2-methoxyphenyl)-1-butanone

The title compound was prepared from addition of 3-fluoro-6-methoxyphenyl magnesium bromide 4-((8aS,12aR)-6,7,9,10,12,12a-hexahydro-5H-pyrido[4,3-b][1,4]thiazepino[2,3,4-hi]indol-11(8aH)-yl)-N-methoxy-N-methylbutanamide following the General procedure of Example 223. ¹H NMR (300 MHz, CD₃OD) δ 7.40 - 7.48 (m, 1H), 7.24 - 7.34 (m, 1H), 7.10 - 7.18 (m, 1H), 6.92 -7.00 (m, 2H), 6.65 - 6.75 (m, 1H), 3.74 - 3.92 (m, 2H), 3.90 (s, 3H), 3.36 - 3.59 (m, 4H), 3.26 - 3.34 (m, 4H), 3.05 - 3.20 (m, 6H), 2.85 - 2.95 (m, 1H), 1.96 - 2.21(m, 4H) ppm. MS (CI, NH₃) *m*/*e* 440 (base, M+H⁺).

### EXAMPLE 230

### 5-(cis-(8a,12a)-6,7,9,10,12,12a-hexahydro-5H-pyrido[4,3-b][1,4]thiazepino[2,3,4-hi]indol-11(8aH)-yl)-1-phenyl-1-pentanone

The title compound was prepared from addition of phenyl magnesium bromide to 5-((8aS,12aR)-6,7,9,10,12,12a-hexahydro-5H-pyrido[4,3-b][1,4]thiazepino[2,3,4-hi]indol-11(8aH)-yl)-N-methoxy-N-methylpentanamide following the General procedure of Example 223. ¹H NMR (300 MHz, CDCl₃) δ 7.84 - 7.94 (m, 2H), 7.50 - 7.58 (m, 1H), 7.44 - 7.50 (m, 2H), 6.92 -7.00 (m, 2H), 6.70 - 6.79 (m, 1H), 3.76 - 3.82 (m, 1H), 3.58 - 3.68 (m, 2H), 3.45 - 3.56 (m, 1H),3.18 - 3.21 ( m, 2H), 2.64 - 2.98 ( m, 7H), 2.30 - 2.35 (m, 1H), 1.80 -1.92 (m, 4H), 1.60 - 1.72 (m, 4H) ppm. MS (CI, NH₃) *m*/*e* 406 (base, M+H⁺).

### EXAMPLE 231

### 4-((8aS,12aR)-6,7,9,10,12,12a-hexahydro-5H-pyrido[4,3-b][1,4]thiazepino[2,3,4-hi]indol-11(8aH)-yl)-1-(4-fluoro-1-naphthyl)-1-butanone

### General procedure:

To a solution of 4-((8aS,12aR)-6,7,9,10,12,12a-hexahydro-5H-pyrido[4,3-b][1,4]thiazepino[2,3,4-hi]indol-11(8aH)-yl)-N-methoxy-N-methylbutanamide from Example 26 (0.1 mmol) in THF (1 mL) at room temperature, was added the corresponding aryl magnesium bromide (0.5 mmol) in THF dropwise. The reaction mixture was stirred at ambient temperature for 18 -20 hours, then heated at 72°C for 1 hour. The reaction was concentrated to a residue. The residue was purified by prep. TLC (Silica gel; CH₂Cl₂:CH₃OH 9:1) to afford the title compounds in 36% - 40% yields.

The title compound was prepared from addition of 4-fluoro-1-naphthyl magnesium bromide to 4-((8aS,12aR)-6,7,9,10,12,12a-hexahydro-5H-pyrido[4,3-b][1,4]thiazepino[2,3,4-hi]indol-11(8aH)-yl)-N-methoxy-N-methylbutanamide following the General procedure above. ¹H NMR (300 MHz, CD₃OD) δ 8.72 - 8.80 (m, 1H), 8.10 - 8.18 (m, 2H), 7.62 - 7.68 (m, 2H), 7.24 -7.30 (m, 1H), 6.92 - 7.00 (m, 2H), 6.64 - 6.70 (m, 1H), 3.82 - 3.92 (m, 1H), 3.52 - 3.64 (m, 2H), 3.24 - 3.44 (m, 7H), 2.90 - 3.14 (m, 2H), 2.68 - 2.84 (m, 2H), 2.30 - 2.44 (m, 2H), 1.99 - 2.11(m, 4H) ppm. MS - ESI: 461 [MH]⁺

### EXAMPLE 232

### 5-((8aS,12aR)-6,7,9,10,12,12a-hexahydro-5H-pyrido[4,3-b][1,4]thiazepino[2,3,4-hi]indol-11(8aH)-yl)-1-phenyl-2-pentanone

The title compound was prepared from addition of benzyl magnesium bromide to 4-((8aS,12aR)-6,7,9,10,12,12a-hexahydro-5H-pyrido[4,3-b][1,4]thiazepino[2,3,4-hi]indol-11(8aH)-yl)-N-methoxy-N-methylbutanamide following the General procedure of Example 231. ¹H NMR (300 MHz, CD₃OD) δ 7.22 - 7.28 (m, 5H), 6.92 - 6.98 (m, 2H), 6.62 - 6.68 (m, 1H), 4.88 (s, 2H), 3.82 - 3.92 (m, 1H), 3.52 - 3.64 (m, 2H), 3.24 - 3.44 (m, 7H), 2.82 - 3.18 (m, 4H), 2.32 - 2.44 (m, 2H), 1.92 - 2.30 (m, 4H) ppm. MS (CI, NH₃) *m*/*e* 406 (base, M+H⁺).

### EXAMPLE 233

### 3-((8aS,12aR)-6,7,9,10,12,12a-hexahydro-5H-pyrido[4,3-b][1,4]thiazepino[2,3,4-hi]indol-11(8aH)-yl)-N-(4-fluorophenyl)propanamide

### General procedure:

To 3-((8a*S*,12a*R*)-6,7,9,10,12,12a-hexahydro-5*H*-pyrido[4,3-*b*][1,4]thiazepino[2,3,4-*hi*]indol-11(8a*H*)-yl)-*N*-methoxy-*N*-methylpropanamide or 4-((8aS,12aR)-6,7,9,10,12,12a-hexahydro-5H-pyrido[4,3-b][1,4]thiazepino[2,3,4-hi]indol-11(8aH)-yl)-N-methoxy-N-methylbutanamide (0.06 - 0.1 mmol) in methanesulfonic acid (0.5 mL), was added NaN₃ (1.5 equiv.). The resultant mixture was stirred at ambient temperature for 1 hour, then was added water (5 mL). Ammonium hydroxide solution was added to adjust the pH to 11. Extracted with CH₂Cl₂(20 mL). The organic layer was dried over MgSO₄, filtered and concentrated to a residue. The residue was purified by prep. TLC (Silica gel; CH₂Cl₂:CH₃OH 9/1). The product was dissolved in ether (1 mL) and stirred at 0°C for 10 minutes, added 1N HCl in ether (0.5 mL) at 0°C. The white crystalline solid was collected by filtration to afford the title compounds in 50% - 52% yields.

The title compound was prepared from 3-((8a*S*,12a*R*)-6,7,9,10,12,12a-hexahydro-5*H*-pyrido[4,3-*b*][1,4]thiazepino[2,3,4-*hi*]indol-11(8a*H*)-yl)-*N*-methoxy-*N*-methylpropanamide following the General procedure above: ¹H NMR (300 MHz, CDCl₃) δ 7.30 - 7.35 (m, 2H), 6.90 - 7.15 (m, 3H), 6.87 (d, J = 7.0 Hz, 1H), 6.59 (dd, J = 7.7 Hz, 7.3 Hz, 1H), 3.62 - 3.72 (m, 1H), 3.22 - 3.48 (m, 5H), 3.04 - 3.12 (m, 1H), 2.55 - 2.86 ( m, 7H), 2.07 - 2.15 ( m, 2H), 1.97 - 2.00 (m, 2H) ppm. MS (CI, NH₃) *m*/*e* 411 (base, M+H⁺).

### EXAMPLE 234

### 4-((8aS,12aR)-6,7,9,10,12,12a-hexahydro-5H-pyrido[4,3-b][1,4]thiazepino[2,3,4-hi]indol-11(8aH)-yl)-N-(4-fluorophenyl)butanamide

The title compound was prepared from 4-((8aS,12aR)-6,7,9,10,12,12a-hexahydro-5H-pyrido[4,3-b][1,4]thiazepino[2,3,4-hi]indol-11(8aH)-yl)-N-methoxy-N-methylbutanamide following the General procedure of Example 233. ¹H NMR (300 MHz, CDCl₃) δ 7.33 - 7.37 (m, 2H), 6.88 - 6.97 (m, 3H), 6.78 (d, J = 7.0 Hz, 1H), 6.54 (dd, J = 7.7 Hz, 7.3 Hz, 1H), 3.64 - 3.74 (m, 1H), 3.37 - 3.48 (m, 1H), 3.21 - 3.26 (m, 1H), 3.00 - 3.17 ( m, 2H), 2.86 - 2.94 ( m, 1H), 2.70 - 2.76 (m, 1H), 2.58 - 2.62 (m, 1H), 2.33 - 2.45 (m, 4H), 1.94 - 2.09 (m, 4H), 1.83 - 1.90 (m, 4H) ppm. MS (CI, NH₃) *m*/*e* 425 (base, M+H⁺).

### EXAMPLE 235

### 4-((8aS,12aR)-6,7,9,10,12,12a-hexahydro-5H-pyrido[4,3-b][1,4]thiazepino[2,3,4-hi]indol-11(8aH)-yl)-1-(4-fluorophenyl)-1-butanol

To 4-((8aS,12aR)1-6,7,9,10,12,12a-hexahydro-5H-pyrido[4,3-b][1,4]thiazepino[2,3,4-hi]indol-11(8aH)-yl)-1-(4-fluorophenyl)-1-butanone or 1-(4-pyridyl)-4-(2,3,6b,7,8,9,10,10a-octahydro-3-methyl-1H-pyrido[3',4':4,5]- pyrrolo[1,2,3-de]quinoxalin-8-yl)-1-butanone (0.06 mmol) in methanol (1 mL) was added sodium borohydride (0.36 mmol) in three portions at 0°C. The reaction mixture was stirred at ambient temperature for 2 hours, followed by addition of two drops of conc. HCl to destroy the excess of NaBH₄. The NH₄OH (1 mL) was added and extracted with CH₂Cl₂ (10 mL). The organic layer was dried over MgSO₄, filtered and concentrated to a residue. The residue was dissolved in ether (1 mL), added 1N HCl in ether. Concentrated to a residue to afford the title compounds in 60% -65% yields.

The title compound was prepared from 4-((8aS,12aR)1-6,7,9,10,12,12a-hexahydro-5H-pyrido[4,3-b][1,4]thiazepino[2,3,4-hi]indol-11(8aH)-yl)-1-(4-fluorophenyl)-1-butanone following the General procedure above: ¹H NMR (300 MHz, CD₃OD) δ 7.34 - 7.40 (m, 2H), 6.98 - 7.10 (m, 2H), 6.90 -6.95 (m, 2H), 6.62 - 6.70 (m, 1H), 4.70 (m, 1H), 3.76 - 3.86 (m, 1H), 3.44 - 3.59 (m, 4H), 3.24 - 3.30 (m, 1H), 2.90 - 3.14 (m, 4H), 2.05 - 2.45 (m, 4H), 1.80 - 2.02 (m, 4H) ppm. MS - ESI: 413 [MH]⁺

### EXAMPLE 236

### 4-((8aS,12aR)-6,7,9,10,12,12a-hexahydro-5H-pyrido[4,3-b][1,4]thiazepino[2,3,4-hi]indol-11(8aH)-yl)-1-(4-pyridinyl)-1-butanol

The title compound was prepared from 1-(4-pyridyl)-4-(2,3,6b,7,8,9,10,10a-octahydro-3-methyl-1H-pyrido[3',4':4,5]- pyrrolo[1,2,3-de]quinoxalin-8-yl)-1-butanone following the General procedure of Example 235. ¹H NMR (300 MHz, CDCl₃) δ 8.53 - 8.57 (m, 2H), 7.32 - 7.36 (m, 2H), 6.90 -6.98 (m, 1H), 6.84 - 6.87 (m, 1H), 6.60 - 6.66 (m, 1H), 4.66 - 4.72 (m, 1H), 3.80 - 3.92 (m, 1H), 3.55 - 3.71 (m, 3H), 3.22 - 3.30 (m, 2H), 2.64 - 3.02 (m, 4H), 2.31 - 2.54 (m, 3H), 1.69 - 2.03 (m, 8H) ppm. MS (CI, NH₃) *m*/*e* 395 (base, M+H⁺).

### EXAMPLE 237

### 4-((8aS,12aR)-6,7,9,10,12,12a-hexahydro-5H-pyrido[4,3-b][1,4]thiazepino[2,3,4-hi]indol-11(8aH)-yl)-1-(2,3-dimethoxyphenyl)-1-butanol

### Step A:

Oxalyl chloride (55 mmol) was dissolved in CH₂Cl₂ (25 mL), cooled down to -60°C, DMSO (120 mmol) in CH₂Cl₂ (10 mL) solution was added dropwise. The reaction mixture was stirred at -60°C for 10 minutes. 1-chlorobutan-4-ol ( 50 mmol) in CH₂Cl₂ (10 mL) was added slowly in 10 minutes. The reaction mixture was stirred at same temperature for 15 minute. Added Et₃N in approximately 5 minutes at -60°C. The cooling bath was removed and water was added at rt, stirring was continued for 10 minutes. The organic layer was separated. The aqueous phase was extracted with CH₂Cl₂ (3 x 50 mL). Combined the organic layer, dried over MgSO₄, filtered, and concentrated to a residue to afford 1-chlorobutan-4-al in 64% yield. The product was distilled under reduced pressure to afford the aldehyde in 60% yield (5 mm Hg, 88-90°C).

### Step B:

To a solution of TMEDA (6.6 mmol) in dry THF (15 mL), was added *Sec*-BuLi (6.6 mmol) slowly at -78°C. The reaction mixture was stirred at -78°C for 10 minute, veratrole (6.0 mmol) in THF (3 mL) was added slowly. The reaction was stirred at -78°C for 30 minutes, 1-chlorobutan-4-al (6.6 mmol) was added and stirred at -78°C for 2 hr.. The reaction mixture was warmed to rt, added brine (1 mL), and filtered. The filtrate was dried over MgSO₄, filtered and concentrated to a residue. The residue was purified by flash column chromatography (Silica gel; Ethyl acetate / Hexane: 3/7) to afford 1-(2,3-dimethoxyphenyl)-4-chlorobutan-1-ol in 20% yield. ¹H NMR (300 MHz, CDCl₃) δ 7.05 (dd, J = 8.0 Hz, 7.7 Hz, 1H), 6.94 (bd, J = 7.7 Hz, 1H), 6.85 (bd, J = 8.1 Hz, 1H), 4.91 - 4.97 (m, 1H), 3.88 (s, 3H), 3.84 (s, 3H), 3.56 - 3.60 (m, 2H), 2.44 - 2.45 (m, 1H), 1.81 - 2.00 (m, 4H) ppm. MS (CI, NH₃) *m*/*e* 244 (base, M+H⁺).

### Step C:

To a suspension of (8a*S*,12a*R*)-6,7,8a,9,10,11,12,12a-octahydro-5*H*-pyrido[4,3-*b*][1,4]thiazepino[2,3,4-*hi*]indole ( 1.17 mmol) in 1,4-dioxane (4 mL) was added the alcohol from Step B 1-(2,3-dimethoxyphenyl)-4-chlorobutan-1-ol (0.78 mmol), potassium iodine (100 mg) and potassium carbonate (300 mg). The reaction mixture was heated at reflux for 2 days. The solvent was removed under reduced pressure. The residue was treated with water (50 mL) and extracted with methylene chloride (3 x 50 mL). The CH₂Cl₂ extract was washed with brine (150 mL), dried over MgSO₄, filtered and concentrated to a residue. The residue was purified by flash column chromatography (Silica gel, CH₂Cl₂:CH₃OH 9:1). The product was dissolved in ether (2 mL) and stirred at 0°C for 10 minutes, added 1N HCl in ether (0.5 mL) at 0°C. The white crystalline solid was collected by filtration to give the title compound in 62% yield. ¹H NMR (300 MHz, CDCl₃) δ 7.07 (d, J = 7.6 Hz, 1H), 6.99 (dd, J = 7.7 Hz, 8.1 Hz, 1H), 6.89 (dd, J = 1.5 Hz, 8.0 Hz, 1H), 6.83 (dd, J = 1.1 Hz, 7.4 Hz, 1H), 6.75 (dd, J = 1.5 Hz, 7.7 Hz, 1H), 6.57 (dd, J = 7.3 Hz, 7.7 Hz, 1H), 4.92 (m, 1H), 3.79 (s, 6H), 3.46 - 3.56 (m, 1H), 3.16 - 3.20 (m, 2H), 2.67 - 2.95 (m, 4H), 2.31 - 2.34 (m, 3H), 1.80 - 2.05 (m, 5H), 1.64 - 1.76 (m, 6H) ppm. MS (CI, NH₃) *m*/*e* 455 (base, M+H⁺).

### EXAMPLE 238

### 4-((8aS,12aR)-6,7,9,10,12,12a-hexahydro-5H-pyrido[4,3-b][1,4]thiazepino[2,3,4-hi]indol-11(8aH)-yl)-1-(2,3-dimethoxyphenyl)-1-butanone

To a solution of the alcohol 4-((8aS,12aR)-6,7,9,20,12,12a-hexahydro-5H-pyrido[4,3-b][1,4]thiazepino [2,3,4-hi]indol-11(8aH)-yl)-1-(2,3-dimethoxyphenyl)-1-butanol (0.11 mmol) and N-methylmorpholine N-oxide (0.17 mmol) in CH₂Cl₂ (2 mL) with powder 4 A molecular sieves at rt, was added solid tetrapropyl ammonium perruthenate (0.006 mmol) in one portion. The reaction mixture was stirred at rt for 4 hr., filtered and concentrated to a residue. The residue was purified by flash column chromatography (Silica gel; CH₂Cl₂/ CH₃OH: 9:1) to afford the title compound in 94% yield. ¹H NMR (300 MHz, CDCl₃) δ 7.05 (d, J = 7.3 Hz, 1H), 7.02 (d, J = 7.4 Hz, 1H), 6.97 (dd, J = 6.9 Hz, 7.7 Hz, 1H), 6.87 (d, J = 7.7 Hz, 1H), 6.77 (d, J = 6.6 Hz, 1H), 6.53 (dd, J = 7.7 Hz, 7.3 Hz, 1H), 3.82 (s, 3H), 3.80 (s, 3H), 3.70 - 3.80 (m, 1H), 3.43 - 3.56 (m, 1H), 3.17 - 3.22 (m, 1H), 2.82 - 3.05 (m, 5H), 2.56 - 2.76 (m, 2H), 2.10 - 2.30 (m, 3H), 1.80 - 2.05 (m, 7H) ppm.

### EXAMPLE 240

### cis-(8a,12a)-11-(4,4-diphenylbutyl)-6,7,8a,9,10,11,12,12a-octahydro-5H-pyrido[4,3-b][1,4]thiazepino[2,3,4-hi]indole

### Step A:

To a solution of 4-chloro-1,1-diphenyl-1-butene (200 mg, 0.82 mmol) in EtOAc (8.0 mL) was added Pd/C (10%, 50 mg). The reaction mixture was stirred under H₂ atmosphere for 15 h at 20°C. The reaction mixture was filtered through celite and the filterate was concentrated to give the analytically pure 4-Chloro-1,1-diphenylbutane (201mg, 99%) as a colorless oil. ¹H NMR (CDCl₃, 300 MHz) δ 1.70-1.81 (m, 2H), 2.16-2.25 (m, 2H), 3.54 (t, 2H, J = 6.5 Hz), 3.91 (t, 1H, J = 7.8 Hz), 7.15-7.31 (m, 10H) ppm.

### Step B:

The title compound was prepared by following the general coupling procedure of Example 43 as a pale yellow oil (48 mg, 88%) cis-(8a,12a)-6,7,8a,9,10,11,12,12a-octahydro-5*H*-pyrido[4,3-*b*][1,4]thiazepino[2,3,4-*hi*]indole (30 mg, 0.12 mmol) and 4-chloro-1,1-diphenylbutane (44 mg, 0.18 mmol). ¹H NMR (CDCl₃, 300 Mhz) δ 1.42-1.53 (m, 2H), 1.78 (t, 1H, J = 12.0 Hz), 1.82-1.92 (m, 2H), 1.95-2.20 (m, 5H), 2.25-2.37 (m, 2H), 2.56-2.63 (m, 1H), 2.65-2.73 (m, 1H), 2.88-2.97 (m, 1H), 3.00-3.17 (m, 2H), 3.22-3.28 (m, 1H), 3.50-3.63 (m, 1H), 3.76-3.85 (m, 1H), 3.89 (t, 1H, J = 7.9 Hz), 6.60 (t, 1H, J = 7.7 Hz), 6.82 (d, 1H, J = 7.3 Hz), 6.93 (d, 1H, J = 7.7 Hz), 7.13-7.35 (m, 10H) ppm.

### EXAMPLE 241

### cis-(8a,12a)-11-(4,4-diphenyl-3-butenyl)-6,7,8a,9,10,11,12,22a-octahydro-5H-pyrido[4,3-b][1,4]thiazepino[2,3,4-hi]indole

### Step A:

Cyclopropyldiphenylmethanol (500 mg, 2.23 mmol) was dissolved in 1M HCl in i-PrOH (4.0 mL). The reaction mixture was then heated at 60°C for 1h. The reaction was cooled 20°C and diluted with Et₂O (100 mL). The organic solution was successively washed with H₂O, NaHCO₃ saturated aqueous solution and brine. It was then dried over MgSO4, filtered, concentrated *in vacuo* and chromatographed on a silica gel column by elution with Hexanes to give 4-Chloro-1,1-diphenyl-1-butene (506 mg, 93%) as a colorless oil. ¹H NMR (CDCl₃, 300 MHz) δ 2.59 (q, 2H, J = 6.9 Hz), 3.58 (t, 2H, J = 6.9 Hz), 6.12 (t, 1H, J = 7.3 Hz), 7.16-7.42 (m, 10H) ppm.

### Step B:

The title compound was prepared by following the general coupling procedure of Example 43 as a pale yellow oil (33 mg, 61%) from cis-(8a,12a)-6,7,8a,9,10,11,12,12a-octahydro-5*H*-pyrido[4,3-*b*][1,4]thiazepino[2,3,4-*hi*]indole (30 mg, 0.12 mmol) and 4-chloro-1,1-diphenyl-1-butene (44 mg, 0.18 mmol). ¹H NMR (CDCl₃, 300 MHz) δ 1.79-1.92 (m, 3H), 1.94-2.17 (m, 2H), 2.20-2.37 (m, 3H), 2.40-2.49 (m, 2H), 2.55-2.63 (m, 1H), 2.65-2.73 (m, 1H), 2.88-2.97 (m, 1H), 3.00-3.17 (m, 2H), 3.23-3.29 (m, 1H), 3.50-3.62 (m, 1H), 3.76-3.87 (m, 1H), 6.06 (t, 1H, J = 7.3 Hz), 6.61 (t, 1H, J = 7.3 Hz), 6.82 (d, 1H, J = 6.6 Hz), 6.94 (dd, 1H, J = 1.1, 7.7 Hz), 7.17-7.41 (m, 10H) ppm.

### EXAMPLE 242

### cis-(8a,12a)-11-[4,4-bis(4-fluorophenyl)butyl]-6,7,8a,9,10,11,12,12a-octahydro-5H-pyrido[4,3-b][1,4]thiazepino[2,3,4-hi]indole

The title compound was prepared by following the general coupling procedure of Example 43 as a pale yellow oil (89 mg, 51%) from cis-(8a,12a)-6,7,8a,9,10,11,12,12a-octahydro-5*H*-pyrido[4,3-*b*][1,4]thiazepino[2,3,4-*hi*]indole (68 mg, 0.27 mmol) and 1,1-bis-(4'-fluorophenyl)-4-chlorobutane (90 mg, 0.32 mmol). ¹H NMR (CDCl₃, 300 MHz) δ 1.38-1.48 (m, 2H), 1.79 (t, 1H, J = 11.2 Hz), 1.83-1.91 (m, 2H), 1.95-2.21 (m, 5H), 2.23-2.35 (m, 2H), 2.53-2.62 (m, 1H), 2.63-2.75 (m, 1H), 2.89-2.99 (m, 1H), 3.01-3.16 (m, 2H), 3.22-3.27 (m, 1H), 3.52-3.62 (m, 1H), 3.76-3.85 (m, 1H), 3.86 (m, 1H, J = 8.1 Hz), 6.61 (t, 1H, J = 7.3 Hz), 6.82 (d, 1H, J = 6.6 Hz), 6.90-6.99 (m, 5H), 7.13-7.22 (m, 4H) ppm.

### EXAMPLE 243

### cis-(8a,12a)-11-[4,4-bis(4-fluorophenyl)-3-butenyl]-6,7,8a,9,10,11,12,12a-octahydro-5H-pyrido[4,3-b][1,4]thiazepino[2,3,4-hi]indole

### Step A:

To a solution of cyclopropyl-(4-fluorophenyl)ketone (520 mg, 3.17 mmol) in THF (10 mL) was added 4-fluorophenyl magnesium bromide dropwise at 0°C under N₂ atmosphere. The reaction mixture was stirred for 1 h at 0°C, quenched by addition of brine and extracted with Et₂O. The organic layer was then washed with NaHCO₃ saturated aqueous solution and brine. It was then dried over MgSO₄, filtered, concentrated *in vacuo* to give crude bis(4-fluorophenyl)-cyclopropylmethanol. It was used for ring opening reaction by following the procedure for formation of 4-chloro-1,1-diphenyl-1-butene without further purification. 1,1-Bis(4-fluorophenyl)-4-chloro-1-butene (780 mg, 88%) was obtained as a colorless oil. ¹H NMR (CDCl₃, 300 MHz) δ 2.57 (q, 2H, J = 6.9 Hz), 3.58 (t, 2H, J = 6.9 Hz), 6.04 (t, 1H, J = 7.3 Hz), 6.92-7.21 (m, 8H) ppm.

### Step B:

The title compound was prepared by following the general coupling procedure of Example 43 as a pale yellow oil (40 mg, 68%) from cis-(8a,12a)-6,7,8a,9,10,11,12,12a-octahydro-5*H*-pyrido[4,3-*b*][1,4]thiazepino[2,3,4-*hi*]indole (30 mg, 0.12 mmol) and 1,1-bis-(4'-fluorophenyl)-4-chlorolbutene (51 mg, 0.18 mmol). ¹H NMR (CDCl₃, 300 MHz) δ 1.79-1.93 (m, 3H), 1.98-2.18 (m, 2H), 2.19-2.37 (m, 3H), 2.38-2.48 (m, 2H), 2.55-2.63 (m, 1H), 2.63-2.73 (m, 1H), 2.90-2.99 (m, 1H), 3.01-3.18 (m, 2H), 3.22-3.28 (m, 1H), 3.48-3.60 (m, 1H), 3.75-3.86 (m, 1H), 5.98 (t, 1H, J = 7.3 Hz), 6.61 (t, 1H, J = 7.3 Hz), 6.83 (d, 1H, J = 7.3 Hz), 6.88-7.01 (m, 3H), 7.01-7.20 (m, 6H) ppm.

### EXAMPLE 244

### N-[2-(cis-(8a,12a)-6,7,9,10,12,12a-hexahydro-5H-pyrido[4,3-b][1,4]thiazepino[2,3,4-hi]indol-11(8aH)-yl)ethyl]benzamide

### Step A:

To a solution of ethanolamine (1.09 g, 17.8 mmol) in THF was added benzoyl chloride (520 mg, 3.60 mmol) dropwise at 0°C under N₂ atmosphere. The reaction mixture was stirred for 10 min and then quenched with 1M HCl. The mixture was then diluted with EtOAc and washed with sat. aq. NaHCO₃ and brine. The organic layer was dried over MgSO₄, filtered and concentrated *in vacuo.* The residue was crystalized to give N-(2-hydroxyethyl)benzamide (593 mg, 97%) as a white crystalline solid. ¹H NMR (CDCl₃, 300 MHz) δ 3.41 (s, 1H), 3.49-3.59 (m, 2H), 3.75 (t, 2H, *J* = 5.2 Hz), 7.10 (s, 1H), 7.32-7.38 (m, 2H), 7.42-7.48 (m, 1H), 7.72-7.76 (m, 2H) ppm.

2-Benzamidoethyl methanesulfonate was prepared by following the general procedure of Example 43 for mesylation as a colorless oil (291 mg, 84%) from N-(2-hydroxyethyl)benzamide (245 mg, 1.30 mmol) and methanesulfonyl chloride (223 mg, 1.94 mmol). ¹H NMR (CDCl₃, 300 MHz) δ 2.85 (s, 3H), 4.48 (t, 2H, J = 9.5 Hz), 5.10 (t, 2H, J = 10.2 Hz), 7.58-7.63 (m, 2H), 7.74-7.80 (m, 1H), 8.20-8.23 (m, 2H) ppm.

### Step B:

The title compound was prepared by following the general coupling procedure of Example 43 as a pale yellow oil (38 mg, 79%) from cis-(8a,12a)-6,7,8a,9,10,11,12,12a-octahydro-5*H*-pyrido[4,3-*b*][1,4]thiazepino[2,3,4-*hi*]indole (30 mg, 0.12 mmol) and 2-benzamidoethyl methanesulfonate (119 mg, 0.49 mmol). ¹H NMR (CDCl₃, 300 MHz) δ 1.87-1.92 (m, 2H), 2.05-2.12 (m, 2H), 2.16-2.23 (m, 1H), 2.40-2.50 (m, 1H), 2.55-2.60 (m, 2H), 2.77-2.84 (m, 2H), 2.92-3.05 (m, 1H), 3.15-3.24 (m, 1H), 3.31-3.34 (m, 1H), 3.44-3.57 (m, 2H), 3.69-3.78 (m, 2H), 4.45-4.51 (m, 1H), 6.59 (t, 1H, J = 7.7 Hz), 6.83-6.86 (m, 1H), 6.91-6.97 (m, 1H), 7.15-7.23 (m, 1H), 7.35-7.5 (m, 2H), 7.61 (dd, 1H, J = 1.1, 8.4 Hz) ppm.

### EXAMPLE 245

### N-[2-(cis-(8a,12a)-6,7,9,10,12,12a-hexahydro-5H-pyrido[4,3-b][1,4]thiazepino[2,3,4-hi]indol-11(8aH)-yl)ethyl]-2-fluorobenzamide

### Step A:

Ethanolamine (1.00 mg, 15.8 mmol) and 2-fluorobenzoyl chloride (504 mg, 3.15 mmol) were coupled to give N-(2-hydroxyethyl)-2'-fluorobenzamide (460 mg, 79%) by following the procedure for preparation of N-(2-hydroxyethyl)benzamide. ¹H NMR (CD₃OD, 300 MHz) δ 3.49 (t, 2H, *J* = 5.9 Hz), 3.68 (t, 2H, *J* = 2.9 Hz), 7.15-7.28 (m, 2H), 7.47-7.55 (m, 1H), 7.72-7.78 (m, 1H) ppm. N-(2-Chloroethyl)-2'-fluorobenzamide was prepared by following the chlorination procedure in synthesis of 2-(2-chloroethyl)isoindolinone as a colorless oil (130 mg, 70%) from N-(2-hydroxyethyl)-2'-fluorobenzamide (130 mg, 0.71 mmol) and methanesulfonyl chloride (122 mg, 1.06 mmol). ¹H NMR (CDCl₃, 300 MHz) ¹H NMR (CDCl₃) δ 3.72-3.76 (m, 2H), 3.82-3.87 (m, 2H), 7.11-7.18 (m, 2H), 7.25-7.28 (m, 1H), 7.46-7.53 (m, 1H), 8.08-8.13 (m, 1H) ppm.

### Step B:

The title compound was prepared by following the general coupling procedure of Example 43 as a pale yellow oil (56 mg, 90%) from cis-(8a,12a)-6,7,8a,9,10,11,12,12a-octahydro-5*H*-pyrido[4,3-*b*][1,4]thiazepino [2,3,4-*hi*]indole (30 mg, 0.12 mmol) and N-(2-chloroethyl)-2'-fluorobenzamide (74 mg, 0.37 mmol). ¹H NMR (CDCl₃, 300 MHz) δ 1.92-2.15 (m, 6H), 2.37-2.39 (m, 1H), 2.55-2.58 (m, 1H), 2.61-2.65 (m, 1H), 2.75-2.80 (m, 1H), 2.91-2.99 (m, 1H), 3.11-3.18 (m, 2H), 3.27-3.31 (m, 1H), 3.49-3.60 (m, 4H), 3.78-3.82 (m, 1H), 6.59 (t, 1H, J = 7.3 Hz), 6.85 (d, 1H, J = 7.0 Hz), 6.94 (dd, 1H, J = 1.5, 8.1 Hz), 7.08, 7.15 (m, 1H), 7.24-7.29 (m, 1H), 7.43-7.51 (m, 1H), 8.08-8.14 (dt, 1H, J = 1.8, 8.1 Hz) ppm.

### EXAMPLE 246

### N-[2-(cis-(8a,12a)-6,7,9,10,12,12a-hexahydro-5H-pyrido[4,3-b][1,4]thiazepino[2,3,4-hi]indol-11(8aH)-yl)ethyl]-4-fluorobenzamide

### Step A:

Ethanolamine (0.96 mg, 15.8 mmol) and 4-fluorobenzoyl chloride were coupled to give N-(2-hydroxyethyl)-4'-fluorobenzamide (482 mg, 81%) by following the procedure for preparation of N-(2-hydroxyethyl)benzamide. ¹H NMR (CD₃OD, 300 MHz) δ 3.47 (t, 2H, J = 5.9 Hz), 3.68 (t, 2H, J = 5.7), 7.13-7.20 (m, 2H), 7.82-7.92 (m, 2H) ppm. 2-(4'-Fluorobenamido)ethyl methanesulfonate was prepared by following the general procedure of Example 43 for mesylation as a white crystal (130 mg, 70%) from N-(2-hydroxyethyl)-4'-fluorobenzamide (130 mg, 0.71 mmol) and methanesulfonyl chloride (122 mg, 1.06 mmol). ¹H NMR (CDCl₃, 300 MHz) δ 2.85 (s, 3H), 4.48 (t, 2H, *J* = 9.5 Hz), 5.10 (t, 2H, *J* = 10.2 Hz), 7.08-7.18 (m, 2H), 7.70-7.82 (m, 3H) ppm.

### Step B:

The title compound was prepared by following the general coupling procedure of Example 43 as a pale yellow oil (45 mg, 91%) from cis-(8a,12a)-6,7,8a,9,10,11,12,12a-octahydro-5*H*-pyrido[4,3-*b*][1,4]thiazepino[2,3,4-*hi*]indole (30 mg, 0.12 mmol) and N-[2-(methylsulfonyl)ethyl]-4-fluorobenzamide (63mg, 0.24 mmol). ¹H NMR (CDCl₃, 300 MHz) δ 1.85-1.93 (m, 2H), 2.05-2.15 (m, 2H), 2.24 (dd, 1H, J = 8.7, 11.3 Hz), 2.39-2.47 (m, 1H), 2.52-2.61 (m, 3H), 2.71-2.80 (m, 1H), 2.98-3.07 (m, 1H), 3.10-3.20 (m, 1H), 3.20-3.30 (m, 1H), 3.31-3.39 (m, 1H), 3.40-3.50 (m, 1H), 3.54 (q, 2H, J = 6.2 Hz), 3.65-3.76 (m, 1H), 6.58 (t, 1H, J = 7.7 Hz), 6.82-6.92 (m, 2H), 6.95 (dd, 1H, J = 1.1, 8.1 Hz), 7.05-7.17 (m, 2H), 7.70-7.80 (m, 2H) ppm.

### EXAMPLE 247

### cis-(8a,12a)-11-[3-(1H-indol-3-yl)propyl]-6,7,8a,9,10,11,12,12a-octahydro-5H-pyrido[4,3-b][1,4]thiazepino[2,3,4-hi]indole

### Step A:

3-(3-Indolyl)-1-propyl methanesulfonate was prepared by following the general procedure of Example 43 for mesylation as a colorless oil (170 mg, 81%) from 3-(3-indolyl)-1-propanol (145 mg, 0.83 mmol) and methanesulfonyl chloride (142 mg, 1.24 mmol). ¹H NMR (CDCl₃, 300 MHz) δ 2.17 (qu, 2H, J = 7.3 Hz), 2.92 (t, 2H, J = 7.0 Hz), 2.99 (s, 3H), 4.27 (t, 2H, J = 6.4 Hz), 7.04 (d, 1H, J = 2.2 Hz), 7.12 (dt, 1H, J = 1.1, 7.0 Hz), 7.21 (dt, 1H, J = 1.1, 7.0 Hz), 7.38 (d, 1H, J = 8.1 Hz), 7.59 (d, 1H, J = 7.3 Hz), 8.00 (br, 1H) ppm.

### Step B:

The title compound was prepared by following the general coupling procedure of Example 43 as a pale yellow oil (20 mg, 59%) cis-(8a,12a)-6,7,8a,9,10,11,12,12a-octahydro-5*H*-pyrido[4,3-*b*][1,4]thiazepino[2,3,4-*hi*]indole (21 mg, 0.084 mmol) and 3-(3-indolyl)propyl methylsulfonate (32 mg, 0.13 mmol). ¹H NMR (CDCl₃, 300 MHz) δ 1.93-2.22 (m, 7H), 2.40-2.53 (m, 1H), 2.55-2.65 (m, 2H), 2.77-2.95 (m, 5H), 2.97-3.07 (m, 1H), 3.26-3.40 (m, 2H), 3.48-3.60 (m, 1H), 3.77-3.88 (m, 1H), 6.63 (t, 1H, J = 7.7 Hz), 6.85 (d, 1H, J = 7.3 Hz), 6.96 (dd, 1H, J = 1.1, 8.1 Hz), 7.01 (d, 1H, 1.8 Hz), 7.10 (t, 1H, J = 7.3 Hz), 7.19 (t, 1H, J = 7.4 Hz), 7.36 (d, 1H, J = 8.1 Hz), 7.58 (d, 1H, J = 7.7 Hz), 8.00 (s, 1H) ppm.

### EXAMPLE 248

### cis-(8a,12a)-11-[3-(1-methyl-1H-indol-3-yl)propyl]-6,7,8a,9,10,11,12,12a-octahydro-5H-pyrido[4,3-b][1,4]thiazepino[2,3,4-hi]indole

### Step A:

To a solution of KOH (2.24 g, 40.0 mmol), in dry DMSO (10 mL) was added 3-indolpropionic acid (946 mg, 5.0 mmol) followed immediately by MeI (3.0 g, 4.0 mmol). The reaction mixture was stirred for 1.5 h at 20°C, quenched by pouring into water and extracted with CHCl₃ (3 x 20 mL). The organic layer was then washed with brine, dried over MgSO4, filtered, concentrated *in vacuo* and chromatographed on a silica gel column by elution with EtOAc/ Hexanes to give methyl 3-(1-methyl-3-indolyl)propionate (1.00 g, 92%) as a colorless oil. ¹H NMR (CDCl₃, 300 MHz) δ 2.71 (t, 2H, J = 8.1 Hz), 3.09 (t, 2H, J = 7.0 Hz), 3.68 (s, 3H), 3.74 (s, 3H), 6.87 (s, 1H), 7.11 (dt, 1H, J = 1.1, 6.8 Hz), 7.22 (dt, 1H, J = 1.1, 8.0 Hz), 7.29 (d, 1H, J = 8.1 Hz), 7.59 (d, 1H, J = 7.7 Hz) ppm.

To a solution of methyl 3-(1-methyl-3-indolyl)propionate (300 mg, 1.38 mmol) in Et₂O (3.0 mL) was added LiAlH₄ at 0°C under N₂ atmosphere. The reaction mixture was stirred for 30 min at 0°C and quenched by careful addition of H₂O. EtOAc was added to the quenched reaction mixture. The organic layer was separated, washed with brine and dried over MgSO₄. It was then concentrated *in vacuo* and chromatographed on a silica gel column by elution with EtOAc/ Hexanes to give 3-(1-methyl-3-indolyl)-1-propanol (250 mg, 96%) as a colorless oil. ¹H NMR (CDCl₃, 300 MHz) δ 1.28 (t, 1H, J = 6.2 Hz), 1.98 (qu, 2H, J = 7.7 Hz), 2.85 (t, 2H, J = 7.3 Hz), 3.70-3.78 (m, 4H), 6.86 (s, 1H), 7.10 (dt, 1H, J = 1.1, 7.0 Hz), 7.22 (dt, 1H, J = 1.1, 7.5 Hz), 7.29 (d, 1H, J = 8.1 Hz), 7.60 (d, 1H, J = 7.7 Hz) ppm.

3-(1-methyl-3-indolyl)-1-propyl methanesulfonate was prepared by following the general procedure of Example 43 for mesylation as a colorless oil (291 mg, 84%) from 3-(1-methyl-3-indolyl)-1-propanol (245 mg, 1.30 mmol) and methanesulfonyl chloride (223 mg, 1.94 mmol). ¹H NMR (CDCl₃, 300 MHz) δ 2.15 (qu, 2H, J = 7.0 Hz), 2.90 (t, 2H, J = 7.0 Hz), 2.99 (s, 3H), 3.76 (s, 3H), 4.27 (t, 2H, J = 6.2 Hz), 6.88 (s, 1H), 7.11 (dt, 1H, J = 1.1, 6.9 Hz), 7.23 (dt, 1H, J = 1.1, 7.0 Hz), 7.29 (d, 1H, J = 8.5 Hz), 7.57 (d, 1H, J = 8.1 Hz) ppm.

### Step B:

The title compound was prepared by following the general coupling procedure of Example 43 as a pale yellow oil (36 mg, 86%) from cis-(8a,12a)-6,7,8a,9,10,11,12,12a-octahydro-5*H*-pyrido[4,3-*b*][1,4]thiazepino[2,3,4-*hi*]indole (25 mg, 0.10 mmol) and 3-(1-methyl-3-indolyl)propyl methylsulfonate (32 mg, 0.12 mmol). ¹H NMR (CDCl₃, 300 MHz) δ 1.92-2.18 (m, 7H), 2.35-2.46 (m, 1H), 2.48-2.60 (m, 2H), 2.74-2.95 (m, 5H), 2.96-3.07 (m, 1H), 3.25-3.35 (m, 2H), 3.46-3.58 (m, 1H), 3.74 (s, 3H), 3.77-3.87 (m, 1H), 6.63 (t, 1H, J = 7.3 Hz), 6.82-6.88 (m, 2H), 6.96 (d, 1H, J = 7.7 Hz), 7.09 (t, 1H, J = 7.0 Hz), 7.20-7.32 (m, 2H), 7.57 (d, 1H, J = 7.7 Hz) ppm.

### EXAMPLE 249

### cis-(8a,12a)-11-[2-(1H-indol-3-yl)ethyl]-6,7,8a,9,10,11,12,12a-octahydro-5H-pyrido[4,3-b][1,4]thiazepino[2,3,4-hi]indole

The title compound was prepared by following the general coupling procedure of Example 43 as a pale yellow oil (33 mg, 71%) from cis-(8a,12a)-6,7,8a,9,10,11,12,12a-octahydro-5*H*-pyrido[4,3-*b*][1,4]thiazepino[2,3,4-*hi*]indole (30 mg, 0.12 mmol) and 3-(2-bromoethyl)indole (54 mg, 0.24 mmol). ¹H NMR (CDCl₃, 300 MHz) δ 1.93-2.20 (m, 5H), 2.35-2.43 (m, 1H), 2.64-2.77 (m, 2H), 2.78-2.87 (m, 1H), 2.89-3.15 (m, 5H), 3.20-3.35 (m, 2H), 3.49-3.61 (m, 1H), 3.77-3.87 (m, 1H), 6.64 (t, 1H, J = 7.7 Hz), 6.90 (d, 1H, J = 7.3 Hz), 6.97 (dd, 1H, J = 1.1, 8.1 Hz), 7.02 (d, 1H, 2.2 Hz), 7.08-7.21 (m, 2H), 7.35 (d, 1H, J = 8.0 Hz), 7.61 (d, 1H, J = 8.1 Hz), 7.98 (s, 1H) ppm.

### EXAMPLE 250

### cis-(8a,12a)-11-[3-(1H-indol-1-yl)propyl]-6,7,8a,9,10,11,12,12a-octahydro-5H-pyrido[4,3-b][1,4]thiazepino[2,3,4-hi]indole

### Step A:

To a solution of 1-bromo-3-chloropropane (2.00 g, 12.7 mmol) in DMF (7.0 mL) was added indole (500 mg, 4.24 mmol) followed by powdered KOH (262 mg, 4.66 mmol) at 20°C. The reaction mixture was then stirred for 15 h at 20°C. The reaction was quenched by addition of H₂O and the product was extracted with Et₂O. The organic solution was washed with H₂O and brine, dried over MgSO₄, filtered and concentrated *in vacuo*. 1-(3-Chloro-1-propyl)indole (580 mg, 71%) was isolated by flash chromatography on a silica gel column by elution with EtOAc/Hexanes a pale yellow oil. ¹H NMR (CDCl₃, 300 MHz) δ 2.28 (qu, 2H, J = 6.3 Hz), 3.46 (t, 2H, J = 6.2 Hz), 4.36 (t, 2H, J = 6.6 Hz), 6.51 (d, 1H, J = 3.3 Hz), 7.09-7.18 (m, 2H), 7.20-7.25 (m, 1H), 7.38 (d, 1H, J = 8.5 Hz), 7.64 (d, 1H, J = 7.7 Hz) ppm.

### Step B:

The title compound was prepared by following the general coupling procedure of Example 43 as a pale yellow oil (46 mg, 95%) from cis-(8a,12a)-6,7,8a,9,10,11,12,12a-octahydro-5*H*-pyrido[4,3-*b*][1,4]thiazepino[2,3,4-*hi*]indole (30 mg, 0.12 mmol) and 1-(3-chloropropyl)indole (46 mg, 0.24 mmol). ¹H NMR (CDCl₃, 300 MHz) δ 1.90-2.15 (m, 7H), 2.18-2,33 (m, 3H), 2.52-2.60 (m, 1H), 2.66-2.75 (m, 1H), 2.89-2.98 (m, 1H), 3.02-3.18 (m, 2H), 3.26-3.33 (m, 1H), 3.48-3.59 (m, 1H), 3.77-3.87 (m, 1H), 4.21 (t, 2H, J = 7.0 Hz), 6.62 (t, 1H, J = 7.3 Hz), 6.85 (d, 1H, J = 6.6 Hz), 6.95 (dd, 1H, J = 1.1, 7.7 Hz), 7.05-7.16 (m, 2H), 7.20 (dt, 1H, J = 1.1, 7.0 Hz), 7.38 (d, 1H, J = 8.4 Hz), 7.63 (d, 1H, J = 8.1 Hz) ppm.

### EXAMPLE 251

### cis-(8a,12a)-11-[3-(2,3-dihydro-1H-indol-1-yl)propyl]-6,7,8a,9,10,11,12,12a-octahydro-5H-pyrido[4,3-b][1,4]thiazepino[2,3,4-hi]indole

### Step A:

To a solution of 1-bromo-3-chloropropane (1.98 g, 12.6 mmol) and indoline (500 mg, 4.20 mmol) in 1,4-dioxane (6.0 mL) was added Et₃N (2.12 g, 21.0 mmol) at 20°C. The reaction mixture was then stirred for 15 h at 70°C. The reaction mixturewas cooled to 20°C and quenched by addition of H₂O. The product was extracted with Et₂O. The organic solution was then washed with H₂O and brine, dried over MgSO₄, filtered and concentrated *in vacuo*. 1-(3-Chloro-1-propyl)indoline (373 mg, 45%) was isolated by flash chromatography on a silica gel column by elution with EtOAc/Hexanes a pale yellow oil. ¹H NMR (CDCl₃, 300 MHz) δ 2.07 (qu, 2H, J = 6.2 Hz), 2.97 (t, 2H, J = 8.2 Hz), 3.24 (t, 2H, J = 6.6 Hz), 3.35 (t, 2H, J = 8.3 Hz), 3.68 (t, 2H, J = 6.2 Hz), 6.50-6.56 (m, 1H), 6.66 (t, 1H, J = 6.6 Hz), 7.04-7.10 (m, 2H) ppm.

### Step B:

The title compound was prepared by following the general coupling procedure of Example 43 as a pale yellow oil (36 mg, 74%) from (±)-*cis*-6,7,8a,9,10, 11,12,12a-octahydro-5H-pyrido[3',4':4,5]pyrrolo[1,2,3-*ef*][2,5]benzothiazepine (30 mg, 0.12 mmol) and 1-(3-chloropropyl)indoline (47 mg, 0.24 mmol). ¹H NMR (CDCl₃, 300 Mhz) δ 1.80-2.18 (m, 7H), 2.23-2.35 (m, 2H), 2.43-2.55 (m, 1H), 2.70-2.85 (m, 2H), 2.89-2.98 (m, 3H), 3.02-3.19 (m, 4H), 3.28-3.38 (m, 3H), 3.48-3.61 (m, 1H), 3.78-3.90 (m, 1H), 6.45-6.52 (m, 1H), 6.59-6.70 (m, 2H), 6.82-6.90 (m, 1H), 6.92-6.98 (m, 1H), 7.02-7.13 (m, 2H) ppm.

### EXAMPLE 252

### cis-(8a,12a)-11-[3-(1H-benzimidazol-1-yl)propyl]-6,7,8a,9,10,11,12,12a-octahydro-5H-pyrido[4,3-b][1,4]thiazepina[2,3,4-hi]indole

### Step A:

To a solution of benzimidazol (355 mg, 3.00 mmol) in dry DMF (10 mL) was added NaH (83 mg, 3.3 mmol) at 20°C under N₂ atmosphere. The reaction mixture was stirred for 30 min, and then 1,3-dibromopropane (1.82 g, 9.00 mmol) was added and stirres for additional 15 h at 20°C. The reaction was quenched by addition of H2O, and the product was extracted with EtOAc. The organic solution was then washed with H₂O and brine, dried over MgSO₄, filtered and concentrated *in vacuo*. 1-(3-Bromo-1-propyl)benzimidazole (530 mg, 74%) was isolated by flash chromatography on a silica gel column by elution with EtOAc/Hexanes a pale yellow oil. ¹H NMR (CDCl₃, 300 MHz) δ 2.40 (qu, 2H, J = 6.6 Hz), 3.33 (t, 2H, J = 6.2 Hz), 4.42 (t, 2H, J = 6.6 Hz), 7.12-7.20 (m, 1H), 7.27-7.48 (m, 2H), 7.43-7.49 (m, 1H), 7.78-7.86 (m, 1H) ppm.

### Step B:

The title compound was prepared by following the general coupling procedure of Example 43 as a pale yellow oil (21 mg, 43%) from (±)-*cis*-6,7,8a,9,10, 11,12,12a-octahydro-5H-pyrido[3',4':4,5]pyrrolo[1,2,3-*ef*][1,5]benzothiazepine (30 mg, 0.12 mmol) and 1-(3-chloropropyl)benzimidazole (58 mg, 0.24 mmol). ¹H NMR (CDCl₃, 300 MHz) δ 1.83-1.95 (m, 3H), 2.00-2.17 (m, 4H), 2.20-2.33 (m, 3H), 2.50-2.60 (m, 1H), 2.64-2.72 (m, 1H), 2.90-2.99 (m, 1H), 3.05-3.18 (m, 2H), 3.29-3.35 (m, 1H), 3.50-3.59 (m, 1H), 3.77-3.87 (m, 1H), 4.28 (dt, 2H, J = 2.2, 6.2 Hz), 6.63 (t, 1H, J = 7.7 Hz), 6.85 (d, 1H, J = 6.6 Hz), 6.95 (dd, 1H, J = 1.1, 7.7 Hz), 7.27-7.35 (m, 2H), 7.40-7.47 (m, 1H), 7.78-7.86 (m, 1H), 7.92 (s, 1H) ppm.

### EXAMPLE 253

### 2-[2-(cis-(8a,12a)-6,7,9,10,12,12a-hexahydro-5H-pyrido[4,3-b][1,4]thiazepino[2,3,4-hi]indol-11(8aH)-yl)ethyl]-1H-isoindole-1,3(2H)-dione

The title compound was prepared by following the general coupling procedure of Example 43 as a pale yellow oil (40 mg, 79%) from (±)-*cis*-6,7,8a,9,10, 11,12,12a-octahydro-5H-pyrido[3',4':4,5]pyrrolo[1,2,3-*ef*][1,5]benzothiazepine (30 mg, 0.12 mmol) and N-(2-bromoethyl)phthalimide (61 mg, 0.24 mmol). ¹H NMR (CDCl₃, 300 MHz) δ 1.80-2.20 (m, 5H), 2.25-2.39 (m, 1H), 2.58 (t, 2H, J = 6.6 Hz), 2.69-2.77 (m, 1H), 2.79-2.89 (m, 1H), 2.90-2.98 (m, 1H), 3.02-3.13 (m, 2H), 3.17-3.27 (m, 1H), 3.50-3.61 (m, 1H), 3.77-3.87 (m, 3H), 6.57 (t, 1H, J = 7.7 Hz), 6.84 (d, 1H, J = 7.0 Hz), 6.92 (dd, 1H, J = 1.1, 7.7 Hz), 7.68-7.78 (m, 2H), 7.82-7.89 (m, 2H) ppm.

### EXAMPLE 254

2-[2-(cis-(8a,12a)-6,7,9,10,12,12a-hexahydro-5H-pyrido[4,3-b][1,4]thiazepino[2,3,4-hi]indol-11(8aH)-yl)ethyl]-1-isoindolinone

### Step A:

Ethanolamine (4.78 g, 78.3 mmol) and phthalide (10.0 g, 74.6 mmol) were placed in a round-bottom flask equipped with a Dean-stark trap. The reaction mixture was heated at 150°C for 4 h then 18 h at 205°C. The product was solidified upon cooling. Pure 2-(2-hydroxyethyl)isoindolinone (10.8 g, 82%) was isolated by recrystalization in CHCl3 / hexanes as a white crystal. ¹H NMR (CDCl₃, 300 MHz) δ 3.02-3.15 (br, 1H), 3.78 (t, 2H, J = 5.0 Hz), 3.93 (t, 2H, J = 4.4 Hz), 4.52 (s, 2H), 7.42-7.58 (m, 3H), 7.84 (d, 1H, J = 7.4 Hz) ppm.

To a solution of 2-(2-hydroxyethyl)isoindolinone (1.0 g, 5.64 mmol) in toluene (3.5 mL) was added thionyl chloride (1.34 g, 11.3 mmol). The reaction mixture was stirred at 20°C for 3 h then 4h at 60°C. The reaction mixture was concentrated *in vacuo* to remove excess thionyl chloride and toluene. 2-(2-Chloroethyl)isoindolinone (1.01g, 92%) was obtained by flash chromatography on a silica gel column by elution with EtOAc/Hexanes a white solid. ¹H NMR (CDCl₃, 300 MHz) δ 3.81 (t, 2H, J = 5.5 Hz), 3.97 (t, 2H, J = 5.9 Hz), 4.59 (s, 2H), 7.44-7.58 (m, 3H), 7.86 (d, 1H, J = 6.9 Hz) ppm.

### Step B:

The title compound was prepared by following the general coupling procedure of Example 43 as a pale yellow oil (42 mg, 86%) from (±)-*cis*-6,7,8a,9,10, 11,12,12a-octahydro-5H-pyrido[3',4':4,5]pyrrolo[1,2,3-*ef*][1,5]benzothiazepine (30 mg, 0.12 mmol) and 2-(2-chloroethyl)isoindolinone (47 mg, 0.24 mmol). ¹H NMR (CDCl₃, 300 MHz) δ 1.80-1.95 (m, 3H), 2.00-2.18 (m, 3H), 2.37 (dt, 1H, J = 3.6, 11 Hz), 2.60 (t, 2H, J = 6.6 Hz), 2.63-2.72 (m, 1H), 2.75-2.82 (m, 1H), 2.89-2.98 (m, 1H), 3.05-3.18 (m, 2H), 3.26-3.33 (m, 1H), 3.48-3.59 (m, 1H), 3.73-3.84 (m, 3H), 4.49 (s, 2H), 6.58 (t, 1H, J = 7.3 Hz), 6.83 (d, 1H, J = 6.6 Hz), 6.94 (dd, 1H, J = 1.1, 7.7 Hz), 7.42-7.55 (m, 3H), 7.85 (d, 1H, J = 7.3 Hz) ppm.

### EXAMPLE 266

### cis-4-((6b,10a)-6-methyl-1,2,6b,9,10,10a-hexahydro[1,4]oxazino[2,3,4-hi]pyrido[4,3-b]indol-8(7H)-yl)-1-(4-fluorophenyl)-1-butanone

Treatment of cis-(6b,10a)-6-methyl-1,2,6b,7,8,9,10,10a-octahydro[1,4]oxazino[2,3,4-*hi*]pyrido[4,3-*b*]indole according to the procedure of Example 261 afforded the title compound in good yield as a viscous light brown liquid. Yield 53%. ¹H NMR (CDCl₃, 300 MHz) δ 1.82-2.10 (m, 5H), 2.18 (s, 3H), 2.20-2.35 (m, 1H), 2.43 (t, J = 6.9 Hz, 2H), 2.60-2.80 (m, 2H), 2.88-3.05 (m,1H), 2.99 (t, J = 7.3 Hz, 2H), 3.07-3.20 (m, 2H), 3.25 (d, J = 11 Hz, 1H), 4.35-4.45 (m, 2H), 6.44 (d, J = 8.1 Hz, 1H), 6.53 (d, J = 8.1 Hz, 1H), 7.13 (t, 8.4 Hz, 2H), 7.99-8.04 (m, 2H) ppm. MS (CI): 395 (M+H⁺).

### EXAMPLE 268

### 4-(cis-(8a,12a)-2-fluoro-6,7,9,10,12,12a-hexahydro-5H-pyrido[4,3-b][1,4]thiazepino[2,3,4-hi]indol-11(8aH)-yl)-1-(4-fluorophenyl)-1-butanone

Treatment of cis-(8a,12a)-2-fluoro-6,7,8a,9,10,11,12,12a-octahydro-5*H*-pyrido[4,3-*b*][1,4]thiazepino[2,3,4-*hi*]indole according to the procedure of Example 261 afforded the title compound in good yield as a viscous oil, M.P. 226-227°C. Yield 27%. ¹H NMR (CDCl₃, 300 MHz) δ 1.50-2.20 (m, 8H), 2.20-2.32 (m, 1H), 2.32-2.50 (m 1H),2.50-2.63 (m,1H), 2.63-2.78 (m, 1H), 2.78=3.30 (m, 6H), 3.45-3.60 (m, 1H), 3.60-3.77 (m,1H), 6.57 (d, J = 7.7 Hz, 1H), 6.67 (t, J = 6.2 Hz, 1H), 7.13 (t, *J* = 7.8 Hz, 2H), 7.97-8.02 (m, 2H) ppm. MS (CI): 429 (M+H⁺).

### EXAMPLE 270

### cis-(6b,10a)-8-[3-(4-fluorophenoxy)propyl]-1,2,6b,7,8,9,10,10a-octahydro[1,4]oxazino[2,3,4-hi]pyrido[4,3-b]indole

Treatment of cis-(6b,10a)-1,2,6b,7,8,9,10,10a-octahydro[1,4]oxazino[2,3,4-*hi*]pyrido[4,3-*b*]indole according to the procedure of Example 203 afforded the title compound in good yield as a viscous liquid. Yield 32%. ¹H NMR (CDCl₃, 300 MHz) δ 1.91-2.02 (m, 3H), 2.06 (t, J = 11.4 Hz, 1H), 2.26-2.40 (m, 2H), 2.40-2.60 (m, 2H), 2.65-2.80 (m, 2H), 2.80-2.95 (m, 1H), 3.05-3.22 (m, 1H), 3.22-3.32 (m, 2H), 3.98 (t, J = 6.3, Hz, 2H), 4.40-4.50 (m, 2H), 6.60-6.65 (m, 2H), 6.65-6.75 (m, 1H), 6.75-6.85 (m, 2H), 6.85-7.0 (m, 2H) ppm. MS (CI): 369 (M+H⁺).

### EXAMPLE 272

### cis-(6b,10a)-1,2,6b,7,8,9,10,10a-octahydro[1,4]oxazino[2,3,4-hi]pyrido[4,3-b]indole

Treatment of benzo[b]morpholine according to the procedure of Example 4, Step E, followed by the procedure of Example 128, Steps A-C, afforded the title compound as colorless crystals, m.p. 100-101°C. ¹H NMR (CDCl₃, 300 MHz) δ 1.77-1.95 (m, 2H), 2.15 (s, 1H), 2.61-2.85 (m, 2H), 2.85-3.00 (m,2H), 3.03-3.21 (m, 2H), 3.28-3.41 (m, 2H), 4.40-4.51 (m,2H), 6.60-6.68 (m, 2H), 6.68-6.73 (m, 1H) ppm. MS (CI): 217 (M+H⁺).

### EXAMPLE 286

### N-[2-((8aS,12aR)-6,7,9,10,12,12a-hexahydro-5H-pyrido[4,3-b][1,4]thiazepino[2,3,4- hi]indol-11(8aH)-yl)ethyl]-2,4-difluorobenzamide

### Step A:

To a solution of ethanolamine (1.10 g, 15.8 mmol) in THF at 0°C under N₂ was added 2,4-difluorobenzoyl chloride (500 mg, 2.83 mmol). The reaction mixture was stirred at 0°C for 4 h, then diluted with ethyl acetate (100 mL) and washed with 1 N HCl (50 mL), saturated NaHCO₃ (50 mL), and saturated NaCl (50 mL). The organic solution was dried over MgSO₄ and concentrated *in vacuo* to yield 2,4-difluoro-*N*-(2-hydroxyethyl)benzamide as a white solid (495 mg, 87%).

### Step B:

To a solution of 2,4-difluoro-*N*-(2-hydroxyethyl)benzamide (299 mg, 1.49 mmol) and triethylamine (302 mg, 2.98 mmol) in CH₂Cl₂ under nitrogen at 0°C was added methanesulfonyl chloride (335 mg, 2.98 mmol) The reaction mixture was stirred for 1 h at 0°C. 1 N HCl (5 mL) was added to quench the reaction and the solution was diluted with ethyl acetate (100 mL), washed with saturated NaHCO₃ (100 mL) and saturated NaCl (100 mL), dried over MgSO₄, filtered and concentrated *in vacuo*. Purification by column chromatography (hexanes:EtOAc 4:1) yielded 2-[(2,4-difluorobenzoyl)amino]ethyl methanesulfonate (300 mg, 72%) as a clear liquid.

### Step C:

To a solution of (8a*S*,12a*R*)-6,7,8a,9,10,11,12,12a-octahydro-5*H*-pyrido[4,3-*b*][1,4]thiazepino[2,3,4-*hi*]indole (30 mg, 0.12 mmol) and 2-[(2,4-difluorobenzoyl)amino]ethyl methanesulfonate (102 mg, .0.36 mmol) in 1,4-dioxane (0.6 mL) were added K₂CO₃ (24 mg, 0.17 mmol) and KI (catalytic amount) and the reaction mixture was stirred at 100°C for 48 h. The reaction mixture was diluted with CHCl₃ (50 mL) and filtered. The title compound was isolated as a light yellow oil (43 g, 77%) after column purification (CHCl₃:MeOH 99:1). ¹H NMR (CDCl₃) δ 1.65 (br-s,1H), 1.86-2.13 (m, 5H), 2.39 (td, 1H, *J* = 3.3, 11.0 Hz), 2.52-2.66 (m, 3H), 2.72-2.78 (m, 1H), 2.93-3.00 (m, 1H), 3.08-3.18 (m, 2H), 3.28-3.33 (m, 1H), 3.49-3.59 (m, 3H), 3.77-3.85 (m, 1H), 6.59 (t, 1H, *J* = 7.4 Hz), 6.82-7.03 (m, 4H), 8.10-8.18 (m, 1H) ppm.

### EXAMPLE 287

### N-[2-((8aS,12aR)-6,7,9,10,12,12a-hexahydro-5H-pyrido[4,3-b][1,4]thiazepino[2,3,4- hi]indol-11(8aH)-yl)ethyl]-N-methylbenzamide

2-(Benzoyl(methyl)amino)ethyl methanesulfonate (987 mg, 49%) was prepared from benzoyl chloride (545 mg, 3.6 mmol) and 2(methylamino)ethanol (1.35 g, 18 mmol) according to the procedure of Example 286, Steps A and B. The title compound was isolated as a yellow oil according to the method of Example 286, Step C (35mg, 33%) from (8a*S*,12a*R*)-6,7,8a,9,10,11,12,12a-octahydro-5*H*-pyrido[4,3-*b*][1,4]thiazepino[2,3,4-*hi*]indole (30mg, 0.12mmol) and 2-(benzoyl(methyl)amino)ethyl methanesulfonate (48 mg, 0.24 mmol). ¹H NMR (CDCl₃) δ 1.69-1.95 (m, 3H), 2.01-2.19 (m, 2H), 2.34-2.51 (m, 2H), 2.57-2.81 (m, 2H), 2.93-3.10 (m, 6H), 3.25-3.58 (m, 2H), 2.61-2.85 (m, 3H), 6.61 (t, 1H, *J* = 7.5 Hz), 6.94 (d, 1H, *J* = 7.7 Hz), 7.26-7.50 (m, 4H) ppm. MS (ESI): 408 (base, M + H).

### EXAMPLE 288

### N-[2-((8aS,12aR)-6,7,9,10,12,12a-hexahydro-5H-pyrido[4,3-b][1,4]thiazepino[2,3,4- hi]indol-11(8aH)-yl)ethyl]-2-fluoro-N-methylbenzamide

2-[(2-Fluorobenzoyl(methyl)amino)ethyl methanesulfonate (531 mg, 85%) was prepared from 2-fluorobenzoyl chloride (1.12 g, 6.4 mmol) and 2(methylamino)ethanol (2.70 g, 32 mmol) according to the procedure of Example 286, Steps A and B. The title compound was isolated as a yellow oil according to the method of Example 286, Step C (35mg, 66%) from (8a*S*,12a*R*)-6,7,8a,9,10,11,12,12a-octahydro-5*H*-pyrido[4,3-*b*][1,4]thiazepino[2,3,4-*hi*]indole (30 mg, 0.12 mmol) and 2-[(2-fluorobenzoyl)(methyl)amino)ethyl methanesulfonate (48 mg, 0.24 mmol). ¹H NMR (CDCl₃) δ 1.65-1.93 (m, 1H), 2.01-2.17 (m, 3H), 2.25-2.44 (m, 3H), 2.54-2.91 (m, 3H), 2.95-3.05 (m, 3H), 3.10-3.22 (m, 3H), 3.27-3.40 (m, 1H), 3.45-3.90 (m, 4H), 6.58-6.64 (m, 1H), 6.81 (dd, 1H, *J* = 6.6, 38.9Hz), 6.92-6.96 (m, 1H), 7.06-7.19 (m, 1H), 7.19 (t, 1H, *J* = 6.9 Hz), 7.32-7.40 (m, 2H) ppm. MS (ESI): 426 (base, M + H).

### EXAMPLE 289

### N-[2-((8aS,12aR)-6,7,9,10,12,12a-hexahydro-5H-pyrido[4,3-b][1,4]thiazepino[2,3,4- hi]indol-11(8aH)-yl)ethyl]-2,4-difluoro-N-methylbenzamide

2-[(2,4-Difluorobenzoyl(methyl)amino)ethyl methanesulfonate (482 mg, 86%) was prepared from 2,4-difluorobenzoyl chloride (1.04 g, 5.9 mmol) and 2(methylamino)ethanol (2.13 g, 28 mmol) according to the procedure of Example 286, Steps A and B. The title compound was isolated as a yellow oil according to the method of Example 286, Step C (17 mg, 31%) from (8a*S*,12a*R*)-6,7,8a,9,10,11,12,12a-octahydro-5*H*-pyrido[4,3-*b*][1,4]thiazepino[2,3,4-*hi*]indole (30 mg, 0.12 mmol) and 2-[(2,4-difluorobenzoyl)(methyl)amino)ethyl methanesulfonate (57 mg, 0.24 mmol). ¹H NMR (CDCl₃) δ 1.89-2.01 (m, 2H), 2.05-2.14 (m, 2H), 2.38-2.52 (m, 3H), 3.55-3.78 (m, 2H), 2.81-2.91 (m, 1H), 2.94-3.07 (m, 3H), 3.10-3.19 (m, 2H), 3.24-3.36 (m, 2H), 3.45-3.60 (m, 1H), 3.61-3.85 (m, 3H), 6.61 (t, 1H, *J* = 7.5 Hz), 6.74-6.96 (m, 4H), 7.33-7.38 (m, 1H) ppm.

### EXAMPLE 290

*N*-[2-((8a*S*,12a*R*)-6,7,9,10,12,12a-hexahydro-5*H*-pyrido[4,3-*b*][1,4]thiazepino[2,3,4- *hi*]indol-11(8a*H*)-yl)ethyl]-4-fluoro-*N*-methylbenzamide

2-[(4-Fluorobenzoyl(methyl)amino)ethyl methanesulfonate (482 mg, 86%) was prepared from 4-fluorobenzoyl chloride (1.12 g, 3.2 mmol) and 2(methylamino)ethanol (2.70 g, 16 mmol) according to the procedure of Example 286, Steps A and B. The title compound was isolated as a yellow oil according to the method of Example 286, Step C (37mg, 69%) from (8a*S*,12a*R*)-6,7,8a,9,10,11,12,12a-octahydro-5*H*-pyrido[4,3-*b*][1,4]thiazepino[2,3,4-*hi*]indole (30 mg, 0.12 mmol) and 2-[(4-fluorobenzoyl)(methyl)amino)ethyl methanesulfonate (53 mg, 0.24 mmol). ¹H NMR (CDCl₃) δ 1.73-1.95 (m, 3H), 1.98-2.21 (m, 3H), 2.35-2.55 (m, 3H), 3.60-3.78 (m, 1H), 2.86-2.97 (m, 1H), 2.98-3.21 (m, 5H), 3.27-3.45 (m, 1H), 3.52-3.59 (m, 2H), 3.72-3.86 (m, 2H), 6.62 (t, 1H, *J* = 7.5 Hz), 6.71-6.83 (m, 1H), 6.95 (dd, 1H, *J* = 1.1 7.8 Hz), 7.08 (t, 2H, J = 8.6 Hz), 7.30-7.50 (m, 2H) ppm.

### EXAMPLE 291

### (8aS,12aR)-11-[3-(1H-1,2,3-benzotriazol-1-yl)propyl]-6,7,8a,9,10,11,12,12a- octahydro-5H-pyrido[4,3-b][1,4]thiazepino[2,3,4-hi]indole

### Step A:

To a solution of benzotriazole (303 mg, 2.54 mmol) and powdered NaOH (101mg, 2.52mmol) in DMSO was added 1-bromo-3-chloro propane (437 mg, 2.77 mmol). The reaction mixture was stirred for 16 h at 20°C. The reaction mixture was then diluted with EtOAc (100 mL) and washed with H₂O (100 mL) and saturated NaCl (100 mL), dried over MgSO₄ and concentrated *in vacuo*. Column purification (hexanes:EtOAc 4:1) yielded 1-(3-chloropropyl)-1*H*-1,2,3-benzotriazole (167mg, 34%). The title compound was prepared according to Example 286, Step C as a yellow oil (21 mg, 42%) from (8a*S*,12a*R*)-6,7,8a,9,10,11,12,12a-octahydro-5*H*-pyrido[4,3-*b*][1,4]thiazepino[2,3,4-*hi*]indole (30 mg, 0.12 mmol) and 1-(3-chloropropyl)-1*H*-1,2,3-benzotriazole (48 mg, 0.24 mmol). ¹H NMR (CDCl₃) δ 1.88-1.98 (m, 3H), 1.99-2.18 (m, 2H), 2.20-2.41 (m, 5H), 2.51-2.60 (m, 1H), 2.64-2.71 (m, 1H), 2.89-3.00 (m, 1H), 3.03-3.13 (m, 2H), 3.21-3.27 (m, 1H), 3.47-3.60 (m, 1H), 3.76-3.88 (m, 1H), 4.73 (t, 2H, *J =* 6.6 Hz), 6.61 (t, 1H, *J =* 7.4 Hz), 6.84 (d, 1H, *J =* 6.6 Hz), 6.94 (dd, 1H, *J =* 1.1, 8.1 Hz), 7.37 (td, 1H, *J =* 1.1, 7.4 Hz), 7.49 (td, 1H, *J =* 1.1, 7.8 Hz), 7.57 (d, 1H, *J =* 8.0 Hz), 8.07 (d, 1H, *J* = 8.4 Hz) ppm.

### EXAMPLE 292

### (8aS,12aR)-11-[3-(2H-1,2,3-benzotriazol-2-yl)propyl]-6,7,8a,9,10,11,12,12a- octahydro-5H-pyrido[4,3-b][1,4]thiazepino[2,3,4-hi]indole

Benzotriazole (303 mg, 2.54 mmol) and 1-bromo-3-chloro propane (437 mg, 2.77 mmol) were used according to the method of Example 291, Step A to yield 2-(3-chloropropyl)-1*H*-1,2,3-benzotriazole (182mg, 37%). The title compound was prepared according to Example 286, Step C as a yellow oil (20 mg, 40%) from (8aS,12aR)-6,7,8a,9,10,11,12,12a-octahydro-5*H*-pyrido[4,3-*b*][1,4]thiazepino[2,3,4-*hi*]indole (30 mg, 0.12 mmol) and 2-(3-chloropropyl)-1*H*-1,2,3-benzotriazole (48 mg, 0.24 mmol). ¹H NMR (CDCl₃) δ 1.84-1.94 (m, 3H), 1.99-2.20 (m, 2H), 2.25-2.52 (m, 5H), 2.64-2.72 (m, 1H), 2.74-2.82 (m, 1H), 2.88-3.00 (m, 1H), 3.02-3.18 (m, 2H), 3.22-3.28 (m, 1H), 3.51-3.61 (m, 1H), 3.74-3.85 (m, 1H), 4.80 (t, 2H, *J* = 6.7 Hz), 6.61 (t, 1H, *J* = 7.5 Hz), 6.92 (dd, 2H, *J* = 6.6, 24.9 Hz), 7.35-7.41 (m, 2H), 7.83-7.85 (m, 2H) ppm.

### EXAMPLE 293

### (8aS,12aR)-11-{[(2S)-1-benzoylpyrrolidinyl]methyl}-6,7,8a,9,10,11,12,12a- octahydro-5H-pyrido[4,3-b][1,4]thiazepino[2,3,4-hi]indole

[(2*S*)-1-Benzoylpyrrolidinyl]methyl methanesulfonate (94 mg, 50%) was prepared from (*S*)-2-pyrrolidine-methanol (150 mg, 1.48 mmol) and benzoyl chloride (208 mg, 1.48 mmol) as in Example 286, Steps A-B. The title compound was isolated as a yellow oil (52 mg, 100%) according to the method of Example 286, Step C from (8a*S*,12a*R*)-6,7,8a,9,10,11,12,12a-octahydro-5*H*-pyrido[4,3-*b*][1,4]thiazepino[2,3,4-*hi*]indole (30 mg, 0.12 mmol) and [(2*S*)-1-benzoylpyrrolidinyl]methyl methanesulfonate (55 mg, 0.24 mmol). ¹H NMR (CDCl₃) δ 1.26 (s, 1H), 1.58-2.30 (m, 8H), 2.35-2.86 (m, 3H), 2.90-3.30 (m, 4H), 3.37-3.61 (m, 4H), 3.75-3.86 (m, 2H), 4.38-4.50 (m, 1H), 6.55-6.96 (m, 2H), 7.31-7.96 (m, 6H) ppm. MS (ESI): 434 (base, M + H).

### EXAMPLE 294

### (8aS,12aR)-11-{[(2R)-1-benzoylpyrrolidinyl]methyl}-6,7,8a,9,10,11,12,12a- octahydro-5H-pyrido[4,3-b][1,4]thiazepino[2,3,4-hi]indole

[(2*R*)-1-Benzoylpyrrolidinyl]methyl methanesulfonate (98 mg, 52%) was prepared from (*R*)-2-pyrrolidine-methanol (150 mg, 1.48 mmol) and benzoyl chloride (208 mg, 1.48 mmol) as in Example 286, Steps A-B. The title compound was isolated as a yellow oil (36 mg, 68%) according to the method of Example 286, Step C from (8a*S*,12a*R*)-6,7,8a,9,10,11,12,12a-octahydro-5*H*-pyrido[4,3-*b*][1,4]thiazepino[2,3,4-*hi*]indole (30 mg, 0.12 mmol) and [(2*R*)-1-benzoylpyrrolidinyl]methyl methanesulfonate (55 mg, 0.24 mmol). ¹H NMR (CDCl₃) δ 1.58-2.27 (m, 12H), 2.64-3.30 (m, 4H), 3.49-3.61 (m, 3H), 3.72-3.84 (m, 2H), 6.60 (td, 1H, *J* = 1.9, 7.5 Hz), 6.93 (d, 1H, *J* = 7.5 Hz), 7.31-7.55 (m, 6H) ppm. MS (ESI): 434 (base, M + H).

### EXAMPLE 295

### (8aS,12aR)-11-{[(2S)-1-(4-fluorobenzoyl)pyrrolidinyl]methyl}-6,7,8a,9,10,11,12,12a-octahydro-5H-pyrido[4,3-b][1,4]thiazepino[2,3,4-hi]indole

[(2*S*)-1-(4-Fluorobenzoyl)pyrrolidinyl]methyl methanesulfonate (152 mg, 68%) was prepared from (*S*)-2-pyrrolidine-methanol (155 mg, 1.53 mmol) and 4-fluorobenzoyl chloride (235 mg, 1.48 mmol) as in Example 286, Steps A-B. The title compound was isolated as a yellow oil (24 mg, 44%) according to the method of Example 286, Step C from (8a*S*,12a*R*)-6,7,8a,9,10,11,12,12a-octahydro-5*H*-pyrido[4,3-*b*][1,4]thiazepino[2,3,4-*hi*]indole (30 mg, 0.12 mmol) and [(2*S*)-1-(4-fluorobenzoyl)pyrrolidinyl]methyl methanesulfonate (59 mg, 0.24 mmol). ¹H NMR (CDCl₃) δ 1.63 (s, 2H), 1.70-2.20 (m, 10H), 2.67-3.31 (m, 5H), 3.37-3.55 (m, 4H), 3.71-3.82 (m, 2H), 6.61 (td, 1H, *J* = 2.0, 7.5 Hz), 6.93 (d, 1H, *J* = 7.7 Hz), 7.03-7.18 (m, 3H), 7.45-7.56 (m, 2H) ppm. MS (ESI): 452 (base, M + H).

### EXAMPLE 296

### (8aS,12aR)-11-{[(2R)-1-(4-fluorobenzoyl)pyrrolidinyl]methyl}-6,7,8a,9,10,11,12,12a-octahydro-5H-pyrido[4,3-b][1,4]thiazepino[2,3,4-hi]indole

[(2*R*)-1-(4-Fluorobenzoyl)pyrrolidinyl]methyl methanesulfonate (147 mg, 80%) was prepared from (*R*)-2-pyrrolidine-methanol (155 mg, 1.53 mmol) and 4-fluorobenzoyl chloride (235 mg, 1.48 mmol) as in Example 286, Steps A-B. The title compound was isolated as a yellow oil (27 mg, 49%) according to the method of Example 286, Step C from (8a*S*,12a*R*)-6,7,8a,9,10,11,12,12a-octahydro-5*H*-pyrido[4,3-*b*][1,4]thiazepino[2,3,4-*hi*]indole (30 mg, 0.12 mmol) and [(2*R*)-1-(4-fluorobenzoyl)pyrrolidinyl]methyl methanesulfonate (59 mg, 0.24 mmol). ¹H NMR (CDCl₃) δ 1.59-2.21 (m, 11H), 2.94-3.35 (m, 4H), 3.47-3.60 (m, 4H), 3.73-3.84 (m, 2H), 4.39-4.47 (1H, m), 4.77-4.02 (m, 1H), 6.61 (td, 1H, *J* = 1.8, 7.4 Hz), 6.83 (d, 1H, *J* = 9.5 Hz), 7.01-7.14 (m, 3H), 7.47-7.64 (m, 2H) ppm.

### EXAMPLE 297

### (8aS,12aR)-11-[2-(1H-1,2,3-benzotriazol-1-yl)ethyl]-6,7,8a,9,10,11,12,12a- octahydro-5H-pyrido[4,3-b][1,4]thiazepino[2,3,4-hi]indole

1-(2-Chloroethyl)-1*H*-1,2,3-benzotriazole (164mg, 36%) was prepared from benzotriazole (300 mg, 2.52 mmol) and 1-bromo-2-chloro ethane (397 mg, 2.77 mmol) according to the procedure of Example 291. The title compound was isolated as a yellow oil (48 mg, 98%) according to the method of Example 286, Step C from (8a*S*,12a*R*)-6,7,8a,9,10,11,12,12a-octahydro-5*H*-pyrido[4,3-*b*][1,4]thiazepino[2,3,4-*hi*]indole (30 mg, 0.12 mmol) and 1-(2-chloroethyl)-1*H*-1,2,3-benzotriazole (44 mg, 0.24 mmol). ¹H NMR (CDCl₃) δ 1.69-2.20 (m, 4H), 2.39-2.45 (m, 1H), 2.61-2.68 (m, 1H), 2.72-2.81 (m, 1H), 2.84-3.01 (m, 2H), 3.03-3.12 (m, 2H), 3.20-3.27 (m, 1H), 3.49-3.60 (m, 1H), 3.74-3.84 (m, 1H), 4.06 (t, 1H, *J* = 6.7 Hz), 4.76 (t, 1H, *J* = 6.6 Hz), 4.96 (t, 1H, *J* = 6.2 Hz), 6.61 (t, 1H, *J* = 7.5 Hz), 6.82 (d, 1H, *J* = 7 Hz), 6.95 (dd, 1H, *J* = 1.1, 7.7 Hz), 7.34-7.62 (m, 3H), 8.08 (t, 1H, *J* = 7.6 Hz) ppm.

### EXAMPLE 298

### (8aS,12aR)-11-[2-(2H-1,2,3-benzotriazol-2-yl)propyl]-6,7,8a,9,10,11,12,12a- octahydro-5H-pyrido[4,3-b][1,4]thiazepino[2,3,4-hi]indole

2-(3-Chloropropyl)-1*H*-1,2,3-benzotriazole (200 mg; 44%) was prepared from benzotriazole (300 mg, 2.52 mmol) and 1-bromo-3-chloro propane (397 mg, 2.77 mmol) according to the procedure of Example 291. The title compound was isolated as a yellow oil (26 mg, 54%) according to the method of Example 286, Step C from (8a*S*,12a*R*)-6,7,8a,9,10,11,12,12a-octahydro-5*H*-pyrido[4,3-*b*][1,4]thiazepino[2,3,4-*hi*]indole (30 mg, 0.12 mmol) and 2-(3-chloropropyl)-1*H*-1,2,3-benzotriazole (44 mg, 0.24 mmol). ¹H NMR (CDCl₃) δ 1.81-1.93 (m, 2H), 1.96-2.14 (m, 3H), 2.41 (td, 1H, J = 3.7 , 11.7 Hz), 2.64-2.72 (m, 1H), 2.80-2.90 (m, 1H), 2.92-3.00 (m, 1H), 3.02-3.16 (m, 4H), 3.21-3.26 (m, 1H), 3.51-3.62 (m, 1H), 3.78-3.86 (m, 1H), 4.85 (t, 2H, *J* = 7.0 Hz), 6.61 (t, 1H, *J* = 7.5 Hz), 6.83 (d, 1H, *J* = 6.6 Hz), 6.94 (dd, 1H, *J* = 1.2, 8.1 Hz), 7.36-7.41 (m, 2H), 7.84-7.88 (m, 2H) ppm.

### EXAMPLE 299

### (8aS,12aR)-11-[3-(3,4-dihydro-1(2H)-quinolinyl)propyl]-6,7,8a,9,10,11,12,12a- octahydro-5H-pyrido[4,3-b][1,4]thiazepino[2,3,4-hi]indole

To a solution of 1,2,3,4-tetrahydroquinoline (505 mg, 3.79 mmol) and 1-bromo-3-chloro propane (1.77 g, 11 mmol) in 1,4-dioxane (6 mL) was added Et₃N (1.90 g, 19 mmol). The reaction mixture was stirred at 70°C for 17 h, and quenched by addition of H₂O (2 mL). Reaction was diluted with Et₂O (100 mL) and washed with brine (100 mL), dried over MgSO₄ and concentrated *in vacuo*. Purification by column chromatography (hexanes:EtOAc 49:1) yielded 1-(3-chloropropyl)-1,2,3,4-tetrahydroquinoline (187 mg, 24%) as a colorless oil. The title compound was isolated as a yellow oil (22 mg, 43%) according to the method of Example 286, Step C from (8a*S*,12a*R*)-6,7,8a,9,10,11,12,12a-octahydro-5*H*-pyrido[4,3-*b*][1,4]thiazepino[2,3,4-*hi*]indole (30 mg, 0.12 mmol) and 1-(3-chloropropyl)-1,2,3,4-tetrahydroquinoline (51 mg, 0.24 mmol). ¹H NMR (CDCl₃) δ 1.77-2.19 (m, 10H), 2.24-2.41 (m, 2H), 2.61-2.69 (m, 1H), 2.75-2.84 (m, 3H), 2.91-3.02 (m, 1H), 3.10-3.21 (m, 2H), 3.26-3.34 (m, 5H), 3.52-3.61 (m, 1H), 3.77-3.96 (m, 1H), 6.49-6.65 (m, 3H), 6.85 (d, 1H, *J* = 7.3 Hz), 6.92-6.96 (m, 2H), 6.99-7.05 (m, 1H) ppm.

### EXAMPLE 300

### (8aS,12aR)-11-[(3E)-4-(4-fluorophenyl)3-pentenyl]-6,7,8a,9,10,11,12,12a- octahydro-5H-pyrido[4,3-b][1,4]thiazepino[2,3,4-hi]indole

### Step A:

To a solution of cyclopropyl-4-fluorophenyl ketone (523 mg, 3.19 mmol) in THF (10 mL) at 0°C under N₂ was added 3 M methyl magnesium bromide (1.7 mL, 5.1 mmol) in Et₂O. After 90 minutes at 0°C, the reaction was quenched by addition of brine (10 mL) and diluted with Et₂O (100 mL). The organic solution was washed with saturated NaHCO₃ (100 mL) and brine (100 mL). Column purification (hexanes:EtOAc 9:1) yielded 1-cyclopropyl-1-(4-fluorophenyl)ethanol (512 mg, 89%) as a colorless oil.

### Step B:

1-Cyclopropyl-1-(4-fluorophenyl)ethanol (307 mg, 1.70 mmol) in a solution of 1 N HCl in isopropyl alcohol (3.6 mL) was heated at 60°C for 1 h. The reaction mixture was then concentrated *in vacuo*, and column chromatography purification yielded 1-[(1*E*)-4-chloro-1-methyl-1-butenyl]-4-fluorobenzene (282 mg, 84%) as a colorless oil. The title compound was isolated as a yellow oil (36 mg, 70%) according to the method of Example 286, Step C from (8a*S*,12a*R*)-6,7,8a,9,10,11,12,12a-octahydro-5*H*-pyrido[4,3-*b*][1,4]thiazepino[2,3,4-*hi*]indole (30 mg, 0.12 mmol) and 1-[(1*E*)-4-chloro-1-methyl-1-butenyl]-4-fluorobenzene (48 mg, 0.24 mmol). ¹H NMR (CDCl₃) δ 1.62 (br-s, 1H), 1.90-2.20 (m, 9H), 2.31-2.44 (m, 5H), 2.71-2.80 (m, 1H), 2.81-2.89 (m, 1H), 2.92-3.00 (m, 1H), 3.04-3.13 (m, 1H), 3.17-3.24 (m, 2H), 3.27-3.31 (m, 1H), 3.51-3.63 (m, 1H), 3.80-3.91 (m, 1H), 5.64-5.73 (m, 1H), 6.62 (t, 1H, *J* = 7.5 Hz), 6.87 (d, 1H, *J* = 6.6 Hz), 6.91-7.00 (m, 3H), 7.29-7.34 (m, 2H) ppm)

### EXAMPLE 301

### (8aS,12aR)-11-[2-(2,3-dihydro-1H-inden-2-yl)ethyl]-6,7,8a,9,10,11,12,12a- octahydro-5H-pyrido[4,3-b][1,4]thiazepino[2,3,4-hi]indole

### Step A:

To a slurry of NaH (400 mg, 17 mmol) in DME (25 mL)at 20°C under N₂ was added triethylphosphonoacetate (3.39 g, 15 mmol). The reaction mixture was stirred for 45 m. To this white solution was added a solution of 2-indanone (2.00 g, 15 mmol) in DME (5 mL), maintaining the temperature at less than 25°C. The reaction mixture was stirred for 30 m and then quenched with H₂O (5 mL). The reaction mixture was extracted using Et₂O (3 x 100 mL). The organic solution was then washed with H₂O (200 mL), saturated NaHCO₃ (200 mL), and brine (200 mL), dried over MgSO₄ and concentrated *in vacuo*. Ethyl 1,3-dihydro-2*H*-inden-2-ylideneacetate (2.04 g, 67%) was isolated as a yellow oil.

### Step B:

To a solution of ethyl 1,3-dihydro-2*H*-inden-2-ylideneacetate (302 mg, 1.49 mmol) in EtOAc (10 mL) was added 5% Pd/C (76 mg) and H₂ was bubbled through the slurry for 20 h. The slurry was then filtered through Celite and the organic solution was concentrated *in vacuo*. Ethyl 2,3-dihydo-1*H*-inden-2-ylacetate (285 mg, 94%) was isolated as a colorless oil without purification.

### Step C:

To a solution of ethyl 2,3-dihydo-1*H*-inden-2-ylacetate (285 mg, 1.39 mmol) in Et₂O (5 mL) at 0°C under N₂ was added LAH (53 mg, 1.39 mmol). After stirring for 40 minutes at 0 °C, 0.4 mL of H₂O was added to quench the reaction. The reaction mixture was diluted with EtOAc (100mL) and MgSO₄ was added with stirring. The reaction mixture was then filtered and concentrated *in vacuo* to a clear oil. 2-(2,3-dihydro-1*H*-inden-2-yl)ethanol was isolated without further purification.

2-(2,3-Dihydro-1*H*-inden-2-yl)ethyl methanesulfonate (128 mg, 98%) was isolated as a clear oil by the method of Example 286, Step B from 2-(2,3-dihydro-1*H*-inden-2-yl)ethanol (88 mg, 0.54 mmol). The title compound was isolated as a yellow oil (28 mg, 61%) according to the method of Example 286, Step C from (8a*S*,12a*R*)-6,7,8a,9,10,11,12,12a-octahydro-5*H*-pyrido[4,3-*b*][1,4]thiazepino[2,3,4-*hi*]indole (30 mg, 0.12 mmol) and 2-(2,3-dihydro-1*H*-inden-2-yl)ethyl methanesulfonate (59 mg, 0.24 mmol). ¹H NMR (CDCl₃) δ 1.65-1.79 (m, 3H), 1.82-1.94 (m, 3H), 2.00-2.21 (m, 2H), 2.24-2.35 (m, 1H), 2.36-2.51 (m, 2H), 2.57-2.78 (m, 3H), 2.77-2.85 (m, 1H), 2.89-2.99 (m, 1H), 3.02-3.14 (m, 3H), 3.17-3.22 (m, 1H), 3.28-3.32 (m, 1H), 3.57-3.63 (m, 1H), 3.80-3.92 (m, 1H), 6.62 (t, 1H, *J* = 7.5 Hz), 6.86 (d, 1H, *J* = 6.6 Hz), 6.95 (dd, 1H, *J* = 1.1, 7.7 Hz), 7.10-7.19 (m, 4H) ppm.

### EXAMPLE 302

4-((8a*S*,12a*R*)-6,7,,9,10,,12,12a-hexahydro-5*H*-pyrido[4,3-*b*][1,4]thiazepino[2,3,4-*hi*]indol-11(8a*H*)-yl)-1-(2-aminophenyl)-1-butanone

### Step A:

To a solution of BCl₃-Me₂S (2.12 g, 12 mmol) in benzene (10 mL) under N₂ and ice cooling was added a solution of aniline (1.00 g, 11 mmol) in benzene (10 mL). This was stirred for 30 minutes and 4-chlorobutyronitrile (1.33 g, 12.8 mmol) was added, followed immediately by the addition of AlCl₃ (1.57 g, 12 mmol) was added in one portion. The reaction mixture was then refluxed for 16 h. The reaction mixture was cooled to 0°C and 2 N HCl (16ml) was added dropwise, then heated to 80°C and stirred for 1 h. The reaction mixture was extracted with CHCl₃ (3 x 100 mL), washed with H₂O (100 mL) and brine (100 mL), dried over MgSO₄ and concentrated *in vacuo*. Purification by column chromatography afforded 1-(2-aminophenyl)-4-chloro-1-butanone as a yellow solid (394 mg, 19%).

### Step B:

The title compound was isolated as a yellow oil (19 mg, 37%) according to the method of Example 286, Step C from (8a*S*,12a*R*)-6,7,8a,9,10,11,12,12a-octahydro-5*H*-pyrido[4,3-*b*][1,4]thiazepino[2,3,4-*hi*]indole (30 mg, 0.12 mmol) and 1-(2-aminophenyl)-4-chloro-1-butanone (48 mg, 0.24 mmol). ¹H NMR (CDCl₃) δ 1.85-2.21 (m, 7H), 2.23-2.37 (n, 1H), 2.37-2.44 (m, 2H), 2.63-2.70 (m, 1H), 2.71-2.82 (m, 1H), 2.94-3.16 (m, 5H), 3.24-3.29 (m, 1H), 3.53-3.62 (m, 1H), 3.78-3.86 (m, 1H), 6.26 (br-s, 2H), 6.59-6.67 (m, 3H), 6.85 (d, 1H, *J* = 7.4 Hz), 6.94 (dd, 1H, *J* = 1.1, 8. 1 Hz), 7.23-7.29 (m, 1H), 7.77 (dd, 1H, *J* = 1.5, 8.4 Hz) ppm.

### EXAMPLE 303

4-((8a*R*,12a*S*)-6,7,,9,10,,12,12a-hexahydro-5*H*-pyrido[4,3-*b*][1,4]thiazepino[2,3,4-*hi*]indol-11(8a*H*)-yl)-1-(2-aminophenyl)-1-butanone

The title compound was isolated as a yellow oil (45 mg, 18%) according to the method of Example 286, Step C from (8a*R*,12a*S*)-6,7,8a,9,10,11,12,12a-octahydro-5*H*-pyrido[4,3-*b*][1,4]thiazepino[2,3,4-*hi*]indole (150 mg, 0.61 mmol) and 1-(2-aminophenyl)-4-chloro-1-butanone (241 mg, 1.2 mmol). ¹H NMR (CDCl₃) δ 1.85-2.21 (m, 7H), 2.23-2.37 (n, 1H), 2.37-2.44 (m, 2H), 2.63-2.70 (m, 1H), 2.71-2.82 (m, 1H), 2.94-3.16 (m, 5H), 3.24-3.29 (m, 1H), 3.53-3.62 (m, 1H), 3.78-3.86 (m, 1H), 6.26 (br-s, 2H), 6.59-6.67 (m, 3H), 6.85 (d, 1H, *J* = 7.4 Hz), 6.94 (dd, 1H, *J* = 1.1, 8.1 Hz), 7.23-7.29 (m, 1H), 7.77 (dd, 1H, *J* = 1.5, 8.4 Hz) ppm.

### EXAMPLE 304

### 4-((8aS,12aR)-6,7,,9,10,,12,12a-hexahydro-5H-pyrido[4,3-b][1,4]thiazepino[2,3,4-hi]indol-11(8aH)-yl)-1-(2-amino-5-fluorophenyl)-1-butanone

1-(2-Amino-5-fluorophenyl)-4-chloro-1-butanone (462 mg, 24%) was afforded as a yellow solid according to the procedure of Example 302, Step A from 4-fluoroaniline (1.00g, 9.0 mmol). The title compound was isolated as a yellow oil (13 mg, 25%) according to the method of Example 286, Step C from (8a*S*,12a*R*)-6,7,8a,9,10,11,12,12a-octahydro-5*H*-pyrido[4,3-*b*][1,4]thiazepino[2,3,4-*hi*]indole (30 mg, 0.12 mmol) and 1-(2-amino-5-fluorophenyl)-4-chloro-1-butanone (53 mg, 0.24 mmol). ¹H NMR (CDCl₃) δ 1.83-2.20 (m, 7H), 2.24-2.42 (m, 3H), 2.62-2.72 (m, 1H), 2.74-2.83 (m, 1H), 2.87-3.00 (m, 3H), 3.02-3.18 (m, 2H), 3.24-3.30 (m, 1H), 3.51-3.63 (m, 1H), 3.78-3.87 (m, 1H), 6.12 (br-s, 2H), 6.57-6.63 (m, 2H), 6.85 (d, 1H, *J* = 6.6 Hz), 6.94 (dd, 1H, *J* = 1.1, 7.7 Hz), 7.01-7.08 (m, 1H), 7.47 (dd, 1H, *J* = 2.7, 10.1 Hz) ppm.

### EXAMPLE 305

### 4-((8aS,12aR)-6,7,,9,10,,12,12a-hexahydro-5H-pyrido[4,3-b][1,4]thiazepino[2,3,4-hi]indol-11(8aH)-yl)-1-(2-amino-3-fluorophenyl)-1-butanone

1-(2-Amino-3-fluorophenyl)-4-chloro-1-butanone (238 mg, 12%) was afforded as a yellow solid according to the procedure of Example 302, Step A from 2-fluoroaniline (1.00g, 9.0 mmol). The title compound was isolated as a yellow oil (12 mg, 23%) according to the method of Example 286, Step C from (8a*S*,12a*R*)-6,7,8a,9,10,11,12,12a-octahydro-5*H*-pyrido[4,3-*b*][1,4]thiazepino[2,3,4-*hi*]indole (30 mg, 0.12 mmol) and 1-(2-amino-3-fluorophenyl)-4-chloro-1-butanone (53 mg, 0.24 mmol). ¹H NMR (CDCl₃) δ 1.87-2.19 (m, 7H), 2.24-2.45 (m, 3H), 2.63-2.69 (m, 1H), 2.72-2.81 (m, 1H), 2.93-3.15 (m, 5H), 3.24-3.30 (m, 1H), 3.50-3.61 (m, 1H), 3.78-3.86 (m, 1H), 6.32 (br-s, 2H), 655-6.64 (m, 2H), 6.84 (d, 1H, J = 7.4 Hz), 6.94 (dd, 1H, *J* = 1.1, 7.7 Hz), 7.07-7.14 (m, 1H), 7.56 (d, 1H, *J* = 8.4 Hz) ppm.

### EXAMPLE 306

### 4-((8aS,12aR)-6,7,,9,10,,12,12a-hexahydro-5H-pyrido[4,3-b][1,4]thiazepino[2,3,4-hi]indol-11(8aH)-yl)-1-(2-amino-4-chlorophenyl)-1-butanone

1-(2-Amino-4-chlorophenyl)-4-chloro-1-butanone (586 mg, 32%) was afforded as a yellow solid according to the procedure of Example 302, Step A from 3-chloroaniline (1.00g, 7.9 mmol). The title compound was isolated as a yellow oil (10 mg, 19%) according to the method of Example 286, Step C from (8a*S*,12a*R*)-6,7,8a,9,10,11,12,12a-octahydro-5*H*-pyrido[4,3-*b*][1,4]thiazepino[2,3,4-*hi*]indole (30 mg, 0.12 mmol) and 1-(2-amino-4-chlorophenyl)-4-chloro-1-butanone (53 mg, 0.24 mmol). ¹H NMR (CDCl₃) δ 1.83-2.17 (m, 7H), 2.19-2.41 (m, 3H), 2.57-2.61 (m, 1H), 2.68-2.77 (m, 1H), 2.83-2.92 (m, 3H), 2.96-3.13 (m, 2H), 3.20-3.26 (m, 1H), 3.45-3.58 (m, 1H), 3.71-3.82 (m, 1H), 6.28 (br-s, 2H), 6.51-6.58 (m, 3H), 6.78 (d, 1H, *J* = 6.6 Hz), 6.87 (dd, 1H, *J* = 1.2, 7.9 Hz), 7.62 (d, 1H, *J* = 8.8 Hz)ppm.

### EXAMPLE 307

### 4-((8aS,12aR)-6,7,,9,10,,12,12a-hexahydro-5H-pyrido[4,3-b][1,4]thiazepino[2,3,4-hi]indol-11(8aH)-yl)-1-(2-amino-4-hydroxyphenyl)-1-butanone

1-(2-Amino-4-hydroxyphenyl)-4-chloro-1-butanone (100 mg, 5%) was afforded as a yellow solid according to the procedure of Example 302, Step A from meta-anisidine (1.00g, 8.2 mmol). The title compound was isolated as a yellow oil (5 mg, 9%) according to the method of Example 286, Step C from (8a*S*,12a*R*)-6,7,8a,9,10,11,12,12a-octahydro-5*H*-pyrido[4,3-*b*][1,4]thiazepino[2,3,4-*hi*]indole (30 mg, 0.12 mmol) and 1-(2-amino-4-hydroxyphenyl)-4-chloro-1-butanone (56 mg, 0.24 mmol). ¹H NMR (CDCl₃) δ 1.90-2.19 (m, 6H), 2.31-2.48 (m, 3H), 2.68-2.77 (m, 1H), 2.78-2.84 (m, 1H), 2.89-3.00 (m, 5H), 3.02-3.18 (m, 1H), 3.24-3.31 (m, 1H), 3.54-3.61 (m, 1H), 3.78-3.87 (m, 1H), 4.21 (br-s, 2H), 6.11-6.16 (m, 2H), 6.61 (t, 1H, *J* = 7.5 Hz), 6.85 (d, 1H, *J* = 6.6), 6.94 (dd, 1H, *J* = 1.1, 7.7 Hz), 7.56 (d, 1H, *J* = 8.4 Hz), 12.93 (br-s, 1H) ppm.

### EXAMPLE 308

### 4-((8aS,12aR)-6,7,,9,10,,12,12a-hexahydro-5H-pyrido[4,3-b][1,4]thiazepino[2,3,4-hi]indol-11(8aH)-yl)-1-(2-amino-4-bromophenyl)-1-butanone

1-(2-Amino-4-bromophenyl)-4-chloro-1-butanone (558 mg, 17%) was afforded as a yellow solid according to the procedure of Example 302, Step A from 3-bromoaniline (2.00 g, 11.7 mmol). The title compound was isolated as a yellow oil (21 mg, 35%) according to the method of Example 286, Step C from (8a*S*,12a*R*)-6,7,8a,9,20,11,12,12a-octahydro-5*H*-pyrido[4,3-*b*][2,4]thiazepino[2,3,4-*hi*]indole (30 mg, 0.12 mmol) and 1-(2-amino-4-bromophenyl)-4-chloro-1-butanone (68 mg, 0.24 mmol). ¹H NMR (CDCl₃) δ1.89-2.22 (m, 8H), 2.24-2.45 (m, 3H), 2.61-2.70 (m, 1H), 2.73-2.81 (m, 1H), 2.91-3.01 (m, 2H), 3.04-3.17 (m, 2H), 3.24-3.29 (m, 1H), 3.51-3.62 (m, 1H), 3.78-3.87 (m, 1H), 6.33 (br-s, 2H), 6.61 (t, 1H, *J* = 7.5 Hz), 6.75 (dd, 1H, *J* = 2.0, 8.6 Hz), 6.81-6.87 (m, 2H), 6.94 (dd, 1H, *J* = 1.1, 7.7 Hz), 7.60 (d, 1H, *J* = 8.4 Hz) ppm.

### EXAMPLE 309

### (8aS,12aR)-11-[3-(1H-indazol-3-yl)propyl]-6,7,8a,9,10,11,12,12a-octahydro-5H-pyrido[4,3-b][1,4]thiazepino[2,3,4-hi]indole

To a suspension of 1-(2-aminophenyl)-4-chloro-1-butanone (508 mg, 2.6 mmol) in concentrated HCl (3.5 mL) at -5°C was added a solution of NaNO₂ (193mg, 2.8 mmol) in H₂O (0.75 mL), and the reaction mixture was stirred for 1 h. A solution of SnCl₂-2H₂O (1.37g, 6.07 mmol) in concentrated HCl (1.9 mL) was added at -5°C to the solution, and this stirred for 1 h under ice cooling. The reaction was quenched with H₂O and extracted into Et₂O (100 mL). The organic solution was washed with H₂O (50 mL) and brine (50 mL), dried over MgSO₄ and concentrated *in vacuo*. Purification by column chromatography afforded 3-(3-chloropropyl)-1*H*-indazole (126 mg, 25%) as a yellow solid. The title compound was isolated as a yellow oil (38 mg, 77%) according to the method of Example 286, Step C from (8a*S*,12a*R*)-6,7,8a,9,10,11,12,12a-octahydro-5*H*-pyrido[4,3-*b*][1,4]thiazepino[2,3,4-*hi*]indole (30 mg, 0.12 mmol) and 3-(3-chloropropyl)-1*H*-indazole (44 mg, 0.24 mmol). ¹H NMR (CDCl₃) δ 1.83-2.19 (m, 7H), 2.23-2.35 (m, 1H), 2.43-2.54 (m, 2H), 2.72-2.79 (m, 1H), 2.81-2.88 (m, 1H), 2.92-3.11 (m, 4H), 3.12-3.21 (m, 1H), 3.24-3.29 (m, 1H), 3.51-3.62 (m, 1H), 3.80-3.92 (m, 1H), 6.61 (t, 1H, *J* = 7.5 Hz), 6.84 (d, 1H, *J* = 6.6 Hz), 6.94 (dd, 1H, *J* = 1.1, 7.7 Hz), 7.11-7.16 (m, 1H), 7.34-7.48 (m, 2H), 7.70 (d, 1H, *J* = 8.0 Hz) ppm.

### EXAMPLE 310

### (8aS,12aR)-11-[3-(5-fluoro-1H-indazol-3-yl)propyl]-6,7,8a,9,10,11,12,12a-octahydro-5H-pyrido[4,3-b][1,4]thiazepino[2,3,4-hi]indole

3-(3-Chloropropyl)-6-fluoro-1*H*-indazole (91 mg, 46%) was afforded according to the procedure of Example 309 from 1-(2-amino-5-fluorophenyl)-4-chloro-1-butanone (200 mg, 0.93 mmol). The title compound was isolated as a yellow oil (35 mg, 66%) according to the method of Example 286, Step C from (8a*S*,12a*R*)-6,7,8a,9,10,11,12,12a-octahydro-5*H*-pyrido[4,3-*b*][1,4]thiazepino[2,3,4-*hi*]indole (30 mg, 0.12 mmol) and 3-(3-chloropropyl)-6-fluoro-1*H*-indazole (52 mg, 0.24 mmol). ¹H NMR (CDCl₃) δ 1.92-2.15 (m, 7H), 2.34-2.42 (m, 1H), 2.42-2.55 (m, 2H), 2.72-3.10 (m, 6H), 3.20-3.34 (m, 2H), 3.50-3.61 (m, 1H), 3.77-3.86 (m, 1H), 6.61 (t, 1H, *J* = 7.5 Hz), 6.84 (d, 1H, *J* = 7.4 Hz), 6.95 (dd, 1H, *J* = 1.1, 7.7 Hz), 7.13 (td, 1H, J = 2.3, 8.8 Hz), 7.29-7.39 (m, 2H) ppm.

### EXAMPLE 311

### (8aS,12aR)-11-[3-(7-fluoro-1H-indazol-3-yl)propyl]-6,7,8a,9,10,11,12,12a-octahydro-5H-pyrido[4,3-b][1,4]thiazepino[2,3,4-hi]indole

3-(3-Chloropropyl)-7-fluoro-1*H*-indazole (14 mg, 10%) was afforded according to the procedure of Example 309 from 1-(2-amino-3-fluorophenyl)-4-chloro-1-butanone (136 mg, 0.63 mmol). The title compound was isolated as a yellow oil (10 mg, 66%) according to the method of Example 286, Step C from (8a*S*,12a*R*)-6,7,8a,9,10,11,12,12a-octahydro-5*H*-pyrido[4,3-*b*][1,4]thiazepino[2,3,4-*hi*]indole (17 mg, 0.07 mmol) and 3-(3-chloropropyl)-7-fluoro-1*H*-indazole (14 mg, 0.07 mmol). ¹H NMR (CDCl₃) δ 1.81-2.11 (m, 7H), 2.24-3.33 (m, 1H), 2.42-2.54 (m, 2H), 2.67-3.04 (m, 6H), 3.18-3.26 (m, 2H), 3.42-3.57 (m, 1H), 3.71-3.85 (m, 1H), 6.54 (d, 1H, *J* = 7.5 Hz), 6.77 (dd, 1H, *J* = 1.1, 7.0 Hz), 6.88 (dd, 1H, *J* = 1.3, 7.9 Hz), 6.94-7.01 (m, 2H), 7.37-7.41 (m, 1H) ppm.

### EXAMPLE 312

### (8aS,12aR)-11-[3-(6-chloro-1H-indazol-3-yl)propyl]-6,7,8a,9,10,11,12,12a-octahydro-5H-pyrido[4,3-b][1,4]thiazepino[2,3,4-hi]indole

3-(3-Chloropropyl)-6-chloro-1*H*-indazole (107 mg, 54%) was afforded according to the procedure of Example 309 from 1-(2-amino-4-chlorophenyl)-4-chloro-1-butanone (202 mg, 0.87 mmol). The title compound was isolated as a yellow oil (37 mg, 69%) according to the method of Example 286, Step C from (8a*S*,12a*R*)-6,7,8a,9,10,11,12,12a-octahydro-5*H*-pyrido[4,3-*b*][1,4]thiazepino[2,3,4-*hi*]indole (30 mg, 0.12 mmol) and 3-(3-chloropropyl)-6-chloro-1*H*-indazole (56 mg, 0.24 mmol). ¹H NMR (CDCl₃) δ 1.93-2.20 (m, 8H), 2.37-3.38 (m, 1H), 2.41-2.48 (m, 2H), 2.69-2.77 (m, 1H), 2.79-2.86 (m, 1H), 2.91-3.10 (m, 4H), 3.16-3.23 (m, 1H), 3.26-3.31 (m, 1H), 3.50-3.61 (m, 1H), 3.77-3.85 (m, 1H), 6.61 (t, 1H, *J* = 7.5 Hz), 6.89 (d, 1H, *J* = 6.6 Hz), 6.94 (dd, 1H, *J* = 1.1, 7.7 Hz), 7.09 (dd, 1H, *J* = 1.7, 7.8 Hz), 7.42 (d, 1H, *J* = 1.1 Hz), 7.61 (d, 1H, *J* = 8.4 Hz)

### EXAMPLE 313

### (8aS,12aR)-11-[3-(6-bromo-1H-indazol-3-yl)propyl]-6,7,8a,9,10,11,12,12a-octahydro-5H-pyrido[4,3-b][1,4]thiazepino[2,3,4-hi]indole

3-(3-Chloropropyl)-6-bromo-1*H*-indazole (228 mg, 74%) was afforded according to the procedure of Example 309 from 1-(2-amino-4-bromophenyl)-4-chloro-1-butanone (311 mg, 1.1 mmol). The title compound was isolated as a yellow oil (40 mg, 69%) according to the method of Example 286, Step C from (8a*S*,12a*R*)-6,7,8a,9,10,11,12,12a-octahydro-5*H*-pyrido[4,3-*b*][1,4]thiazepino[2,3,4-*hi*]indole (30 mg, 0.07 mmol) and 3-(3-chloropropyl)-6-bromo-1*H*-indazole (67 mg, 0.24 mmol). ¹H NMR (CDCl₃) δ 1.94-2.17 (m, 7H), 2.34-2.41 (m, 1H), 2.45-2.56 (m, 2H), 2.73-2.81 (m, 1H), 2.83-2.90 (m, 1H), 2.90-3.11 (m, 4H), 3.24-3.35 (m, 2H), 3.49-3.61 (m, 1H), 3.77-3.89 (m, 1H), 6.61 (t, 1H, *J* = 7.5 Hz), 6.84 (d, 1H, *J* = 7.4 Hz), 6.95 (d, 1H, *J* = 7.7 Hz), 7.20-7.26 (m, 1H), 7.53-7.60 (m, 1H) ppm.

### EXAMPLE 314

### 4-((8aS,12aR)-6,7,,9,10,,12,12a-hexahydro-5H-pyrido[4,3-b][1,4]thiazepino[2,3,4-hi]indol-11(8aH)-yl)-1-(2-(methylamino)phenyl)-1-butanone

1-(2-Methylaminophenyl)-4-chloro-1-butanone (886 mg, 22%) was afforded as a yellow solid according to the procedure of Example 302, Step A from *N*-methylaniline (2.00 g, 18 mmol). The title compound was isolated as a yellow oil (23 mg, 45%) according to the method of Example 286, Step C from (8a*S*,12a*R*)-6,7,8a,9,10,11,12,12a-octahydro-5*H*-pyrido[4,3-*b*][1,4]thiazepino[2,3,4-*hi*]indole (30 mg, 0.12 mmol) and 1-(2-methylaminophenyl)-4-chloro-1-butanone (52 mg, 0.24 mmol). ¹H NMR (CDCl₃) δ 1.91-2.23 (m, 6H), 2.24-3.36 (m, 1H), 2.37-2.44 (m, 2H), 2.63-2.71 (m, 1H), 2.75-2.82 (m, 1H), 2.89-3.17 (m, 9H), 3.24-3.30 (m, 1H), 3.47-3.62 (m, 1H), 3.79-3.87 (m, 1H), 6.56-6.63 (m, 2H), 6.69 (d, 1H, *J* = 8.4 Hz), 6.84 (d, 1H, *J* = 7.0 Hz), 6.94 (dd, 1H, *J* = 1.1, 7.7 Hz), 7.35-7.41 (m, 1H), 7.80 (dd, 1H, *J* = 8.6 Hz), 8.81 (br-s, 1H) ppm.

### EXAMPLE 315

### (8aS,12aR)-11-[3-(1-benzothien-3-yl)propyl]-6, 7, 8a, 9,10,11,12,12a-octahydro-5H-pyrido[4,3-b][1,4]thiazepino[2,3,4-hi]indole

The title compound was isolated as a yellow oil (49 mg, 52%) according to the method of Example 286, Step C from (8a*S*,12a*R*)-6,7,8a,9,10,11,12,12a-octahydro-5*H*-pyrido[4,3-*b*][1,4]thiazepino[2,3,4-*hi*]indole (55mg, 0.23 mmol) and 3-(1-benzothien-3-yl)propyl methanesulfonate (122 mg, 0.45 mmol). ¹H NMR (CDCl₃) δ 1.97-2.20 (m, 7H), 2.31-2.41 (m, 1H), 2.44-2.56 (m, 2H), 2.71-2.79 (m, 1H), 2.81-2.99 (m, 4H), 3.04-3.11 (m, 1H), 3.19-3.35 (m, 2H), 3.50-3.62 (m, 1H), 3.79-3.88 (m, 1H), 6.62 (t, 1H, *J* = 7.5 Hz), 6.85 (d, 1H, *J* = 7.3 Hz), 6.95 (dd, 1H, *J* = 1.3, 7.9 Hz), 7.11 (s, 1H), 7.34-7.41 (m, 2H), 7.74-7.78 (m, 1H), 7.85 (dd, 1H, *J* = 1.5, 6.2 Hz) ppm.

### EXAMPLE 355

### 2- [2-((±)-cis-6,7,9,10,12a-hexahydro-5H-pyrido[4,3-b][1,4]thiazepino[2,3,4-hi]indol-11(8aH)-yl)ethyl][1,2,4]trizolo[4,3-a]pyridin-3(2H)-one

### Step A:

To a solution of [1,2,4]trizolo[4,3-*a*]pyridin-3(2*H*)-one (200 mg, 1.47 mmol) in DMF (7.0 mL) was added NaH (43 mg, 1.76 mmol) at 0°C under N₂. The reaction mixture was stirred for 30 min at 0°C then 1-bromo-2-chloroethane (424 mg, 2.96 mmol) was added dropwise. The reaction mixture was stirred for 15 h at 20°C then quenched by addition of H₂O and extracted with CHCl₃. The combined organic solution was successively washed with saturated NaHCO₃ aqueous solution and brine, dried over MgSO₄, filtered and concentrated *in vacuo.* The residue was chromatographed (silica gel; CHCl₃: MeOH 99:1) to give 2-(2-chloroethyl) [1,2,4]trizolo[4,3-*a*]pyridin-3(2*H*)-one as a white solid (260 mg, 90%).

### Step B:

To a solution of (±)-*cis*-6,7,8a,9,10,11,12,12a-octahydro-5H-pyrido[4,3-*b*][1,4]thiazepino[2,3,4-*hi*]indole (30 mg, 0.12 mmol)) in 1,4-dioxane (0.72 mL) were added 2-(2-chloroethyl)[1,2,4]trizolo[4,3-*a*]pyridin-3(2*H*)-one (36 mg, 0.18 mmol, KI (catalytic amount) and K₂CO₃ (25 mg, 0.18 mmol). The reaction mixture was heated at 100°C for 48 h. The reaction mixture was cooled to 20°C then diluted with CHCl₃. The solution was filtered to remove excess K₂CO₃ and the filtrate was concentrated *in vacuo* and chromatographed (silica gel, CHCl₃: MeOH 98:2) to give the title compound (32 mg, 65%) as a pale yellow oil. ¹H NMR (CDCl₃, 300 MHz) δ 1.65-1.77 (br-s, 1H), 1.82-1.95 (m, 2H), 1.97-2.20 (m, 3H), 2.39 (dt, J = 11.0, 4.1 Hz, 1H), 2.70-2.90 (m, 3H), 2.92-2.99 (m, 1H), 3.02-3.18 (m,2H), 3.20-3.24 (m, 1H), 3.49-3.60 (m, 1H), 3.77-3.85 (m, 1H), 4.13 (t, J = 16.5 Hz, 2H), 6.48 (qu, J = 3.5 Hz, 1H), 6.60 (t, J = 7.7 Hz, 1H), 6.85 (d, J = 7.0 Hz, 1H), 6.93 (d, J = 7.7 Hz, 1H), 7.05-7.09 (m, 2H), 7.75 (d, J = 7.0 Hz, 1H) ppm.

### EXAMPLE 356

### (±)-cis-11-[3-(6-fluoro-1H-indol-1-yl)propyl]-6,7,8a,9,10,11,12,12a-octahydro-5H-pyrido[4,3-b][1,4]thiazepino[2,3,4-hi]indole

### Step A:

To a solution of 1-bromo-3-chloropropane (700 mg, 4.44 mmol) in DMF (2.4 mL) was added 6-fluoroindole (200 mg, 1.48 mmol) and powder KOH (92 mg, 1.63 mmol) at 20°C. The reaction mixture was stirred for 15 h at 20°C then quenched by addition of H₂O and extracted with Et₂O. The combined organic solution was successively washed with brine, dried over MgSO₄, filtered and concentrated *in vacuo*. The residue was chromatographed to give 1-(3-chloropropyl)-6-fluoroindole (190 mg, 66%) as a colorless oil.

### Step B:

The title compound was prepared by the method of Example 355 Step B as a yellow oil (50 mg, 99%) from (±)-*cis*-6,7,8a,9,10,11,12,12a-octahydro-5H-pyrido[4,3-*b*][1,4]thiazepino[2,3,4-*hi*]indole (30 mg, 0.12 mmol) and 1-(3-chloropropyl)-6-fluoroindole (36 mg, 0.18 mmol). ¹H NMR (CDCl₃, 300 MHz) δ 1.85-2.30 (m, 10H), 2.57-2.60 (m, 1H), 2.62-2.70 (m, 1H), 2.92-2.98 (m, 1H), 3.03-3.20 (m,2H), 3.27-3.32 (m, 1H), 3.49-3.60 (m, 1H), 3.78-3.85 (m, 1H), 4.16 (t, J = 16.6 Hz, 2H), 6.46 (d, J = 2.9 Hz, 1H), 6.62 (t, J = 7.3 Hz, 1H), 6.82-6.90 (m, 2H), 6.95 (dd, J = 8.1, 0.8 Hz, 1H), 7.07-7.13 (m, 2H), 7.51 (dd, J = 8.8, 5.5 Hz, 1H) ppm.

### EXAMPLE 357

### (±)-cis-11-[3-(5-fluoro-1H-indol-1-yl)propyl]-6,7,8a,9,10,11,12,12a-octahydro-5H-pyrido[4,3-b][1,4]thiazepino[2,3,4-hi]indole

### Step A:

1-(3-chloropropyl)-5-fluoroindole (190 mg, 66%) was prepared by the method of Example 355 Step A as a colorless oil from 5-fluoroindole (200 mg, 1.48 mmol).

### Step B:

The title compound was prepared by the method of Example 355 Step B as a yellow oil (48 mg, 95%) from (±)-*cis*-6,7,8a,9,10,11,12,12a-octahydro-5H-pyrido[4,3-*b*][1,4]thiazepino[2,3,4-*hi*]indole (30 mg, 0.12 mmol) and 1-(3-chloropropyl)-5-fluoroindole (36 mg, 0.18 mmol). ¹H NMR (CDCl₃, 300 MHz) δ 1.86-2.30 (m, 10H), 2.55-2.59 (m, 1H), 2.65-2.72 (m, 1H), 2.92-3.00 (m, 1H), 3.05-3.21 (m, 2H), 3.27-3.33 (m, 1H), 3.47-3.59 (m, 1H), 3.75-3.86 (m, 1H), 4.20 (t, J = 16.6 Hz, 2H), 6.44 (d, J = 3.3 Hz, 1H), 6.62 (t, J = 7.3 Hz, 1H), 6.85 (d, J = 6.9 Hz, 1H), 6.90-6.98 (m, 2H), 7.14 (d, J = 2.9 Hz, 1H), 7.23-7.31 (m, 2H) ppm.

### EXAMPLE 358

### (±)-cis -11-[3-(6-fluoro-2,3-dihydro-1H-indol-1-yl)propyl]-6,7,8a,9,10,11,12,12a-octahydro-5H-pyrido[4,3-b][1,4]thiazepino[2,3,4-hi]indole

To a solution of (±)-*cis*-11-[3-(6-fluoro-1*H*-indol-1-yl)propyl]-6,7,8a,9,10,11,12,12a-octahydro-5*H*-pyrido[4,3-*b*][1,4]thiazepino[2,3,4-*hi*]indol (25 mg, 0.054 mmol) in acetic acid (0.8 mL) was added NaCNBH₃ (10.2 mg, 0.16 mmol) slowly at 10°C. The reaction mixture was slowly warmed to 20°C and stirred for 2h. The reaction was quenched by addition of ice followed by 1N NaOH. The product was extracted with CHCl₃ and the combine organic solution was dried over MgSO₄. The title compound was obtained by flash column chromatography as a colorless oil (19 mg, 83%). ¹H NMR (CDCl₃, 300 MHz) δ 1.77 (qu, J = 7.0 Hz, 2H), 1.80-2.20 (m, 5H), 2.22-2.45 (m, 3H), 2.62-2.72 (m, 1H), 2.73-2.82 (m, 1H), 2.87-2.98 (m, 3H), 3.03-3.22 (m,4H), 3.25-3.32 (m, 1H), 3.40 (t, J = 8.5 Hz, 2H), 3.50-3.61 (m, 1H), 3.78-3.88 (m, 1H), 6.16 (dd, J = 10.6, 2.2 Hz, 1H), 6.26 (dt, J = 8.0, 2.5 Hz, 1H), 6.62 (t, J = 7.3 Hz, 1H), 6.84-6.97 (m, 3H) ppm. MS (CI, NH₃): 424.3 (base, M+H).

### EXAMPLE 359

### (±)-cis -11-[3-(5-fluoro-2,3-dihydro-1H-indol-1-yl)propyl]-6,7,8a,9,10,11,12,12a-octahydro-5H-pyrido[4,3-b][1,4]thiazepino[2,3,4-hi]indole

The title compound was prepared by the method of Example 358 as a colorless oil (19 mg, 69%) from (8a*S*,12a*R*)-11-[3-(6-fluoro-1*H*-indol-1-yl)propyl]-6,7,8a,9,10,11,12,12a-octahydro-5*H*-pyrido[4,3-*b*][1,4]thiazepino[2,3,4-*hi*]indol (30 mg, 0.065 mmol). ¹H NMR (CDCl₃, 300 MHz) δ 1.74-1.84 (m, 2H), 1.86-2.20 (m, 5H), 2.22-2.45 (m, 3H), 2.64-2.82 (m, 2H), 2.87-3.22 (m, 7H), 3.25-3.38 (m, 3H), 3.50-3.62 (m, 1H), 3.78-3.85 (m, 1H), 6.35 (dd, J = 8.8, 4.4 Hz, 1H), 6.62 (t, J = 7.3 Hz, 1H), 6.70-6.90 (m, 3H), 6.95 (dd, J = 8.0, 1.0 Hz, 1H) ppm.

### EXAMPLE 360

### (±)-cis -11-[3-(6-fluoro-1H-indol-3-yl)propyl]-6,7,8a,9,10,11,12,12a-octahydro-5H-pyrido[4,3-b][1,4]thiazepino[2,3,4-hi]indole

### Step A:

The solution of 6-fluoroindol (300 mg, 2.22 mmol), acrylic acid (352 mg, 4.88 mmol) and acetic anhydride (453 mg, 4.44 mmol) in acetic acid (1.1 mL) was heated at 90°C for 15 h. The reaction mixture was cooled to 20°C then concentrated *in vacuo*. The residue was dissolved in 3N NaOH. The solution was filtered to remove insoluble material and the filtrate was acidified with conc. HCl and extracted with CHCl₃. The organic solution was dried over MgSO₄, filtered and concentrated *in vacuo* to give 3-(6-fluoroindolyl)propionic acid (240 mg, 52%) as a yellow solid.

### Step B:

To a solution of 3-(6-fluoroindolyl)propionic acid (235 mg, 1.13 mmol) in THF (5.3 mL) was added LiAlH₄ (86 mg, 2.26 mmol) slowly at 0°C under N₂. The reaction mixture was warmed to 20°C and stirred for 15 h. The reaction was quenched by addition of H₂O (0.5 mL) and diluted with EtOAc. The resulting solution was dried over MgSO₄, filtered through Celite and concentrated *in vacuo*. The residue was chromatographed to give 6-fluoro-3-(3-hydroxypropyl)indole (142 mg, 65%) as a colorless oil.

### Step C:

To a solution of 6-fluoro-3-(3-hydroxypropyl)indole (140 mg, 0.72 mmol) in CH₂Cl₂ (4.5 mL) and Et₃N (147 mg, 1.45 mmol) was added methane sulfonyl chloride (125 mg, 1.09 mmol) at 0°C under N₂. The reaction mixture was stirred for 2h at 0°C. The reaction was quenched by addition of 1N HCl and diluted with Et₂O. The layer was separated and the organic layer was dried over MgSO₄, filtered and concentrated *in vacuo.* The residue was chromatographed to give 3-(6-fluoroindolyl)-propyl methanesulfonate (140 mg, 71%) as a colorless oil.

### Step D:

The title compound was prepared by the method of Example 355 Step B as a yellow oil (35 mg, 69%) from (±)-*cis*-6,7,8a,9,10,11,12,12a-octahydro-5H-pyrido[4,3-*b*][1,4]thiazepino[2,3,4-*hi*]indole (30 mg, 0.12 mmol) and 3-(6-fluoroindolyl)-propyl methanesulfonate (49 mg, 0.18 mmol). ¹H NMR (CDCl₃, 300 MHz) δ 1.86-2.20 (m, 7H), 2.25-2.36 (m, 1H), 2.39-2.46 (m, 2H), 2.67-2.85 (m, 4H), 2.85-2.96 (m, 1H), 3.02-3.15 (m, 1H), 3.17-3.25 (m, 1H), 3.28 (qu, J = 3.3 Hz, 1H) 3.50-3.62 (m, 1H), 3.77-3.87 (m, 1H), 6.62 (t, J = 7.4 Hz, 1H), 6.83-6.91 (m, 2H), 6.93-6.97 (m, 2H), 7.01 (dd, J = 9.9, 2.2 Hz, 1H), 7.48 (dd, J = 8.5, 5.2 Hz, 1H), 7.93-7.99 (br-s, 1H) ppm.

### EXAMPLE 361

### (8aS,12aR)-11-[3-(6-fluoro-1H-indol-3-yl)propyl]-6,7,8a,9,10,11,12,12a-octahydro-5H-pyrido[4,3-b][1,4]thiazepino[2,3,4-hi]indole

To a solution of (8a*S*,12a*R*)-6,7,8a,9,10,11,12,12a-octahydro-5H-pyrido[4,3-*b*][1,4]thiazepino[2,3,4-*hi*]indole HCl salt (20 mg, 0.063 mmol) in 1,4-dioxane (0.4 mL) and *N,N*-diisopropylethylamine (82 mg, 0.63 mmol) were added 3-(6-fluoroindolyl)-propyl methanesulfonate (26 mg, 0.89 mmol) and KI (catalytic amount). The reaction mixture was heated at 100°C for 15h. The reaction mixture was cooled to 20°C then concentrated *in vacuo* and chromatographed (silica gel CHCl₃: MeOH 98:2) to give desired the title compound (24 mg, 91%) as a yellow oil. The title compound was spectroscopically identical to Example 360.

### EXAMPLE 362

### (±)-cis -11-[3-(5-fluoro-1H-indol-3-yl)propyl]-6,7,8a,9,10,11,12,12a-octahydro-5H-pyrido[4,3-b][1,4]thiazepino[2,3,4-hi]indole

### Step A:

3-(5-Fluoroindolyl)propionic acid (272 mg, 60%) was prepared by the method of Example 360 Step A as a yellow solid from 5-fluoroindole (300 mg, 2.22 mmol)

### Step B:

5-Fluoro-3-(3-hydroxypropyl)indole (185 mg, 74%) was prepared by the method of Example 360 Step B as a colorless solid from 3-(5-Fluoroindolyl)propionic acid (270 mg, 1.30 mmol)

### Step C:

3-(5-Fluoroindolyl)-propyl methanesulfonate (185 mg, 74%) was prepared by the method of Example 360 Step C as a colorless solid from 5-fluoro-3-(3-hydroxypropyl)indole (167 mg, 0.86 mmol)

### Step D:

The title compound was prepared by the method of Example 355 Step B as a yellow oil (35 mg, 69%) from (±)-*cis*-6,7,8a,9,10,11,12,12a-octahydro-5H-pyrido[4,3-*b*][1,4]thiazepino[2,3,4-*hi*]indole (30 mg, 0.12 mmol) and 3-(5-fluoroindolyl)-propyl methanesulfonate (49 mg, 0.18 mmol). ¹H NMR (CDCl₃, 300 MHz) δ 1.88-2.19 (m, 7H), 2.24-2.35 (m, 1H), 2.37-2.47 (m, 2H), 2.68-2.85 (m, 4H), 2.88-2.97 (m, 1H), 3.02-3.12 (m, 1H), 3.15-3.30 (m, 2H), 3.48-3.60 (m, 1H), 3.77-3.87 (m, 1H), 6.62 (t, J = 7.3 Hz, 1H), 6.85 (d, J = 6.6 Hz, 1H), 6.89-6.97 (m, 2H), 7.03 (s, 1H), 7.21-7.30 (m, 2H), 7.93-8.01 (br-s, 1H) ppm.

### EXAMPLE 363

### (8aS,12aR)-11-[3-(5-fluoro-1H-indol-3-yl)propyl]-6,7,8a,9,10,11,12,12a-octahydro-5H-pyrido[4,3-b][1,4]thiazepino[2,3,4-hi]indole

The title compound was prepared by the method of Example 361 as a yellow oil (19 mg, 72%) from (8a*S*,12a*R*)-6,7,8a,9,10,11,12,12a-octahydro-5H-pyrido[4,3-*b*][1,4]thiazepino[2,3,4-*hi*]indole HCl salt (20 mg, 0.063 mmol) and 3-(5-fluoroindolyl)-propyl methanesulfonate (26 mg, 0.094 mmol). The title compound was spectroscopically identical to Example 362.

### EXAMPLE 364

### (±)-cis -11-[3-(9H-purin-9-yl)propyl]-6,7,8a,9,10,11,12,12a-octahydro-5H-pyrido[4,3-b][1,4]thiazepino[2,3,4-hi]indole

### Step A:

To a solution of purine (360 mg, 3.00 mmol) and 1-bromo-3-chloropropane (1.42 g, 9.0 mmol) in DMF (10 mL) was added K₂CO₃ (622 mg, 4.5 mmol) at 20°C. The reaction mixture was stirred for 24h at 20°C and filtered. The filtrate was concentrated *in vacuo* and the residue was chromatographed to give 9-(3-chloropropyl)purine (400 mg, 68%) and 7-(3-chloropropyl)purine (136 mg, 23%) as colorless oils.

### Step B:

The title compound was prepared by the method of Example 355 Step B as a yellow oil (40 mg, 82%) from (±)-*cis*-6,7,8a,9,10,11,12,12a-octahydro-5H-pyrido[4,3-*b*][1,4]thiazepino[2,3,4-*hi*]indole (30 mg, 0.12 mmol) and 9-(3-chloropropyl)purine (36 mg, 0.18 mmol). ¹H NMR (CDCl₃, 300 MHz) δ 1.78-2.20 (m, 7H), 2.22-2.33 (m, 3H), 2.47-2.55 (m, 1H), 2.60-2.68 (m, 1H), 2.94 (dt, J = 14.3, 4.5 Hz, 1H), 3.03-3.15 (m, 2H), 3.28 (qu, J = 3.0 Hz, 1H), 3.47-3.55 (m, 1H), 3.72-3.82 (m, 1H), 4.38 (dt, J = 6.6, 1.1 Hz, 2H), 6.62 (t, J = 7.3 Hz, 1H), 6.85 (d, J = 6.6 Hz, 1H), 6.94 (dd, J = 7.7, 0.9 Hz, 1H), 8.12 (s, 1H), 8.98 (s, 1H), 9.14 (s, 1H) ppm.

### EXAMPLE 365

### (±)-cis -11-[3-(7H-purin-7-yl)propyl]-6,7,8a,9,10,11,12,12a-octahydro-5H-pyrido[4,3-b][1,4]thiazepino[2,3,4-hi]indole

The title compound was prepared by the method of Example 355 Step B as a yellow oil (34 mg, 70%) from (±)-*cis*-6,7,8a,9,10,11,12,12a-octahydro-5H-pyrido[4,3-*b*][1,4]thiazepino[2,3,4-*hi*]indole (30 mg, 0.12 mmol) and 7-(3-chloropropyl)purine (36 mg, 0.18 mmol). ¹H NMR (CDCl₃, 300 MHz) δ 1.80-2.30 (m, 10H), 2.45-2.53 (m, 1H), 2.58-2.65 (m, 1H), 2.94 (dt, J = 13.9, 4.6 Hz, 1H), 3.05-3.17 (m, 2H), 3.30 (qu, J = 3.6 Hz, 1H), 3.45-3.55 (m, 1H), 3.75-3.83 (m, 1H), 4.36-4.45 (m, 2H), 6.63 (t, J = 7.6 Hz, 1H), 6.85 (d, J = 6.9 Hz, 1H), 6.95 (d, J = 7.7 Hz, 1H), 8.25 (s, 1H), 9.00 (s, 1H), 9.16 (s, 1H) ppm.

### EXAMPLE 366

### 4-[5-((±)-cis 6,7,9,10,12,12a-hexahydro-5H-pyrido[4,3-b][1,4]thiazepino[2,3,4-hi]indol-11(8aH)-ylmethyl)4,5-dihydro-3-isoxazolyl]benzonitrile

### Step A:

[3-(4-Cyanophenyl)-4,5-dihydro-5-isoxazolyl]methyl methanesulfonate (69 mg, 99%) was prepared by the method of Example 360 Step C as a yellow solid from 4-[5-(hydroxymethyl)-4,5-dihydro-3-isoxazolyl]benzonitrile (51 mg, 0.25 mmol)

### Step B:

The title compound was prepared by the method of Example 355 Step B as a yellow oil (50 mg, 96%) from (±)-*cis*-6,7,8a,9,10,11,12,12a-octahydro-5H-pyrido[4,3-*b*][1,4]thiazepino[2,3,4-*hi*]indole (30 mg, 0.12 mmol) and {3-(4-cyanophenyl)-4,5-dihydro-5-isoxazolyl}methyl methanesulfonate (50 mg, 0.18 mmol). ¹H NMR (CDCl₃, 300 MHz) δ 1.89 (qu, J = 4.4 Hz, 2H), 2.02-2.20 (m, 3H), 2.40-2.80 (m, 4H), 2.88-2.99 (m, 1H), 3.05-3.35 (m, 5H), 3.43-3.55 (m, 2H), 3.75-3.83 (m, 1H), 4.92-5.01 (m, 1H), 6.58 (td, J = 13.9, 7.2 Hz, 1H), 6.84 (dd, J = 7.0, 3.0 Hz, 1H), 6.92-6.97 (m, 1H), 7.67-7.60 (m, 4H) ppm.

### EXAMPLE 367

### (±)-cis -11-[3-(6-fluoro-1H-indazol-3-yl)propyl]-6,7,8a,9,10,11,12,12a-octahydro-5H-pyrido[4,3-b][1,4]thiazepino[2,3,4-hi]indole

### Step A:

To a solution of BCl₃·Me₂S (1.78 g, 9.9 mmol), in anhydrous benzene (9 mL) was added dropwise a solution of 3-fluoroaniline (1.00 g, 9.0 mmol) in benzene (9 mL) under N₂ with ice cooling. To this reaction mixture was added 4-chlorobutyronitrile (1.12 g, 10.8 mmol) and AlCl₃, successively. The reaction mixture was refluxed for 20 h then cooled to 20°C. Ice cold 2N HCl was added to the reaction mixture to form yellow solid. The resulting mixture was reheated to 80°C for 1h. The solution was cooled to 20°C and extracted with CHCl₃. The organic solution was washed with H₂O and brine, dried over MgSO₄, filtered and concentrated *in vacuo*. The residue was chromatographed to give 1-(2-amino-4-fluorophenyl)-4-chloro-1-butanone (1.07 g, 55%) as a white solid.

### Step B:

To a suspension of 1-(2-amino-4-fluorophenyl)-4-chloro-1-butanone (500 mg, 2.3 mmol) in conc. HCl (3.2 mL) was added a solution of NaNO₂ in H₂O (0.7 mL) and stirred for 1 h at -6 - 0°C. A solution of SnCl₂·2H₂O (1.25 g, 5.51 mmol) in conc. HCl (1.7 mL) was added the reaction mixture and stirred for additional 1h at 0°C. The reaction was quenched by addition of ice water and extracted with Et₂O. The combined organic layer was washed with H₂O and brine, dried over MgSO₄, filtered and concentrated *in vacuo* to give a white solid. It was recrystalized to give pure 3-(3-chloropropyl)-6-fluoroindazole (420 mg, 86%).

### Step C:

The title compound was prepared by the method of Example 355 Step B as a yellow oil (34 mg, 67%) from (±)-*cis*-6,7,8a,9,10,11,12,12a-octahydro-5H-pyrido[4,3-*b*][1,4]thiazepino[2,3,4-*hi*]indole (30 mg, 0.12 mmol) and 3-(3-chloropropyl)-6-fluoroindazole (39 mg, 0.18 mmol). ¹H NMR (CDCl₃, 300 MHz) δ 1.86-2.20 (m, 7H), 2.22-2.33 (m, 1H), 2.39-2.47 (m, 2H), 2.65-2.73 (m, 1H), 2.75-2.83 (m, 1H), 2.88-3.08 (m, 4H), 3.12-3.20 (m, 1H), 3.27 (qu, J = 3.3 Hz, 1H) 3.50-3.60 (m, 1H), 3.77-3.87 (m, 1H), 6.61 (t, J = 7.5 Hz, 1H), 6.83 (d, J = 7.9 Hz, 1H), 6.86-6.95 (m, 2H), 7.06 (dd, J = 9.1, 2.2 Hz, 1H), 7.62 (dd, J = 8.8, 5.1 Hz, 1H), 9.85-10.15 (br-s, 1H) ppm.

### EXAMPLE 368

### (8aS,12aR)-11-[3-(6-fluoro-1H-indazol-3-yl)propyl]-6,7,8a,9,10,11,12,12a-octahydro-5H-pyrido[4,3-b][1,4]thiazepino[2,3,4-hi]indole

The title compound was prepared by the method of Example 361 as a yellow oil (21 mg, 50%) from (8a*S*, 12a*R*)-6,7,8a,9,10,11,12,12a-octahydro-5H-pyrido[4,3-*b*][1,4]thiazepino[2,3,4-*hi*]indole HCl salt (32 mg, 0.10 mmol) and 3-(3-chloropropyl)-6-fluoroindazole (32 mg, 0.15 mmol). The title compound was spectroscopically identical to Example 367.

### EXAMPLE 369

### (8aR,12aS)-11-[3-(6-fluoro-1H-indazol-3-yl)propyl]-6,7,8a,9,10,11,12,12a-octahydro-5H-pyrido[4,3-b][1,4]thiazepino[2,3,4-hi]indole

The title compound was prepared by the method of Example 361 as a yellow oil (31 mg, 73%) from (8a*R*,12a*S*)-6,7,8a,9,10,11,12,12a-octahydro-5H-pyrido[4,3-*b*][1,4]thiazepino[2,3,4-*hi*]indole HCl salt (32 mg, 0.10 mmol) and 3-(3-chloropropyl)-6-fluoroindazole (32 mg, 0.15 mmol). The title compound was spectroscopically identical to Example 367.

### EXAMPLE 370

4-((±)-*cis*-6,7,9,10,12,12a-hexahydro-5*H*-pyrido[4,3-*b*][1,4]thiazepino[2,3,4-*hi*]indol-11(8a*H*)-yl)-1-(2-amino-4-fluorophenyl)-1-butanone

The title compound was prepared by the method of Example 355 Step B as a red oil (330 mg, 64%) from (±)-*cis*-6,7,8a,9,10,11,12,12a-octahydro-5H-pyrido[4,3-*b*][1,4]thiazepino[2,3,4-*hi*]indole (300 mg, 0.121 mmol) and 1-(2-amino-4-fluorophenyl)-4-chloro-1-butanone (400 mg, 1.85 mmol). ¹H NMR (CDCl₃, 300 MHz) δ 1.83-2.18 (m, 7H), 2.22-2.40 (m, 3H), 2.60-2.69 (m, 1H), 2.72-2.80 (m, 1H), 2.89-2.99 (m, 3H), 3.03-3.17 (m, 2H), 3.25 (qu, J = 2.9 Hz, 1H), 3.49-3.59 (m, 1H), 3.78-3.87 (m, 1H), 6.28-6.38 (m, 2H), 6.40-6.48 (br-s, 2H), 6.61 (t, J = 7.4 Hz, 1H), 6.85 (d, J = 7.0 Hz, 1H), 6.94 (dd, J = 8.1, 1.1 Hz, 1H), 7.77 (dd, J = 8.8, 6.4 Hz, 1H) ppm.

### EXAMPLE 371

### 4-((8aS,12aR)-6,7,9,10,12,12a-hexahydro-5H-pyrido[4,3-b][1,4]thiazepino[2,3,4-hi]indol-11(8aH)-yl)-1-(2-amino-4-fluorophenyl)-1-butanone

The title compound was prepared by the method of Example 355 Step B as a yellow oil (130 mg, 24%) from (8a*S*,12a*R*)-6,7,8a,9,10,11,12,12a-octahydro-5H-pyrido[4,3-*b*][1,4]thiazepino[2,3,4-*hi*]indole (320 mg, 1.3 mmol) and 1-(2-amino-4-fluorophenyl)-4-chloro-1-butanone (564 mg, 2.6 mmol). The title compound was spectroscopically identical to Example 370.

### EXAMPLE 372

### N-{2-[4-((±)-cis-6,7,9,10,12,12a-hexahydro-5H-pyrido[4,3-b][1,4]thiazepino[2,3,4-hi]indol-11(8aH)-yl)butanoyl]-5-fluorophenyl}methanesulfonamide

To a solution of 4-((±)-*cis*-6,7,9,10,12,12a-hexahydro-5*H*-pyrido[4,3-*b*][1,4]thiazepino[2,3,4-*hi*]indol-11(8a*H*)-yl)-1-(2-amino-4-fluorophenyl)-1-butanone (30 mg, 0.070 mmol) in CH₂Cl₂ (0.5 mL) was added Et₃N (15 mg, 0.14 mmol) followed by methanesulfonyl chloride (12 mg, 0.11 mmol) at 0°C under N₂. The reaction mixture was stirred for 4h at 0°C then quenched by addition of HCl (1.0N, 1.0 mL). The resulting solution was extracted with CHCl₃. The combine organic solution was dried over MgSO₄. The title compound was obtained by flash column chromatography (silica gel; CHCl₃: MeOH 99:1) as a white amorphous solid (35 mg, 99%). ¹H NMR (CDCl₃, 300 MHz) δ 1.85-2.18 (m, 7H), 2.24-2.42 (m, 3H), 2.58-2.66 (m, 1H), 2.69-2.77 (m, 1H), 2.89-3.17 (m, 5H), 3.23-3.30 (m, 1H), 3.49-3.59 (m, 4H), 3.74-3.85 (m, 1H), 6.60 (t, J = 7.7 Hz, 1H), 6.80-88 (m, 2H), 6.94 (d, J = 8.1 Hz, 1H), 7.13 (dd, J = 8.5, 2.2 Hz, 1H), 7.20-7.25 (m, 1H), 7.77 (dd, J = 8.8, 5.9 Hz, 1H) ppm.

### EXAMPLE 373

### N-{2-[4-((±)-cis-6,7,9,10,12,12a-hexahydro-5H-pyrido[4,3-b][2,4]thiazepino[2,3,4-hi]indol-11(8aH)-yl)butanoyl]-5-fluorophenyl)acetamide

To a solution of 4-((±)-*cis*-6,7,9,10,12,12a-hexahydro-5*H*-pyrido[4,3-*b*][1,4]thiazepino[2,3,4-*hi*]indol-11(8a*H*)-yl)-1-(2-amino-4-fluorophenyl)-1-butanone (28 mg, 0.066 mmol) in CH₂Cl₂ (0.5 mL) was added pyridine (16 mg, 0.20 mmol) followed by acetic anhydride (13 mg, 0.13 mmol) at 20°C under N₂. The reaction mixture was stirred for 15h at 20°C then quenched by addition of H₂O. The resulting solution was extracted with CHCl₃. The combine organic solution was dried over MgSO₄. The title compound was obtained by flash column chromatography (silica gel; CHCl₃: MeOH 99:1) as a white amorphous solid (28 mg, 91%). ¹H NMR (CDCl₃, 300 MHz) δ 1.90-2.18 (m, 10H), 2.49-2.69 (m, 3H), 2.88-3.07 (m, 6H), 3.27-3.38 (m, 2H), 3.49-3.59 (m, 1H), 3.79-3.90 (m, 1H), 6.25-6.38 (m, 2H), 6.41-6.50 (br-s, 1H), 6.64 (t, J = 7.3 Hz, 1H), 6.87 (d, J = 7.0 Hz, 1H), 6.97 (dd, J = 7.7, 1.1 Hz, 1H), 7.73 (dd, J = 9.2, 6.6 Hz, 1H) ppm.

### EXAMPLE 374

### N-{2-[4-((8aS,12aR)-6,7,9,10,12,12a-hexahydro-5H-pyrido[4,3-b][1,4]thiazepino[2,3,4-hi]indol-11(8aH)-yl)butanoyl]-5-fluorophenyl}acetamide

The title compound (37 mg, 80%) was prepared by the method of Example 373 from 4-((8a*S*,12a*R*)-6,7,9,10,12,12a-hexahydro-5*H*-pyrido[4,3-*b*][1,4]thiazepino[2,3,4-*hi*]indol-11(8a*H*)-yl)-1-(2-amino-4-fluorophenyl)-1-butanone (43 mg, 0.10 mmol) as a white amorphous solid (28 mg, 91%). The title compound was spectroscopically identical to Example 373.

### EXAMPLE 375

### Ethyl 2-[4-((±)-cis-6,7,9,10,12,12a-hexahydro-5H-pyrido[4,3-b][1,4]thiazepino[2,3,4-hi]indol-11(8aH)-yl)butanoyl]-5-fluorophenylcarbamate

To a solution of 4-((±)-*cis*-6,7,9,10,12,12a-hexahydro-5*H*-pyrido[4,3-*b*][1,4]thiazepino[2,3,4-*hi*]indol-11(8a*H*)-yl)-1-(2-amino-4-fluorophenyl)-1-butanone (35 mg, 0.082 mmol) in pyridine (0.2 mL) was added ethyl chloroformate (16 mg, 0.10 mmol) at 0°C under N₂. The reaction mixture was stirred for 40 min at 0°C then concentrated *in vacuo*. The residue was chromatographed (silica gel; CHCl₃: MeOH 99:1) to give the title compound as a yellow oil (6.0 mg, 51%). ¹H NMR (CDCl₃, 300 MHz) δ 1.32 (t, J = 7.3 Hz, 3H), 1.83-2.18 (m, 7H), 2.25-2.50 (m, 3H), 2.70-2.87 (br-s, 2H), 2.89-3.17 (m, 5H), 3.25-3.32 (m, 1H), 3.50-3.61 (m, 1H), 3.77-3.87 (m, 1H), 4.23 (q, J = 7.3 Hz, 2H), 6.61 (t, J = 7.4 Hz, 1H), 6.72-6.80 (m, 1H), 6.85 (d, J = 7.4 Hz, 1H), 6.94 (dd, J = 7.7, 1.1 Hz, 1H), 7.94 (dd, J = 9.1, 6.3 Hz, 1H), 8.28 (dd, J = 12.1, 2.5 Hz, 1H), 11.39-11.43 (br-s, 1H) ppm.

### EXAMPLE 376

### N-{2-[4-((±)-cis-6,7,9,10,12,12a-hexahydro-5H-pyrido[4,3-b][1,4]thiazepino[2,3,4-hi]indol-11(8aH)-yl)butanoyl]-5-fluorophenyl-N'-ethylurea

4-((±)-C*is*-6,7,9,10,12,12a-Hexahydro-5*H*-pyrido[4,3-*b*][1,4]thiazepino[2,3,4-*hi*]indol-11(8a*H*)-yl)-1-(2-amino-4-fluorophenyl)-1-butanone (32 mg, 0.075 mmol) was dissolved in ethyl isocyanate (50 µL), and the solution was stirred for 20 h at 20°C under N₂. The reaction mixture was concentrated *in vacuo* then chromatographed (silica gel; CHCl₃: MeOH 99:1) to give the title compound as a pale yellow oil (30 mg, 81%). ¹H NMR (CDCl₃, 300 MHz) δ 1.21 (t, J = 7.0 Hz, 3H), 1.90-2.18 (m, 7H), 2.25-2.50 (m, 3H), 2.65-2.87 (br-s, 2H), 2.90-3.17 (m, 4H), 3.19-3.35 (m, 4H), 3.50-3.59 (m, 1H), 3.78-3.87 (m, 1H), 4.73-4.79 (br-s, 1H), 6.57-6.77 (m, 2H), 6.84 (d, J = 6.2 Hz, 1H), 6.95 (dd, J = 7.7, 1.1 Hz, 1H), 7.91 (dd, J = 8.7, 6.2 Hz, 1H), 8.41 (dd, J = 12.4, 2.6 Hz, 1H), 11.39-11.43 (br-s, 1H) ppm. MS (CI, NH₃): 497.2 (base, M+H).

### EXAMPLE 377

### 2-[4-((±)-cis-6,7,9,10,12,12a-hexahydro-5H-pyrido[4,3-b][1,4]thiazepino[2,3,4-hi]indol-11(8aH)-yl)butanoyl]-5-fluorophenylformamide

4-((±)-*Cis*-6,7,9,10,12,12a-Hexahydro-5*H*-pyrido[4,3-*b*][1,4]thiazepino[2,3,4-*hi*]indol-11(8a*H*)-yl)-1-(2-amino-4-fluorophenyl)-1-butanone (38 mg, 0.090 mmol) was dissolved in acetic formic anhydride (0.2 mL). The reaction mixture was stirred for 2h at 60°C under N₂. The reaction mixture was concentrated *in vacuo*, and the residue was chromatographed (silica gel; CHCl₃: MeOH 99:1) to give the title compound as a pale yellow oil (31 mg, 76%). ¹H NMR (CDCl₃, 300 MHz) δ 1.89-2.18 (m, 7H), 2.37-2.57 (m, 3H), 2.75-2.97 (m, 3H), 2.99-3.12 (m, 3H), 3.15-3.30 (m, 2H), 3.48-3.58 (m, 1H), 3.77-3.87 (m, 1H), 6.62 (t, J = 7.5 Hz, 1H), 6.82-6.90 (m, 2H), 6.95 (dd, J = 7.7, 1.1 Hz, 1H), 7.99 (dd, J = 9.1, 6.2 Hz, 1H), 8.50 (d, J = 1.1 Hz, 1H), 8.55 (dd, J = 11.8, 2.5 Hz, 1H), 11.83-11.87 (br-s, 1H) ppm.

### EXAMPLE 378

### 4-((±)-cis-6,7,9,10,12,12a-hexahydro-5H-pyrido[4,3-b][1,4]thiazepino[2,3,4-hi]indol-11(8aH)-yl)butanoyl]-1-(4-fluoro-2-hydroxyphenyl)-1-butanone

The title compound was prepared by the method of Example 355 Step B as a yellow oil (36 mg, 21%) from (±)-*cis*-6,7,8a,9,10,11,12,12a-octahydro-5H-pyrido[4,3-*b*][1,4]thiazepinc[2,3,4-*hi*]indole (100 mg, 0.41 mmol) and 4-chloro-1-(4-fluoro-2-hydroxyphenyl)-1-butanone (176 mg, 0.81 mmol). ¹H NMR (CDCl₃, 300 MHz) δ 1.80-2.18 (m, 7H), 2.22-2.45 (m, 3H), 2.57-2.77 (m, 2H), 2.85-3.15 (m, 5H), 3.22-3.27 (m, 1H), 3.47-3.59 (m, 1H), 3.77-3.85 (m, 1H), 6.57-6.70 (m, 3H), 6.83 (d, J = 6.7 Hz, 1H), 6.94 (dd, J = 7.7, 1.1 Hz, 1H), 7.80 (dd, J = 8.7, 6.4 Hz, 1H) ppm.

### EXAMPLE 379

### 4-((±)-cis-6,7,9,10,12,12a-hexahydro-5H-pyrido[4,3-b][1,4]thiazepino[2,3,4-hi]indol-11(8aH)-yl)-1-[4-fluoro-2-(methylsulfanyl)phenyl]-1-butanone

### Step A:

To a solution of 3-fluorothiophenol (4.73 g, 37.0 mmol) in diethyl carbonate (11 mL, 129 mmol) was added K₂CO₃ (7.67 g, 55.5 mmol) and 18-crown-6 ether (100 mg, 0.37 mmol). The reaction mixture was refluxed at 100°C for 12h. The reaction mixture was cooled to 20°C then quenched by addition of H₂O and extracted with Et₂O. The combine organic solution was washed with H₂O and brine, dried over MgSO₄.to give 1-fluoro-3-(methylsulfanyl)benzene (5.10g, 97%) as a colorless oil.

### Step B:

To a solution of 1-fluoro-3-(methylsulfanyl)benzene (1.98 g, 14.0 mmol) and 4-chlorobutyryl chloride in CH₂Cl₂ (15 mL) was added AlCl₃ (2.06 g, 15.4 mmol) at 20°C under N₂. The reaction mixture was stirred for 15h at 20°C for 12h then quenched by addition of H₂O and extracted with Et₂O. The combine organic solution was washed with H₂O and brine, dried over MgSO₄, filtered and concentrated *in vacuo*. The resulting white solid was recrystalized to give 4-chloro-1-[4-fluoro-2-(methylsulfanyl)phenyl]-1-butanone (2.80 g, 81%) as a white needle shape crystal.

### Step C:

The title compound was prepared by the method of Example 355 Step B as a yellow oil (45 mg, 45%) from (±)-*cis*-6,7,8a,9,10,11,12,12a-octahydro-5H-pyrido[4,3-*b*][1,4]thiazepino[2,3,4-*hi*]indole (100 mg, 0.41 mmol) and 4-chloro-1-[4-fluoro-2-(methylsulfanyl)phenyl]-1-butanone (50 mg, 0.20 mmol). ¹H NMR (CDCl₃, 300 MHz) δ 1.82-2.18 (m, 7H), 2.24-2.49 (m, 3H), 2.51 (s, 3H), 2.60-2.82 (m, 2H), 2.85-3.15 (m, 5H), 3.21-3.27 (m, 1H), 3.48-3.60 (m, 1H), 3.76-3.87 (m, 1H), 6.61 (t, J = 7.3, 1H), 6.83-6.97 (m, 3H), 7.03 (dd, J = 8.5, 1.9 Hz, 1H), 7.79 (t, J = 8.0 Hz, 1H) ppm.

### EXAMPLE 438

### 1-(2-aminophenyl)-4-((6bR,10aS)-1,2,6b,9,10,10a-hexahydropyrido[4,3-b][1,4]thiazino[2,3,4-hi]indol-8(7H)-yl)-1-butanone

The title compound was isolated as a yellow oil (95 mg, 37%) according to the method of Example 286, Step C from (6b*R*,10a*S*)-1,2,6b,7,8,9,10,10a-octahydropyrido[4,3-*b*] [1,4]thiazino[2,3,4-*hi*]indole (150 mg, 0.65 mmol) and 1-(2-aminophenyl)-4-chloro-1-butanone (255 mg, 1.29 mmol). ¹H NMR (CDCl₃) δ 1.94-2.03 (M, 5H), 2.21-2.33 (m, 1H), 2.37-2.44 (m, 2H), 2.63-2.71 (m, 1H), 2.82-2.89 (m, 1H), 2.90-3.21 (m, 5H), 3.33-3.40 (m, 1H), 3.45-3.64 (m, 2H), 6.60-6.67 (m, 3H), 6.80-6.85 (m, 2H), 7.22-7.28 (m, 1H), 7.76 (dd, 1H, *J* = 1.5, 8.5 Hz) ppm.

### EXAMPLE 439

### 1-(2-aminophenyl)-4-((6bS,10aR)-1,2,6b,9,10,10a-hexahydropyrido[4,3-b][1,4]thiazino[2,3,4-hi]indol-8(7H)-yl)-1-butanone

The title compound was isolated as a yellow oil (98 mg, 38%) according to the method of Example 286, Step C from (6b*S*,10a*R*)-1,2,6b,7,8,9,10,10a-octahydropyrido[4,3-*b*][1,4]thiazino[2,3,4-*hi*]indole (150 mg, 0.65 mmol) and 1-(2-aminophenyl)-4-chloro-1-butanone (255 mg, 1.29 mmol). ¹H NMR (CDCl₃) δ 1.94-2.03 (M, 5H), 2.21-2.33 (m, 1H), 2.37-2.44 (m, 2H), 2.63-2.71 (m, 1H), 2.82-2.89 (m, 1H), 2.90-3.21 (m, 5H), 3.33-3.40 (m, 1H), 3.45-3.64 (m, 2H), 6.60-6.67 (m, 3H), 6.80-6.85 (m, 2H), 7.22-7.28 (m, 1H), 7.76 (dd, 1H, J = 1.5, 8.5 Hz) ppm.

### EXAMPLE 440

### 1-(2-amino-4-fluorophenyl)-4-((6bR,10aS)-1,2,6b,9,10,10a-hexahydro[4,3-b][1,4]thiazino-[2,3,4-hi]indol-8(7H)-yl)-1-butanone

The title compound was prepared by the method of Example 355 Step B as a yellow oil (103 mg, 59%) from (6b*R*,10a*S*)-1,2,6b,7,8,9,10,10a-octahydro[4,3-*b*][1,4]thiazino-[2,3,4-*hi*]indole (100 mg, 0.43 mmol) and 1-(2-amino-4-fluorophenyl)-4-chloro-1-butanone (186 mg, 0.85 mmol). ¹H NMR (CDCl₃, 300 MHz) δ 1.90-2.05 (m, 5H) , 2.23-2.33 (m, 1H), 2.39-2.47 (m, 2H), 2.63-2.75 (m, 1H), 2.82-2.98 (m, 4H), 3.02-3.10 (m, 1H), 3.12-3.20 (m, 1H), 3.35-3.40 (m, 1H), 3.43-3.60 (m, 2H), 6.27-6.39 (m, 2H), 6.40-6.50 (br-s, 2H), 6.64 (t, J = 7.3 Hz, 1H), 6.80-6.89 (m, 2H), 7.76 (dd, J = 9.1, 6.6 Hz, 1H) ppm.

### EXAMPLE 441

### 1-(2-amino-4-fluorophenyl)-4-((6bS,10aR)-1,2,6b,9,10,10a-hexahydro[4,3-b][1,4]thiazino-[2,3,4-hi]indol-8(7H)-yl)-1-butanone

The title compound was prepared by the method of Example 355 Step B as a yellow oil (101 mg, 58%) from (6b*S*,10a*R*)-1,2,6b,7,8,9,10,10a-octahydro[4,3-*b*][1,4]thiazino-[2,3,4-*hi*]indole (100 mg, 0.43 mmol) and 1-(2-amino-4-fluorophenyl)-4-chloro-1-butanone (186 mg, 0.85 mmol). The title compound was spectroscopically identical to Example 440. *N*-{2-[4-((±)-*cis*-6,7,9,10,12,12a-hexahydro-5*H*-pyrido[4,3-*b*][1,4]thiazepino[2,3,4-*hi*]indol-11(8a*H*)-yl)butanoyl]-5-fluorophenyl}methanesulfonamide

To a solution of 4-((±)-*cis*-6,7,9,10,12,12a-hexahydro-5*H*-pyrido[4,3-*b*][1,4]thiazepino[2,3,4-*hi*]indol-11(8a*H*)-yl)-1-(2-amino-4-fluorophenyl)-1-butanone (30 mg, 0.070 mmol) in CH₂Cl₂ (0.5 mL) was added Et₃N (15 mg, 0.14 mmol) followed by methanesulfonyl chloride (12 mg, 0.11 mmol) at 0°C under N₂. The reaction mixture was stirred for 4h at 0°C then quenched by addition of HCl (1.0N, 1.0 mL). The resulting solution was extracted with CHCl₃. The combine organic solution was dried over MgSO₄. The title compound was obtained by flash column chromatography (silica gel; CHCl₃: MeOH 99:1) as a white amorphous solid (35 mg, 99%). ¹H NMR (CDCl₃, 300 MHz) δ 1.85-2.18 (m, 7H), 2.24-2.42 (m, 3H), 2.58-2.66 (m, 1H), 2.69-2.77 (m, 1H), 2.89-3.17 (m, 5H), 3.23-3.30 (m, 1H), 3.49-3.59 (m, 4H), 3.74-3.85 (m, 1H), 6.60 (t, J = 7.7 Hz, 1H), 6.80-88 (m, 2H), 6.94 (d, J = 8.1 Hz, 1H), 7.13 (dd, J = 8.5, 2.2 Hz, 1H), 7.20-7.25 (m, 1H), 7.77 (dd, J = 8.8, 5.9 Hz, 1H) ppm.

### EXAMPLE 456

### 4-((6bS,10aR)-1,2,6b,9,10,10a-hexahydropyrido[4,3-b][1,4]thiazino[2,3,4-hi]indol-8(7H)-yl)-1-(4-fluorophenyl)-1-butanone hydrochloride

### Step A:

Typical procedure for alkylation of amines:
A mixture of indoline hydrochloride (approx 200 mg) in dioxane (4 mL) was treated with Hunig's base (10 equivs) and heated to reflux for 15 min. To the cooled reaction mixture was added 4-chloro-1-(4-fluorophenyl)-1-butanone (5 equivs), KI (0.9 equivs), then the whole mixture was refluxed for 48 h. The reaction was then diluted with chloroform (20 mL) and extracted once with saturated solution of ammonium chloride (10 mL) and twice with ice-cold water (100 mL). The organic layer was dried over sodium sulfate and concentrated to dryness under reduced pressure. The residue was purified by flash chromatography eluting with a gradient of hexane/ethylacetate (e.g. 96:4 to 50:50), following with a gradient methanol/dichloromethane (e.g.1:99 to 3:97) to give the desired product.

The 4-((6b*S*,10a*R*)-1,2,6b,9,10,10a-hexahydropyrido[4,3-*b*][1,4]thiazino[2,3,4-*hi*]indol-8(7*H*)-yl)-1-(4-fluorophenyl)-1-butanone(478 mg, 52%) was obtained from (6b*S*,10a*R*)-1,2,6b,7,8,9,10,10a-octahydropyrido[4,3-*b*][1,4]thiazino[2,3,4-*hi*]indole hydrochloride (750 mg, 2.79 mmol) and 4-chloro-1-(4-fluorophenyl)-1-butanone (3.0 mL, 15.24 mmol) using the procedure described above.

### Step B:

The title compound (483 mg, 81%) was prepared from 4-((6b*S*,10a*R*)-1,2,6b,9,10,10a-hexahydropyrido[4,3-*b*][1,4]thiazino[2,3,4-*hi*]indol-8(7*H*)-yl)-1-(4-fluorophenyl)-1-butanone (578 mg) using the procedure described in EXAMPLE 448 Step B. ¹H NMR (DMSO, 300 MHz) δ 1.92-2.13 (m, 2H), 2.28 (br-s, 2H), 2.80 (t, 1H, J=9.9 Hz), 2.92-3.63 (m, 12H), 3.63-3.75 (m, 1H), 6.67 (t, 1H, J=7.9), 6.87 (d, 1H, J=8.9), 6.96 (d, 1H, J=6.9), 7.30-7.48 (m, 2H), 7.48-8.12 (m, 2H), 10.43 (br-s, 1H) ppm. m/z= 397[C₂₃H₂₅FN₂OS+H]⁺.

### EXAMPLE 457

### 4-((6bR,10aS)-1,2,6b,9,10,10a-hexahydropyrido[4,3-b][1,4]thiazino[2,3,4-hi]indol-8(7H)-yl)-2-(4-fluorophenyl)-1-butanone hydrochloride

### Step A:

The 4-((6b*R*,10a*S*)-1,2,6b,9,10,10a-hexahydropyrido[4,3-*b*][1,4]thiazino[2,3,4-*hi*]indol-8(7*H*)-yl)-1-(4-fluorophenyl)-1-butanone (349 mg, 25%) was prepared from (6b*R*,10a*S*)-1,2,6b,7,8,9,10,10a-octahydropyrido[4,3-*b*][1,4]thiazino[2,3,4-*hi*]indolehydrochloride (750 mg, 2.79 mmol) with 4-chloro-1-(4-fluorophenyl)-1-butanone (3.0 mL, 15.24 mmol) using the procedure described in Example 456 Step A.

### Step B:

The title compound (72 mg, 25%) was prepared from 4-((6b*R*,10a*S*)-1,2,6b,9,10,10a-hexahydropyrido[4,3-*b*][1,4]thiazino[2,3,4-*hi*]indol-8(7*H*)-yl)-1-(4-fluorophenyl)-1-butanone (340 mg) using the procedure described in EXAMPLE 448 Step B. ¹H NMR (DMSO, 300 MHz) δ 1.92-2.13 (m, 2H), 2.28 (br-s, 2H), 2.80 (t, 1H, J=9.9 Hz), 2.92-3.63 (m, 12H), 3.63-3.75 (m, 1H), 6.67 (t, 1H, J=7.9), 6.87 (d, 1H, J=8.9), 6.96 (d, 1H, J=6.9), 7.30-7.48 (m, 2H), 7.48-8.12 (m, 2H), 10.43 (br-s, 1H) ppm. m/z= 397[C₂₃H₂₅FN₂OS+H]⁺.

### EXAMPLE 472

### 4-[6,7,8a,9,10,12,13,13a-octahydro-5H,11H-azepino[4,5-b][1,4]thiazepino[2,3,4-hi]indol-11-yl]-1-(4-fluorophenyl)-1-butanone

6,7,9,20,11,12,13,13a-Octahydro-5*H*,8a*H*-azepino[4,5-*b*][1,4]thiazepino[2,3,4-*hi*]indole (200 mg, 0.78 mmol), 4-chloro-4'-fluorobutyrophenone (213 mg, 1.6 mmol), KI (129 mg, 0.78 mmol), K₂CO₃ (322 mg, 2.3 mmol), and 2 drops of TEA were suspended in MEK (4 mL). The mixture was heated for 60 hrs. The reaction was cooled to room temperature and concentrated. The residue was purified by column chromatography (5, 7, 10% MeOH/CH₂Cl₂) to afford the title compound (234 mg, 0.55 mmol, 71%) as a pale-brown viscous oil. ¹H NMR (CDCl₃, 300 MHz) δ 6.95 (2 H, ddd, 2.9 Hz, 5.1 Hz, 12.1 Hz), 7.06 (2H, m), 6.91 (1H, 7.7 Hz), 6.74 (1H, d, 7.3 Hz), 6.55 (1H, t, 7.7 Hz), 3.65-3.75 (1H, m), 3.45-3.61 (2H, m), 3.29-3.38 (1H, m), 2.85-3.0 (4H, m), 2.2-2.8 (6H, m), 1.7-2.2 (8H, m). MS (ESI): 425.3 (base, M+H)

### EXAMPLE 473

### (8aS,13aS)-11-[3-(4-fluorophenoxy)propyl]-6,7,9,10,11,12,13,13a-octahydro-5H,8aH-azepino[4,5-b][1,4]thiazepino[2,3,4-hi]indole

6,7,9,10,11,12,13,13a-Octahydro-5*H*,8a*H*-azepino[4,5-*b*][1,4]thiazepino[2,3,4-*hi*]indole (43.3 mg, 0.17 mmol), 1-(3-chloropropoxy)-4-fluorobenzene (40.8 mg, 0.22 mmol), KI (27.6 mg, 0.17 mmol), and K₂CO₃ (69mg, 0.50 mmol) were suspended in 4 mL of MEK. The suspension was heated at 80°C for 18 hrs. The reaction was cooled to rt and concentrated. The residue was purified by column chromatography (5, 7, 10% MeOH/CH₂Cl₂) to afford the racemic title compound (24.5 mg, 0.06 mmol, 35%) as a pale-brown viscous oil. The enantiomers were separated on a Chiracel OD column using hexane/IPA/TFA (75/25/0.1) as the eluent to give the title compound. ¹H NMR (CDCl₃, 300 MHz) δ 6.85-6.95 (2 H, m), 7.06 (2H, m), 6.68-6.80 (2H, m), 6.53 (1H, t, 7.6 Hz), 3.90 (2H, t, 6.2 Hz), 3.70-3.80 (1H, m), 3.32-3.59 (3H, m), 2.71-2.94 (3H, m), 2.46-2.58 (5H, m), 1.7-2.1 (8H, m). MS (ESI): 413.3 (base, M+H)

### EXAMPLE 474

### 11-[2-(6-fluoro-1,2-benzisoxazol-3-yl)propyl]-6,7,9,10,11,12,13,13a-octahydro-5H,8aH-azepino[4,5-b][1,4]thiazepino[2,3,4-hi]indole

The title compound was prepared by the same general method as example 473 from 6,7,9,10,11,12,13,13a-octahydro-5*H*,8a*H*-azepino[4,5-*b*][1,4]thiazepino[2,3,4-*hi*]indole (45 mg, 0.17 mmol) and 3-(3-chloropropyl)-1,2-benzisoxazole (61.3 mmol, 0.35 mmol) to afford the desired product (46.1 mg, 11 mmol, 62%) after chromatographic purification. . ¹H NMR (CDCl₃, 300 MHz) δ 7.71 (1 H, dd, 5.1 Hz, 8.8 Hz), 7.23 (1H, dd, 1.8 Hz, 8.4 Hz), 7.06 (1H, dt, 2.2 Hz, 8.4 Hz), 6.94 (1H, br-d, 7.6 Hz), 6.80 (1h, 7.4 Hz), 6.59 (1H, t, 7.3 Hz), 3.81 (1H, ddd, 5.1 Hz, 9.9 Hz, 13.6 Hz), 3.38-3.65 (3H, m), 2.92-3.00 (3H, m), 2.68-2.88 (2H, m), 2.44-2.61 (5H, m), 1.87-2.12 (8H, m). MS (ESI): 438.2 (base, M+H)

### EXAMPLE 475

### 4-[6,7,8a,9,10,12,13,13a-octahydro-5H,11H-azepino[4,5-b][1,4]thiazepino[2,3,4-hi]indol-11-yl]-1-(4-pyridinyl)-1-butanone

The title compound was prepared by the same general method as example 473 from 6,7,9,10,11,12,13,13a-octahydro-5*H*,8a*H*-azepino[4,5-*b*][1,4]thiazepino[2,3,4-*hi*]indole (275 mg, 1.1 mmol) and 4-chloro-1-(4-pyridinyl)-1-butanone (387 mmol, 2.1 mmol) to afford the desired product (60.2 mg, 0.15 mmol, 14%) after chromatographic purification.. ¹H NMR (CDCl₃, 300 MHz) δ 8.81 (2H ,dd, 1.4 Hz, 4.4 Hz), 7.73 (2H, dd, 1.4 Hz, 4.4 Hz), 6.99 (1H, d, 7.7 Hz), 6.81 (1H, d, 7.3 Hz), 6.61-6.60 (1H, m), 3.5-3.8 (3H, m), 3.3-3.5 (1H, m), 2.5-3.1 (9H, m), 1.8-2.4 (10 H, m). MS (ESI): 408.4 (base, M+H)

### EXAMPLE 483

### 4-[2-bromo-6,7,8a,9,10,12,13,13a-octahydro-5H,11H-azepino[4,5-b][1,4]thiazepino[2,3,4-hi]indol-11-yl]-1-(4-fluorophenyl)-1-butanone

2-Bromo-6,7,9,10,11,12,13,13a-octahydro-5*H*,8a*H*-azepino[4,5-*b*][1,4]thiazepino[2,3,4-*hi*]indole (121mg, 0.36 mg), 4-chloro-4'-fluorobutyrophenone (107 mg, 0.53 mmol), KI (59.2 mg, 0.36 mmol), and K2CO3 (148 mg, 1.1 mmol) were added to 4 mL of MEK. The reaction was refluxed for 18 hr. The reaction was then cooled to rt and concentrated. The resulting residue was immediately purified by column chromatography (2, 5, 7% MeOH/CH2Cl) to afford the title compound (115.6 mg, 64%) as a light-brown oil. ¹H NMR (CDCl₃, 300 MHz) δ 7.96-8.02 (2H, m), 7.10-7.16 (2H, m), 7.05-7.06 (1h, m), 6.86-6.87 (1H, m), 3.73-3.83 (1H, m), 3.59 (1H, dt, 4Hz, 10.3 Hz), 3.36-3.51 (2H, m), 2.91-3.00 (3H, m), 2.76-2.84 (2H, m), 2.59-2.65 (1H, m), 2.42-2.53 (4H, m), 1.81-2.10 (9H, m). MS (ESI): 505.3(base, M+H)

### EXAMPLE 484

### 3-[2-bromo-6,7,8a,9,10,12,13,13a-octahydro-5H,11H-azepino[4,5-b][1,4]thiazepino[2,3,4-hi]indol-11-yl]propyl 4-fluorophenyl ether

3-[2-Bromo-6,7,8a,9,10,12,13,13a-octahydro-5*H*,11*H*-azepino[4,5-*b*][1,4]thiazepino[2,3,4-*hi*]indol-11-yl]propyl 4-fluorophenyl ether was prepared by the same general method as example 483 from 2-bromo-6,7,9,10,11,12,13,13a-octahydro-5*H*,8a*H*-azepino[4,5-*b*][1,4]thiazepino[2,3,4-*hi*]indole (141 mg, 0.42 mmol) and 1-(3-chloropropoxy)-4-fluorobenzene (118 mg, 0.62 mmol) to afford the title compound (158 mg, 77%) after chromatographic purification. ¹H NMR (CDCl₃, 300 MHz) δ 7.08 (1H, 1.8 Hz), 6.93-6.99 (2H, m), 6.88-.689 (1H, m), 6.78-6.85 (2H, m), 3.97 (2H, t, 6.2 Hz), 3.72-3.82 (1H, m), 3,64 (1H, dt, 3.6 Hz, 10.2 Hz), 3.36-3.56 (2H, m), 2.78-2.99 (3H, m), 2.59-2.76 (5H, m), 1.89-2.11 (8H, m). MS (ESI): 491.2 +(base, M+H)

### EXAMPLE 485

### Methyl 4-(6,7,9,10,12,12a-hexahydro-5H-pyrido[4,3-b][1,4]thiazepino[2,3,4-hi]indol-11(8aH)-yl)-2-methylbutanoate

### Step A:

3-Methyldihydro-2(3*H*)-furanone (2.13 g, 21,3 mmol) was dissolved in CH₂Cl₂ (15 mL). BBr₃ was added drop-wise as a solution in 1M CH₂Cl₂. The reaction was stirred at rt for 18 hrs. MeOH (2 mL)was added at room temperature. Saturated aqueous Na₂CO₃ (20 mL) and CH₂Cl₂ (10 mL) were added. The layers were separated, and the aqueous was reextracted with CH₂Cl₂ (2 X 20 mL). The combined organic layers were washed with brine, dried, and concentrated to afford 3.77 g of a brown liquid. This crude product was purified by column chromatography to afford methyl 4-bromo-2-methylbutanoate (3.39 g, 82%) as a clear liquid. ¹H NMR (CDCl₃, 300 MHz) δ 3.69 (3H, s), 3.42 (2H, t, 6.6 Hz), 2.68-2.75 (1H, m), 2.20-2.32 (1H, m), 1.86-1.97 (1H, m), 1.19 (3H, d, 7.0 Hz).

### Step B:

6,7,8a,9,10,11,12,12a-Octahydro-5*H*-pyrido[4,3-*b*][1,4]thiazepino[2,3,4-*hi*]indole (302 mg, 1.24 mmol), methyl 4-bromo-2-methylbutanoate (315 mg, 1.62 mmol), KI (206 mg 1.24 mmol), and K₂CO₃ (514 mg, 3.72 mmol) were suspended in MEK. The mixture was refluxed for 18 hrs. The reaction was cooled and concentrated. The residue was purified by column chromatography (3, 5, 7% MeOH/CH₂Cl₂) to afford the title compound (477 mg, 99%) as a clear, amorphous solid. ¹H NMR (CDCl₃, 300 MHz) δ 6.93 (1H, d, 7.7 Hz), 6.84 (1H, d, 7.3 Hz), 6.61 (1H, m), 3.71-3.86 (1H, m), 3.67 (3H, d, 5.1 Hz), 2.5-3.3 ( 10H, m), 1.4-2.4 (10H, m), 1.12 (3H, d, 6.9 Hz). CI MS (NH3): 390 (base, M+H)

### EXAMPLE 486

### 4-(6,7,9,10,12,12a-hexahydro-5H-pyrido[4,3-b][2,4]thiazepino[2,3,4-hi]indol-11(8aH)-yl)-1,1-bis(4-fluorophenyl)-2-methyl-1-butanol

Methyl 4-(6,7,9,10,12,12a-hexahydro-5*H*-pyrido[4,3-*b*][1,4]thiazepino[2,3,4-*hi*]indol-11(8a*H*)-yl)-2-methylbutanoate (110.5 mg, 0.28 mmol) was dissolved in THF (0.5 mL). 4-Fluorophenylmagnesium bromide (1.4 mL, 1.4 mmol) was added as a 1M solution in THF. The reaction was stirred at room temperature for 20 hrs. The reaction was quenched with 1M HCl (5mL). The reaction was extracted with CH₂Cl₂ (3 X 15 mL). The combined organic layers were washed with brine, dried, and concentrated to afford 125 mg of a viscous oil. This crude material was purified by column chromatography to afford the title compound (72.9 mg, 50%) as a light-yellow oil. ¹H NMR (CDCl₃, 300 MHz) δ 7.61-7.68 (2H, m), 7.46-7.51 (2H, m), 6.8-7.1 (6H, m), 6.5-6.7 (2H, m), 3.7-4.0 (1H, m), 3.4-3.7 (1H, m), 3.1-3.4 (2H, m), 2.5-3.1 (5H, m), 2.3-2.45 (1H, m), 1.7-2.2 (8H, m), 1.2-1.4 (1H, m) , 0.88 (3H, d, 7.0 Hz). CI MS (NH3) : 521 (base, M+H)

### EXAMPLE 487

### 4-(6,7,9,10,12,12a-hexahydro-5H-pyrido[4,3-b][1,4]thiazepino[2,3,4-hi]indol-11(8aH)-yl)-1,1-bis(4-chlorophenyl)-2-methyl-1-butanol

4-(6,7,9,10,12,12a-Hexahydro-5*H*-pyrido[4,3-*b*] [1,4]thiazepino[2,3,4-*hi*]indol-11(8a*H*)-yl)-1,1-bis(4-chlorophenyl)-2-methyl-1-butanol was prepared by the same general method as example 486 from methyl 4-((8a*S*,12a*R*)-6,7,9,10,12,12a-hexahydro-5*H*-pyrido[4,3-*b*][1,4]thiazepino[2,3,4-*hi*]indol-11(8a*H*)-yl)-2-methylbutanoate (108 mg, 0.36 mmol) and 4-chlorophenylmagnesium bromide (1.8 mL, 1.8 mmol) to afford the title compound (106.9 mg, 54%) as a pale yellow amorphous solid. ¹H NMR (CDCl₃, 300 MHz) δ 7.5-7.7 (2H, m), 7.2-7.5 (4H, m), 7.15-7.25 (2H, m), 6.8-7.0 (2H, m), 6.5-6.7 (2H, m), 3.7-3.9 (1H, m), 3.4-3.65 (1H, m), 3.2-3.4 (2H, m), 2.5-3.1 (5H, m), 2.3-2.5 (1H, m), 1.7-2.2 (7 H, m), 1.2-1.5 (2H, m), 0.88 (3H, d, 7.0 Hz). ESI MS: 553.2 (base, M+H)

### EXAMPLE 489

### 4-(6,7,9,10,12,12a-hexahydro-5H-pyrido[4,3-b][1,4]thiazepino[2,3,4-hi]indol-11(8aH)-yl)-1-(4-fluorophenyl)-2-methyl-1-butanone

### Step A:

*N*-*O*-Dimethylhydroxylamine hydrochloride (1.11 g 11.4 mmol) was dissolved in toluene (30mL). This solution was cooled to 0°C, and a 2M solution of AlMe₃ (8.5 mL, 17 mmol) in toluene was added. The reaction was warmed to room temperature and stirred for 1 hr. Th reaction was then recooled to 0°C, and then methyl 4-(6,7,9,10,12,12a-hexahydro-5*H*-pyrido[4,3-*b*][1,4]thiazepino[2,3,4-*hi*]indol-11(8a*H*)-yl)-2-methylbutanoate (2.05 g, 5.71 mmol) was added as a solution in toluene (20 mL). The reaction was stirred at room temperature for 1hr, and then at 4°C for 18 hrs. The reaction was warmed to room temperature and stirred for 2 hrs. More *N*-O-dimethylhydroxylamine hydrochloride (277 mg, 2.83 mmol) and AlMe₃ (1.4 mL, 2.8 mmol) were added. The reaction was stirred 2 more hrs at rt. It was cooled back to 0°C and quenched with 1M aqueous tartaric acid (25 mL). The layers were separated, and the aqueous was extracted with CHCl₃ (3 X 15mL). The combined organic layers were washed with brine, dried, and concentrated to afford 3.0 g of an amorphous solid. The crude product was purified by column chromatography (5, 7% MeOH/CH₂Cl₂) to afford 4-(6,7,9,10,12,12a-hexahydro-5*H*-pyrido[4,3-*b*][1,4]thiazepino[2,3,4-*hi*]indol-11(8a*H*)-yl)-*N*-methoxy-*N*,2-dimethylbutanamide (1.81g, 81%) as a light-yellow amorphous solid.

### Step B:

4-(6,7,9,10,12,12a-Hexahydro-5*H*-pyrido[4,3-*b*][1,4]thiazepino[2,3,4-*hi*]indol-11(8a*H*)-yl)-*N*-methoxy-*N*,2-dimethylbutanamide (109.9 mg, 0.28 mmol) was dissolved in THF (0.5 mL). 4-fluorophenylmagnesium bromide (1.41 mL, 1.41 mmol) was added as a 1M solution in THF. The reaction was stirred at room temperature for 18 hrs. More THF (1mL) and 4-fluorophenylmagnesium bromide (1.41 mL, 1.41 mmol) were added. The reaction was stirred for 2 more hrs and then it was quenched with 1M HCl (3 mL). The layers were separated, and then the aqueous was basified with 1M NaOH (aq) until pH=14. The aqueous phase was extracted with CH₂Cl₂ (3 x 10 mL). The combined organic layers were washed with brine, dried, and concentrated to afford 167.7 mg of a brown oil. This material was purified by column chromatography (5, 7, 10% MeOH/CH2Cl2) to afford the title compound (36.4 mg, 31%) as a light-yellow amorphous solid. ¹H NMR (CDCl3, 300 MHz) δ 7.9-8.1 (2H, m), 7.0-7.2 (2H, m), 6.93 (1H, d, 7.7 Hz), 6.7-6.9 (1H, m), 6.5-6.6 (1H, m), 3.6-3.9 (1H, m), 3.4-3.6 (2H, m), 2.7-3.2 (4H, m), 2.3-2.7 (2 H, m), 1.7-2.3 (7H, m), 1.18 (3H, d, 7.0 Hz). ESI MS: 425.3 (base, M+H).

### EXAMPLE 490

### 4-(6,7,9,10,12,12a-hexahydro-5H-pyrido[4,3-b][1,4]thiazepino[2,3,4-hi]indol-11(8aH)-yl)-1-(4-fluoro-2-methoxyphenyl)-2-methyl-1-butanone

4-(6,7,9,10,12,12a-Hexahydro-5*H*-pyrido[4,3-*b*][1,4]thiazepino[2,3,4-*hi*]indol-11(8a*H*)-yl)-*N*-methoxy-*N*,2-dimethylbutanamide (48 mg, 0.12 mmol) was dissolved in THF (0.5 mL). 4-Fluoro-2-methoxyphenylmagnesium bromide (0.62 mL, 0.62 mmol) was added as a 1M solution in THF. The reaction was stirred at room temperature for 8 hrs, and then more 4-fluoro-2-methoxyphenylmagnesium bromide (0.62 mL, 0.62 mmol) was added. The reaction was stirred for 18 hrs, and then quenched with 1M HCl (3mL). The layers were separated, and the aqueous was basified with 1M NaOH until pH=14. The aqueous was extracted with CH₂Cl₂ (3 X 10 mL). The combined organic layers were washed with brine, dried and concentrated to afford 65 mg of a brown material. This crude product was purified by column chromatography (5, 7, 10% MeOH/CH₂Cl₂) to afford the title compound (45 mg, 82%) as light-yellow amorphous solid. ¹H NMR (CDCl3, 300 MHz) δ 7.27-7.32 (1H, m), 7.09-7.15 (1 H, m), 6.80-6.95 (3H, m), 6.57-6.62 (1H, m), 3.7-2.9 (4H, m), 3.3-3.6 (3H, m), 3.1-3.3 (1H, m), 2.8-3.1 (3H, m), 2.5-2.7 (2H, m), 1.7-2.4 (10H, m), 1.4-1.7 (1H, m), 1.13 (3H, d, 6.9 Hz). CI MS (NH3): 455 (base, M+H).

### EXAMPLE 491

### 4-(6,7,9,10,12,12a-hexahydro-5H-pyrido[4,3-b][1,4]thiazepino[2,3,4-hi]indol-11(8aH)-yl)-1-(4-fluoro-3-methylphenyl)-2-methyl-1-butanone

4-(6,7,9,10,12,12a-Hexahydro-5*H*-pyrido[4,3-*b*][1,4]thiazepino[2,3,4-*hi*]indol-11(8a*H*)-yl)-1-(4-fluoro-3-methylphenyl)-2-methyl-1-butanone was prepared by the same general method as example 489, step B from 4-(6,7,9,10,12,12a-hexahydro-5*H*-pyrido[4,3-*b*][1,4]thiazepino[2,3,4-*hi*]indol-11(8a*H*)-yl)-*N*-methoxy-*N*,2-dimethylbutanamide (108.2 mg, 0.28 mmol) and 4-fluoro-3-methylphenylmagnesium bromide (1.39 mL, 1.39 mmol) to afford the title compound (45.6 mg, 37%) after chromatographic purification. ¹H NMR (CDCl3, 300 MHz) δ 7.82-7.84 (2H, m), 7.05-7.080 (1 H, m), 6.91-6.94 (1H, m), 6.82-6.85 (1H, m), 6.59-6.60 (1H, m), 3.4-3.9 (4H, m), 2.8-3.2 (4H, m), 2.4-2.7 (2H, m), 1.7-2.4 (13H, m), 1.17 (3H, d, 7.0 Hz). ESI MS: 439.3 (M+H).

### EXAMPLE 492

### 4-(6,7,9,10,12,12a-hexahydro-5H-pyrido[4,3-b][1,4]thiazepino[2,3,4-hi]indol-11(8aH)-yl)-2-methyl-1-(2-methylphenyl)-1-butanone

4-(6,7,9,10,12,12a-Hexahydro-5*H*-pyrido[4,3-*b*][1,4]thiazepino[2,3,4-*hi*]indol-11(8a*H*)-yl)-*N*-methoxy-*N*,2-dimethylbutanamide (92.2 mg, 0.31 mmol) was dissolved in THF (0.5 mL). 2-Methylphenylmagnesium bromide (0.77 mL, 0.77 mmol) was added as a 1M solution in THF. The reaction was stirred for 18 hrs, and then more 2-methylphenylmagnesium bromide (0.39 mL, 0.39 mmol) was added. The reaction was stirred for another 24 hrs, and then quenched with 1M HCl (3mL). The layers were separated, and the aqueous was basified with 1M NaOH until pH=14. The aqueous was extracted with CH₂Cl₂ (3 X 10 mL). The combined organic layers were washed with brine, dried and concentrated to afford 105 mg of a brown material. This crude product was purified by column chromatography (5, 7, 10% MeOH/CH₂Cl₂) to afford the title compound (73.2 mg, 56%) as light-yellow amorphous solid. ¹H NMR (CDCl3, 300 MHz) δ 7.59-7.64 (1H, m), 7.32-7.38 (1 H, m), 7.20-7.26 (1H, m), 7.20-7.22 (1H, m), 6.9-7.0 (1H, m), 6.8-6.9 (1H, m), 6.5-6.7 (1H, m), 3.3-3.9 (3H, m), 3.1-3.3 (1H, m), 2.8-3.1 (3H, m), 1.4-2.8 (15H, m), 1.14 (3H, d, 7.0 Hz). ESI MS: 421.3 (base, M+H).

### EXAMPLE 493

### 4-(6,7,9,10,12,12a-hexahydro-5H-pyrido[4,3-b][1,4]thiazepino[2,3,4-hi]indol-11(8aH)-yl)-2-methyl-1-phenyl-1-butanone

4-(6,7,9,10,12,12a-Hexahydro-5*H*-pyrido[4,3-*b*][1,4]thiazepino[2,3,4-*hi*]indol-11(8a*H*)-yl)-*N*-methoxy-*N*,2-dimethylbutanamide (52.5 mg, 0.13 mmol) was dissolved in THF (0.5 mL). Phenylmagnesium bromide(0.22 mL, 0.66 mmol) was added as a 1M solution in THF. The reaction was stirred at room temperature for 18 hrs, and then quenched with 1M HCl (3mL). The layers were separated, and the aqueous was basified with 1M NaOH until pH=14. The aqueous was extracted with CH₂Cl₂ (3 X 10 mL). The combined organic layers were washed with brine, dried and concentrated to afford 75 mg of a brown material. This crude product was purified by column chromatography (5, 7, 10% MeOH/CH₂Cl₂) to afford the title compound (14.1 mg, 27%) as light-yellow amorphous solid. ¹H NMR (CDCl3, 300 MHz) δ 7.95-7.99 (2H, m), 7.42-7.55 (3 H, m), 6.92 (1H, d, 8.1 Hz), 6.84 (1H, d, 6.6 Hz), 6.77 (1H, d, 6.6 Hz), 6.56-6-63 (1H, m), 3.4-3.7 (3H, m), 2.4-3.2 (5H, m), 1.5-2.4 (11H, m), 1.19 (3H, d, 7.0 Hz). ESI MS: 421.3 (base, M+H). CI MS (NH3): 407 (base, M+H).

### EXAMPLE 494

### 1-(2-aminophenyl)-4-((6bR,10aS)-1,2,6b,9,10,10a-hexahydro[1,4]oxazino[2,3,4-hi]pyrido[4,3-b]indol-8(7H)-yl)-1-butanone

The title compound was isolated as a yellow oil (105 mg, 41%) according to the method of Example 286, Step C from (6b*R*,10a*S*)-1,2,6b,7,8,9,10,10a-octahydro[1,4]oxazino[2,3,4-*hi*]pyrido[4,3-*b*]indole (150 mg, 0.68 mmol) and 1-(2-aminophenyl)-4-chloro-1-butanone (274 mg, 1.38 mmol). ¹H NMR (CDCl₃) δ 1.90-2.00 (m, 3H), 2.03-2.11 (m, 1H), 2.29-2.38 (m, 1H), 2.39-2.47 (m, 2H), 2.67-2.80 (m, 2H), 2.91-3.04 (m, 4H), 3.12-3.21 (m, 1H), 3.24-3.35 (m, 2H), 4.40-4.46 (m, 2H), 6.26 (br-s, 2H), 6.61-6.73 (m, 4H), 7.23-7.29 (m, 2H), 7.77 (dd, 1H, J = 1.3, 8.3 Hz) ppm.

### EXAMPLE 495

### 1-(2-aminophenyl)-4-((6bS,10aR)-1,2,6b,9,10,10a-hexahydro[1,4]oxazino[2,3,4-hi]pyrido[4,3-b]indol-8(7H)-yl)-1-butanone

The title compound was isolated as a yellow oil (115 mg, 44%) according to the method of Example 286, Step C from (6b*S*,10a*R*)-1,2,6b,7,8,9,10,10a-octahydro[1,4]oxazino[2,3,4-*hi*]pyrido[4,3-*b*]indole (150 mg, 0.68 mmol) and 1-(2-aminophenyl)-4-chloro-1-butanone (274 mg, 1.38 mmol). ¹H NMR (CDCl₃) δ 1.90-2.00 (m, 3H), 2.03-2.11 m, 1H), 2.29-2.38 (m, 1H), 2.39-2.47 (m, 2H), 2.67-2.80 (m, 2H), 2.91-3.04 (m, 4H), 3.12-3.21 (m, 1H), 3.24-3.35 (m, 2H), 4.40-4.46 (m, 2H), 6.26 (br-s, 2H), 6.61-6.73 (m, 4H), 7.23-7.29 (m, 2H), 7.77 (dd, 1H, *J* = 1.3, 8.3 Hz) ppm.

### EXAMPLE 496

### (6bR,10aS)-8-[3-(1H-indazol-3-yl)propyl]-1,2,6b,7,8,9,10,10a-octahydro[1,4]oxazino[2,3,4-hi]pyrido[4,3-b]indole

The title compound was isolated as a yellow oil (54 mg, 100%) according to the method of Example 286, Step C from (6b*R*,10a*S*)-1,2,6b,7,8,9,10,10a-octahydro[1,4]oxazino[2,3,4-*hi*]pyrido[4,3-*b*]indole (31 mg, 0.14 mmol) and 3-(3-chloropropyl)-1*H*-indazole (56 mg, 0.28). ¹H NMR (CDCl₃) δ 1.92-2.17 (m, 5H), 2.29-2.40 (m, 1H), 2.47-2.59 (m, 2H), 2.73-2.81 (m, 2H), 2.91-3.07 (m, 3H), 3.21-3.34 (m, 3H), 4.42-4.47 (m, 2H), 6.59-6.69 (m, 3H), 7.13 (t, 1H, *J* = 7.0 Hz), 7.33-7.45 (m, 2H), 7.70 (d, 1H, J = 8.1 Hz) ppm.

### EXAMPLE 528

### (6bR, 10aS)-8-[3-(6-fluoro-1H-indazol-3-yl)propyl]-1,2,6b,7,8,9,10,10a-octahydro[1,4]oxazino-[2,3,4-hi]pyrido[4,3-b]indole

The title compound was prepared by the method of Example 355 Step B as a yellow oil (60 mg, 63%) from (6b*R*, 10a*S*)-1,2,6b,7,8,9,10,10a-octahydro[1,4]oxazino-[2,3,4-*hi*]pyrido[4,3-*b*]indole (63 mg, 0.29 mmol) and 3-(3-chloropropyl)-6-fluoroindazole (93 mg, 0.43 mmol). ¹H NMR (CDCl₃, 300 MHz) δ 1.89-2.15 (m, 5H), 2.27-2.38 (m, 1H), 2.43-2.55 (m, 2H), 2.71-2.80 (m, 2H), 2.88-3.02 (m, 3H), 3.18-3.32 (m, 3H), 4.43 (dd, J = 6.2, 1.8 Hz, 2H) 6.60-6.72 (m, 3H), 6.90 (dt, J = 9.1, 2.2 Hz, 1H), 7.07 (dd, J = 9.2, 1.9 Hz, 1H), 7.63 (dd, J = 8.8, 5.1 Hz, 1H), 9.85-10.15 (br-s, 1H) ppm.

### EXAMPLE 529

### 1-(2-amino-4-fluorophenyl)-4-((±)-cis-1,2,6b,9,10,10a-hexahydro[1,4]oxazino-[2,3,4-hi]pyrido[4,3-b]indol-8(7H)-yl)-1-butanone

The title compound was prepared by the method of Example 361 as a red oil (11 mg, 24%) from (±)-*cis*-1,2,6b,7,8,9,10,10a-octahydro[1,4]oxazino-[2,3,4-*hi*]pyrido[4,3-*b*]indole (30 mg, 0.12 mmol) and 1-(2-amino-4-fluorophenyl)-4-chloro-1-butanone (51 mg, 0.24 mmol). ¹H NMR (CDCl₃, 300 MHz) δ 1.87-2.15 (m, 5H), 2.25-2.45 (m, 3H), 2.65-2.79 (m, 2H), 2.86-2.95 (m, 3H), 3.15-3.30 (m, 3H), 4.44 (dd, J = 6.9, 2.2 Hz, 2H), 6.26-6.38 (m, 2H), 6.41-6.50 (br-s, 2H), 6.60-6.72 (m, 3H), 7.78 (dd, J = 9.1, 6.6 Hz, 1H) ppm.

### EXAMPLE 530

### 1-(2-amino-4-fluorophenyl)-4-((6bR,10aS)-1,2,6b,9,10,10a-hexahydro[1,4]oxazino-[2,3,4-hi]pyrido[4,3-b]indol-8(7H)-yl)-1-butanone

The title compound was prepared by the method of Example 355 Step B as a yellow oil (172 mg, 57%) from (6b*R*,10a*S*)-1,2,6b,7,8,9,10,10a-octahydro[1,4]oxazino[2,3,4-*hi*]pyrido[4,3-*b*]indole (165 mg, 0.76 mmol) and 1-(2-amino-4-fluorophenyl)-4-chloro-1-butanone (329 mg, 1.5 mmol). The title compound was spectroscopically identical to Example 529.

### EXAMPLE 531

### 4-((±)-cis-1,2,6b,9,10,10a-hexahydro[1,4]oxazino[2,3,4-hi]pyrido[4,3-b]indol-8(7H)-yl)-1-(4-fluoro-2-hydroxyphenyl)-1-butanone

The title compound was prepared by the method of Example 361 as a yellow oil (8 mg, 17%) from (±)-*cis*-1,2,6b,7,8,9,10,10a-octahydro[1,4]oxazino-[2,3,4-*hi*]pyrido[4,3-*b*]indole (30 mg, 0.12 mmol) and 4-chloro-1-(4-fluoro-2-hydroxyphenyl)-1-butanone (52 mg, 0.24 mmol). ¹H NMR (CDCl₃, 300 MHz) δ 1.85-2.13 (m, 5H), 2.25-2.47 (m, 3H), 2.63-2.95 (m, 3H), 2.99 (t, J = 7.0 Hz, 2H), 3.17-3.35 (m, 3H), 4.43 (dd, J = 6.9, 2.2 Hz, 2H), 6.59-6.75 (m, 5H), 7.81 (dd, J = 8.7, 6.6 Hz, 1H) ppm.

### EXAMPLE 539

### 3-(1,2,6b,7,8,10,11,11a-octahydro-9H-azepino[4,5-b][1,4]oxazino[2,3,4-hi]indol-9-yl)propyl 4-fluorophenyl ether hydrochloride

### Typical procedure for alkylation of amines:

A mixture of indoline hydrochloride (approx 200 mg) in dioxane (4 mL) was treated with Hunig's base (10 equivs) and heated to reflux for 15 min. To the cooled reaction mixture was added appropriate side chain (5 equivs), KI (0.9 equivs), then the whole mixture was refluxed for 48 hr. The reaction was then diluted with chloroform (20 mL) and extracted once with saturated solution of ammonium chloride (10 mL) and twice with ice-cold water (100 mL). The organic layer was dried over sodium sulfate and concentrated to dryness under reduced pressure. The residue was purified by flash chromatography eluting with a gradient of hexane/ethylacetate (e.g. 96:4 to 50:50), following with a gradient methanol/ dichloromethane (e.g.1:99 to 3:97) to give the desired product.

3-(1,2,6b,7,8,10,11,11a-octahydro-9*H*-azepino[4,5-*b*][1,4]oxazino[2,3,4-*hi*]indol-9-yl)propyl 4-fluorophenyl ether (400 mg, 62%) was obtained from the alkylation of 1,2,7,8,9,10,11,11a-octahydro-6b*H*-azepino[4,5-*b*][1,4]oxazino[2,3,4-*hi*]indole (560 mg, 1.69 mmol) with 1-(3-chloropropoxy)-4-fluorobenzene (1.32 mL, 8.47 mmol) using the procedures described above. ¹H NMR (300 MHz, CDCl₃) δ 1.79-1.99 (m, 1H), 2.10 (d, 1H, J=12.2 Hz), 2.39-2.57 (m, 1H), 2.72-3.01 (m, 3H), 3.01-3.22 (m, 2H), 3.22-3.51 (m, 3H), 3.61-3.72 (m, 1H), 6.69-6.89 (m, 1H), 7.17 (d, 1H, J=7.0 Hz), 7.42 (d, 1H, J=9.6 Hz) ppm. CI MS m/z= 249 [MH]⁺. The title compound (380 mg, 89%) was obtained by the procedure described in Example 535 Step B. ¹H NMR (300 MHz, CD₃OD) δ 2.19-2.60 (m, 5H), 2.59-2.96 (m, 1H), 3.01-3.19 (m, 1H), 3.25-3.91 (m, 9H), 3.92-4.21 (m, 2H), 4.32-4.61 (m, 2H), 6.53-6.89 (m, 2H), 6.88-7.22 (m, 5H); CI MS m/z= 383 [MH]⁺.

### EXAMPLE 540

### 9-[3-(6-fluoro-1,2-benzisoxazol-3-yl)propyl]-1,2,7,8,9,10,11,11a-octahydro-6bH-azepino[4,5-b][1,4]oxazino[2,3,4-hi]indole hydrochloride

9-[3-(6-Fluoro-1,2-benzisoxazol-3-yl)propyl]-1,2,7,8,9,10,11,11a-octahydro-6b*H*-azepino[4,5-*b*][1,4]oxazino[2,3,4-*hi*]indole (351 mg, 48%) was obtained from the alkylation of 1,2,7,8,9,10,11,11a-octahydro-6b*H*-azepino[4,5-*b*][1,4]oxazino[2,3,4-*hi*]indole (595 mg, 1.80 mmol) with 3-(3-chloropropyl)-6-fluoro-1,2-benzisoxazole (1.54 g, 7.20 mmol) using the procedure described in Example 539. ¹H NMR (CD₃OD, 300 MHz) δ 2.10-2.51 (m, 4H), 2.53-2.80 (m, 2H), 2.80-3.00 (m, 1H), 3.10-3.25 (m, 3H), 3.23-3.65 (m, 4H), 3.70-3.90 (m, 3H), 4.12 (q, 1H, J = 9.0 Hz), 4.40-4.60 (m, 2H), 6.58-6.80 (m, 1H), 6.92 (d, 1H, J = 8.1 Hz), 7.06 (t, 1H, J = 8.1 Hz), 7.12-7.25 (m, 1H), 7.35-7.45 (m, 1H), 7.80-7.90 (m, 1H) ppm. CIMS (Methane) m/z = 408 [MH]⁺.

### EXAMPLE 544

### 4- (6,7,9,10,12,12a-hexahydro-5H-[1,4]oxazepino[2,3,4-hi]pyrido[4,3-b]indol-11(8aH)-yl)-1-(4-fluorophenyl)-1-butanone hydrochloride

4-(6,7,9,10,12,12a-hexahydro-5*H*-[1,4]oxazepino[2,3,4-*hi*]pyrido[4,3-*b*]indol-11(8a*H*)-yl)-1-(4-fluorophenyl)-1-butanone(100 mg, 55%) was obtained from the alkylation of 6,7,8a,9,10,11,12,12a-octahydro-5*H*-[1,4]oxazepino[2,3,4-*hi*]pyrido[4,3-*b*]indole (188 mg, 0.7 mmol) with 4-chloro-4'-fluoro-butyrophenone (608, 3.5 mmoles) using the procedure described in EXAMPLE 539. The title compound was obtained using the procedure described in EXAMPLE 534 Step C. ¹H NMR (CD₃OD, 300 MHz) δ 2.01-2.29 (m, 4H), 2.43-2.61 (m, 2H), 3.11-3.80 (m, 13H), 4.39-4.59 (m, 1H), 6.71-6.83 (m, 2H), 6.82-6.91 (m,, 1H), 7.18-7.20 (m, 2H), 8.03-8.13 (m, 2H) ppm. CIMS (Methane) m/z= 395 [MH]⁺.

### EXAMPLE 546

### 4-(1,2,6b,7,8,10,11,11a-octahydro-9H-azepino[4,5-b][1,4]oxazino[2,3,4-hi]indol-9-yl)-1-(4-fluorophenyl)-1-butanone

1,2,7,8,9,10,11,11a-octahydro-6b*H*-azepino[4,5-*b*][1,4]oxazino[2,3,4-*hi*]indole (97.8 mg, 0.42 mmol), 4-chloro-1-(4-fluorophenyl)-1-butanone (172 mg, 0.86 mmol), KI (71.2 mg, 0.43 mmol), and DIEA (550 mg, 4.3 mmol) were suspended in dioxane (2 mL). The reaction mixture was refluxed for 18 hrs. After cooling to rt, the solution was concentrated. The residue was immediately purified by column chromatography to afford the title compound . MS - ESI: 395[MH] ⁺.

### EXAMPLE 591

### 4-((8aS, 12aR)-3-chloro-6, 7, 9, 10, 12, 12a-hexahydro-5H-pyrido[4,3-b][1,4]thiazepino[2, 3, 4-hi]indol-11(8aH)-yl)-1-(2-amino-4-fluorophenyl)-1-butanone

(8a*S*, 12a*R*)-3-Chloro-6, 7, 8a, 9, 10, 11, 12, 12a-octahydro-5*H*-pyrido[4,3-b][1,4]thiazepino[2, 3, 4-*hi*]indole (0.090 g, 0.38 mmol) was stirred with 1-(2-amino-4-fluorophenyl)-5-1-pentanone (0.164 g, 0.76 mmol), potassium carbonate (0.210 g, 1.52 mmol) and potassium iodide (0.020 g, 0.120 mmol) in 1,4 dioxane (2 mL) and refluxed for 72 hours. H₂O (5 mL) was added and the aqueous layer was extracted with CHCl₃ (3 x 10 mL). The combined extracts were washed with brine (10 mL) and dried (MgSO₄) and evaporated. The resulting residue was purified by silica gel column chromatography (2.5% MeOH/ CH₂Cl₂) affording the title compound in 27% yield (0.046 g). ¹H NMR (CDCl₃, 300 MHz) 7.78 - 7.81 (m, 2H), 3.73 (s, 1H), 6.24 - 6.42 (m, 4H), 3.98 - 4.07 (m, 1H), 3.63 - 3.78 (m, 2H), 3.23 - 3.31 (m, 2H), 2.81 - 3.07 (m, 4H), 2.61 - 2.80 (m, 3H), 2.11 - 2.31 (m, 2H), 1. 82 - 2.01 (m, 6H) ppm.

### UTILITY

The compounds of the present invention have therapeutic utility for illnesses or disorders involving the neurotransmitter serotonin (5-hydroxy tryptamine or 5-HT) and either agonism or antagonism of 5-HT2 receptors, as demonstrated by the assays described below. Therapeutic utility for these illnesses or disorders could involve numerous biological processes affected by serotonin including, but not limited to, appetite, mood, sleep, sexual activity, and arterial constriction. These biological processes may also be important to numerous central nervous system (CNS) disorders including those related to the affective disorders of depression, anxiety, psychosis, and schizophrenia, as well as, disorders of food intake such as anorexia, bulemia, and obesity. The compounds of the present invention potentially have therapeutic utility in other conditions in which serotonin has been implicated, such as migraine, attention deficit disorder or attention deficit hyperactivity disorder, addictive behavior, and obsessive-compulsive disorder, as well as, conditions associated with cephalic pain, social phobias, and gastrointestinal disorders such as dysfunction of the gastrointestinal tract motility. Lastly, compounds of the present invention potentially have therapeutic utility in neurodegenerative diseases and traumatic conditions represented by the examples of Alzheimer's disease and brain/spinal cord trauma.

The pharmacological analysis of each compound fro either antogonism or agonism of at 5-HT2A and 5-HT2C receptors consisted of in vitro and in vivo studies. In vitro analyses included Kᵢ determinations at 5-HT2A and 5-HT2C receptors and an assessment of functional (i.e., agonism or antagonism) activity at each receptor class by IP3 hydrolysis assays. Additional receptor assays were conducted to evaluate receptor specificity of 5-HT2A and 5-HT2C receptors over monoamine and nuisance receptors (e.g. histamine, dopamine, and muscarinic). A compound is considered active as a 5-HT2A antagonist or a 5-HT2C agonist if it has an IC₅₀ value or a Kᵢ value of less than about 1 micromolar; preferably less than about 0.1 micromolar; more preferably less than about 0.01 micromolar. Compounds of the invention have been shown to have an IC₅₀ value of less than about 1 micromolar for 5-HT2A antagonism or a 5-HT2C agonism.

In vivo assays assessed compound activity in a variety of behavioral paradigms including quipazine head twitch, acute and chronic feeding models, anxiety and depression models (learned-helplessness, elevated plus maze, Geller-Siefter, conditioned taste aversion, taste reactivity, satiety sequence). In aggregate, these models reflect activity as a 5-HT2A antagonist (quipazine head twitch, depression models) or 5-HT2C agonist (feeding models, anxiety models, depression models) and provide some indication as to bioavailability, metabolism and pharmacokinetics.

Radioligand binding experiments were conducted on recombinant human 5-HT2A and 5-HT2C receptors expressed in HEK293E cells. The affinities of compounds of the present invention to bind at these receptors is determined by their capacity to compete for [¹²⁵I]-1-(2,5-dimethoxy-4-iodophenyl)-2-amino-propane (DOI) binding at the 5-HT2A or 5-HT2C. General references for binding assays include 1) Lucaites VL, Nelson DL, Wainscott DB, Baez M (1996) Receptor subtype and density determine the coupling repertoire of the 5-HT2 receptor subfamily. Life Sci., 59(13):1081-95. J Med Chem 1988 Jan;31(1):5-7; 2) Glennon RA, Seggel MR, Soine WH, Herrick-Davis K, Lyon RA, Titeler M (1988) [125I]-1-(2,5-dimethoxy-4-iodophenyl)-2-aminopropane: an iodinated radioligand that specifically labels the agonist high-affinity state of 5-HT2 serotonin receptors. J Med. Chem. 31(1):5-7 and 3) Leonhardt S, Gorospe E, Hoffman BJ, Teitler M (1992) Molecular pharmacological differences in the interaction of serotonin with 5-hydroxytryptamine1C and 5-hydroxytryptamine2 receptors. Mol Pharmacol., 42(2):328-35.

The functional properties of compounds (efficacy and potency) were determined in whole cells expressing 5-HT2A or 5-HT2C receptors by assessing their ability to stimulate or inhibit receptor-mediated phosphoinositol hydrolysis. The procedures used are described below.

### In Vitro Binding Assays

### Stable expression of 5-HT2A and 5-HT2C receptors in HEK293E cells.

Stable cell lines were generated by transfecting 293EBNA cells with plasmids containing human 5-HT2A , 5-HT2B, or 5-HT2C (VNV edited isoform) cDNA using calcium phosphate. These plasmids also contained the cytomegalovirus (CMV) immediate early promoter to drive receptor expression and EBV oriP for their maintenance as an extrachromosomal element, and the hph gene from E. Coli to yield hygromycin B resistance (Horlick et al., 1997). Transfected cells were maintained in Dulbecco's Modified Eagle medium (DMEM) containing dialyzed 10% fetal bovine serum at 37°C in a humid environment (5% CO₂) for 10 days. The 5-HT2A cells were adapted to spinner culture for bulk processing whereas it was necessary to maintain the other lines as adherent cultures. On the day of harvest, cells were washed in phosphate-buffered saline (PBS), counted, and stored at -80 °C.

### Membrane Preparation

On the day of assay, pellets of whole cells (containing approximately 1 X 108 cells) expressing the 5-HT2A or 5-HT2C receptor were thawed on ice and homogenized in 50 mM Tris HCl (pH 7.7) containing 1.0 mM EDTA using a Brinkman Polytron (PT-10, setting 6 for 10 sec). The homogenate was centrifuged at 48,000 x g for 10 min and the resulting pellet washed twice by repeated homogenization and centrifugation steps. The final pellet was resuspended in tissue buffer and protein determinations were made by the bichichoninic acid (BCA) assay (Pierce Co., IL) using bovine serum albumin as the standard.

### Radioligand binding assays for the 5-HT2A ,and 5-HT2C receptors.

Radioligand binding studies were conducted to determine the binding affinities (KI values) of compounds for the human recombinant 5-HT2A, 5-HT2B, and 5-HT2C receptors (Fitzgerald et al., 1999). Assays were conducted in disposable polypropylene 96-well plates (Costar Corp., Cambridge, MA) and were initiated by the addition of 5-HT2A , 5-HT2B, or 5-HT2C membrane homogenate in tissue buffer (10-30 (g/well) to assay buffer (50 mM Tris HCl, 0.5 mM EDTA, 10 mM pargyline, 10 mM MgSO₄, 0.05% ascorbic acid, pH 7.5) containing [¹²⁵I]DOI for the 5-HT2A and 5-HT2C receptors (0.3-0.5 nM, final) or [³H]LSD (2-2.5 nM, final) for the 5-HT2B receptor, with or without competing drug (i.e, newly synthesized chemical entity). For a typical competition experiment, a fixed concentration of radioligand was competed with duplicate concentrations of ligand (12 concentrations ranging from 10 picomolar to 10 micromolar). The reaction mixtures were incubated to equilibrium for 45 min at 37°C and terminated by rapid filtration (cell harvestor; Inotech Biosystems Inc., Lansing, MI) over GFF glass-fiber filters that had been pre-soaked in 0.3% polyethyleneimine. Filters were washed in ice-cold 50 mM Tris HCl buffer (pH 7.5) and then counted in a gamma counter for the 5-HT2A and 5-HT2C assays, or by liquid scintillation spectroscopy for the 5-HT2B assay.

### Phosphoinositide hydrolysis studies.

The ability of newly synthesized compounds to stimulate phosphoinositide (PI) hydrolysis was monitored in whole cells using a variant (Egan et al., 1998) of a protocol described previously (Berridge et al., 1982). HEK293E cells expressing the human 5-HT2A, 5-HT2B, or 5-HT2C receptor were lifted with 0.5 mM EDTA and plated at a density of 100,000/well onto poly-D-lysine-coated 24-well plates (Biocoat; Becton Dickinson, Bedford, MA) in Dulbecco's modified Eagle's serum (DMEM; Gibco BRL) containing high glucose, 2mM glutamine, 10% dialyzed fetal calf serum, 250 (g/ml hygromycin B, and 250(g/ml G418. Following a 24-48 hr period, the growth media was removed and replaced with DMEM without fetal calf serum and inositol (Gibco BRL). The cells were then incubated with DMEM (without serum and inositol) containing a final concentration of 0.5 uCi/well myo-[³H]inositol for 16-18 hr. Following this incubation, the cells were washed with DMEM (without serum or inositol) containing 10 mM LiCl and 10 (M pargyline and then incubated for 30 min with the same media but now containing one of several test compounds. Reactions were terminated by aspirating the media and lysing the cells by freeze-thaw. [³H]phosphoinositides were extracted with chloroform/methanol (1:2 v/v), separated by anion exchange chromatography (Bio-Rad AGI-X8 resin), and counted by liquid scintillation spectroscopy as described previously (Egan et al., 1998).

### Data analyses

The equilibrium apparent dissociation constants (Ki's) from the competition experiments were calculated using an iterative nonlinear regression curve-fitting program (GraphPad Prism; San Diego, CA). For the PI hydrolysis experiments, EC50's were calculated using a one-site 'pseudo' Hill model: y=((Rmax-Rmin)/(1+R/EC50)nH)) + Rmax where R= response (DeltaGraph, Monterey, CA). Emax (maximal response) was derived from the fitted curve maxima (net IP stimulation) for each compound. Intrinsic activity (IA) was determined by expressing the Emax of a compound as a percentage of the Emax of 5-HT (IA=1.0).

### In Vivo Experiments for Serotonergic Ligands.

### Preclinical Efficacy, Potency, and Side Effect Liability.

### a) Anti-Serotonin Efficacy.

Antagonism of Quipazine-Induced Head Twitch in Rat. Quipazine, an agonist at 5-HT receptors, produces a characteristic head twitch response in rats. 5-HT receptor antagonists effectively antagonize this 5-HT agonist-induced behavioral effect (Lucki et al., 1984). Accordingly, the quipazine-induced head twitch model in rat can function as an in vivo behavioral correlate to 5-HT receptor binding. Compounds are administered 30 minutes before behavioral testing (and 25 minutes before quipazine), and a dose-related antagonism of the quipazine response is determined.

### b) Antipsychotic Efficacy.

Inhibition of the Conditioned Avoidance Response (CAR) in Rat. Rats are trained to consistently avoid (by climbing onto a pole suspended from the ceiling of the test chamber) an electric foot shock (0.75 mA) delivered to the grid floor of the testing chamber. All antipsychotic drugs effectively inhibit this conditioned avoidance response (Arnt, 1982). The ability of a compound to inhibit this response is used to determine the antipsychotic efficacy of potential drug candidates.

### c) Extrapyramidal Side Effect Liability.

Induction of Catalepsy in Rat. Typical antipsychotic drugs produce extrapyramidal side effects (EPS) at clinically effective doses. The most widely accepted preclinical indicator of EPS liability in humans is a drug-induced catalepsy syndrome in rat (Costall and Naylor, 1975), a condition whereby the animal will remain immobile in an externally imposed posture (analogous to a catatonic stupor in humans). Rats are tested for induction of catalepsy in a dose-response test after oral administration of compounds.

### d) CNS penetration; In vivo brain receptor occupancy.

In Vivo Binding. To determine the level of in vivo receptor occupancy, an in vivo receptor binding protocol is used. This procedure uses an appropriate radioligand to label the receptor of interest. For example, to measure both Dopamine D2 and 5-HT2A receptors in vivo, one can use ³H-N-methyl spiperone (³H -NMSP), (Frost, et. al. 1987) The procedure uses rats (or mice) fasted overnight. To measure the effects of compounds on the receptors of interest, compounds are dosed, usually p.o. for example in 2 microliters/gram body weight in 0.25% Methocel suspension. The radiolabeled compound (in this example, ³H-NMSP) is administered by i.v. tail vein injection (10 microcuries label/200 gram rat). Time course experiments are used to determine the optimal time of binding for both the radiolabeled and unlabeled compound. These optimal time frames are used for all subsequent dose-response experiments. After the appropriate time frame of compound/radioligand exposure, the animals are sacrificed and the relevant brain regions dissected (frontal cortex for 5-HT2A and striatum for D2 receptors) and examined for their content of radioactivity. The level of non-specific binding is determined by examining a brain region known not to contain the receptor of interest (in this case the cerebellum) or by administering an excess of compound known pharmacologically to interact with the receptor.

### REFERENCES

Arnt, J. Acta Pharmacol. et Toxicol. 1982: 51, 321-329.
Berridge M.J., Downes P.C. , Hanley M.R. (1982) Lithium amplifies agonist-dependent phosphotidyinositol response in brain and salivary glands. Biochem. J., 206, 587-595.
Costall, B and Naylor, RJ. Psychopharmacology. 1975: 43, 69-74.
Egan C.T., Herrick-Davis K., Miller K., Glennon R.A., and Teitler M. (1998) Agonist activity of LSD and lisuride at cloned 5-HT2A and 5-HT2C receptors. Psychopharmacology, 136, 409-414.
Fitzgerald LW, Conklin DS, Krause CM, Marshall AP, Patterson JP, Tran DP, Iyer G, Kostich WA, Largent BL, Hartig PR (1999) High-affinity agonist binding correlates with efficacy (intrinsic activity) at the human serotonin 5-HT2A and 5-HT2C receptors: evidence favoring the ternary complex and two-state models of agonist action. J. Neurochem., 72, 2127-2134.
Frost, J.J., Smith, A.C., Kuhar, M.J., Dannals, R.F., Wagner, H.N., 1987, In Vivo Binding of 3H-N-Methylspiperone to Dopamine and Serotonin Receptors. Life Sciences, 40:987-995.
Horlick, R.A., Sperle, K., Breth, L.A., Reid, C.C., Shen, E.S., Robbinds, A.K., Cooke, G.M., Largent, B.L. (1997) Rapid Generation of stable cell lines expressing corticotrophin-releasing hormone receptor for drug discovery. Protein Expr. Purif. 9, 301-308.
Lucki, I, Nobler, M.S., Frazer, A., 1984, Differential actions of serotonin antagonists on two behavioral models of serotonin receptor activation in the rat. J. Pharmacol. Exp. Ther. 228(1):133-139.

### Dosage and Formulation

The serotonin agonist and serotonin antagonist compounds of this invention can be administered as treatment for the control or prevention of central nervous system disorders including obesity, anxiety, depression, psychosis, schizophrenia, sleep and sexual disorders, migraine and other conditions associated with cephalic pain, social phobias, and gastrointestinal disorders such as dysfunction of the gastrointestinal tract motility by any means that produces contact of the active agent with the agent's site of action, i.e., 5-HT2 receptors, in the body of a mammal. It can be administered by any conventional means available for use in conjunction with pharmaceuticals, either as an individual therapeutic agent or in a combination of therapeutic agents. It can be administered alone, but preferably is administered with a pharmaceutical carrier selected on the basis of the chosen route of administration and standard pharmaceutical practice.

The compounds of the present invention can be administered in such oral dosage forms as tablets, capsules (each of which includes sustained release or timed release formulations), pills, powders, granules, elixirs, tinctures, suspensions, syrups, and emulsions. Likewise, they may also be administered in intravenous (bolus or infusion), intraperitoneal, subcutaneous, or intramuscular form, all using dosage forms well known to those of ordinary skill in the pharmaceutical arts.

The dosage administered will, of course, vary depending upon known factors, such as the pharmacodynamic characteristics of the particular agent and its mode and route of administration; the age, health and weight of the recipient; the nature and extent of the symptoms; the kind of concurrent treatment; the frequency of treatment; and the effect desired. By way of general guidance, a daily dosage of active ingredient can be expected to be about 0.001 to about 1000 milligrams per kilogram of body weight, with the preferred dose being about 0.01 to about 100 mg/kg; with the more preferred dose being about 0.1 to about 30 mg/kg. Advantageously, compounds of the present invention may be administered in a single daily dose, or the total daily dosage may be administered in divided doses of two, three, or four times daily.

Dosage forms of compositions suitable for administration contain from about 1 mg to about 100 mg of active ingredient per unit. In these pharmaceutical compositions the active ingredient will ordinarily be present in an amount of about 0.5-95% by weight based on the total weight of the composition. The active ingredient can be administered orally in solid dosage forms, such as capsules, tablets and powders, or in liquid dosage forms, such as elixirs, syrups and suspensions. It can also be administered parenterally, in sterile liquid dosage forms.

Gelatin capsules contain the active ingredient and powdered carriers, such as lactose, starch, cellulose derivatives, magnesium stearate, stearic acid, and the like. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric coated for selective disintegration in the gastrointestinal tract. Liquid dosage forms for oral administration can contain coloring and flavoring to increase patient acceptance.

In general, water, a suitable oil, saline, aqueous dextrose (glucose), and related sugar solutions and glycols such as propylene glycol or polyethylene glycols are suitable carriers for parenteral solutions. Solutions for parenteral administration preferably contain a water soluble salt of the active ingredient, suitable stabilizing agents, and if necessary, buffer substances. Antioxidizing agents such as sodium bisulfite, sodium sulfite, or ascorbic acid, either alone or combined, are suitable stabilizing agents. Also used are citric acid and its salts, and sodium EDTA. In addition, parenteral solutions can contain preservatives, such as benzalkonium chloride, methyl- or propyl-paraben and chlorobutanol. Suitable pharmaceutical carriers are described in *Remington's Pharmaceutical Sciences, supra,* a standard reference text in this field.

Useful pharmaceutical dosage-forms for administration of the compounds of this invention can be illustrated as follows:

### Capsules

A large number of unit capsules can be prepared by filling standard two-piece hard gelatin capsules each with 100 mg of powdered active ingredient, 150 mg of lactose, 50 mg of cellulose, and 6 mg magnesium stearic.

### Soft Gelatin Capsules

A mixture of active ingredient in a digestible oil such as soybean oil, cottonseed oil or olive oil can be prepared and injected by means of a positive displacement pump into gelatin to form soft gelatin capsules containing 100 mg of the active ingredient. The capsules should then be washed and dried.

### Tablets

A large number of tablets can be prepared by conventional procedures so that the dosage unit is 100 mg of active ingredient, 0.2 mg of colloidal silicon dioxide, 5 milligrams of magnesium stearate, 275 mg of microcrystalline cellulose, 11 mg of starch and 98.8 mg of lactose. Appropriate coatings may be applied to increase palatability or delay absorption.

### Suspension

An aqueous suspension can be prepared for oral administration so that each 5 mL contain 25 mg of finely divided active ingredient, 200 mg of sodium carboxymethyl cellulose, 5 mg of sodium benzoate, 1.0 g of sorbitol solution, U.S.P., and 0.025 mg of vanillin.

### Injectable

A parenteral composition suitable for administration by injection can be prepared by stirring 1.5% by weight of active ingredient in 10% by volume propylene glycol and water. The solution is sterilized by commonly used techniques.

The Tables below provide representative Examples, the synthesis of which are described above, of the compounds of Formula (I) of the present invention.

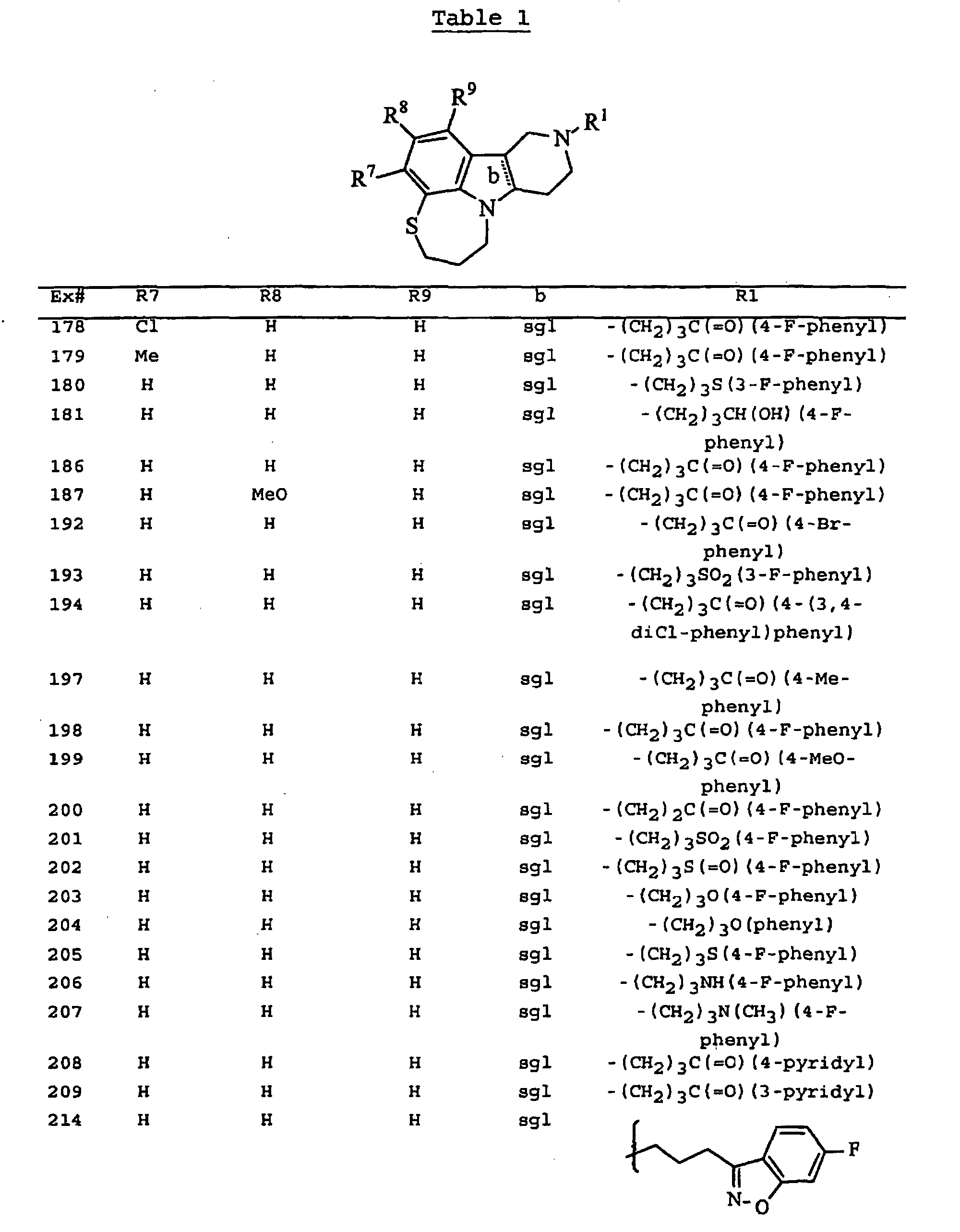

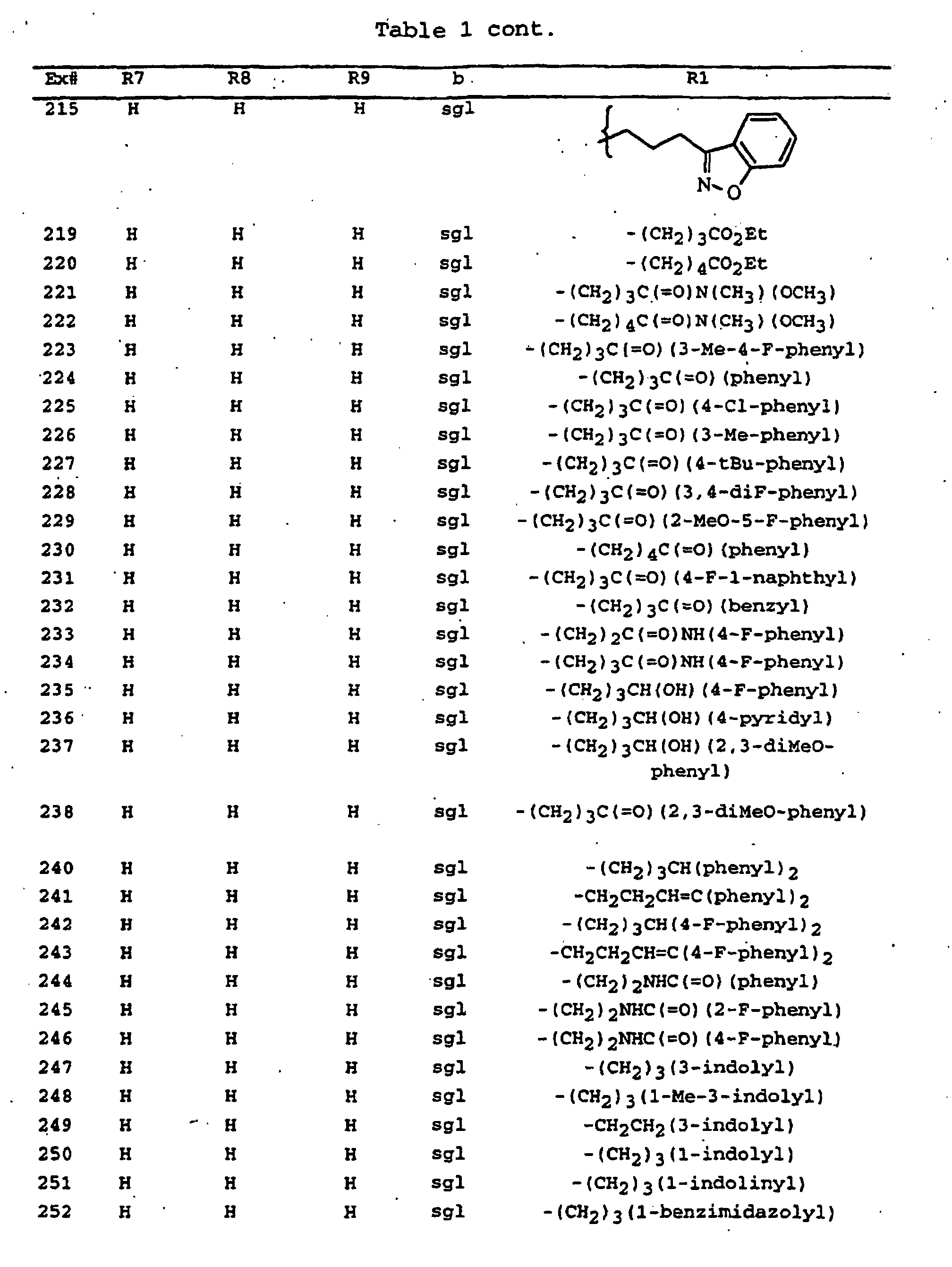

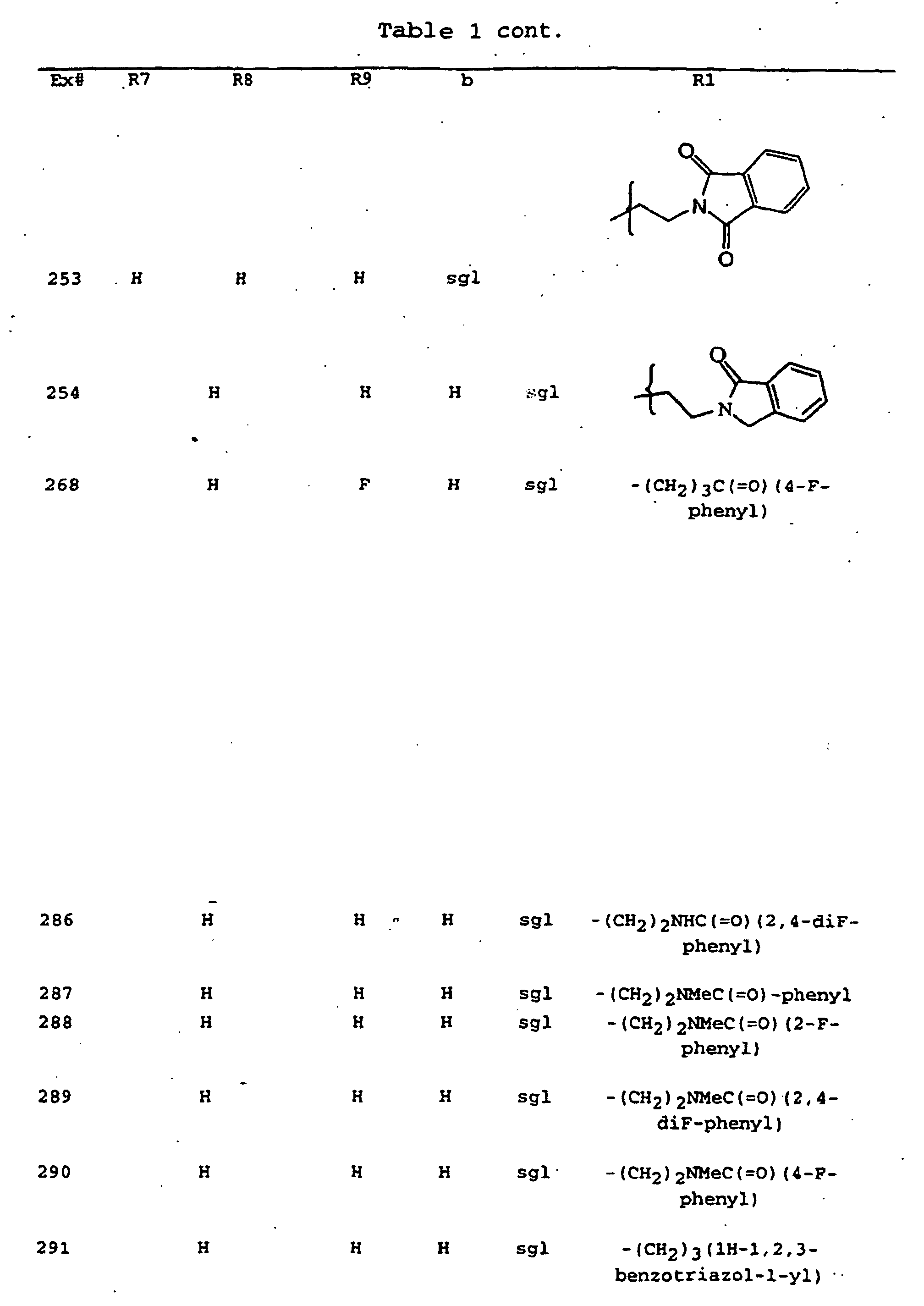

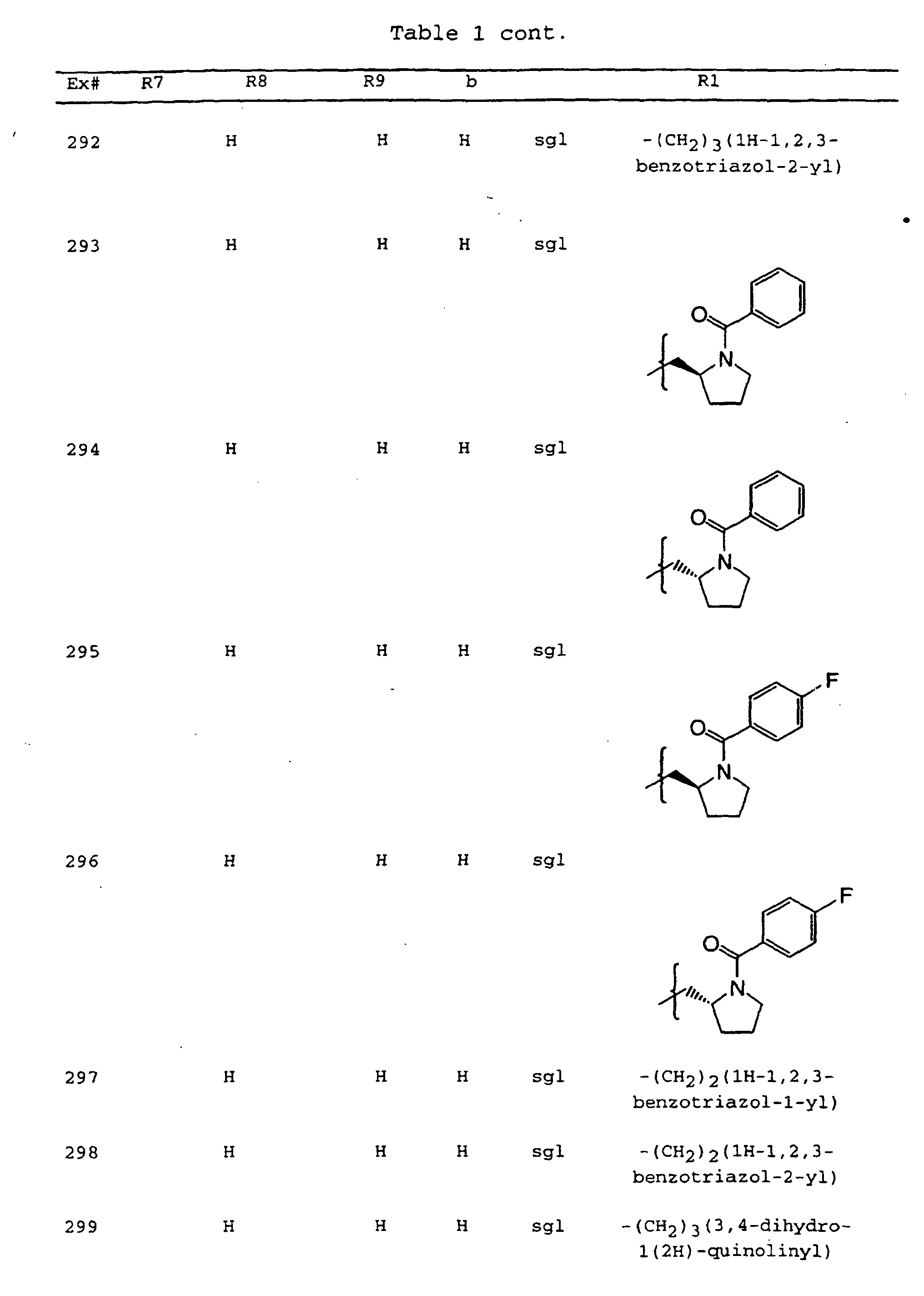

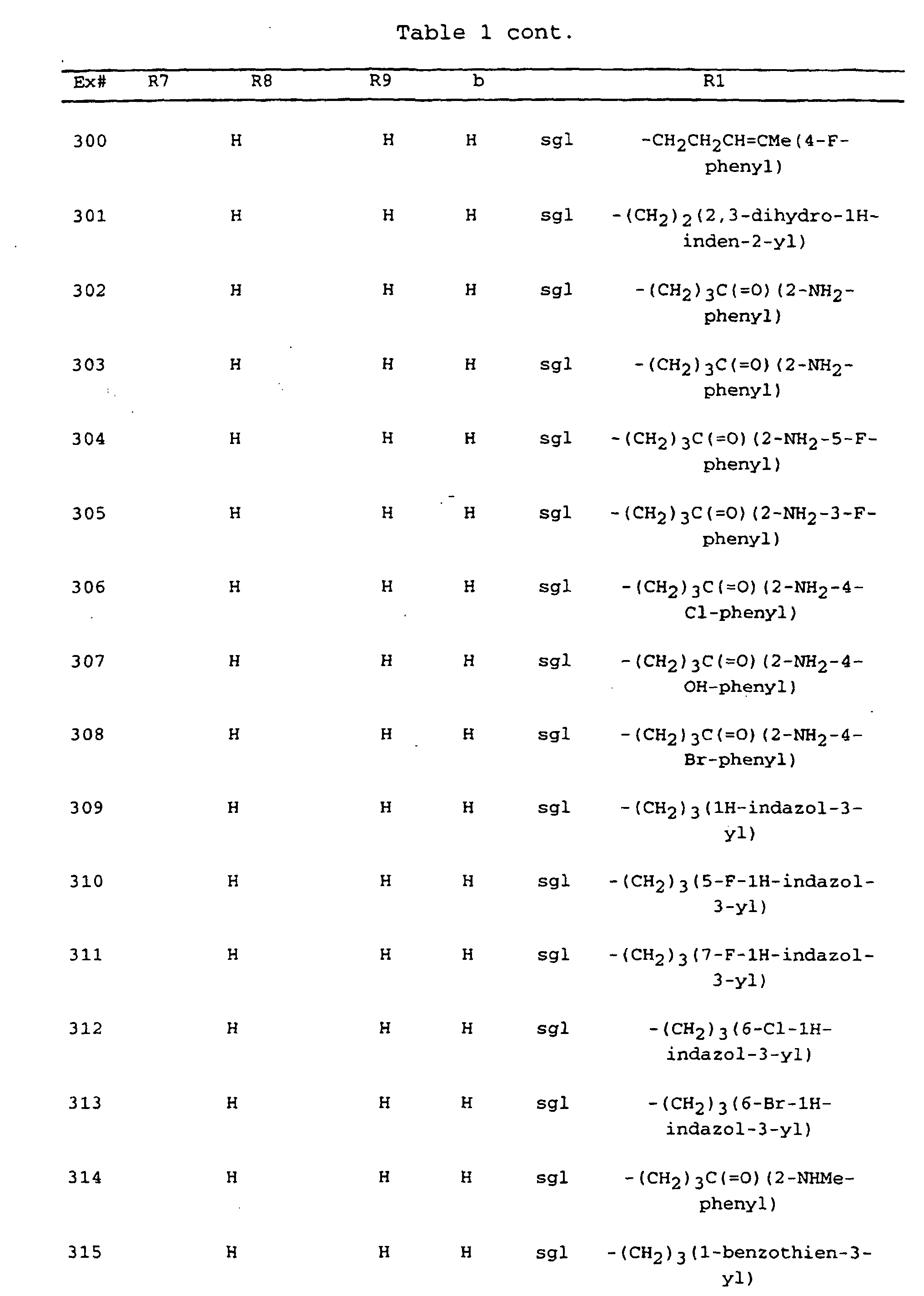

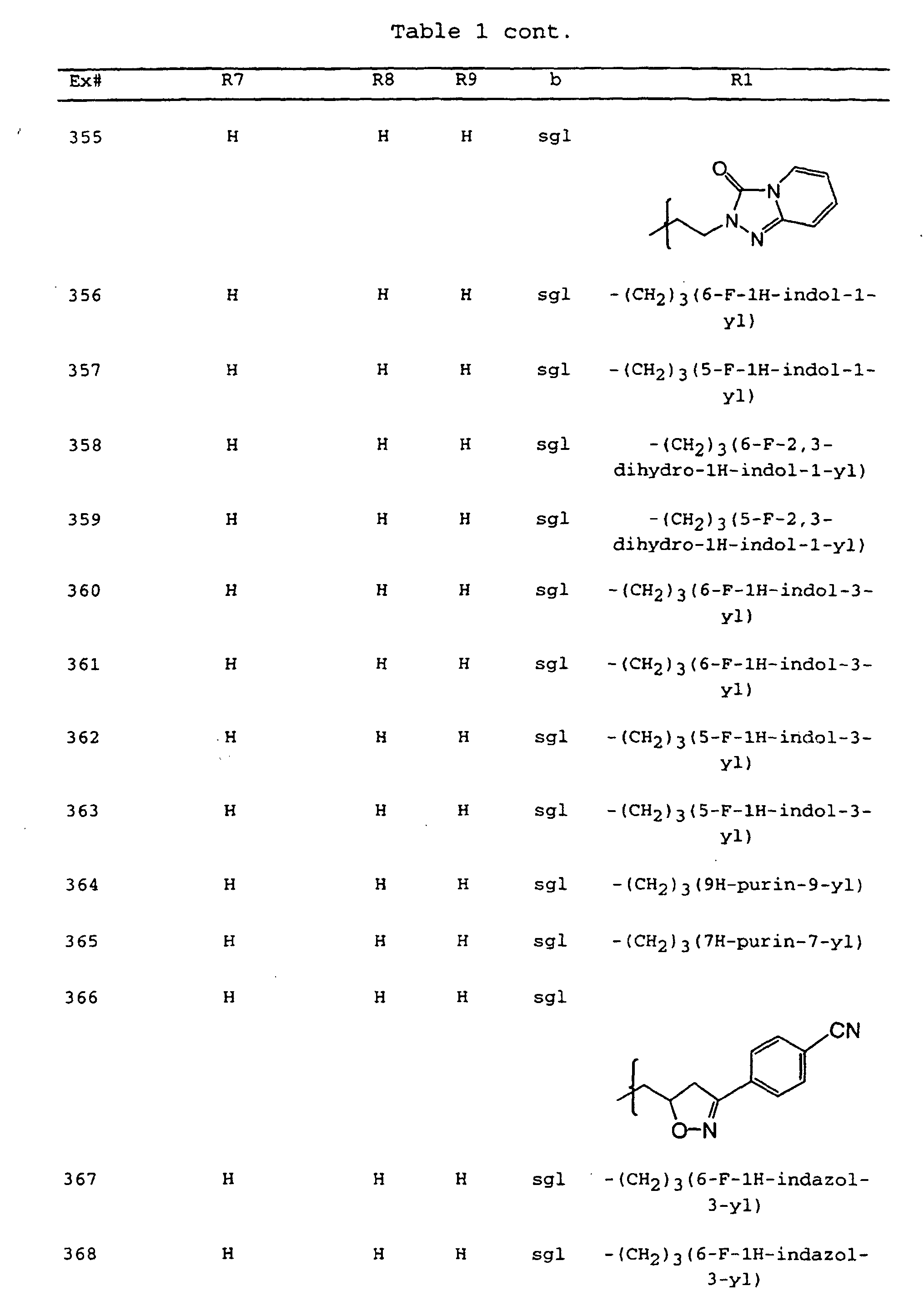

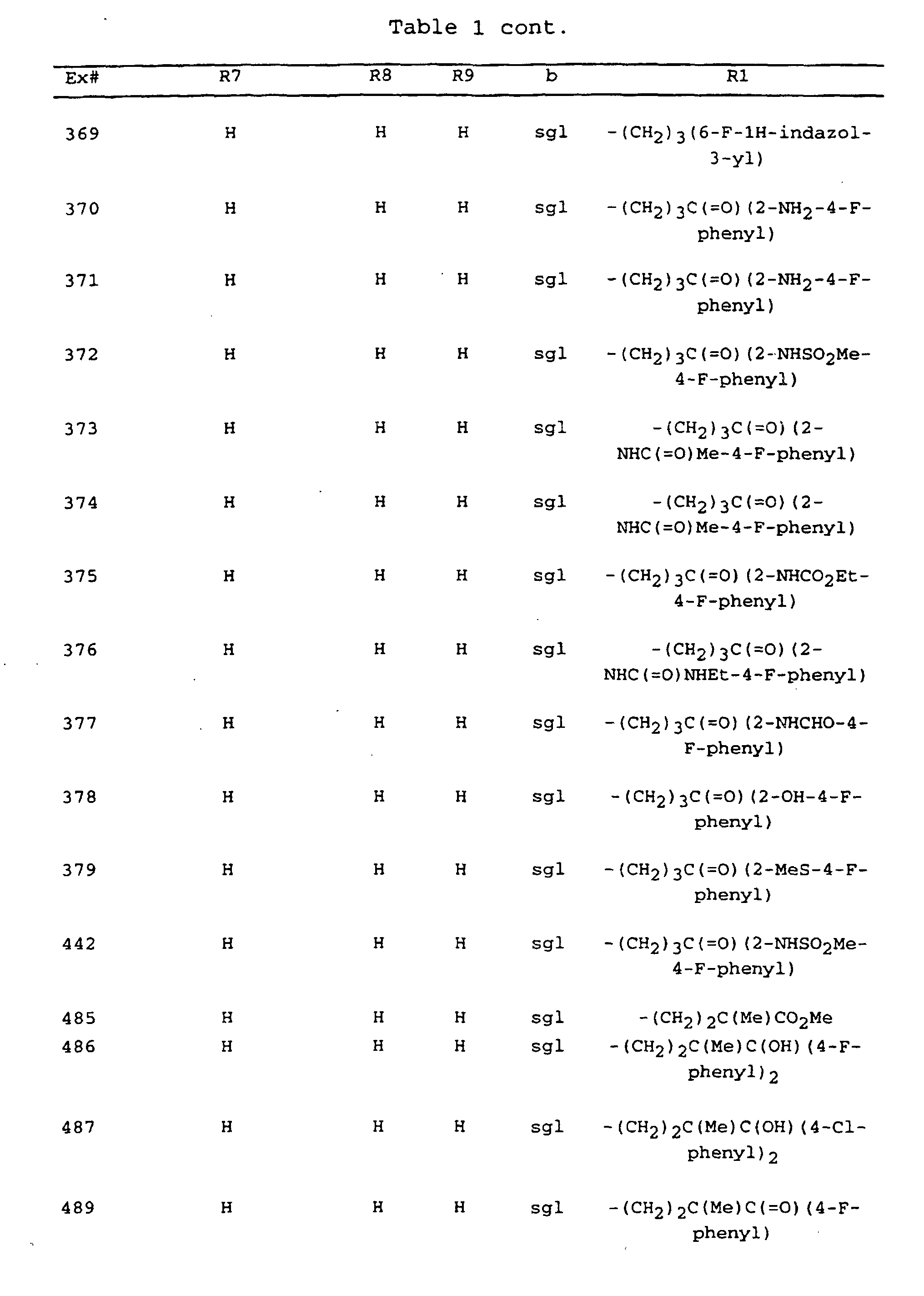

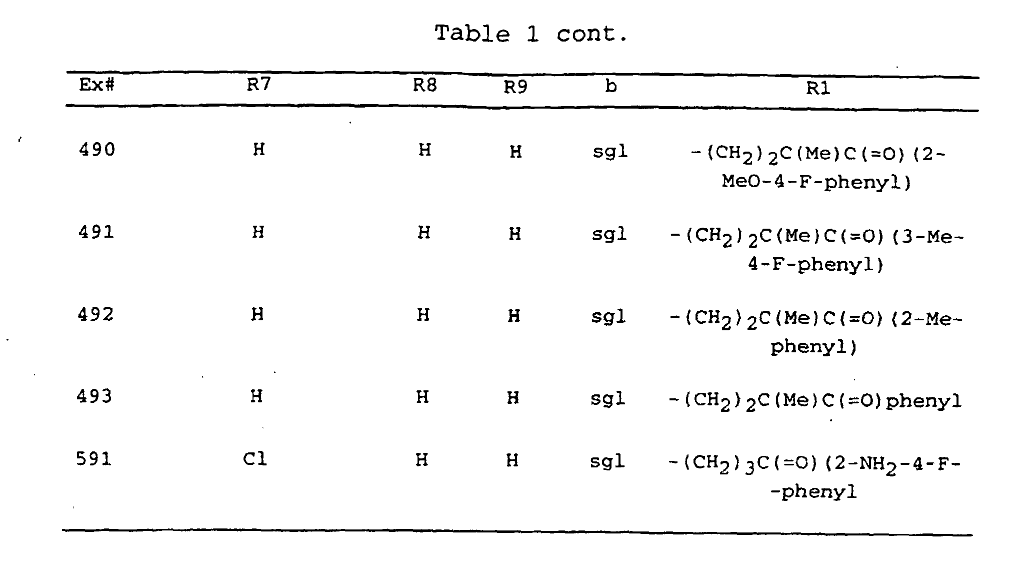

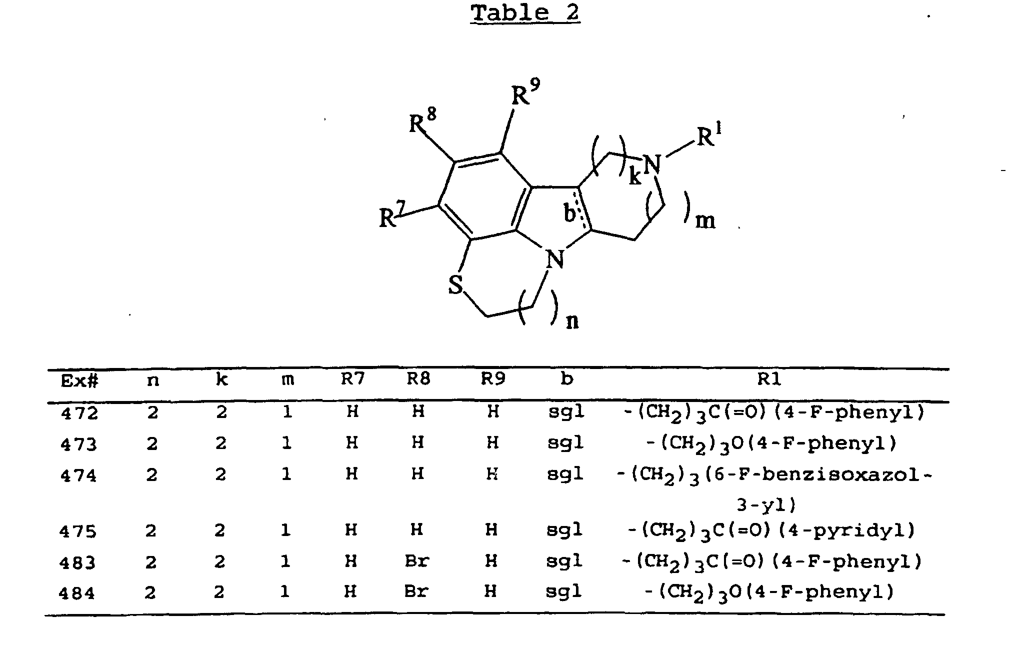

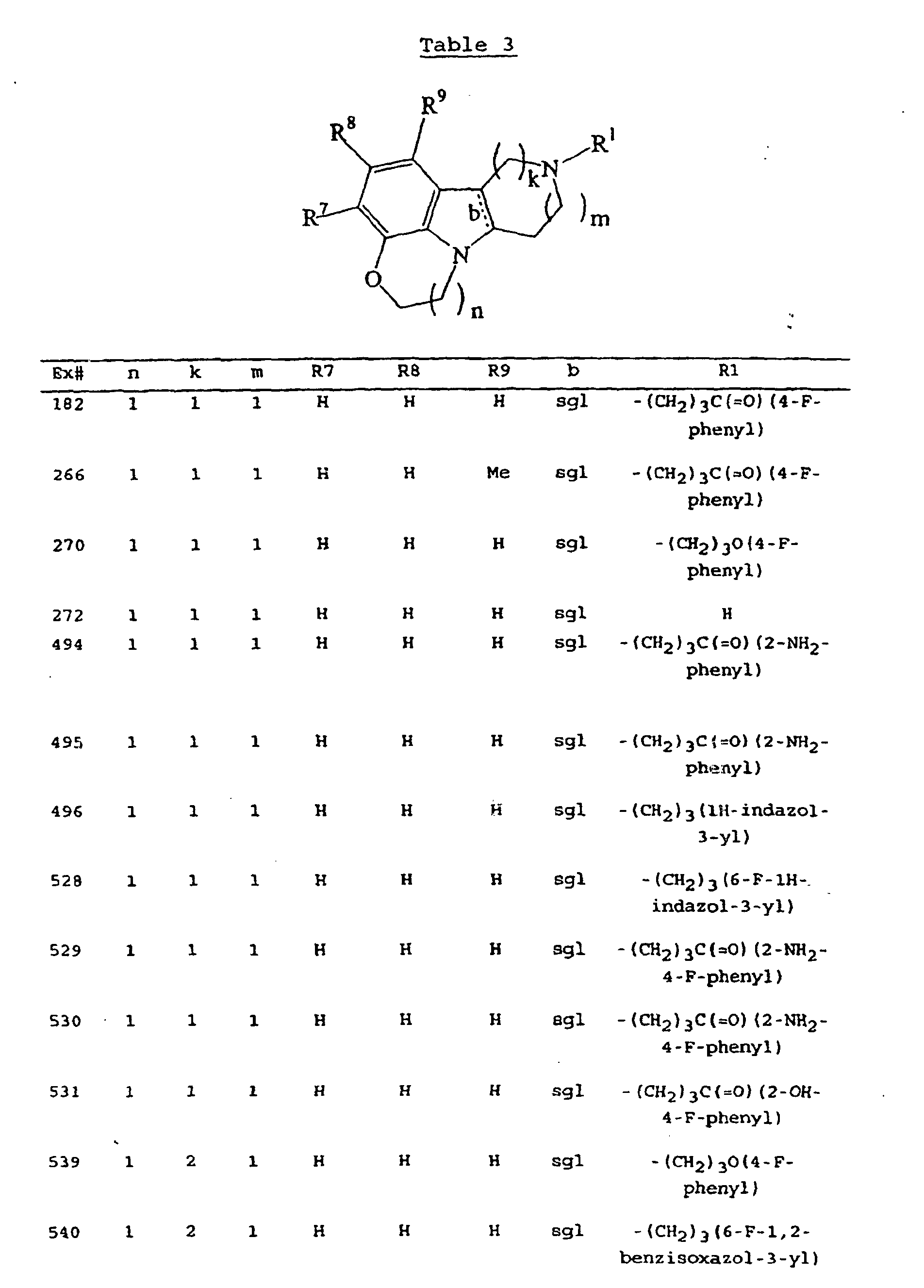

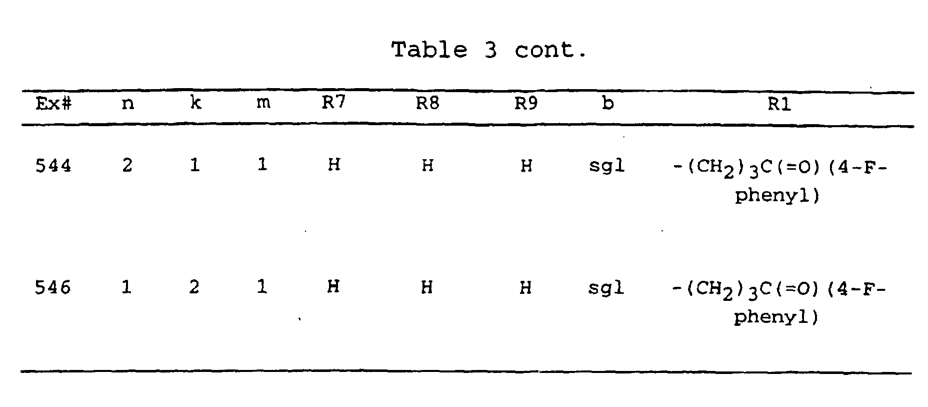

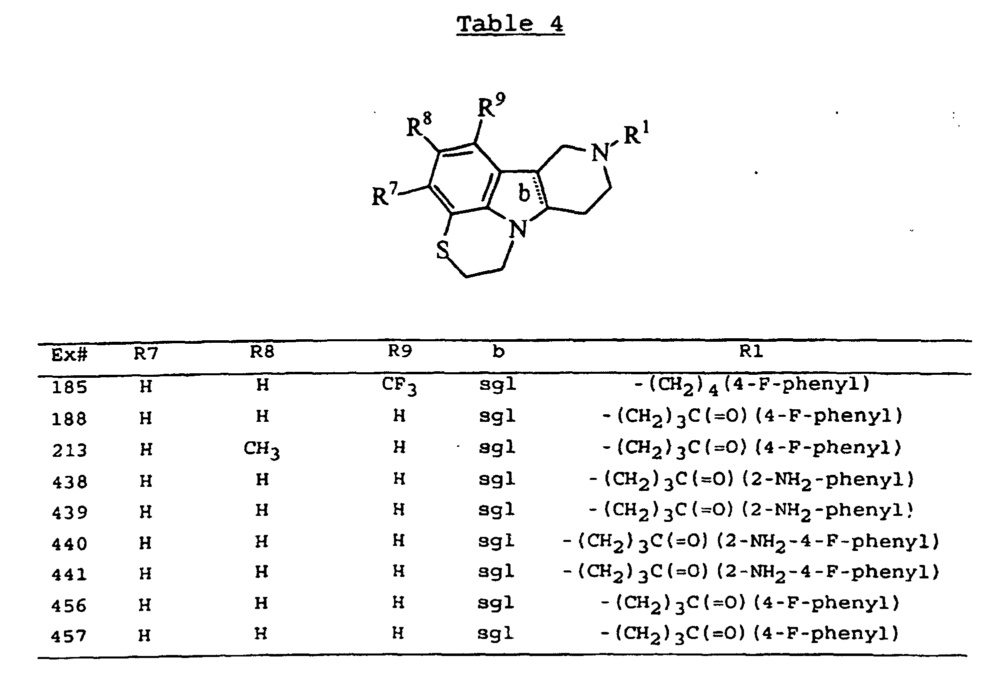

## Claims

1. A compound of the Formula (I-a) 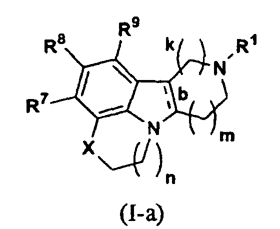 or pharmaceutically acceptable salt forms thereof, wherein:
b is a single bond;
X is -S- or -0-;
R¹ is selected from
-(CH₂)₃C(=O)(4-fluoro-phenyl),
-(CH₂)₃C(=O)(4-bromo-phenyl),
-(CH₂)₃C(=O)(4-methyl-phenyl),
-(CH₂)₃C(=O)(4-methoxy-phenyl),
-(CH₂)₃C(=O)(4-(3,4-dichloro-phenyl)phenyl),
-(CH₂)₃C(=O)(3-methyl-4-fluoro-phenyl),
-(CH₂)₃C(=O)(2,3-dimethoxy-phenyl),
-(CH₂)₃C(=O)(phenyl),
-(CH₂)₃C(=O)(4-chloro-phenyl),
-(CH₂)₃C(=O)(3-methyl-phenyl),
-(CH₂)₃C(=O)(4-t-butyl-phenyl),
-(CH₂)₃C(=O)(3,4-difluoro-phenyl),
-(CH₂)₃C(=O)(2-methoxy-5-fluoro-phenyl),
-(CH₂)₃C(=O)(4-fluoro-1-naphthyl),
-(CH₂)₃C(=O)(benzyl),
-(CH₂)₃C(=O)(4-pyridyl),
-(CH₂)₃C(=O)(3-pyridyl),
-(CH₂)₃CH(OH)(4-fluoro-phenyl),
-(CH₂)₃CH(OH)(4-pyridyl),
-(CH₂)₃CH(OH)(2,3-dimethoxy-phenyl),
-(CH₂)₃S(3-fluoro-phenyl),
-(CH₂)₃S(4-fluoro-phenyl),
-(CH₂)₃S(=O)(4-fluoro-phenyl),
-(CH₂)₃SO₂(3-fluoro-phenyl),
-(CH₂)₃SO₂(4-fluoro-phenyl),
-(CH₂)₃O(4-fluoro-phenyl),
-(CH₂)₃O(phenyl),
-(CH₂)₃O(3-pyridyl),
-(CH₂)₃O(4-pyridyl),
-(CH₂)₃O(2-NH₂-phenyl),
-(CH₂)₃O(2-NH₂-5-F-phenyl),
-(CH₂)₃O(2-NH₂-4-F-phenyl),
-(CH₂)₃O(2-NH₂-3-F-phenyl),
-(CH₂)₃O(2-NH₂-4-Cl-phenyl),
-(CH₂)₃O(2-NH₂-4-OH-phenyl),
-(CH₂)₃O(2-NH₂-4-Br-phenyl),
-(CH₂)₃O(2-NHC(=O)Me-4-F-phenyl),
-(CH₂)₃O(2-NHC(=O)Me-phenyl),
-(CH₂)₃NH(4-fluoro-phenyl),
-(CH₂)₃N(methyl)(4-fluoro-phenyl),
-CH₂)₃CO₂(ethyl),
-(CH₂)₃C(=O)N(methyl)(methoxy),
-(CH₂)₃C(=O)NH(4-fluoro-phenyl),
-(CH₂)₂NHC(=O)(phenyl),
-(CH₂)₂NMeC(=O)(phenyl),
-(CH₂)₂NHC(=O)(2-fluoro-phenyl),
-(CH₂)₂NMeC(=O)(2-fluoro-phenyl),
-(CH₂)₂NHC(=O)(4-fluoro-phenyl),
-(CH₂)₂NMeC(=O)(4-fluoro-phenyl),
-(CH₂)₂NHC(=O)(2,4-difluoro-phenyl),
-(CH₂)₂NMeC(=O)(2,4-difluoro-phenyl),
-(CH₂)₃(3-indolyl),
-(CH₂)₃(1-methyl-3-indolyl),
-(CH₂)₃(1-indolyl),
-(CH₂)₃(1-indolinyl),
-(CH₂)₃(1-benzimidazolyl),
-(CH₂)₃(1H-1,2,3-benzotriazol-1-yl),
-(CH₂)₃(1H-1,2,3-benzotriazol-2-yl),
-(CH₂)₂(1H-1,2,3-benzotriazol-1-yl),
-(CH₂)₂(1H-1,2,3-benzotriazol-2-yl),
-(CH₂)₃(3,4 dihydro-1(2H)-quinolinyl),
-(CH₂)₂C(=O)(4-fluoro-phenyl),
-(CH₂)₂C(=O)NH(4-fluoro-phenyl),
-CH₂CH₂(3-indolyl),
-CH₂CH₂(1-phthalimidyl),
-(CH₂)₄C(=O)N(methyl)(methoxy),
-(CH₂)₄CO₂(ethyl),
-(CH₂)₄C(=O)(phenyl),
-(CH₂)₃CH(phenyl)₂,
-CH₂CH₂CH=C(phenyl)₂,
-CH₂CH₂CH=CMe(4-F-phenyl),
-(CH₂)₃CH(4-fluoro-phenyl)₂,
-CH₂CH₂CH=C(4-fluoro-phenyl)₂,
-(CH₂)₂(2,3-dihydro-1H-inden-2-yl),
-(CH₂)₃C(=O)(2-NH₂-phenyl),
-(CH₂)₃C(=O)(2-NH₂-5-F-phenyl),
-(CH₂)₃C(=O)(2-NH₂-4-F-phenyl),
-(CH₂)₃C(=O)(2-NH₂-3-F-phenyl),
-(CH₂)₃C(=O)(2-NH₂-4-Cl-phenyl),
-(CH₂)₃C(=O)(2-NH₂-4-OH-phenyl),
-(CH₂)₃C(=O)(2-NH₂-4-Br-phenyl),
-(CH₂)₃(1H-indazol-3-yl),
-(CH₂)₃(5-F-1H-indazol-3-yl),
-(CH₂)₃(7-F-1H-indazol-3-yl),
-(CH₂)₃(6-Cl-1H-indazol-3-yl),
-(CH₂)₃(6-Br-1H-indazol-3-yl),
-(CH₂)₃C(=O)(2-NHMe-phenyl),
-(CH₂)₃(1-benzothien-3-yl),
-(CH₂)₃(6-F-1H-indol-1-yl),
-(CH₂)₃(5-F-1H-indol-1-yl),
-(CH₂)₃(6-F-2,3-dihydro-1H-indol-1-yl),
-(CH₂)₃(5-F-2,3-dihydro-1H-indol-1-yl),
-(CH₂)₃(6-F-1H-indol-3-yl),
-(CH₂)₃(5-F-1H-indol-3-yl),
-(CH₂)₃(5-F-1H-indol-3-yl),
-(CH₂)₃(9H-purin-9-yl),
-(CH₂)₃(7H-purin-7-yl),
-(CH₂)₃(6-F-1H-indazol-3-yl),
-(CH₂)₃C(=O)(2-NHSO₂Me-4-F-phenyl),
-(CH₂)₃C(=O)(2-NHC(=O)Me-4-F-phenyl),
-(CH₂)₃C(=O)(2-NHC(=O)Me-phenyl),
-(CH₂)₃C(=O)(2-NHCO₂Et-4-F-phenyl),
-(CH₂)₃C(=O)(2-NHC(=O)NHEt-4-F-phenyl),
-(CH₂)₃C(=O)(2-NHCHO-4-F-phenyl),
-(CH₂)₃C(=O)(2-OH-4-F-phenyl),
-(CH₂)₃C(=O)(2-MeS-4-F-phenyl),
-(CH₂)₃C(=O)(2-NHSO₂Me-4-F-phenyl),
-(CH₂)₂C(Me)CO₂Me,
-(CH₂)₂C(Me)CH(OH)(4-F-phenyl)₂,
-(CH₂)₂C(Me)CH(OH)(4-Cl-phenyl)₂,
-(CH₂)₂C(Me)C(=O)(4-F-phenyl),
-(CH₂)₂C(Me)C(=O)(2-MeO-4-F-phenyl),
-(CH₂)₂C(Me)C(=O)(3-Me-4-F-phenyl),
-(CH₂)₂C(Me)C(=O)(2-Me-phenyl),
-(CH₂)₂C(Me)C(=O)phenyl, 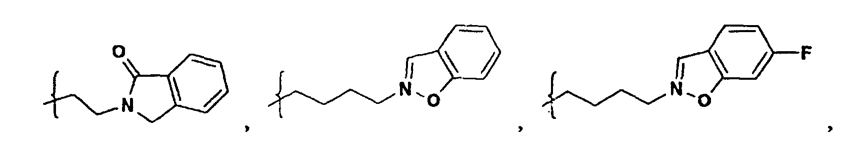 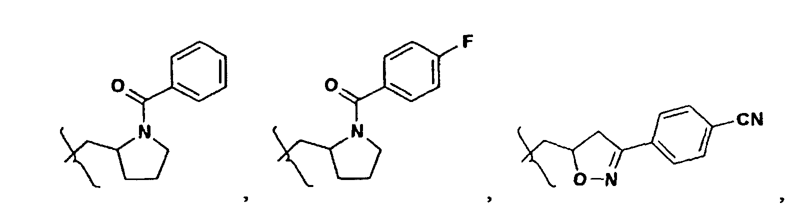 and 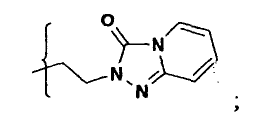
R⁷, R⁸, and R⁹, at each occurrence, are independently selected from hydrogen, fluoro, chloro, bromo, cyano, methyl, ethyl, propyl, isopropyl, butyl, t-butyl, nitro, trifluoromethyl, methoxy, ethoxy, isopropoxy, trifluoromethoxy, phenyl, benzyl, HC(=O)-, methylC(=O)-, ethylC(=O)-, propylC(=O)-, isopropylC(=O)-, n-butylC(=O)-, isobutylC(=O)-, secbutylC(=O)-, tertbutylC(=O)-, phenylC(=O)-, methylC(=O)NH-, ethylC(=O)NH -, propylC(=O)NH-, isopropylC(=O)NH-, n-butylC(=O)-, isobutylC(=O)NH-, secbutylC(=O)NH-, tertbutylC(=O) -, phenylC(=O)NH-, methylamino-, ethylamino-, propylamino-, isopropylamino-, n-butylamino-, isobutylamino-, secbutylamino-, tertbutylamino-, phenylamino-,
provided that two of substituents R⁷, R⁸, and R⁹, are independently selected from hydrogen, fluoro, chloro, bromo, cyano, methyl, ethyl, propyl, isopropyl, butyl, t-butyl, nitro, trifluoromethyl, methoxy, ethoxy, isopropoxy, and trifluoromethoxy;
k is 1 or 2;
m is 1 or 2; and
n is 1 or 2.

2. A compound of Claim 1 of Formula (II-a) or Formula (III-a) 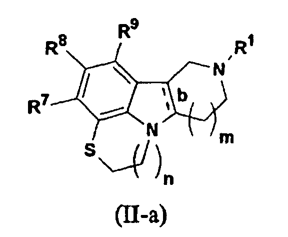 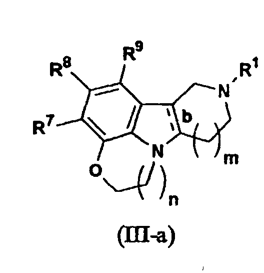 wherein:
b is a single bond, wherein the bridge hydrogens are in a cis position;
R¹ is selected from
-(CH₂)₃C(=O)(4-fluoro-phenyl),
-(CH₂)₃C(=O)(4-bromo-phenyl),
-(CH₂)₃C(=O)(4-methyl-phenyl),
-(CH₂)₃C(=O)(4-methoxy-phenyl),
-(CH₂)₃C(=O)(4-(3,4-dichloro-phenyl)phenyl),
-(CH₂)₃C(=O)(3-methyl-4-fluoro-phenyl),
-(CH₂)₃C(=O)(2,3-dimethoxy-phenyl),
-(CH₂)₃C(=O)(phenyl),
-(CH₂)₃C(=O)(4-chloro-phenyl),
-(CH₂)₃C(=O)(3-methyl-phenyl),
-(CH₂)₃C(=O)(4-t-butyl-phenyl),
-(CH₂)₃C(=O)(3,4-difluoro-phenyl),
-(CH₂)₃C(=O)(2-methoxy-5-fluoro-phenyl),
-(CH₂)₃C(=O)(4-fluoro-1-naphthyl),
-(CH₂)₃C(=O)(benzyl),
-(CH₂)₃C(=O)(4-pyridyl),
-(CH₂)₃C(=O)(3-pyridyl),
-(CH₂)₃CH(OH)(4-fluoro-phenyl),
-(CH₂)₃CH(OH)(4-pyridyl),
-(CH₂)₃CH(OH)(2,3-dimethoxy-phenyl),
-(CH₂)₃S(3-fluoro-phenyl),
-(CH₂)₃S(4-fluoro-phenyl),
-(CH₂)₃S(=O)(4-fluoro-phenyl),
-(CH₂)₃SO₂(3-fluoro-phenyl),
-(CH₂)₃SO₂(4-fluoro-phenyl),
-(CH₂)₃O(4-fluoro-phenyl),
-(CH₂)₃O(phenyl),
-(CH₂)₃NH(4-fluoro-phenyl),
-(CH₂)₃N(methyl)(4-fluoro-phenyl,
-(CH₂)₃CO₂(ethyl),
-(CH₂)₃C(=O)N(methyl)(methoxy),
-(CH₂)₃C(=O)NH(4-fluoro-phenyl),
-(CH₂)₂NHC(=O)(phenyl),
-(CH₂)₂NMeC(=O)(phenyl),
-(CH₂)₂NHC(=O)(2-fluoro-phenyl),
-(CH₂)₂NMeC(=O)(2-fluoro-phenyl),
-(CH₂)₂NHC(=O)(4-fluoro-phenyl),
-(CH₂)₂NMeC(=O)(4-fluoro-phenyl),
-(CH₂)₂NHC(=O)(2,4-difluoro-phenyl),
-(CH₂)₂NMeC(=O)(2,4-difluoro-phenyl),
-(CH₂)₃(3-indolyl),
-(CH₂)₃(1-methyl-3-indolyl),
-(CH₂)₃(1-indolyl),
-(CH₂)₃(1-indolinyl),
-(CH₂)₃(1-benzimidazolyl),
-(CH₂)₃(1H-1,2,3-benzotriazol-1-yl),
-(CH₂)₃(1H-1,2,3-benzotriazol-2-yl),
-(CH₂)₂(1H-1,2,3-benzotriazol-1-yl),
-(CH₂)₂(1H-1,2,3-benzotriazol-2-yl),
-(CH₂)₃(3,4dihydro-1(2H)-quinolinyl),
-(CH₂)₂C(=O)(4-fluoro-phenyl),
-(CH₂)₂C(=O)NH(4-fluoro-phenyl),
-CH₂CH₂(3-indolyl),
-CH₂CH₂(1-phthalimidyl),
-(CH₂)₄C(=O)N(methyl)(methoxy),
-(CH₂)₄CO₂(ethyl),
-(CH₂)₄C(=O)(phenyl),
-(CH₂)₃CH(phenyl)₂,
-CH₂CH₂CH=C(phenyl)₂,
-CH₂CH₂CH=CMe(4-F-phenyl),
-(CH₂)₃CH(4-fluoro-phenyl)₂,
-CH₂CH₂CH=C(4-fluoro-phenyl)₂,
-(CH₂)₂(2,3-dihydro-1H-inden-2-yl),
-(CH₂)₃C(=O)(2-NH₂-phenyl),
-(CH₂)₃C(=O)(2-NH₂-5-F-phenyl),
-(CH₂)₃C(=O)(2-NH₂-4-F-phenyl),
-(CH₂)₃C(=O)(2-NH₂-3-F-phenyl),
-(CH₂)₃C(=O)(2-NH₂-4-Cl-phenyl),
-(CH₂)₃C(=O)(2-NH₂-4-OH-phenyl),
-(CH₂)₃C(=O)(2-NH₂-4-Br-phenyl),
-(CH₂)₃(1H-indazol-3-yl),
-(CH₂)₃(5-F-1H-indazol-3-yl),
-(CH₂)₃(7-F-1H-indazol-3-yl),
-(CH₂)₃(6-Cl-1H-indazol-3-yl),
-(CH₂)₃(6-Br-1H-indazol-3-yl),
-(CH₂)₃C(=O)(2-NHMe-phenyl),
-(CH₂)₃(1-benzothien-3-yl);
-(CH₂)₃(6-F-1H-indol-1-yl),
-(CH₂)₃(5-F-1H-indol-1-yl),
-(CH₂)₃(6-F-2,3-dihydro-1H-indol-1-yl),
-(CH₂)₃(5-F-2,3-dihydro-1H-indol-1-yl),
-(CH₂)₃(6-F-1H-indol-3-yl),
-(CH₂)₃(5-F-1H-indol-3-yl),
-(CH₂)₃(5-F-1H-indol-3-yl),
-(CH₂)₃(9H-purin-9-yl),
-(CH₂)₃(7H-purin-7-yl),
-(CH₂)₃(6-F-1H-indazol-3-yl),
-(CH₂)₃C(=O)(2-NHSO₂Me-4-F-phenyl),
-(CH₂)₃C(=O)(2-NHC(=O)Me-4-F-phenyl),
-(CH₂)₃C(=O)(2-NHC(=O)Me-phenyl),
-(CH₂)₃C(=O)(2-NHCO₂Et-4-F-phenyl),
-(CH₂)₃C(=O)(2-NHC(=O)NHEt-4-F-phenyl),
-(CH₂)₃C(=O)(2-NHCHO-4-F-phenyl),
-(CH₂)₃C(=O)(2-OH-4-F-phenyl),
-(CH₂)₃C(=O)(2-MeS-4-F-phenyl),
-(CH₂)₃C(=O)(2-NHSO₂Me-4-F-phenyl),
-(CH₂)₂C(Me)CO₂Me,
-(CH₂)₂C(Me)CH(OH)(4-F-phenyl)₂,
-(CH₂)₂C(Me)CH(OH)(4-Cl-phenyl)₂,
-(CH₂)₂C(Me)C(=O)(4-F-phenyl),
-(CH₂)₂C(Me)C(=O)(2-MeO-4-F-phenyl),
-(CH₂)₂C(Me)C(=O)(3-Me-4-F-phenyl),
-(CH₂)₂C(Me)C(=O)(2-Me-phenyl),
-(CH₂)₂C(Me)C(=O)phenyl, 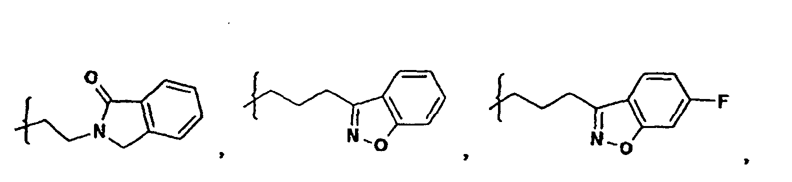 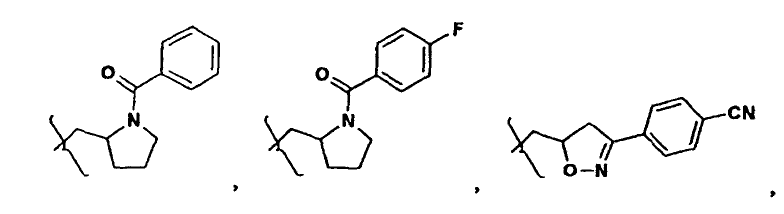 and 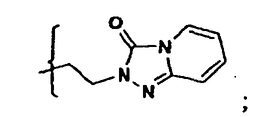
and
R₇, R₈, and R_{9,} at each occurrence, are independently selected from hydrogen, fluoro, chloro, bromo, cyano, methyl, ethyl, propyl, isopropyl, butyl, tbutyl, nitro, trifluoromethyl, methoxy, ethoxy, isopropoxy, trifluoromethoxy, methylC(=O)-, ethylC(=O)-, propylC(=O)-, isopropylC(=O)-, methylC(=O)NH-, ethylC(=O)NH-, propylC(=O)NH-, isopropylC(=O)NH-, methylamino-, ethylamino-, propylamino-, and isopropylamino-,
provided that two of substituents R⁷, R⁸, and R⁹, are independently selected from hydrogen, fluoro, chloro, methyl, trifluoromethyl, methoxy, and trifluoromethoxy.

3. A compound of Claim 2 of Formula (III-a)

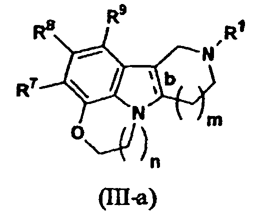

4. A compound of claim 2 of Formula (II-a)

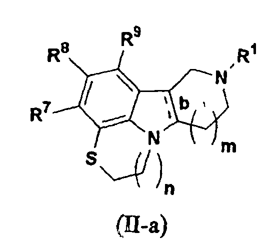

5. A compound of Claim 1 selected from the group consisting of compounds of formula

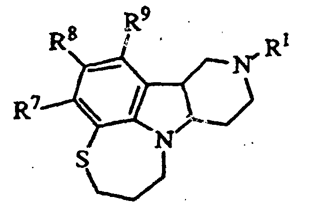

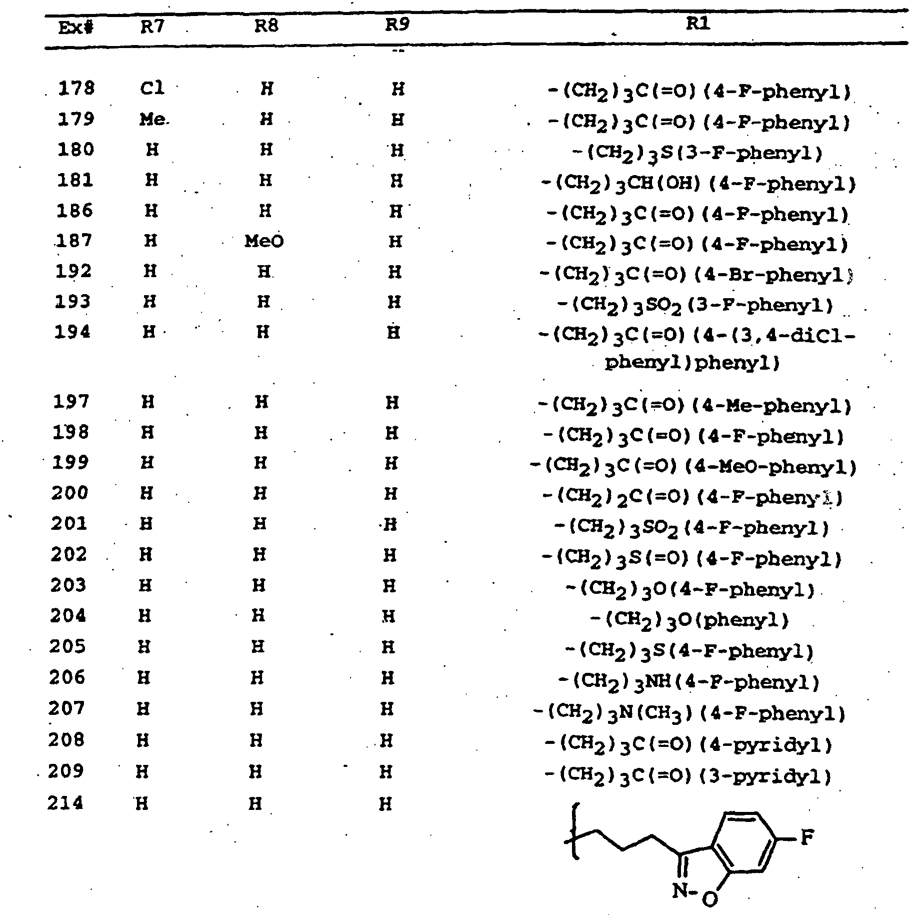

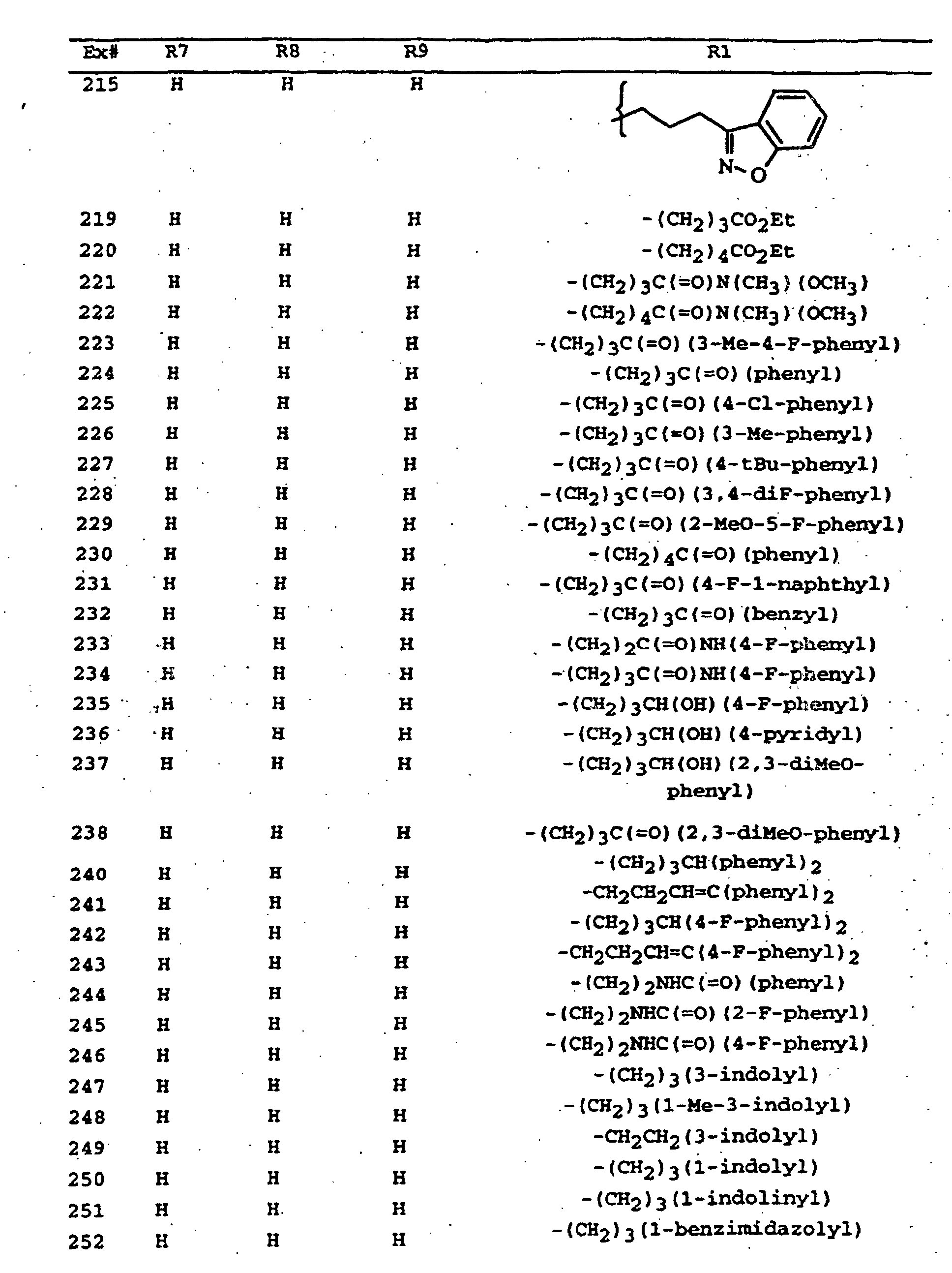

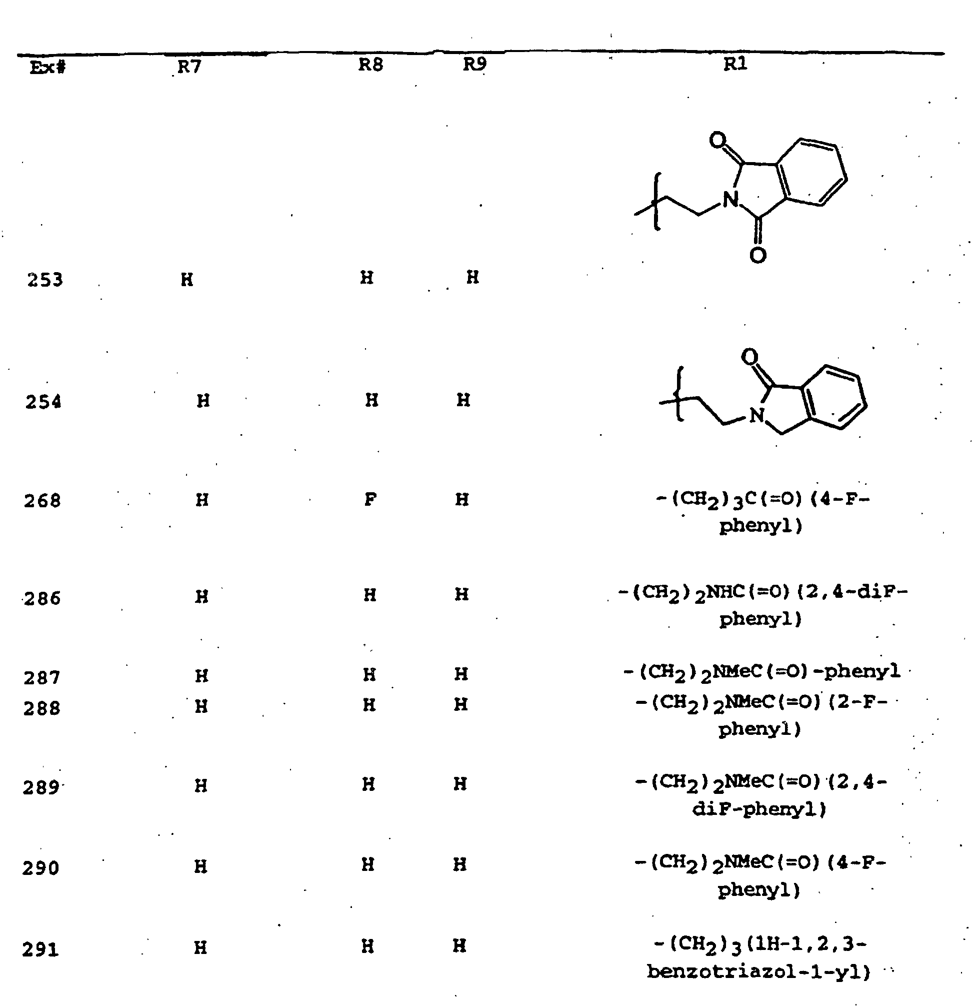

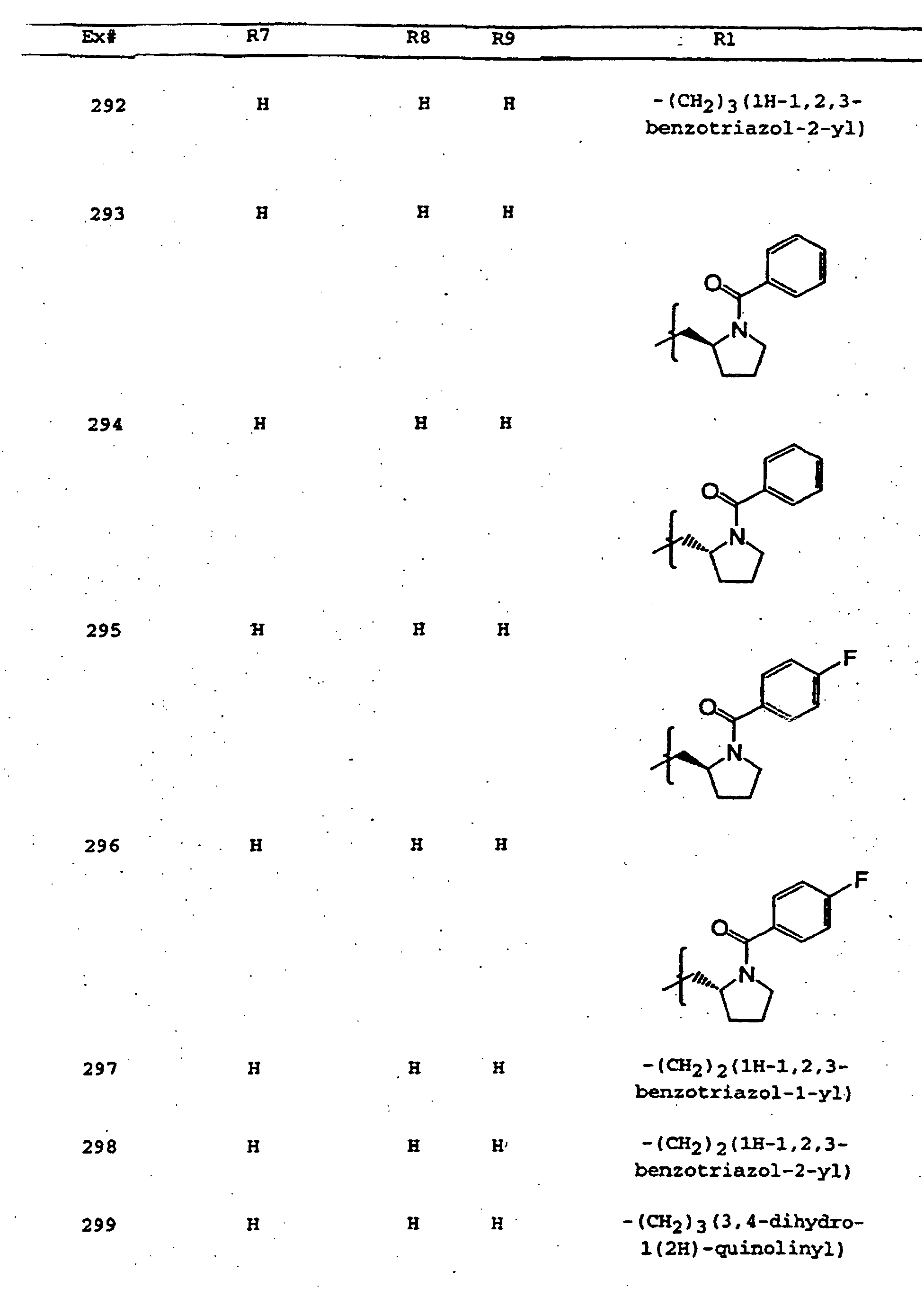

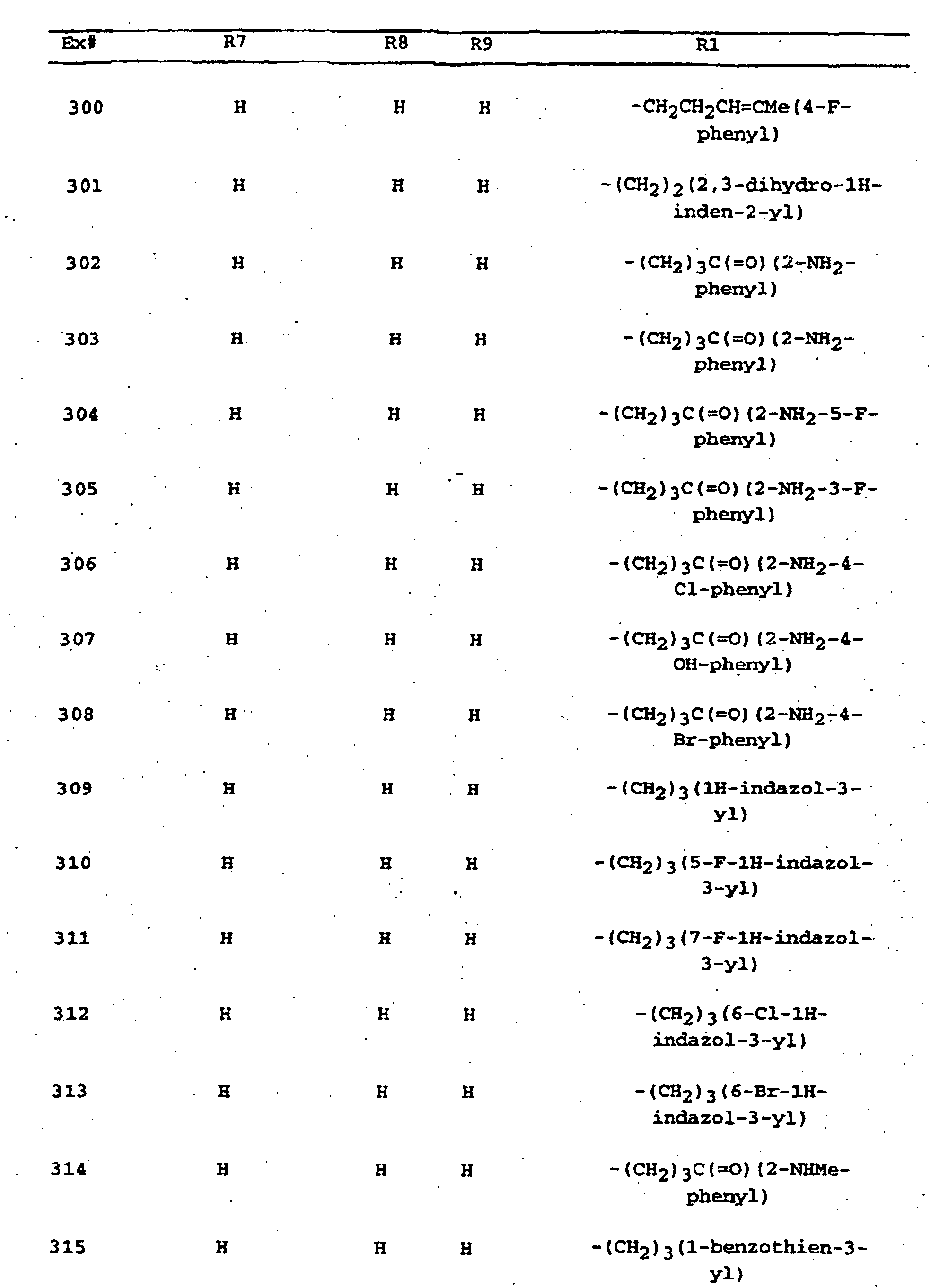

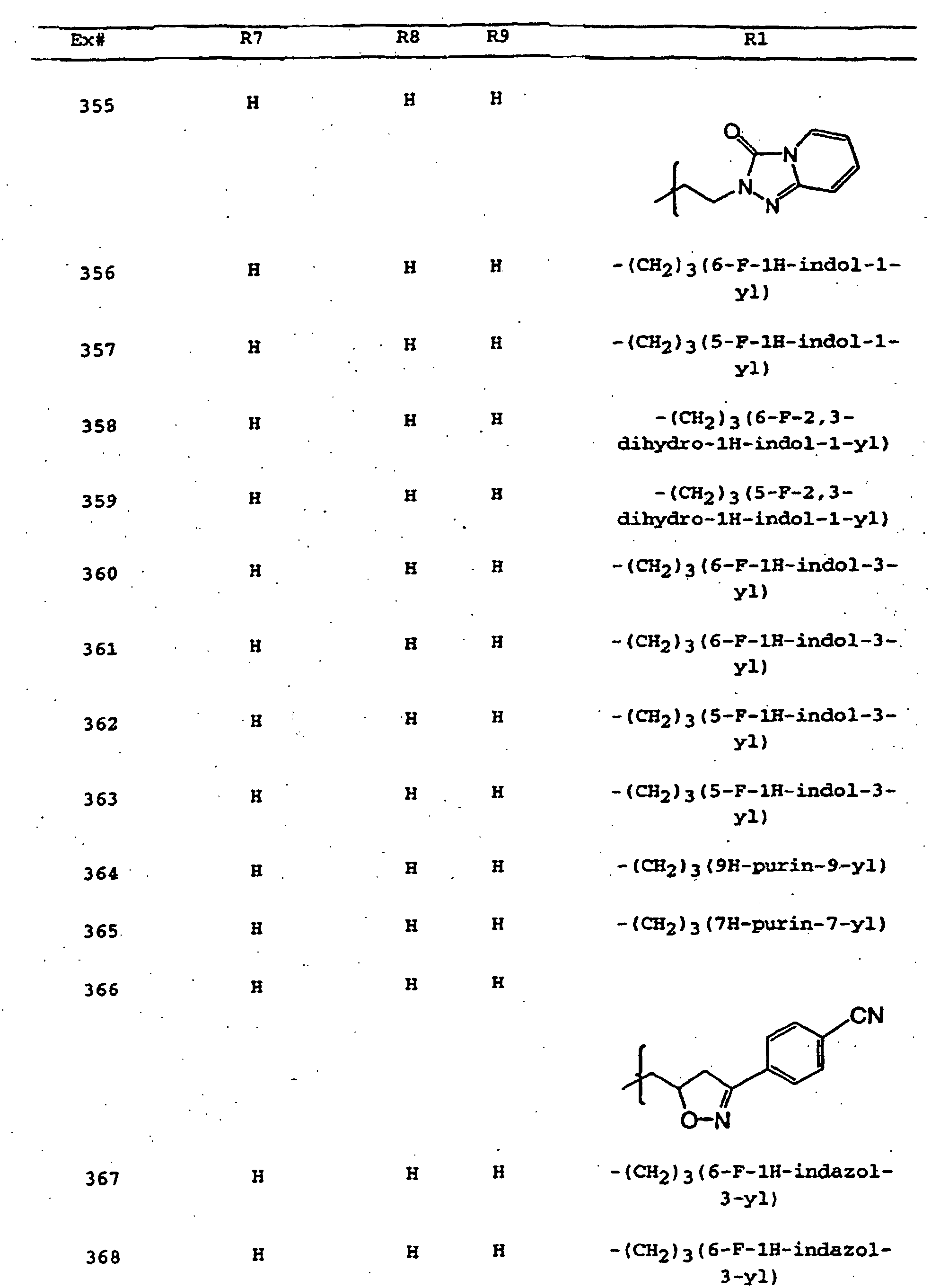

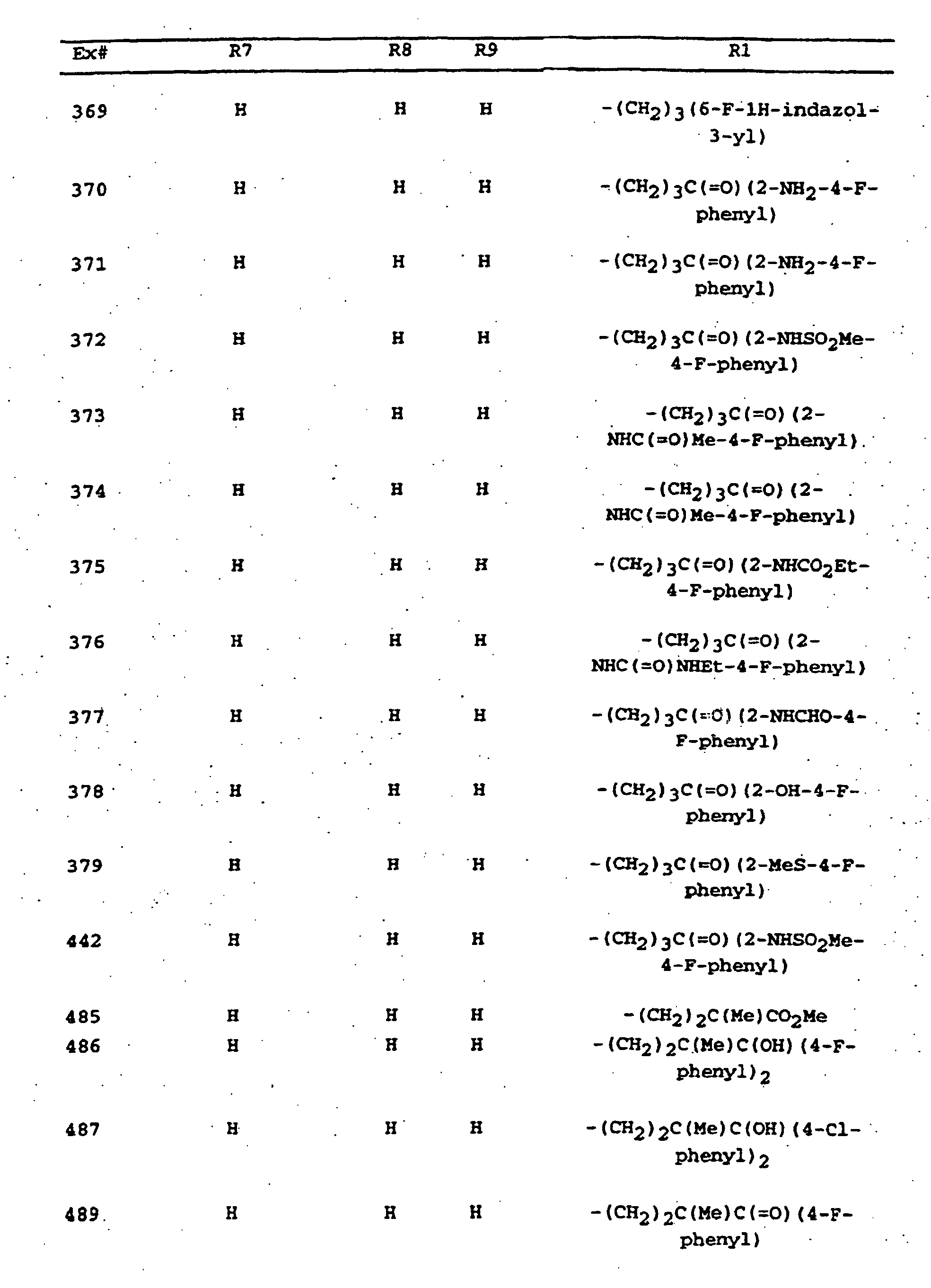

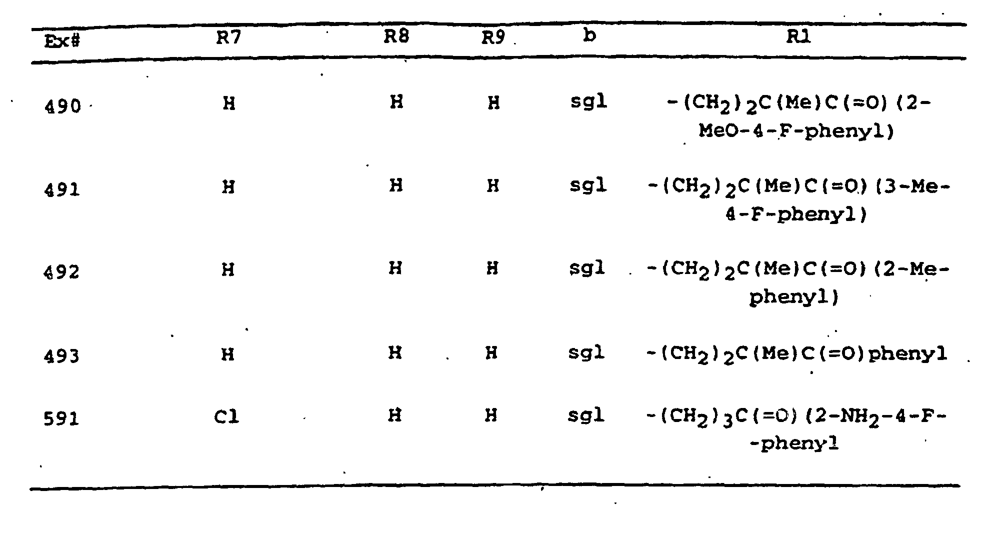

6. A compound of claim 1 selected from the group consisting of compounds of formula 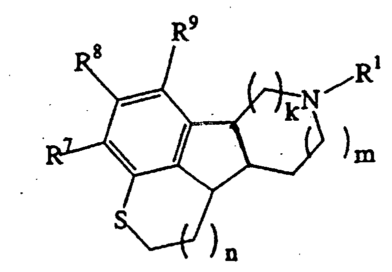
| Ex# | n | k | m | R7 | R8 | R9 | R1 |
|---|---|---|---|---|---|---|---|
| 472 | 2 | 2 | 1 | H | H | H | -(CH₂)₃C(=O)(4-F-phenyl) |
| 473 | 2 | 2 | 1 | H | H | H | -(CH₂)₃O(4-F-phenyl) |
| 474 | 2 | 2 | 1 | H | H | H | -(CH₂)₃(6-F-benzisoxazol-3-yl) |
| 475 | 2 | 2 | 1 | H | H | H | -(CH₂)₃C(=O)(4-pyridyl) |
| 483 | 2 | 2 | 1 | H | Br | H | -(CH₂)₃C(=O)(4-F-phenyl) |
| 484 | 2 | 2 | 1 | H | Br | H | -(CH₂)₃O(4-F-phenyl) |

7. A compound of claim 1 selected from the group consisting of compounds of formula 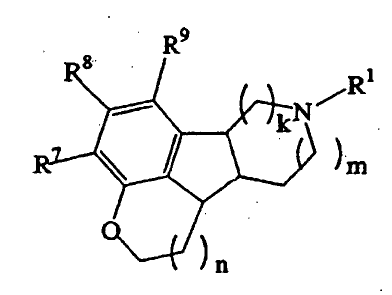
| Ex# | n | k | m | R7 | R8 | R9 | R1 |
|---|---|---|---|---|---|---|---|
| 182 | 1 | 1 | 1 | H | H | H | -(CH₂)₃C(=O)(4-F-phenyl) |
| 266 | 1 | 1 | 1 | H | H | Me | -(CH₂)₃C(=O)(4-F-phenyl) |
| 270 | 1 | 1 | 1 | H | H | H | -(CH₂)₃O(4-F-phenyl) |
| 272 | 1 | 1 | 1 | H | H | H | H |
| 494 | 1 | 1 | 1 | H | H | H | -(CH₂)₃C(=O)(2-NH₂-phenyl) |
| 495 | 1 | 1 | 1 | H | H | H | -(CH₂)₃C(=O)(2-NH₂-phenyl) |
| 496 | 1 | 1 | 1 | H | H | H | -(CH₂)₃(1H-indazol-3-yl) |
| 528 | 1 | 1 | 1 | H | H | H | -(CH₂)₃(6-F-1H-indazol-3-yl) |
| 529 | 1 | 1 | 1 | H | H | H | -(CH₂)₃C(=O)(2-NH₂-4-F-phenyl) |
| 530 | 1 | 1 | 1 | H | H | H | -(CH₂)₃C(=O)(2-NH₂-4-F-phenyl) |
| 531 | 1 | 1 | 1 | H | H | H | -(CH₂)₃C(=O)(2-OH-4-F-phenyl) |
| 539 | 1 | 2 | 1 | H | H | H | -(CH₂)₃O(4-F-phenyl) |
| 540 | 1 | 2 | 1 | H | H | H | -(CH₂)₃(6-F-1,2-benzisoxazol-3-yl) |
| 544 | 2 | 1 | 1 | H | H | H | sgl -(CH₂)₃C(=O)(4-F-phenyl) |
| 546 | 1 | 2 | 1 | H | H | H | sgl -(CH₂)₃C(=O)(4-F-phenyl) |

8. A compound of claim 1 selected from the group consisting of compounds of formula 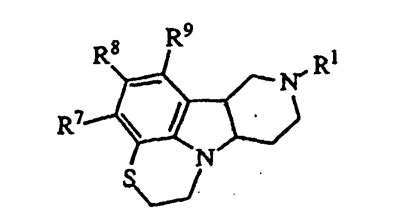
| Ex# | R7 | R8 | R9 | R1 |
|---|---|---|---|---|
| 185 | H | H | CF₃ | -(CH₂)₄(4-F-phenyl) |
| 188 | H | H | H | -(CH₂)₃C(=O)(4-F-phenyl) |
| 213 | H | CH₃ | H | -(CH₂)₃C(=O)(4-F-phenyl) |
| 438 | H | H | H | -(CH₂)₃C(=O)(2-NH₂-phenyl) |
| 439 | H | H | H | -(CH₂)₃C(=O)(2-NH₂-phenyl) |
| 440 | H | H | H | -(CH₂)₃C(=O)(2-NH₂-4-F-phenyl) |
| 441 | H | H | H | -(CH₂)₃C(=O)(2-NH₂-4-F-phenyl) |
| 456 | H | H | H | -(CH₂)₃C(=O)(4-F-phenyl) |
| 457 | H | H | H | -(CH₂)₃C(=O)(4-F-phenyl) |

9. A pharmaceutically composition comprising a pharmaceutically acceptable carrier and a therapeutically effective amount of a compound according to any one of Claims 1-8, or a pharmaceutically acceptable salt thereof

10. A compound of any one of daims 1 to 8, for use in therapy.

11. A compound of any one of claims 1 to 8, for use in treating obesity, schizophrenia or depression.

12. Use of a compound of any one of claims 1 to 8, in the manufacture of a medicament for the treatment of obesity, schizophrenia or depression.

## Patentansprüche

1. Verbindung der Formel (I-a) 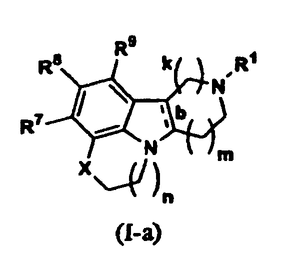 oder pharmazeutisch akzeptable Salzformen davon, worin:
b für eine Einfachbindung steht;
X für -S- oder -O- steht;
R¹ ausgewählt ist unter
-(CH₂)₃C(=O)(4-fluor-phenyl),
-(CH₂)₃C(=O)(4-brom- phenyl),
-(CH₂)₃C(=O)(4-methyl-phenyl),
-(CH₂)₃C(=O)(4-methoxy-phenyl),
-(CH₂)₃C(=O)(4-(3,4-dichlor-phenyl)phenyl),
-(CH₂)₃C(=O)(3-methyl-4-fluor-phenyl),
-(CH₂)₃C(=O)(2,3-dimethoxy-phenyl),
-(CH₂)₃C(=O)(phenyl),
-(CH₂)₃C(=O)(4-chlor-phenyl),
-(CH₂)₃C(=O)(3-methyl-phenyl),
-(CH₂)₃C(=O)(4-t-butyl-phenyl),
-(CH₂)₃C(=O)(3,4-difluor-phenyl),
-(CH₂)₃C(=O)(2-methoxy-5-fluor-phenyl),
-(CH₂)₃C(=O)(4-fluor-1-naphthyl),
-(CH₂)₃C(=O)(benzyl),
-(CH₂)₃C(=O)(4-pyridyl),
-(CH₂)₃C(=O)(3-pyridyl),
-(CH₂)₃CH(OH)(4-fluor-phenyl),
-(CH₂)₃CH(OH)(4-pyridyl),
-(CH₂)₃CH(OH)(2,3-dimethoxy-phenyl),
-(CH₃)₃S(3-fluor-phenyl),
-(CH₂)₃S(4-fluor-phenyl),
-(CH₂)₃S(=O)(4-fluor -phenyl),
-(CH₂)₃SO₂(3-fluor-phenyl),
-(CH₂)₃SO₂(4-fluor -phenyl),
-(CH₂)₃O(4-fluor-phenyl),
-(CH₂)₃O(phenyl),
-(CH₂)₃O(3-pyridyl),
-(CH₂)₃O(4-pyridyl),
-(CH₂)₃O(2-NH₂-phenyl),
-(CH₂)₃O(2-NH₂-5-F-phenyl),
-(CH₂)₃O(2-NH₂-4-F-phenyl),
-(CH₂)₃O(2-NH₂-3-F-phenyl),
-(CH₂)₃O(2-NH₂-4-Cl-phenyl),
-(CH₂)₃O(2-NH₂-4-OH-phenyl),
-(CH₂)₃O(2-NH₂-4-Br-phenyl),
-(CH₂)₃O(2-NHC(=O)Me-4-F-phenyl),
-(CH₂)₃O(2-NHC(=O)Me-phenyl),
-(CH₂)₃NH(4-fluor-phenyl),
-(CH₂)₃N(methyl)(4-fluor-phenyl),
-(CH₂)₃CO₂(ethyl),
-(CH₂)₃C(=O)N(methyl)(methoxy),
-(CH₂)₃C(=O)NH(4-fluor-phenyl),
-(CH₂)₂NHC(=O)(phenyl),
-(CH₂)₂NMeC(=O)(phenyl),
-(CH₂)₂NHC(=O)(2-fluor-phenyl),
-(CH₂)₂NMeC(=O)(2-fluor-phenyl),
-(CH₂)₂NHC(=O)(4-fluor-phenyl),
-(CH₂)₂NMeC(=O)(4-fluor-phenyl),
-(CH₂)₂NHC(=O)(2,4-difluor-phenyl),
-(CH₂)₂NMeC(=O)(2,4-difluor-phenyl),
-(CH₂)₃(3-indolyl),
-(CH₂)₃(1-methyl-3-indolyl),
-(CH₂)₃(1-indolyl),
-(CH₂)₃(1-indolinyl),
-(CH₂)₃(1-benzimidazolyl),
-(CH₂)₃(1H-1,2,3-benzotriazol-1-yl),
-(CH₂)₃(1H-1,2,3-benzotriazol-2-yl),
-(CH₂)₂(1H-1,2,3-benzotriazol-1-yl),
-(CH₂)₂(1H-1,2,3-benzotriazol-2-yl),
-(CH₂)₃(3,4 dihydro-1(2H)-quinolinyl),
-(CH₂)₂C(=O)(4-fluor-phenyl),
-(CH₂)₂C(=O)NH(4-fluor-phenyl),
-CH₂CH₂(3-indolyl),
-CH₂CH₂(1-phthalimidyl),
-(CH₂)₄C(=O)N(methyl)(methoxy),
-(CH₂)₄CO₂(ethyl),
-(CH₂)₄C(=O)(phenyl),
-(CH₂)₃CH(phenyl)₂,
-CH₂CH₂CH=C(phenyl)₂,
-CH₂CH₂CH=CMe(4-F-phenyl),
-(CH₂)₃CH(4-fluor-phenyl)₂,
-CH₂CH₂CH=C(4-fluor-phenyl)₂,
-(CH₂)₂(2,3-dihydro-1H-inden-2-yl),
-(CH₂)₃C(=O)(2-NH₂-phenyl),
-(CH₂)₃C(=O)(2-NH₂-5-F-phenyl),
-(CH₂)₃C(=O)(2-NH₂-4-F-phenyl),
-(CH₂)₃C(=O)(2-NH₂-3-F-phenyl),
-(CH₂)₃C(=O)(2-NH₂-4-Cl-phenyl),
-(CH₂)₃C(=O)(2-NH₂-4-OH-phenyl),
-(CH₂)₃C(=O)(2-NH₂-4-Br-phenyl),
-(CH₂)₃(1H-indazol-3-yl),
-(CH₂)₃(5-F-1H-indazol-3-yl),
-(CH₂)₃(7-F-1H-indazol-3-yl),
-(CH₂)₃(6-Cl-1H-indazol-3-yl),
-(CH₂)₃(6-Br-1H-indazol-3-yl),
-(CH₂)₃C(=O)(2-NHMe-phenyl),
-(CH₂)₃(1-benzothien-3-yl),
-(CH₂)₃(6-F-1H-indol-1-yl),
-(CH₂)₃(5-F-1H-indol-1-yl),
-(CH₂)₃(6-F-2,3-dihydro-1H-indol-1-yl),
-(CH₂)₃(5-F-2,3-dihydro-1H-indol-1-yl),
-(CH₂)₃(6-F-1H-indol-3-yl),
-(CH₂)₃(5-F-1H-indol-3-yl),
-(CH₂)₃(5-F-1H-indol-3-yl),
-(CH₂)₃(9H-purin-9-yl),
-(CH₂)₃(7H-purin-7-yl),
-(CH₂)₃(6-F-1H-indazol-3-yl),
-(CH₂)₃C(=O)(2-NHSO₂Me-4-F-phenyl),
-(CH₂)₃C(=O)(2-NHC(=O)Me-4-F-phenyl),
-(CH₂)₃C(=O)(2-NHC(=O)Me-phenyl),
-(CH₂)₃C(=O)(2-NHCO₂Et-4-F-phenyl),
-(CH₂)₃C(=O)(2-NHC(=O)NHEt-4-F-phenyl),
-(CH₂)₃C(=O)(2-NHCHO-4-F-phenyl),
-(CH₂)₃C(=O)(2-OH-4-F-phenyl),
-(CH₂)₃C(=O)(2-MeS-4-F-phenyl),
-(CH₂)₃C(=O)(2-NHSO₂Me-4-F-phenyl),
-(CH₂)₂C(Me)CO₂Me,
-(CH₂)₂C(Me)CH(OH)(4-F-phenyl)₂,
-(CH₂)₂C(Me)CH(OH)(4-Cl-phenyl)₂,
-(CH₂)₂C(Me)C(=O)(4-F-phenyl),
-(CH₂)₂C(Me)C(=O)(2-MeO-4-F-phenyl),
-(CH₂)₂C(Me)C(=O)(3-Me-4-F-phenyl),
-(CH₂)₂C(Me)C(=O)(2-Me-phenyl),
-(CH₂)₂C(Me)C(=O)phenyl, 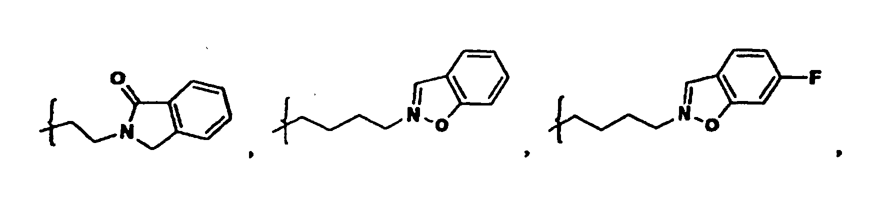 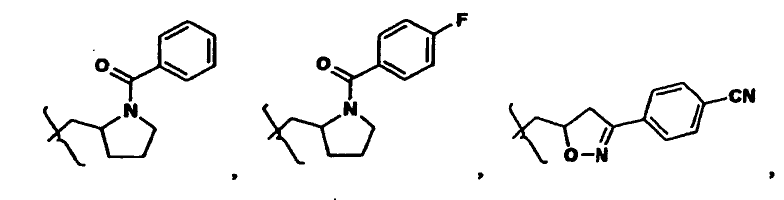 und 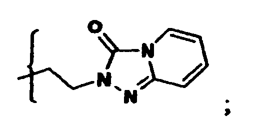
R⁷, R⁸ und R⁹ jeweils unabhängig voneinander ausgewählt sind unter Wasserstoff, Fluor, Chlor, Brom, Cyano, Methyl, Ethyl, Propyl, Isopropyl, Butyl, t-Butyl, Nitro, Trifluormethyl, Methoxy, Ethoxy, Isopropoxy, Trifluormethoxy, Phenyl, Benzyl, HC(=O)-, MethylC(=O)-, EthylC(=O)-, PropylC(=O)-, IsopropylC(=O)-, n-ButylC(=O)-, IsobutylC(=O)-, sec-ButylC(=O)-, tert-ButylC(=O)-, PhenylC(=O)-, MethylC(=O)NH-, EthylC(=O)NH-, PropylC(=O)NH-, IsopropylC(=O)NH-, n-ButylC(=O)-, IsobutylC(=O)NH-, sec-ButylC(=O)NH-, tert-ButylC(=O)-, PhenylC(=O)NH-, Methylamino-, Ethylamino-, Propylamino-, Isopropylamino-, n-Butylamino-, Isobutylamino-, sec-Butylamino-, tert-Butylamino-, Phenylamino-,
unter der Voraussetzung, dass zwei der Substituenten R⁷, R⁸ und R⁹ unabhängig voneinander ausgewählt sind unter Wasserstoff, Fluor, Chlor, Brom, Cyano, Methyl, Ethyl, Propyl, Isopropyl, Butyl, t-Butyl, Nitro, Trifluormethyl, Methoxy, Ethoxy, Isopropoxy, und Trifluormethoxy;
k für 1 oder 2 steht;
m für 1 oder 2 steht; und
n für 1 oder 2 steht.

2. Verbindung nach Anspruch 1 der Formel (II-a) oder der Formel (III-a) 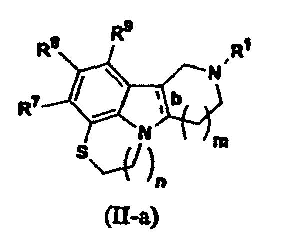 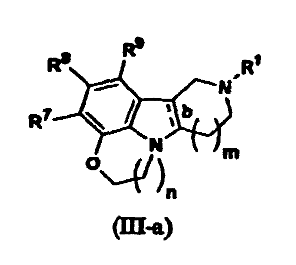 worin
b für eine Einfachbindung steht, wobei sich die Brückenwasserstoff atome in cis-Position befinden;
R¹ ausgewählt ist unter
-(CH₂)₃C(=O)(4-fluor-phenyl),
-(CH₂)₃C(=O)(4-brom-phenyl),
-(CH₂)₃C(=O)(4-methyl-phenyl),
-(CH₂)₃C(=O)(4-methoxy-phenyl),
-(CH₂)₃C(=O)(4-(3,4-dichlor-phenyl)phenyl),
-(CH₂)₃C(=O)(3-methyl-4-fluor-phenyl),
-(CH₂)₃C(=O)(2,3-dimethoxy-phenyl),
-(CH₂)₃C(=O)(phenyl),
-(CH₂)₃C(=O)(4-chlor-phenyl),
-(CH₂)₃C(=O)(3-methyl-phenyl),
-(CH₂)₃C(=O)(4-t-butyl-phenyl),
-(CH₂)₃C(=O)(3,4-difluor-phenyl),
-(CH₂)₃C(=O)(2-methoxy-5-fluor-phenyl),
-(CH₂)₃C(=O)(4-fluor-1-naphthyl),
-(CH₂)₃C(=O)(benzyl),
-(CH₂)₃C(=O)(4-pyridyl),
-(CH₂)₃C(=O)(3-pyridyl),
-(CH₂)₃CH(OH)(4-fluor-phenyl),
-(CH₂)₃CH(OH)(4-pyridyl),
-(CH₂)₃CH(OH)(2,3-dimethoxy-phenyl),
-(CH₂)₃S(3-fluor-phenyl),
-(CH₂)₃S(4-fluor-phenyl),
-(CH₂)₃S(=O)(4-fluor-phenyl),
-(CH₂)₃SO₂(3-fluor-phenyl),
-(CH₂)₃SO₂(4-fluor-phenyl),
-(CH₂)₃O(4-fluor-phenyl),
-(CH₂)₃O(phenyl),
-(CH₂)₃NH(4-fluor-phenyl),
-(CH₂)₃N(methyl)(4-fluor-phenyl,
-(CH₂)₃CO₂(ethyl),
-(CH₂)₃C(=O)N(methyl)(methoxy),
-(CH₂)₃C(=O)NH(4-fluor-phenyl),
-(CH₂)₂NHC(=O)(phenyl),
-(CH₂)₂NMeC(=O)(phenyl),
-(CH₂)₂NHC(=O)(2-fluor-phenyl),
-(CH₂)₂NMeC(=O)(2-fluor-phenyl),
-(CH₂)₂NHC(=O)(4-fluor-phenyl),
-(CH₂)₂NMeC(=O)(4-fluor-phenyl),
-(CH₂)₂NHC(=O)(2,4-difluor-phenyl),
-(CH₂)₂NMeC(=O)(2,4-difluor-phenyl),
-(CH₂)₃(3-indolyl),
-(CH₂)₃(1-methyl-3-indolyl),
-(CH₂)₃(1-indolyl),
-(CH₂)₃(1-indolinyl),
-(CH₂)₃(1-benzimidazolyl),
-(CH₂)₃(1H-1,2,3-benzotriazol-1-yl),
-(CH₂)₃(1H-1,2,3-benzotriazol-2-yl),
-(CH₂)₂(1H-1,2,3-benzotriazol-1-yl),
-(CH₂)₂(1H-1,2,3-benzotriazol-2-yl),
-(CH₂)₃(3,4 dihydro-1(2H)-quinolinyl),
-(CH₂)₂C(=O)(4-fluor-phenyl),
-(CH₂)₂C(=O)NH(4-fluor-phenyl),
-CH₂CH₂(3-indolyl),
-CH₂CH₂(1-phthalimidyl),
-(CH₂)₄C(=O)N(methyl)(methoxy),
-(CH₂)₄CO₂(ethyl),
-(CH₂)₄C(=O)(phenyl),
-(CH₂)₃CH(phenyl)₂,
-CH₂CH₂CH=C(phenyl)₂,
-CH₂CH₂CH=CMe(4-F-phenyl),
-(CH₂)₃CH(4-fluor-phenyl)₂,
-CH₂CH₂CH=C(4-fluor-phenyl)₂,
-(CH₂)₂(2,3-dihydro-1H-inden-2-yl),
-(CH₂)₃C(=O)(2-NH₂-phenyl),
-(CH₂)₃C(=O)(2-NH₂-5-F-phenyl),
-(CH₂)₃C(=O)(2-NH₂-4-F-phenyl),
-(CH₂)₃C(=O)(2-NH₂-3-F-phenyl),
-(CH₂)₃C(=O)(2-NH₂-4-Cl-phenyl),
-(CH₂)₃C(=O)(2-NH₂-4-OH-phenyl),
-(CH₂)₃C(=O)(2-NH₂-4-Br-phenyl),
-(CH₂)₃(1H-indazol-3-yl),
-(CH₂)₃(5-F-1H-indazol-3-yl),
-(CH₂)₃(7-F-1H-indazol-3-yl),
-(CH₂)₃(6-Cl-1H-indazol-3-yl),
-(CH₂)₃(6-Br-1H-indazol-3-yl),
-(CH₂)₃C(=O)(2-NHMe-phenyl),
-(CH₂)₃(1-benzothien-3-yl);
-(CH₂)₃(6-F-1H-indol-1-yl),
-(CH₂)₃(5-F-1H-indol-1-yl),
-(CH₂)₃(6-F-2,3-dihydro-1H-indol-1-yl),
-(CH₂)₃(5-F-2,3-dihydro-1H-indol-1-yl),
-(CH₂)₃(6-F-1H-indol-3-yl),
-(CH₂)₃(5-F-1H-indol-3-yl),
-(CH₂)₃(5-F-1H-indol-3-yl),
-(CH₂)₃(9H-purin-9-yl),
-(CH₂)₃(7H-purin-7-yl),
-(CH₂)₃(6-F-1H-indazol-3-yl),
-(CH₂)₃C(=O)(2-NHSO₂Me-4-F-phenyl),
-(CH₂)₃C(=O)(2-NHC(=O)Me-4-F-phenyl),
-(CH₂)₃C(=O)(2-NHC(=O)Me-phenyl),
-(CH₂)₃C(=O)(2-NHCO₂Et-4-F-phenyl),
-(CH₂)₃C(=O)(2-NHC(=O)NHEt-4-F-phenyl),
-(CH₂)₃C(=O)(2-NHCHO-4-F-phenyl),
-(CH₂)₃C(=O)(2-OH-4-F-phenyl),
-(CH₂)₃C(=O)(2-MeS-4-F-phenyl),
-(CH₂)₃C(=O)(2-NHSO₂Me-4-F-phenyl),
-(CH₂)₂C(Me)CO₂Me,
-(CH₂)₂C(Me)CH(OH)(4-F-phenyl)₂,
-(CH₂)₂C(Me)CH(OH)(4-Cl-phenyl)₂,
-(CH₂)₂C(Me)C(=O)(4-F-phenyl),
-(CH₂)₂C(Me)C(=O)(2-MeO-4-F-phenyl),
-(CH₂)₂C(Me)C(=O)(3-Me-4-F-phenyl),
-(CH₂)₂C(Me)C(=O)(2-Me-phenyl),
-(CH₂)₂C(Me)C(=O)phenyl, 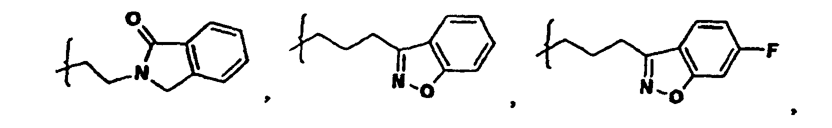 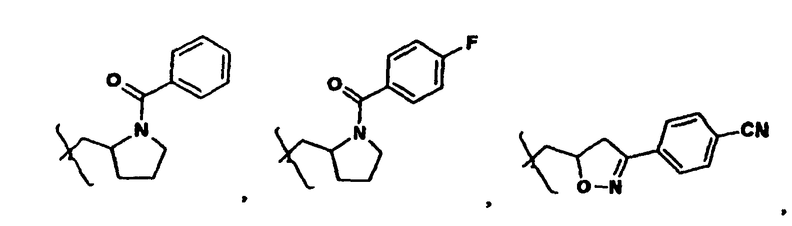 und 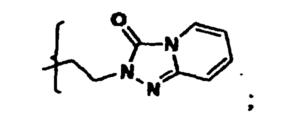
und
R⁷, R⁸ und R⁹ jeweils unabhängig voneinander ausgewählt sind unter Wasserstoff, Fluor, Chlor, Brom, Cyano, Methyl, Ethyl, Propyl, Isopropyl, Butyl, t-Butyl, Nitro, Trifluormethyl, Methoxy, Ethoxy, Isopropoxy, Trifluormethoxy, MethylC(=O)-, EthylC(=O)-, PropylC(=O)-, IsopropylC(=O)-, MethylC(=O)NH-, EthylC(=O)NH-, PropylC(=O)NH-, IsopropylC(=O)NH-, Methylamino-, Ethylamino-, Propylamino-, und Isopropylamino-, vorausgesetzt dass zwei der Substituenten R⁷, R⁸ und R⁹ unabhängig voneinander ausgewählt sind unter Wasserstoff, Fluor, Chlor, Methyl, Trifluormethyl, Methoxy und Trifluormethoxy.

3. Verbindung nach Anspruch 2 der Formel (III-a)

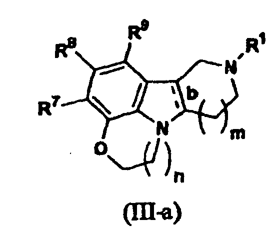

4. Verbindung nach Anspruch 2 der Formel (II-a)

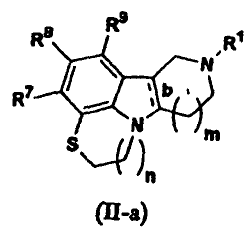

5. Verbindung nach Anspruch 1, ausgewählt aus der Gruppe, bestehend aus Verbindungen der Formel

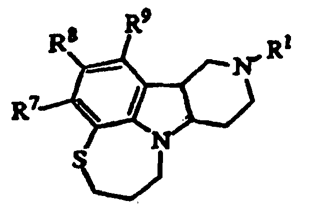

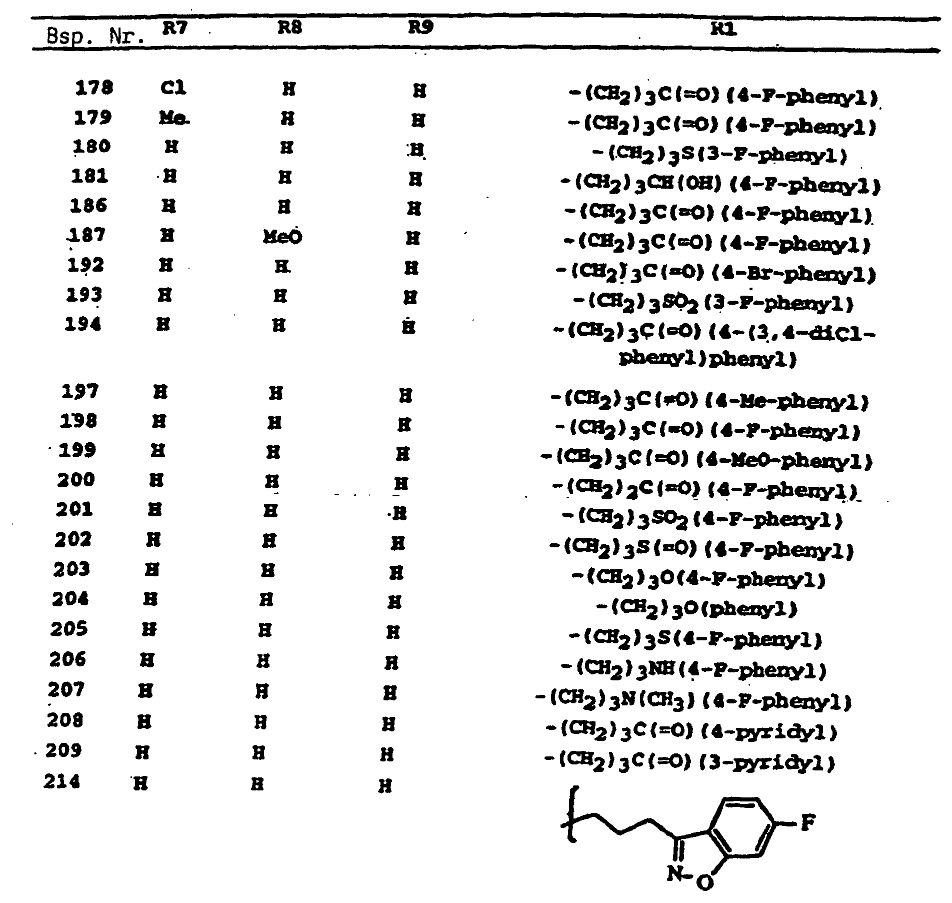

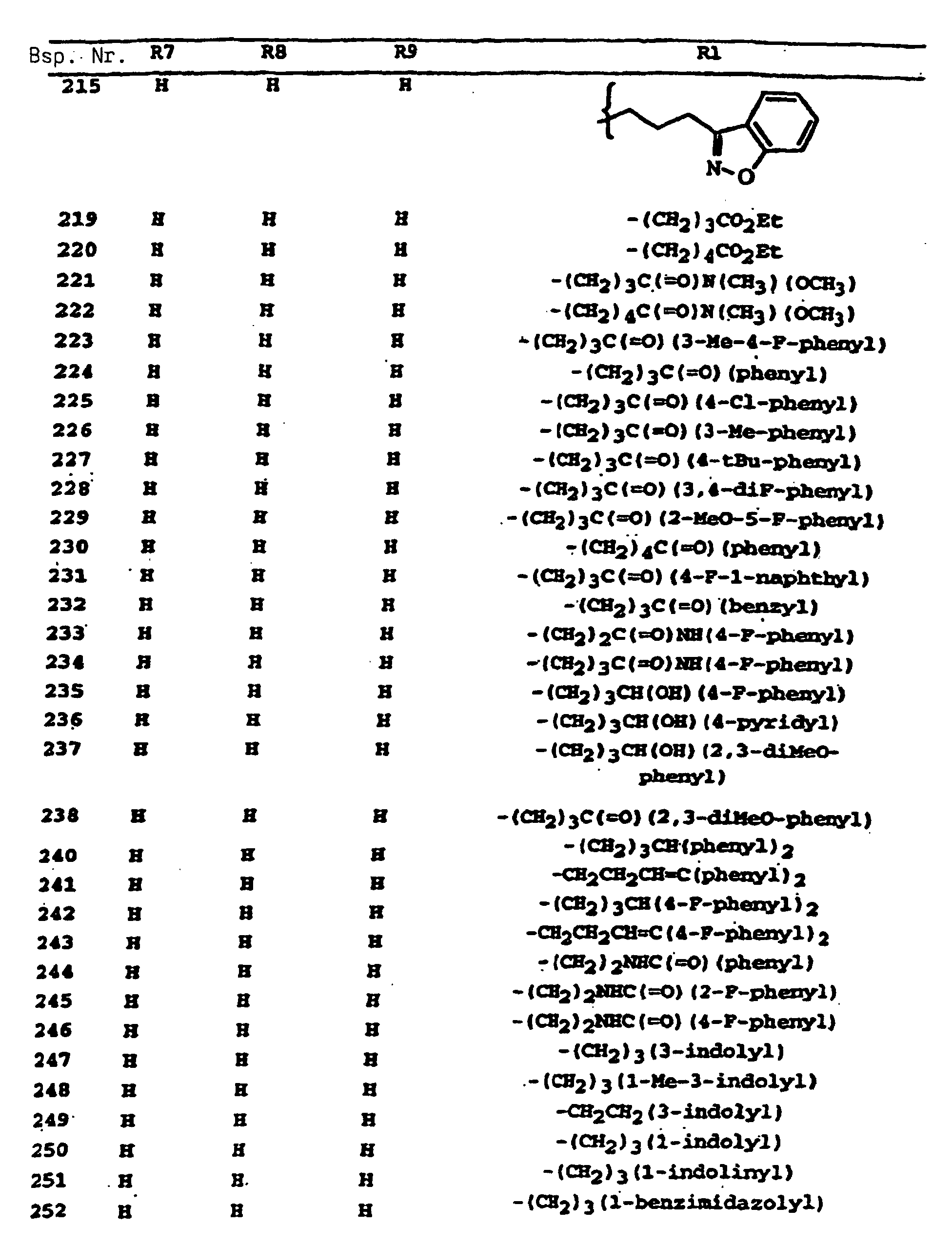

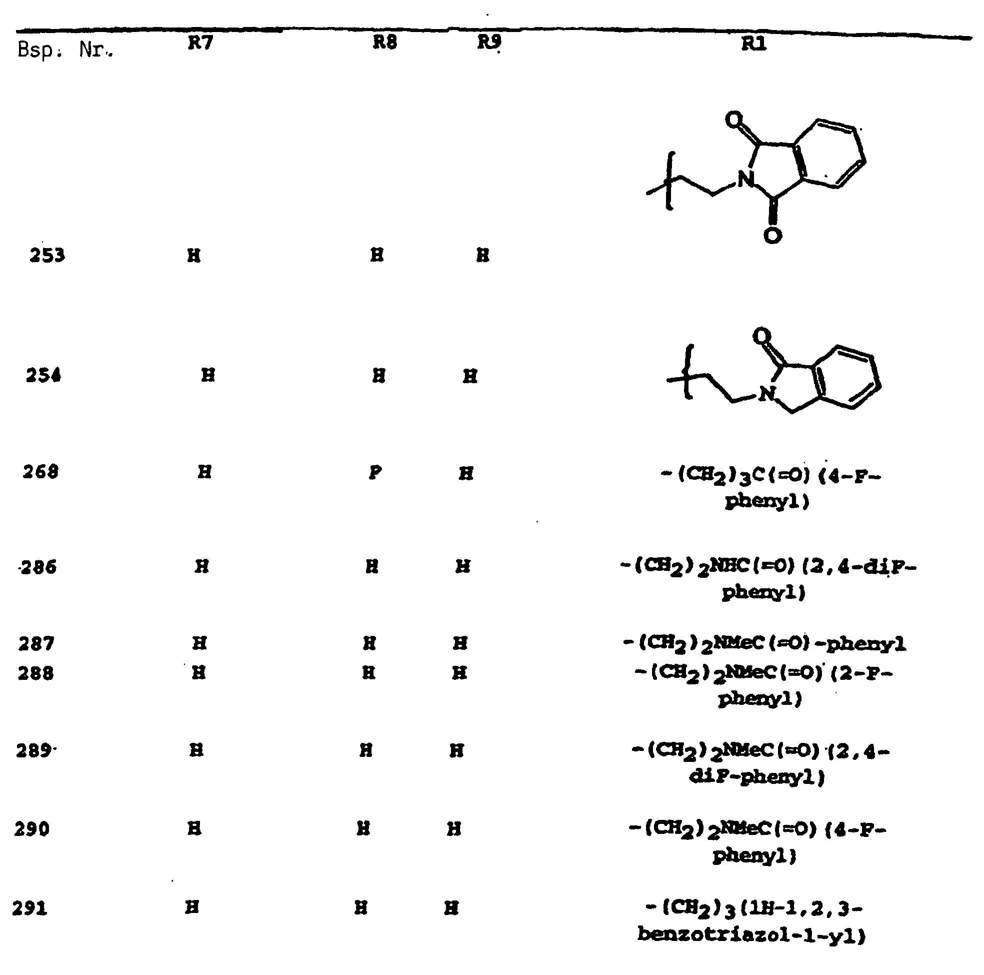

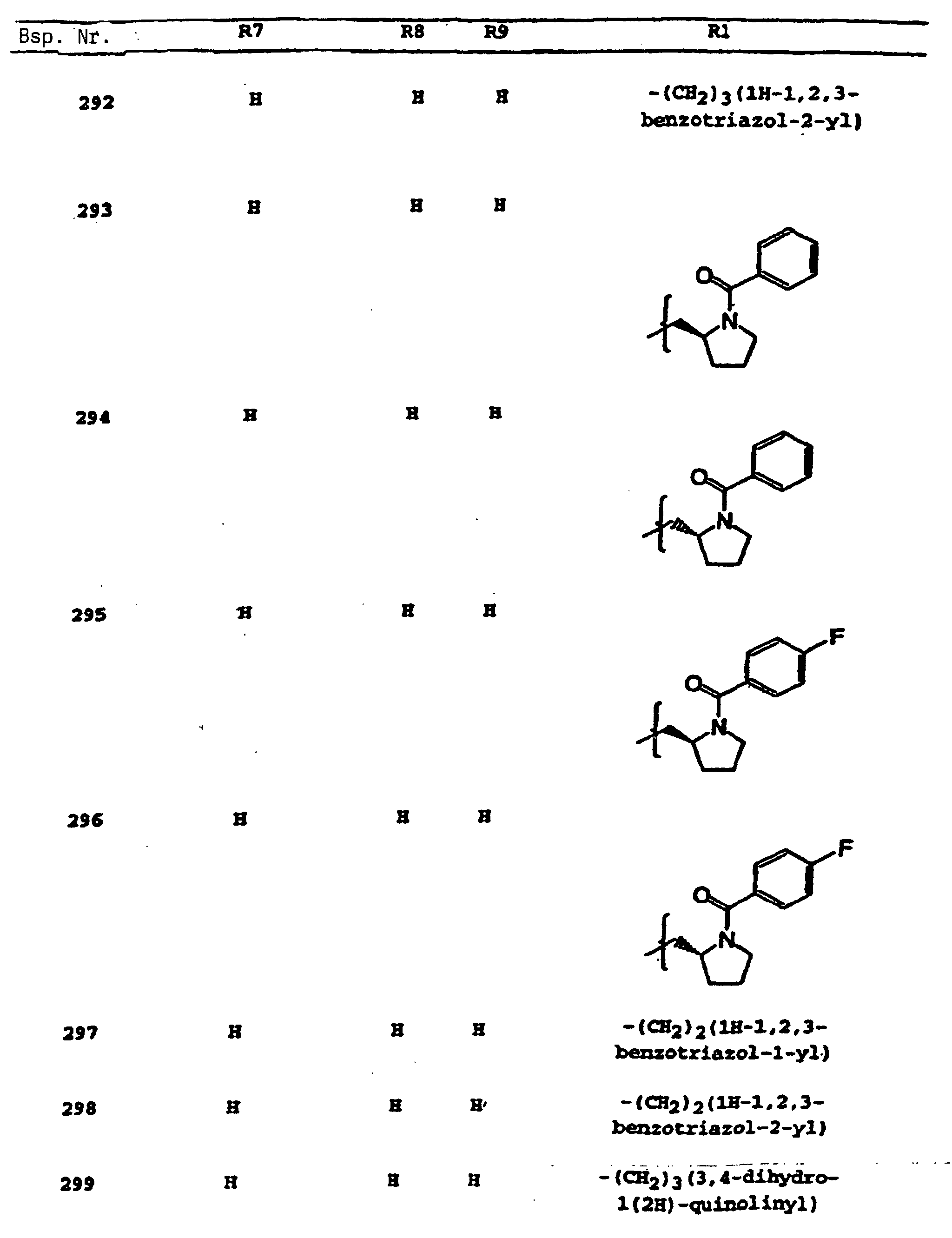

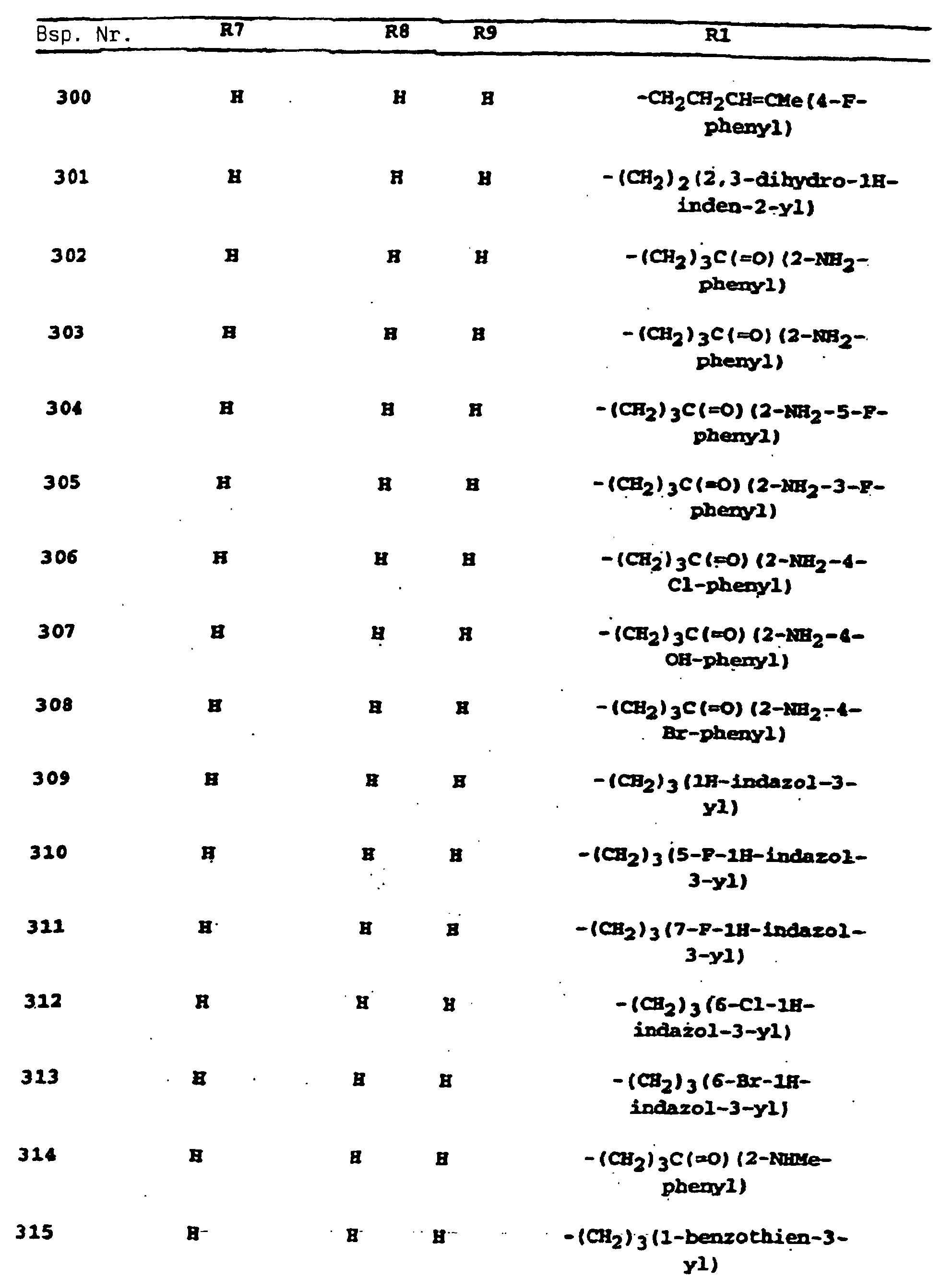

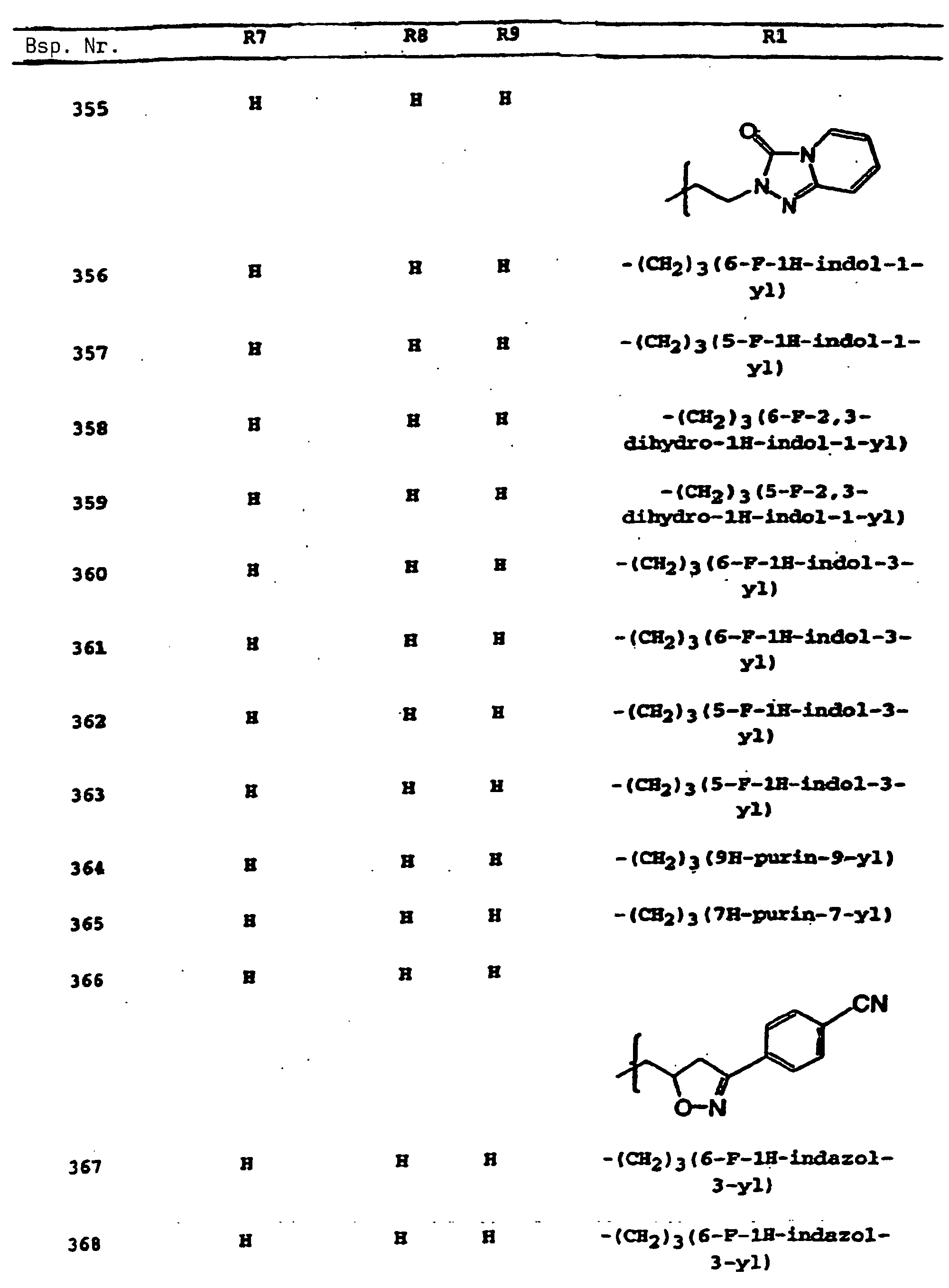

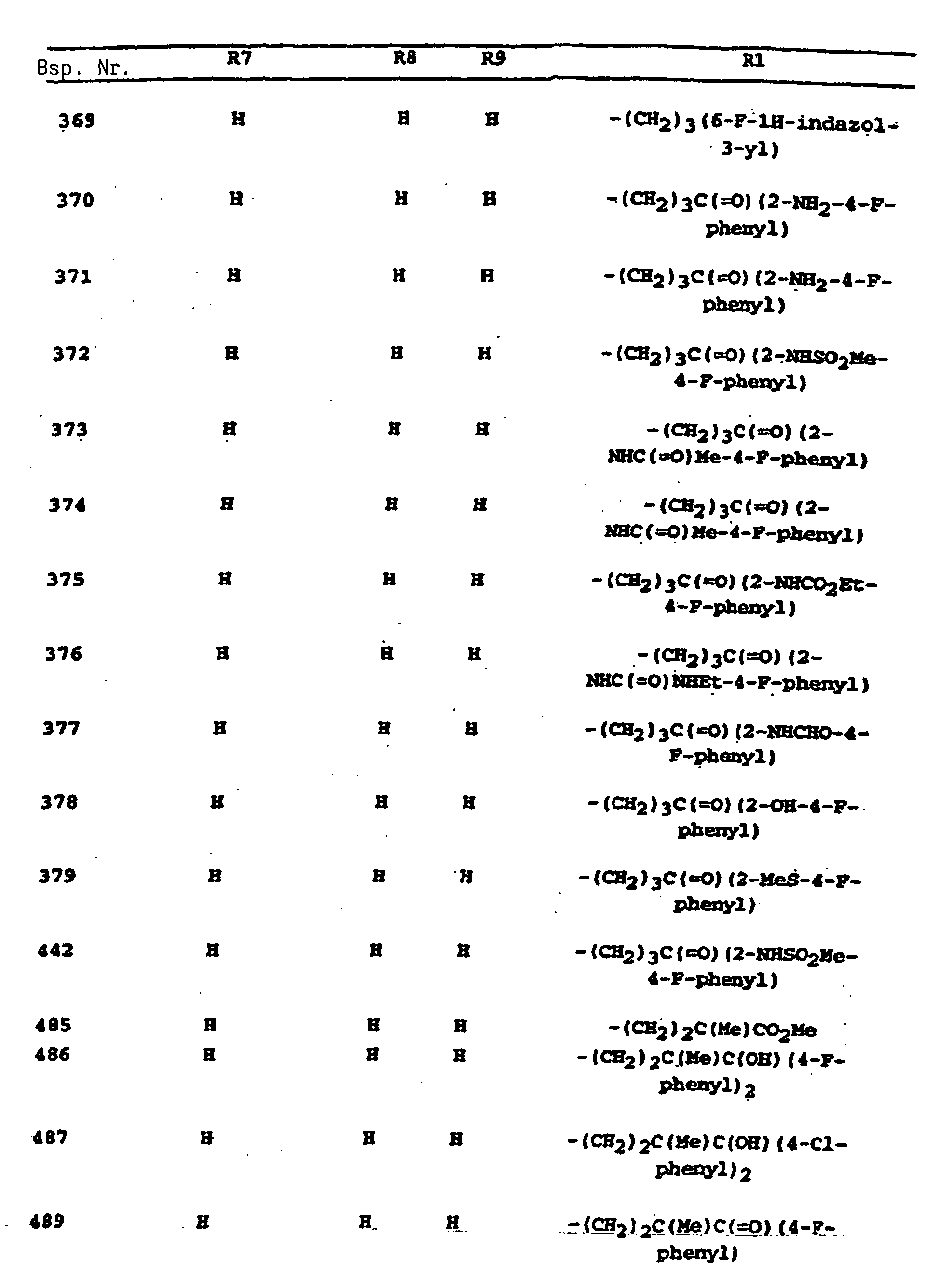

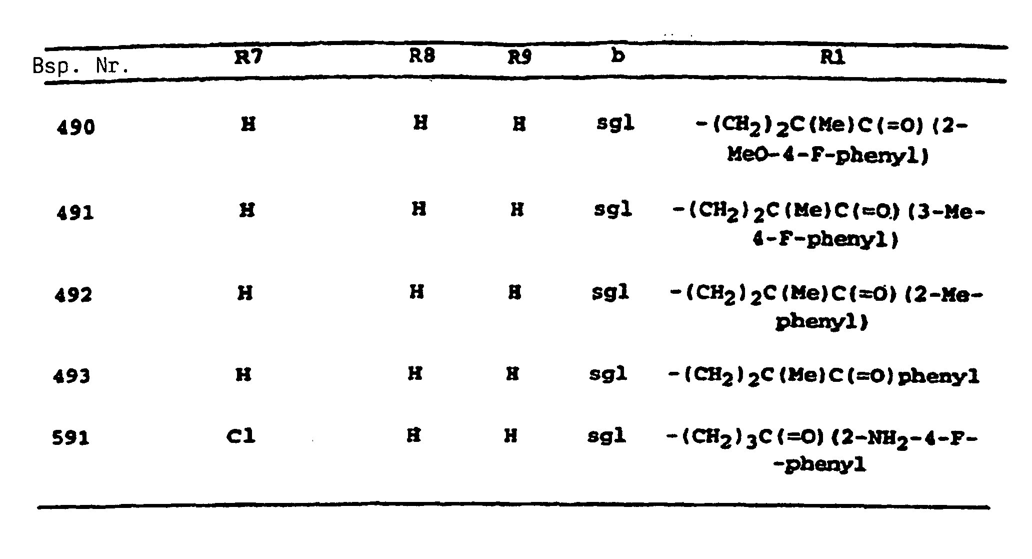

6. Verbindung nach Anspruch 1, ausgewählt aus der Gruppe, bestehend aus Verbindungen der Formel 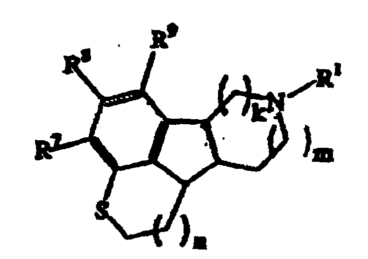
| Bsp. Nr. | n | k | m | R7 | R8 | R9 | R1 |
|---|---|---|---|---|---|---|---|
| 472 | 2 | 2 | 1 | H | H | H | -(CH₂)₃C(=O)(4-F-phenyl) |
| 473 | 2 | 2 | 1 | H | H | H | -(CH₂)₃O(4-F-phenyl) |
| 474 | 2 | 2 | 1 | H | H | H | -(CH₂)₃(6-F-benzisoxazol-3-yl) |
| 475 | 2 | 2 | 1 | H | H | H | -(CH₂)₃C(=O)(4-pyridyl) |
| 483 | 2 | 2 | 1 | H | Br | H | -(CH₂)₃C(=O)(4-F-phenyl) |
| 484 | 2 | 2 | 1 | H | Br | H | -(CH₂)₃O(4-F-phenyl) |

7. Verbindung nach Anspruch 1, susgewählt aus der Gruppe, bestehend aus Verbindungen der Formel 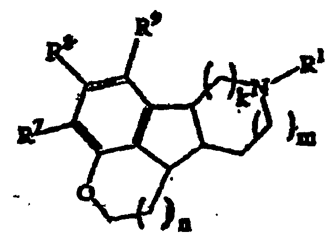
| Bsp. Nr. | n | k | m | R7 | R8 | R9 | R1 |
|---|---|---|---|---|---|---|---|
| 182 | 1 | 1 | 1 | H | H | H | -(CH₂)₃C(=O)(4-F-phenyl) |
| 266 | 1 | 1 | 1 | H | H | Me | -(CH₂)₃C(=O)(4-F-phenyl) |
| 270 | 1 | 1 | 1 | H | H | H | -(CH₂)₃O(4-F-phenyl) |
| 272 | 1 | 1 | 1 | H | H | H | H |
| 494 | 1 | 1 | 1 | H | H | H | -(CH₂)₃C(=O)(2-NH₂-phenyl) |
| 495 | 1 | 1 | 1 | H | H | H | -(CH₂)₃C(=O)(2-NH₂-phenyl) |
| 496 | 1 | 1 | 1 | H | H | H | -(CH₂)₃(1H-indazol-3-yl) |
| 528 | 1 | 1 | 1 | H | H | H | -(CH₂)₃(6-F-1H-indazol-3-yl) |
| 529 | 1 | 1 | 1 | H | H | H | -(CH₂)₃C(=O)(2-NH₂-4-F-phenyl) |
| 530 | 1 | 1 | 1 | H | H | H | -(CH₂)₃C(=O)(2-NH₂-4-F-phenyl) |
| 531 | 1 | 1 | 1 | H | H | H | -(CH₂)₃C(=O)(2-OH-4-F-phenyl) |
| 539 | 1 | 2 | 1 | H | H | H | -(CH₂)₃O(4-F-phenyl) |
| 540 | 1 | 2 | 1 | H | H | H | -(CH₂)₃(6-F-1,2-benzisoxazol-3-yl) |
| 544 | 2 | 1 | 1 | H | H | H | sgl -(CH₂)₃C(=O)(4-F-phenyl) |
| 546 | 1 | 2 | 1 | H | H | H | sgl -(CH₂)₃C(=0)(4-F-phenyl) |

8. Verbindung nach Anspruch 1, ausgewählt aus der Gruppe, bestehend aus Verbindungen der Formel 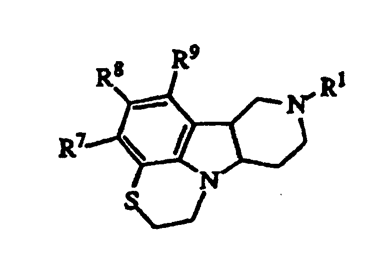
| Bsp. Nr. | R7 | R8 | R9 | R1 |
|---|---|---|---|---|
| 185 | H | H | CF₃ | -(CH₂)₄(4-F-phenyl) |
| 188 | H | H | H | -(CH₂)₃C(=O)(4-F-phenyl) |
| 213 | H | CH₃ | H | -(CH₂)₃C(=O)(4-F-phenyl) |
| 438 | H | H | H | -(CH₂)₃C(=O)(2-NH₂-phenyl) |
| 439 | H | H | H | -(CH₂)₃C(=O)(2-NH₂-phenyl) |
| 440 | H | H | H | -(CH₂)₃C(=O)(2-NH₂-4-F-phenyl) |
| 441 | H | H | H | -(CH₂)₃C(=O)(2-NH₂-4-F-phenyl) |
| 456 | H | H | H | -(CH₂)₃C(=O)(4-F-phenyl) |
| 457 | H | H | H | -(CH₂)₃C(=O)(4-F-phenyl) |

9. Pharmazeutische Zusammensetzung, umfassend einen pharmazeutisch akzeptablen Träger und eine therapeutisch wirksame Menge einer Verbindung nach einem der Ansprüche 1 bis 8 oder ein pharmazeutisch akzeptables Salz davon.

10. Verbindung nach einem der Ansprüche 1 bis 8 zur Verwendung in der Therapie.

11. Verbindung nach einem der Ansprüche 1 bis 8 zur Verwendung bei der Behandlung von Obesitas, Schizophrenie oder Depression.

12. Verwendung einer Verbindung nach einem der Ansprüche 1 bis 8 zur Herstellung eines Medikaments zur Behandlung von Obesitas, Schizophrenie oder Depression.

## Revendications

1. Un composé de Formule (I-a) 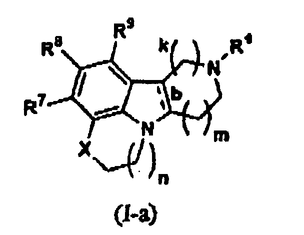 ou l'un de ses sels acceptables pharmaceutiquement, dans laquelle :
b est une simple liaison ;
X est - S - ou -O- ;
R¹ est choisi parmi
-(CH₂)₃C(=O)(4-fluoro-phényle),
-(CH₂)₃C(=O)(4-bromo-phényle),
-(CH₂)₃C(=O)(4-méthyl-phényle),
-(CH₂)₃C(=O)(4-méthoxy-phényle),
-(CH₂)₃C(=O)(4-(3,4-dichloro-phényl)phényle),
-(CH₂)₃C(=O)(3-méthyl-4-fluoro-phényle),
-(CH₂)₃C(=O)(2,3-diméthoxy-phényle),
-(CH₂)₃C(=O)(phényle),
-(CH₂)₃C(=O)(4-chloro-phényle),
-(CH₂)₃C(=O)(3-méthyl-phényle),
-(CH₂)₃C(=O)(4-t-butyl-phényle),
-(CH₂)₃C(=O)(3,4-difluoro-phényle),
-(CH₂)₃C(=O)(2-méthoxy-5-fluoro-phényle),
-(CH₂)₃C(=O)(4-fluoro-1-naphtyle),
-(CH₂)₃C(=O)(benzyle),
-(CH₂)₃C(=O)(4-pyridyle),
-(CH₂)₃C(=O)(3-pyridyle),
-(CH₂)₃CH(OH)(4-fluoro-phényle),
-(CH₂)₃CH(OH)(4-pyridyle),
-(CH₂)₃CH(OH)(2,3-diméthoxy-phényle),
-(CH₂)₃S(3-fluoro-phényle),
-(CH₂)₃S(4-fluoro-phényle),
-(CH₂)₃S(=O)(4-fluoro-phényle),
-(CH₂)₃SO₂(3-fluoro-phényle),
-(CH₂)₃SO₂(4-fluoro-phényle),
-(CH₂)₃O(4-fluoro-phényle),
-(CH₂)₃O(phényle),
-(CH₂)₃O(3-pyridyle),
-(CH₂)₃O(4-pyridyle),
-(CH₂)₃O(2-NH₂-phényle),
-(CH₂)₃O(2-NH₂-5-F-phényle),
-(CH₂)₃O(2-NH₂-4-F-phényle),
-(CH₂)₃O(2-NH₂-3-F-phényle),
-(CH₂)₃O(2-NH₂-4-Cl-phényle),
-(CH₂)₃O(2-NH₂-4-OH-phényle),
-(CH₂)₃O(2-NH₂-4-Br-phényle),
-(CH₂)₃O(2-NHC(=O)Me-4-F-phényle),
-(CH₂)₃O(2-NHC(=O)Me-phényle),
-(CH₂)₃NH(4-fluoro-phényle),
-(CH₂)₃N(méthyl)(4-fluoro-phényle),
-(CH₂)₃CO₂(éthyle),
-(CH₂)₃C(=O)N(méthyl)(méthoxy),
-(CH₂)₃C(=O)NH(4-fluoro-phényle),
-(CH₂)₂NHC(=O)(phényle),
-(CH₂)₂NMeC(=O)(phényle),
-(CH₂)₂NHC(=O)(2-fluoro-phényle),
-(CH₂)₂NMeC(=O)(2-fluoro-phényle),
-(CH₂)₂NHC(=O)(4-fluoro-phényle),
-(CH₂)₂NMeC(=O)(4-fluoro-phényle),
-(CH₂)₂NHC(=O)(2,4-difluoro-phényle),
-(CH₂)₂NMeC(=O)(2,4-dufluoro-phényle),
-(CH₂)₃(3-indolyle),
-(CH₂)₃(1-méthyl-3-indolyle),
-(CH₂)₃(1-indolyle),
-(CH₂)₃(1-indolinyle),
-(CH₂)₃(1-benzimidazolyle),
-(CH₂)₃(1H-1,2,3-benzotriazol-1-yle),
-(CH₂)₃(1H-1,2,3-benzotriazol-2-yle),
-(CH₂)₂(1H-1,2,3-benzotriazol-1-yle),
-(CH₂)₂(1H-1,2,3-benzotriazol-2-yle),
-(CH₂)₃(3,4-dihydro-1(2H)-quinolinyle),
-(CH₂)₂C(=O)(4-fluoro-phényle),
-(CH₂)₂C(=O)NH(4-fluoro-phényle),
-CH₂CH₂(3-indolyle),
-CH₂CH₂(1-phtalimidyle),
-(CH₂)₄C(=O)N(méthyl)(méthoxy),
-(CH₂)₄CO₂(éthyle),
-(CH₂)₄C(=O)(phényle),
-(CH₂)₃CH(phényle)₂,
-CH₂CH₂CH=C(phényle)₂,
-CH₂CH₂CH=CMe(4-F-phényle),
-(CH₂)₃CH(4-fluoro-phényle)₂,
-CH₂CH₂CH=C(4-fluoro-phényle)₂,
-(CH₂)₂(2,3-dihydro-1H-indène-2-yle),
-(CH₂)₃C(=O)(2-NH₂-phényle),
-(CH₂)₃C(=O)(2-NH₂-5-F-phényle),
-(CH₂)₃C(=O)(2-NH₂-4-F-phényle),
-(CH₂)₃C(=O)(2-NH₂-3-F-phényle),
-(CH₂)₃C(=O)(2-NH₂-4-Cl-phényle),
-(CH₂)₃C(=O)(2-NH₂-4-OH-phényle),
-(CH₂)₃C(=O)(2-NH₂-4-Br-phényle),
-(CH₂)₃(1H-indazol-3-yle),
-(CH₂)₃(5-F-1H-indazol-3-yle),
-(CH₂)₃(7-F-1H-indazol-3-yle),
-(CH₂)₃(6-Cl-1H-indazol-3-yle),
-(CH₂)₃(6-Br-1H-indazol-3-yle),
-(CH₂)₃C(=O)(2-NHMe-phényle),
-(CH₂)₃(1-benzothiène-3-yle),
-(CH₂)₃(6-F-1H-indol-1-yle),
-(CH₂)₃(5-F-1H-indol-1-yle),
-(CH₂)₃(6-F-2,3-dihydro-1H-indol-1-yle),
-(CH₂)₃(5-F-2,3-dihydro-1H-indol-1-yle),
-(CH₂)₃(6-F-1H-indol-3-yle),
-(CH₂)₃(5-F-1H-indol-3-yle),
-(CH₂)₃(5-F-1H-indol-3-yle),
-(CH₂)₃(9H-purin-9-yle),
-(CH₂)₃(7H-purin-7-yle),
-(CH₂)₃(6-F-1H-indazol-3-yle),
-(CH₂)₃C(=O)(2-NHSO₂Me-4-F-phényle),
-(CH₂)₃C(=O)(2-NHC(=O)Me-4-F-phényle),
-(CH₂)₃C(=O)(2-NHC(=O)Me-phényle),
-(CH₂)₃C(=O)(2-NHCO₂Et-4-F-phényle),
-(CH₂)₃C(=O)(2-NHC(=O)NHEt-4-F-phényle),
-(CH₂)₃C(=O)(2-NHCHO-4-F-phényle),
-(CH₂)₃C(=O)(2-OH-4-F-phényle),
-(CH₂)₃C(=O)(2-MeS-4-F-phényle),
-(CH₂)₃C(=O)(2-NHSO₂Me-4-F-phényle),
-(CH₂)₃C(=O)(2-NHSO₂Me-4-F-phényle),
-(CH₂)₂C(Me)CO₂Me,
-(CH₂)₂C(Me)CH(OH)(4-F-phényle)₂,
-(CH₂)₂C(Me)CH(OH)(4-Cl-phényle)₂,
-(CH₂)₂C(Me)C(=O)(4-F-phényle),
-(CH₂)₂C(Me)C(=O)(2-MeO-4-F-phényle),
-(CH₂)₂C(Me)C(=O)(3-Me-4-F-phényle),
-(CH₂)₂C(Me)C(=O)(2-Me-phényle),
-(CH₂)₂C(Me)C(=O)phényle, 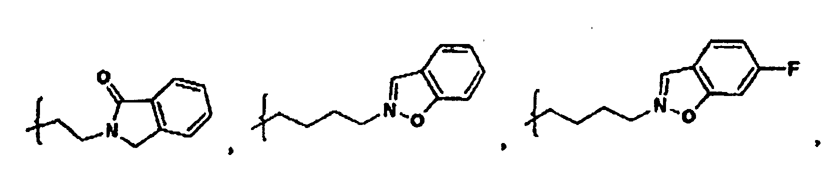 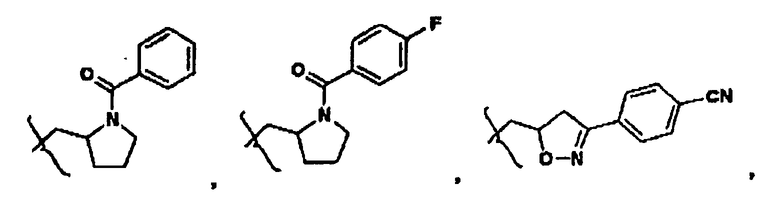 et 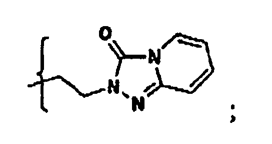
R⁷, R⁸ et R⁹, chaque fois qu'ils apparaissent, sont choisis indépendamment parmi hydrogène, fluoro, chloro, bromo, cyano, méthyle, éthyle, propyle, isopropyle, butyle, t-butyle, nitro, trifluorométhyle, méthoxy, éthoxy, isopropoxy, trifluorométhoxy, phényle, benzyle, HC(=O)-, méthylC(=O)-, éthylC(=O)-, propylC(=O)-, isopropylC(=O)-, n-butylC(=O)-, isobutylC(=O)-, secbutylC(=O)-, tertbutylC(=O)-, phénylC(=O)-, méthylC(=O)NH-, éthylC(=O)NH-, propylC(=O)NH-, isopropylC(=O)NH-, n-butylC(=O)-, isobutylC(=O)NH-, secbutylC(=O)NH-, tertbuylC(=O)-, phénylC(=O)NH-, méthylamino-, éthylamino-, propylamino-, isopropylamino-, n-butylamino-, isobutylamino-, sectubylamino-, tertbutylamino-, phénylamino-,
pourvu que deux des substituants R⁷, R⁸ et R⁹ soient choisis indépendamment parmi hydrogène, fluoro, chloro, bromo, cyano, méthyle, éthyle, propyle, isopropyle, butyle, t-butyle, nitro, trifluorométhyle, méthoxy, éthoxy, isopropoxy et trifluorométhoxy ;
k est 1 ou 2 ;
m est 1 ou 2 ; et
n est 1 ou 2.

2. Un composé suivant la revendication 1 de Formule (II-a) ou de Formule (III-a) 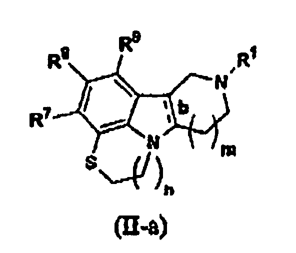 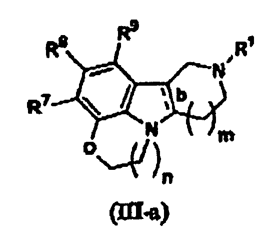 dans lesquelles :
b est une simple liaison, les hydrogènes du pont étant en une position cis ;
R¹ est choisi parmi
-(CH₂)₃C(=O)(4-fluoro-phényle),
-(CH₂)₃C(=O)(4-bromo-phényle),
-(CH₂)₃C(=O)(4-méthyl-phényle),
-(CH₂)₃C(=O)(4-méthoxy-phényle),
-(CH₂)₃C(=O)(4-(3,4-dichloro-phényl)phényle),
-(CH₂)₃C(=O)(3-méthyl-4-fluoro-phényle),
-(CH₂)₃C(=O)(2,3-diméthoxy-phényle),
-(CH₂)₃C(=O)(phényle),
-(CH₂)₃C(=O)(4-chloro-phényle),
-(CH₂)₃C(=O)(3-méthyl-phényle),
-(CH₂)₃C(=O)(4-t-butyl-phényle),
-(CH₂)₃C(=O)(3,4-difluoro-phényle),
-(CH₂)₃C(=O)(2-méthoxy-5-fluoro-phényle),
-(CH₂)₃C(=O)(4-fluoro-1-naphtyle),
-(CH₂)₃C(=O)(benzyle),
-(CH₂)₃C(=O)(4-pyridyle),
-(CH₂)₃C(=O)(3-pyridyle),
-(CH₂)₃CH(OH)(4-fluoro-phényle),
-(CH₂)₃CH(OH)(4-pyridyle),
-(CH₂)₃CH(OH)(2,3-diméthoxy-phényle),
-(CH₂)₃S(3-fluoro-phényle),
-(CH₂)₃S(4-fluoro-phényle),
-(CH₂)₃S(=O)(4-fluoro-phényle),
-(CH₂)₃SO₂(3-fluoro-phényle),
-(CH₂)₃SO₂(4-fluoro-phényle),
-(CH₂)₃O(4-fluoro-phényle),
-(CH₂)₃O(phényle),
-(CH₂)₃NH(4-fluoro-phényle),
-(CH₂)₃N(méthyl)(4-fluoro-phényle),
-(CH₂)₃CO₂(éthyle),
-(CH₂)₃C(=O)N(méthyl)(méthoxy),
-(CH₂)₃C(=O)NH(4-fluoro-phényle),
-(CH₂)₂NHC(=O)(phényle),
-(CH₂)₂NMeC(=O)(phényle),
-(CH₂)₂NHC(=O)(2-fluoro-phényle),
-(CH₂)₂NMeC(=O)(2-fluoro-phényle),
-(CH₂)₂NHC(=O)(4-fluoro-phényle),
-(CH₂)₂NMeC(=O)(4-fluoro-phényle),
-(CH₂)₂NHC(=O)(2,4-difluoro-phényle),
-(CH₂)₂NMeC(=O)(2,4-dufluoro-phényle),
-(CH₂)₃(3-indolyle),
-(CH₂)₃(1-méthyl-3-indolyle),
-(CH₂)₃(1-indolyle),
-(CH₂)₃(1-indolinyle),
-(CH₂)₃(1-benzimidazolyle),
-(CH₂)₃(1H-1,2,3-benzotriazol-1-yle),
-(CH₂)₃(1H-1,2,3-benzotriazol-2-yle),
-(CH₂)₂(1H-1,2,3-benzotriazol-1-yle),
-(CH₂)₂(1H-1,2,3-benzotriazol-2-yle),
-(CH₂)₃(3,4-dihydro-1(2H)-quinolinyle),
-(CH₂)₂C(=O)(4-fluoro-phényle),
-(CH₂)₂C(=O)NH(4-fluoro-phényle),
-CH₂CH₂(3-indolyle),
-CH₂CH₂(1-phtalimidyle),
-(CH₂)₄C(=O)N(méthyl)(méthoxy),
-(CH₂)₄CO₂(éthyle),
-(CH₂)₄C(=O)(phényle),
-(CH₂)₃CH(phényle)₂,
-CH₂CH₂CH=C(phényle)₂,
-CH₂CH₂CH=CMe(4-F-phényle),
-(CH₂)₃CH(4-fluoro-phényle)₂,
-CH₂CH₂CH=C(4-fluoro-phényle)₂,
-(CH₂)₂(2,3-dihydro-1H-indène-2-yle),
-(CH₂)₃C(=O)(2-NH₂-phényle),
-(CH₂)₃C(=O)(2-NH₂-5-F-phényle),
-(CH₂)₃C(=O)(2-NH₂-4-F-phényle),
-(CH₂)₃C(=O)(2-NH₂-3-F-phényle),
-(CH₂)₃C(=O)(2-NH₂-4-Cl-phényle),
-(CH₂)₃C(=O)(2-NH₂-4-OH-phényle),
-(CH₂)₃C(=O)(2-NH₂-4-Br-phényle),
-(CH₂)₃(1H-indazol-3-yle),
-(CH₂)₃(5-F-1H-indazol-3-yle),
-(CH₂)₃(7-F-1H-indazol-3-yle),
-(CH₂)₃(6-Cl-1H-indazol-3-yle),
-(CH₂)₃(6-Br-1H-indazol-3-yle),
-(CH₂)₃C(=O)(2-NHMe-phényle),
-(CH₂)₃(1-benzothiène-3-yle),
-(CH₂)₃(6-F-1H-indol-1-yle),
-(CH₂)₃(5-F-1H-indol-1-yle),
-(CH₂)₃(6-F-2,3-dihydro-1H-indol-1-yle),
-(CH₂)₃(5-F-2,3-dihydro-1H-indol-1-yle),
-(CH₂)₃(6-F-1H-indol-3-yle),
-(CH₂)₃(5-F-1H-indol-3-yle),
-(CH₂)₃(5-F-1H-indol-3-yle),
-(CH₂)₃(9H-purin-9-yle),
-(CH₂)₃(7H-purin-7-yle),
-(CH₂)₃(6-F-1H-indazol-3-yle),
-(CH₂)₃C(=O)(2-NHSO₂Me-4-F-phényle),
-(CH₂)₃C(=O)(2-NHC(=O)Me-4-F-phényle),
-(CH₂)₃C(=O)(2-NHC(=O)Me-phényle),
-(CH₂)₃C(=O)(2-NHCO₂Et-4-F-phényle),
-(CH₂)₃C(=O)(2-NHC(=O)NHEt-4-F-phényle),
-(CH₂)₃C(=O)(2-NHCHO-4-F-phényle),
-(CH₂)₃C(=O)(2-OH-4-F-phényle),
-(CH₂)₃C(=O)(2-MeS-4-F-phényle),
-(CH₂)₃C(=O)(2-NHSO₂Me-4-F-phényle),
-(CH₂)₃C(=O)(2-NHSO₂Me-4-F-phényle),
-(CH₂)₂C(Me)CO₂Me,
-(CH₂)₂C(Me)CH(OH)(4-F-phényle)₂,
-(CH₂)₂C(Me)CH(OH)(4-Cl-phényle)₂,
-(CH₂)₂C(Me)C(=O)(4-F-phényle),
-(CH₂)₂C(Me)C(=O)(2-MeO-4-F-phényle),
-(CH₂)₂C(Me)C(=O)(3-Me-4-F-phényle),
-(CH₂)₂C(Me)C(=O)(2-Me-phényle),
-(CH₂)₂C(Me)C(=O)phényle, 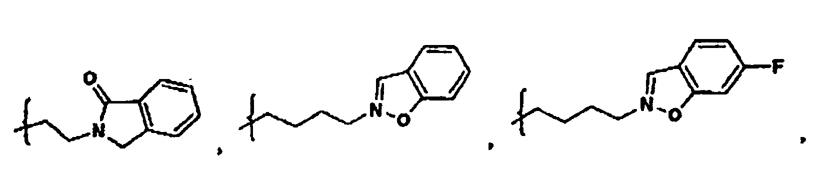 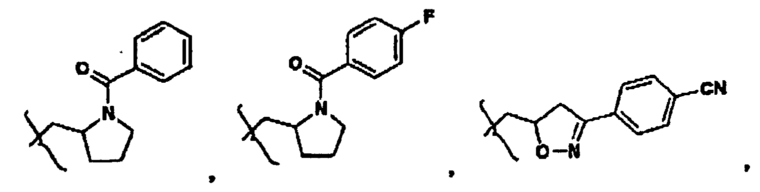 et 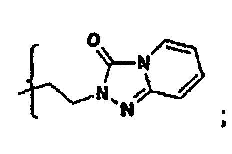
et
R⁷, R⁸ et R⁹ sont, chaque fois qu'ils se présentent, choisis indépendamment parmi hydrogène, fluoro, chloro, bromo, cyano, méthyle, éthyle, propyle, isopropyle, butyle, t-butyle, nitro, trifluorométhyle, méthoxy, éthoxy, isopropoxy, trifluorométhoxy, méthylC(=O)-, éthylC(=O)-, propylC(=O)-, isopropylC(=O)-, méthylC(=O)NH-, éthylC(=O)NH-, propylC(=O)NH-, isopropylC(=O)NH-, méthylamino-, éthylamino-, propylamino- et isopropylamino-,
pourvu que deux des substituants R⁷, R⁸ et R⁹ soient choisis indépendamment parmi hydrogène, fluoro, chloro, méthyle, trifluorométhyle, méthoxy et trifluorométhoxy.

3. Un composé suivant la revendication 2 de Formule (III-a)

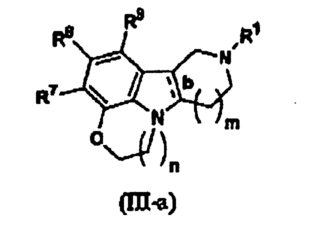

4. Un composé suivant la revendication 2 de Formule (II-a)

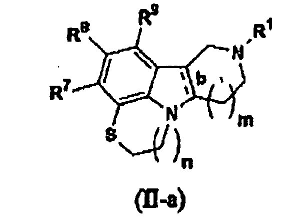

5. Un composé suivant la revendication 1, choisi dans le groupe consistant en des composés de formule

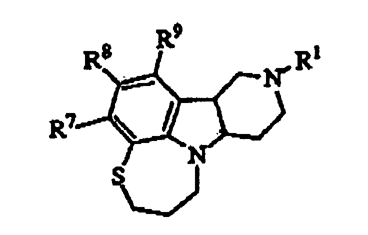

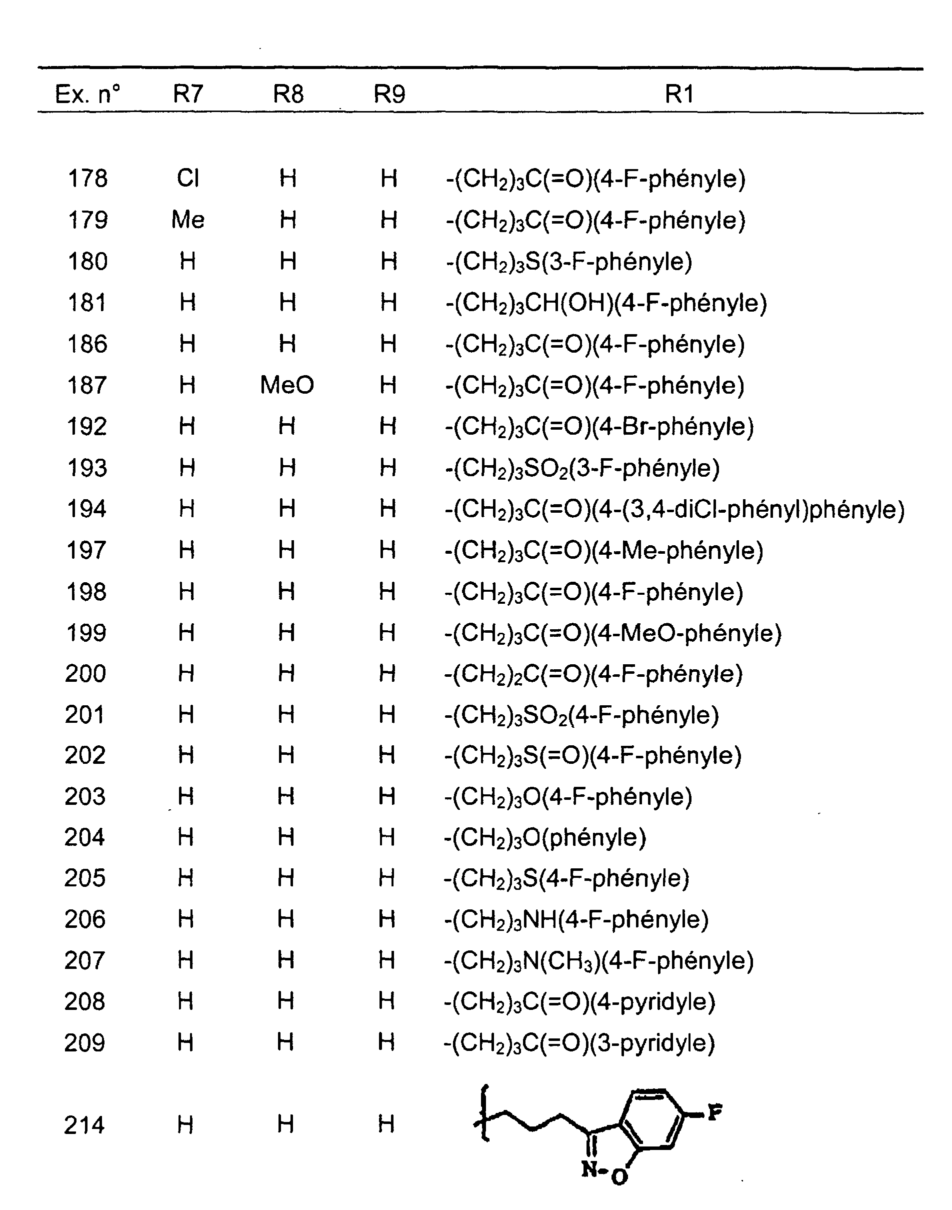

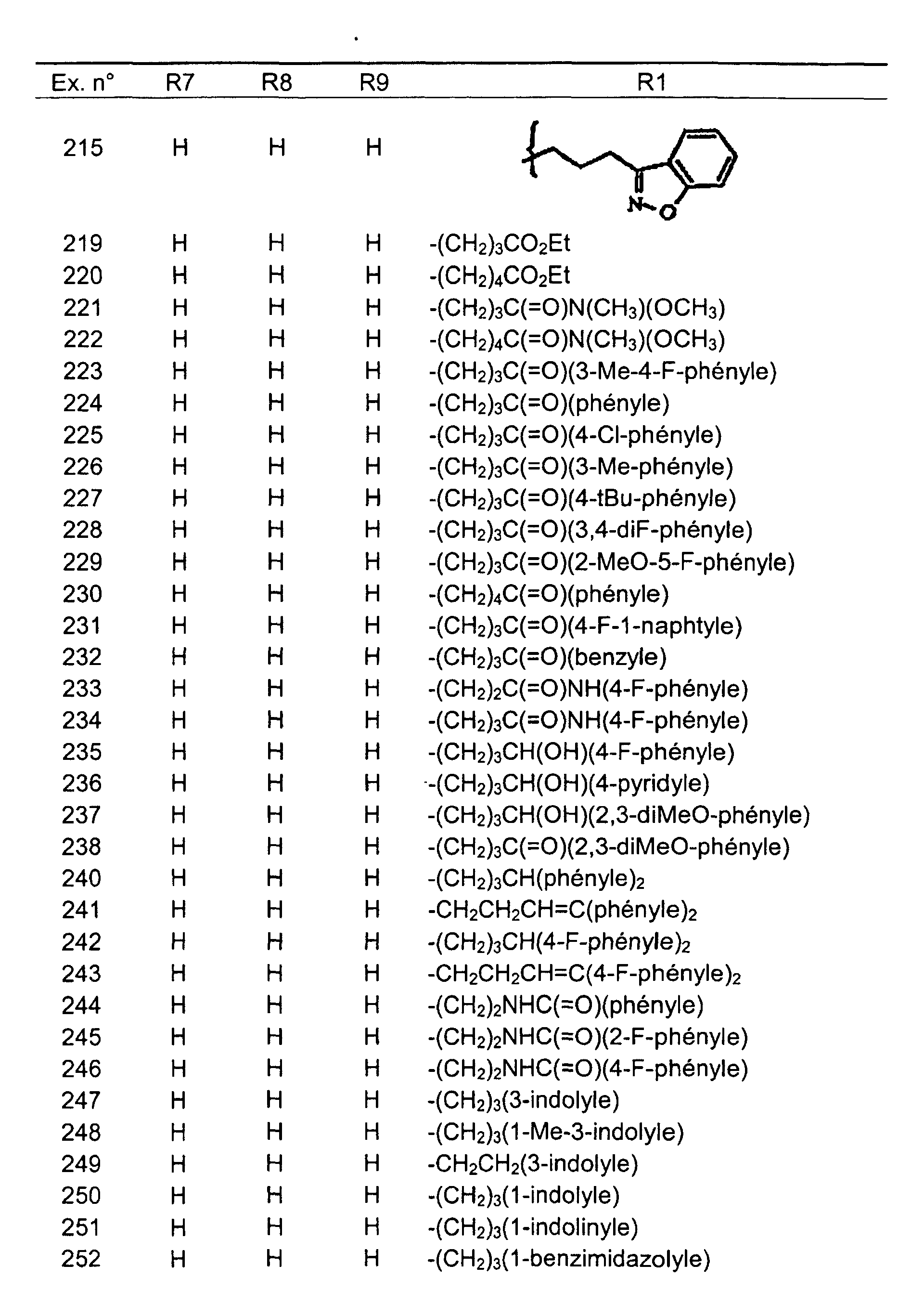

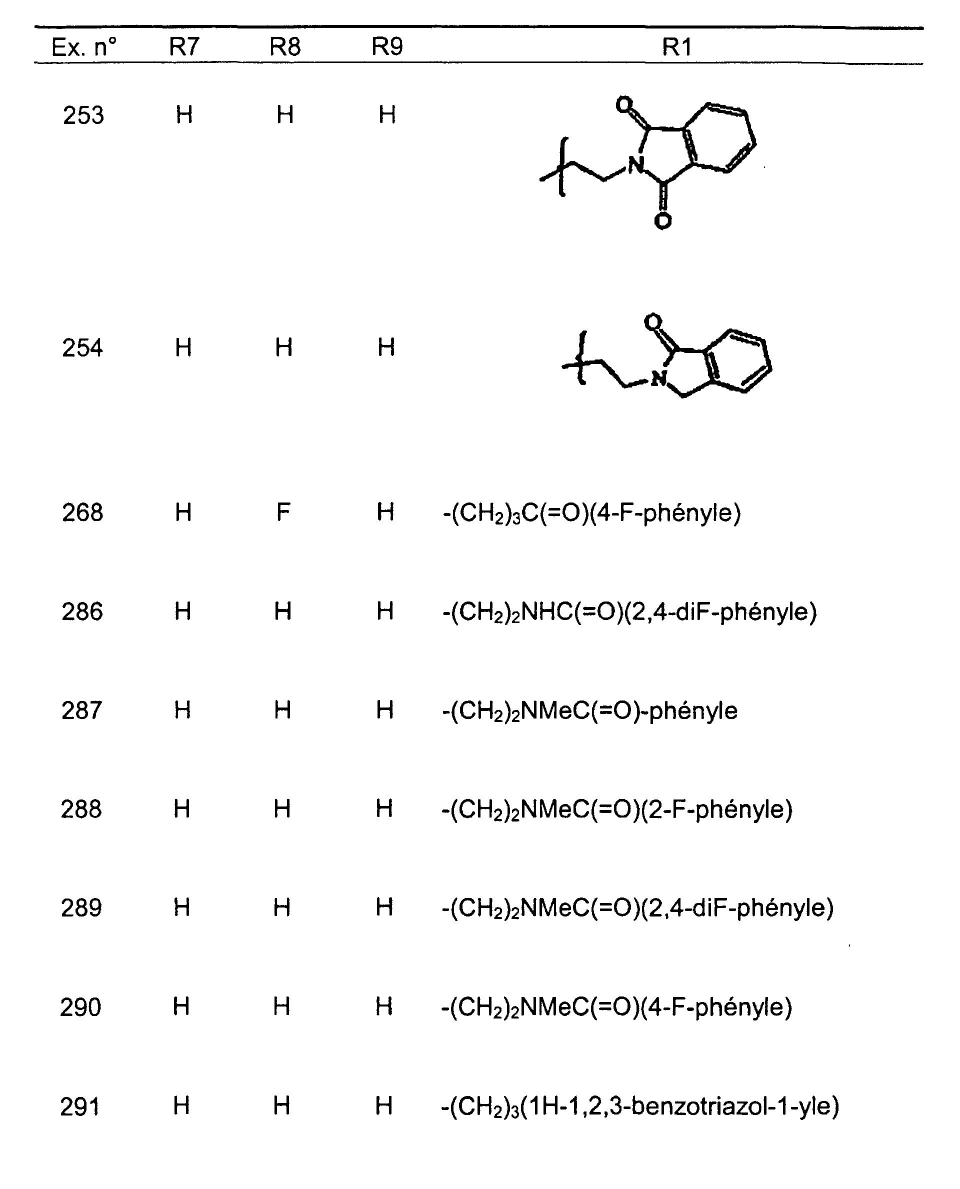

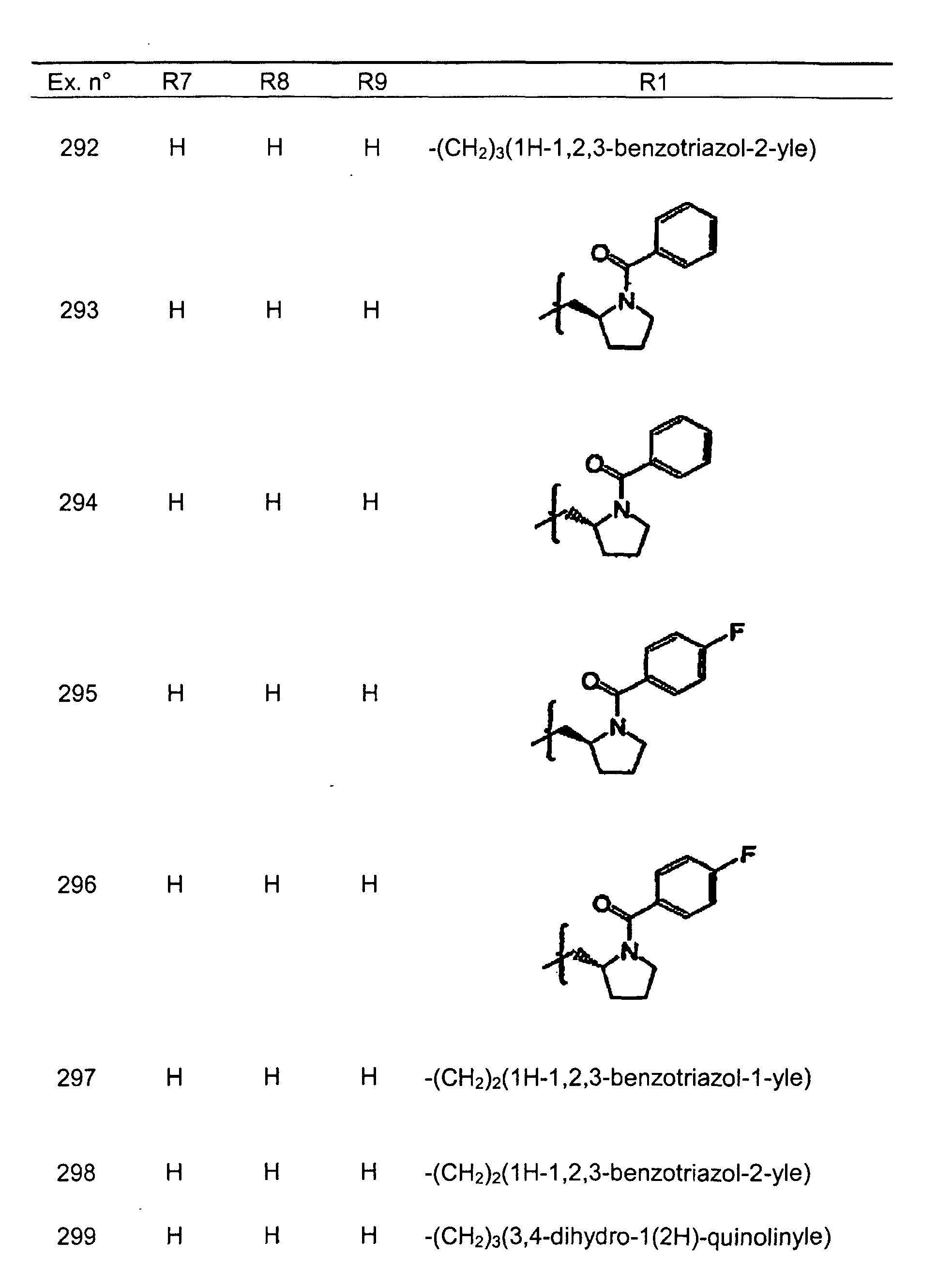

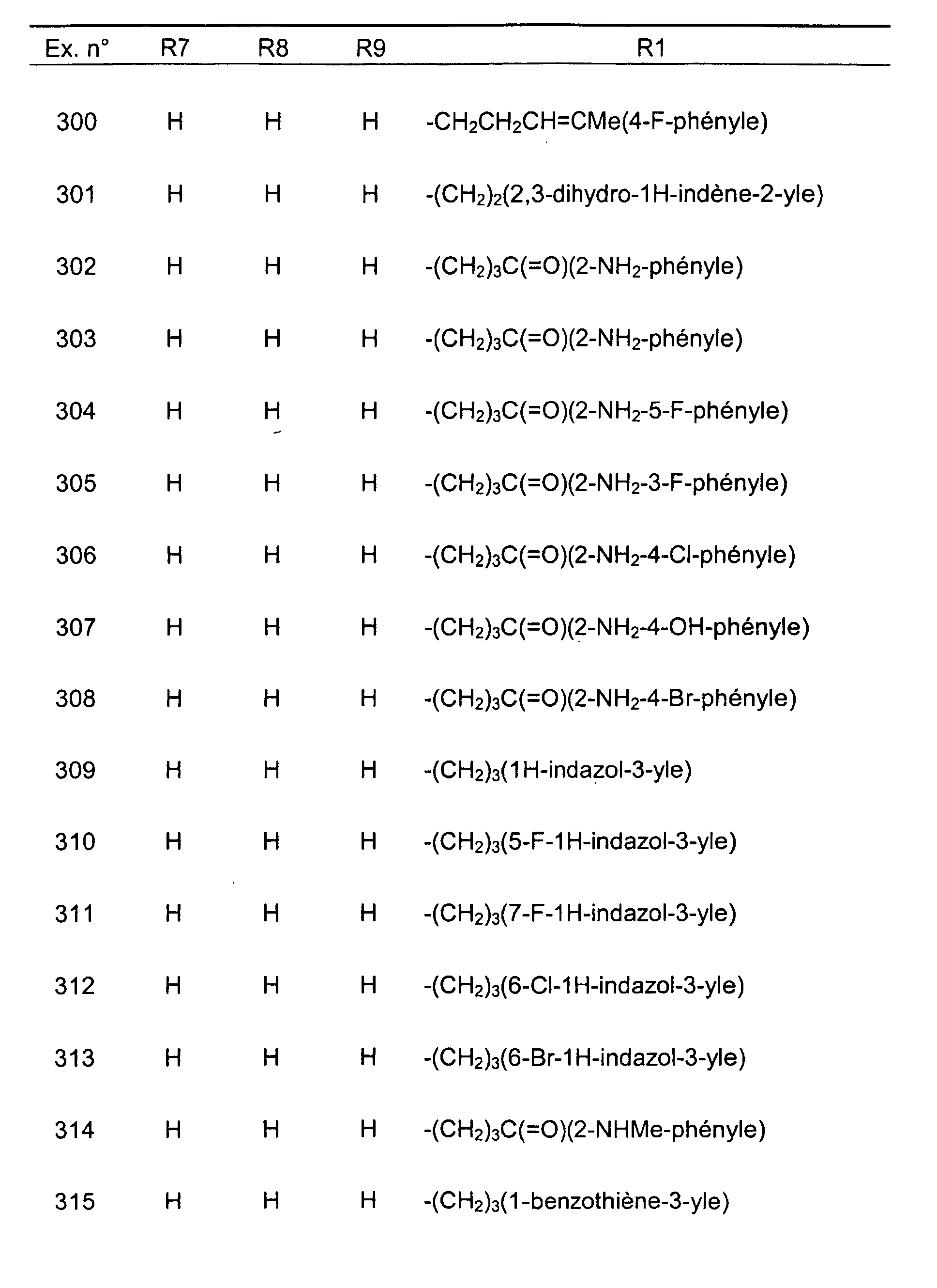

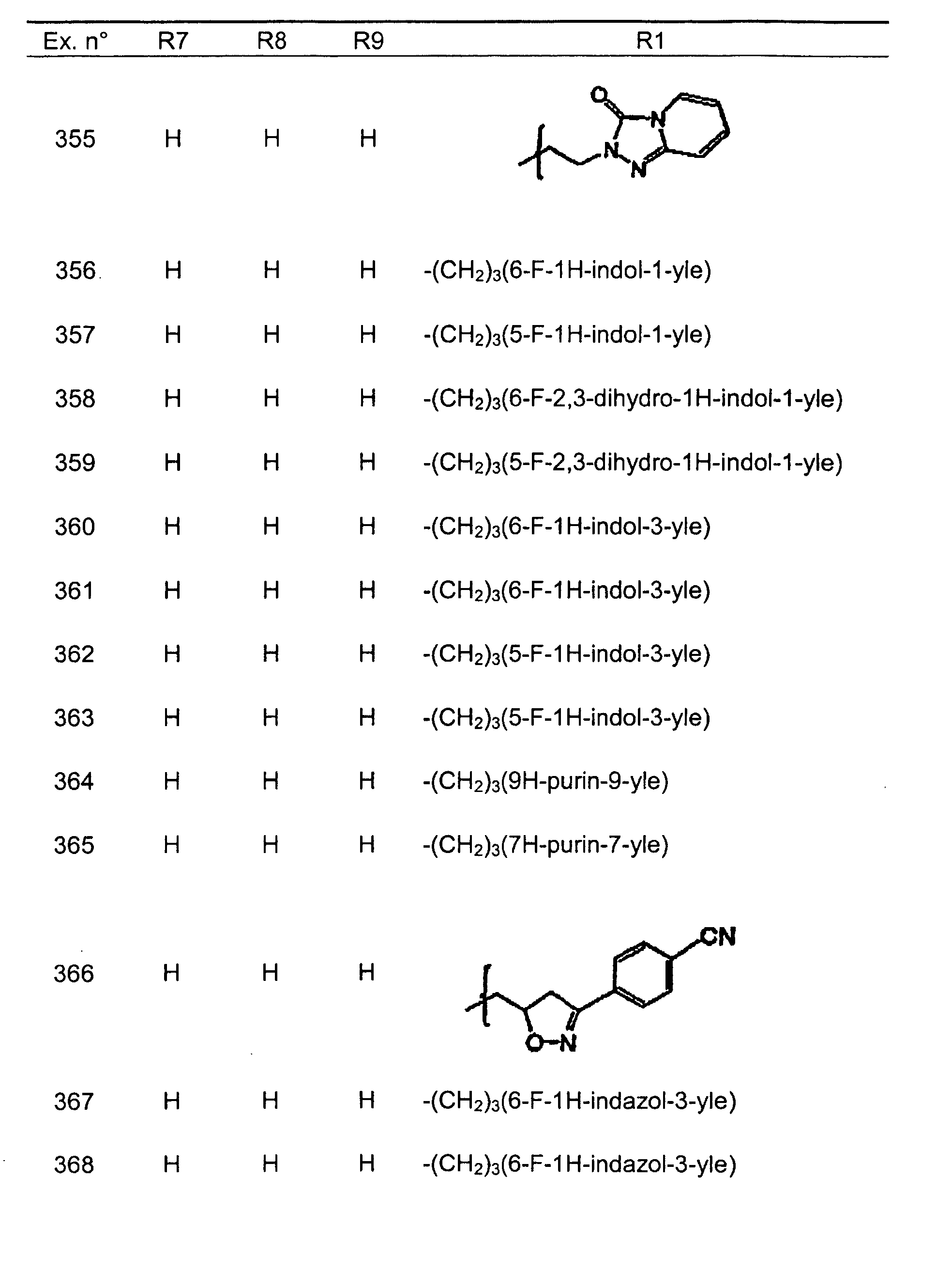

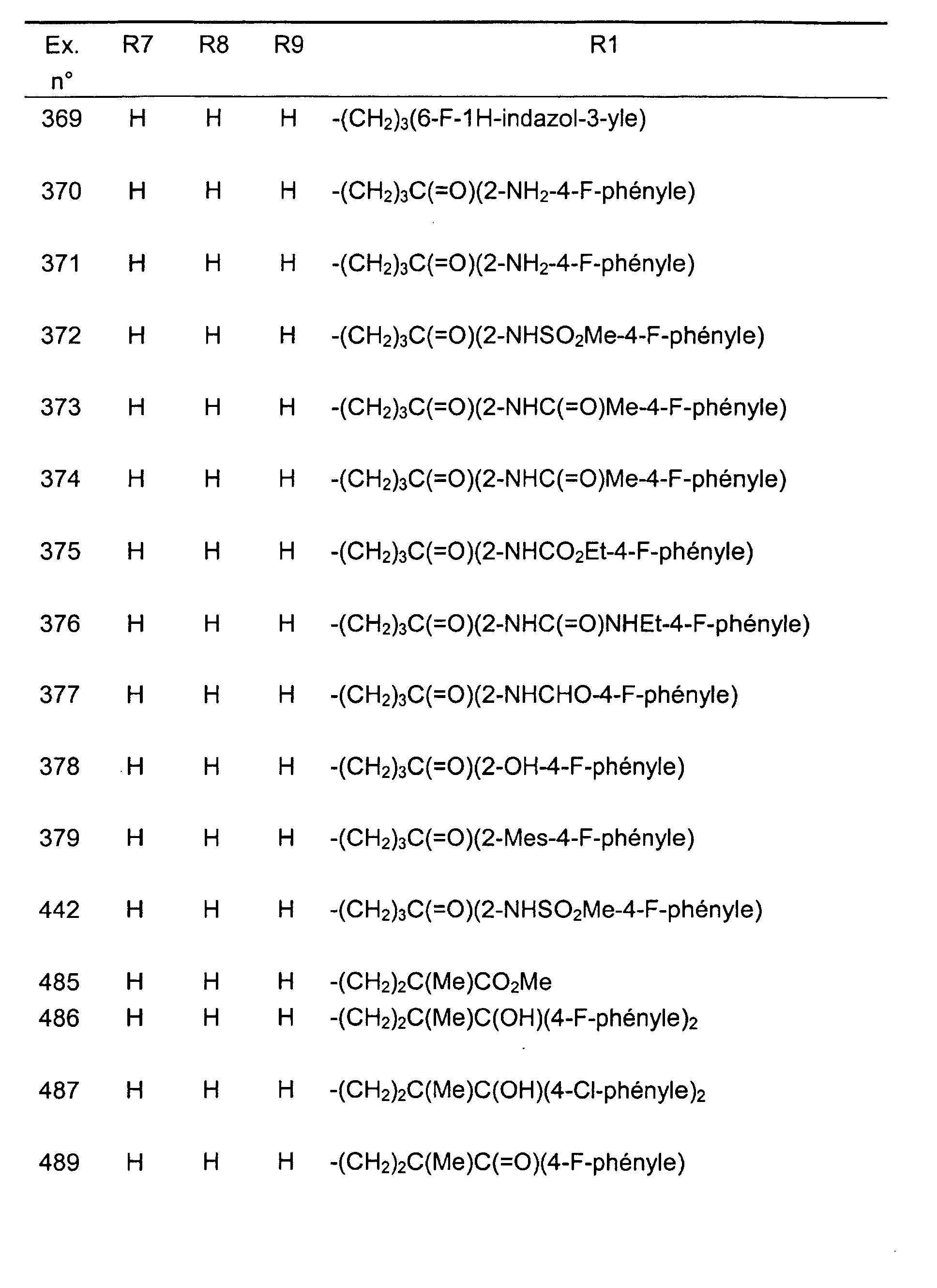

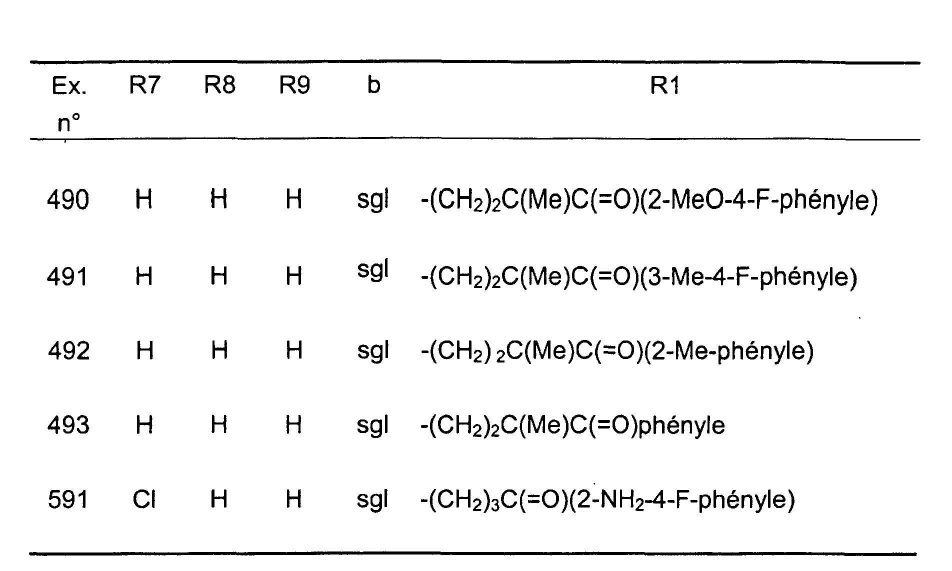

6. Un composé suivant la revendication 1, choisi dans le groupe consistant en des composés de formule 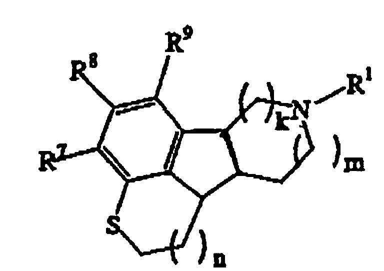
| Ex. n° | N | k | m | R7 | R8 | R9 | R1 |
|---|---|---|---|---|---|---|---|
| 472 | 2 | 2 | 1 | H | H | H | -(CH₂)₃C(=O)(4-F-phényle) |
| 473 | 2 | 2 | 1 | H | H | H | -(CH₂)₃O(4-F-phényle) |
| 474 | 2 | 2 | 1 | H | H | H | -(CH₂)₃(6-F-benzisoxazol-3-yle) |
| 475 | 2 | 2 | 1 | H | H | H | -(CH₂)₃C(=O)(4-pyridyle) |
| 483 | 2 | 2 | 1 | H | Br | H | -(CH₂)₃C(=O)(4-F-phényle) |
| 484 | 2 | 2 | 1 | H | Br | H | -(CH₂)₃O(4-F-phényle) |

7. Un composé suivant la revendication 1, choisi dans le groupe consistant en des composés de formule 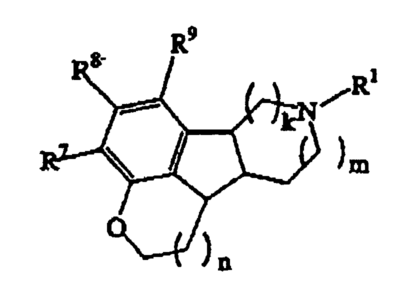
| Ex. n° | n | k | m | R7 | R8 | R9 | R1 |
|---|---|---|---|---|---|---|---|
| 182 | 1 | 1 | 1 | H | H | H | -(CH₂)₃C(=O)(4-F-phényle) |
| 266 | 1 | 1 | 1 | H | H | Me | -(CH₂)₃C(=O)(4-F-phényle) |
| 270 | 1 | 1 | 1 | H | H | H | -(CH₂)₃O(4-F-phényle) |
| | | | | | | | |
| 272 | 1 | 1 | 1 | H | H | H | H |
| 494 | 1 | 1 | 1 | H | H | H | -(CH₂)₃C(=O)(2-NH₂-phényle) |
| | | | | | | | |
| 495 | 1 | 1 | 1 | H | H | H | -(CH₂)₃C(=O)(2-NH₂-phényle) |
| 496 | 1 | 1 | 1 | H | H | H | -(CH₂)₃(1H-indazol-3-yle) |
| 528 | 1 | 1 | 1 | H | H | H | -(CH₂)₃(6-F-1H-indazol-3-yle) |
| 529 | 1 | 1 | 1 | H | H | H | -(CH₂)₃C(=O)(2-NH₂-4-F-phényle) |
| 530 | 1 | 1 | 1 | H | H | H | -(CH₂)₃C(=O)(2-NH₂-4-F-phényle) |
| 531 | 1 | 1 | 1 | H | H | H | -(CH₂)₃C(=O)(2-OH-4-F-phényle) |
| 539 | 1 | 2 | 1 | H | H | H | -(CH₂)₃O(4-F-phényle) |
| 540 | 1 | 2 | 1 | H | H | H | -(CH₂)₃(6-F-1,2-benzisoxazol-3-yle) |
| 544 | 2 | 1 | 1 | H | H | H | sgl -(CH₂)₃C(=O)(4-F-phényle) |
| 546 | 1 | 2 | 1 | H | H | H | sgl -(CH₂)₃C(=O)(4-F-phényle) |

8. Un composé suivant la revendication 1, choisi dans le groupe consistant en des composés de formule 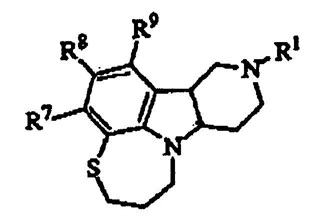
| Ex. n° | R7 | R8 | R9 | R1 |
|---|---|---|---|---|
| 185 | H | H | CF₃ | -(CH₂)₄(4-F-phényle) |
| 188 | H | H | H | -(CH₂)₃C(=O)(4-F-phényle) |
| 213 | H | CH₃ | H | -(CH₂)₃C(=O)(4-F-phényle) |
| 438 | H | H | H | -(CH₂)₃C(=O)(2-NH₂-phényle) |
| 439 | H | H | H | -(CH₂)₃C(=O)(2-NH₂-phényle) |
| 440 | H | H | H | -(CH₂)₃C(=O)(2-NH₂-4-F-phényle) |
| 441 | H | H | H | -(CH₂)₃C(=O)(2-NH₂-4-F-phényle) |
| 456 | H | H | H | -(CH₂)₃C(=O)(4-F-phényle) |
| 457 | H | H | H | -(CH₂)₃C(=O)(4-F-phényle) |

9. Une composition pharmaceutique comprenant un véhicule acceptable pharmaceutiquement et une quantité efficace thérapeutiquement d'un composé suivant l'une quelconque des revendications 1 à 8, ou l'un de ses sels acceptables pharmaceutiquement.

10. Un composé suivant l'une quelconque des revendications 1 à 8, destiné à être utilisé en thérapeutique.

11. Un composé suivant l'une quelconque des revendications 1 à 8, destiné à être utilisé pour le traitement de l'obésité, de la schizophrénie ou de la dépression.

12. Utilisation d'un composé suivant l'une quelconque des revendications 1 à 8 dans la fabrication d'un médicament pour le traitement de l'obésité, de la schizophrénie ou de la dépression.
